# EUROPEAN PATENT APPLICATION

(11) **EP 2 145 964 A1**
(43) Date of publication of application: **20.01.2010**
(21) Application number: 08160649.3
(22) Date of filing: 17.07.2008
(51) Int. Cl.: C12Q 1/68

(54) **A method for lung cancer early detection and prognosis**

(71) Applicant: UNIVERSITÄT ZU KÖLN, 50923 Köln (DE); DKFZ Deutsches Krebsforschungszentrum, Stiftung des öffentlichen Rechts, 69120 Heidelberg (DE)
(72) Inventor: Zander, Thomas, 53115 Bonn (DE); Schultze, Joachim L., 50259 Pulheim (DE); Wolf, Jürgen, 50733 Köln (DE); Staratschek-Jox, Andrea, 50733 Köln (DE); Debey-Pascher, Svenja, 42657 Solingen (DE); Eggle, Daniela, 82377 Penzberg (DE); Boffetta, Paolo, 69372 Lyon (FR); Linseisen, Jakob, 69120 Heidelberg (DE)
(74) Representative: von Kreisler Selting Werner

(57) **Abstract**

The present invention provides method for the prognosis or detection of lung cancer, notably for early detection of lung cancer, as well as a kit and a (micro) array suitable for said method.

## Description

The present invention provides method for the prognosis or detection of lung cancer, notably for early detection of lung cancer, as well as a kit and a (micro)array suitable for said method.

### Background of the Invention

Lung cancer is the most frequent cause of cancer-related death in the western world, with only 15% of patients being alive 5 years after diagnosis (Jemal, A., et al., CA Cancer J Clin, 56:106-130 (2006)). This is almost exclusively due to a lack of approaches affording early diagnosis. About 75% of lung cancer patients present with advanced stage disease, without a chance for cure (Jemal, A., et al., CA Cancer J. Clin., 56:106-130 (2006)). Developing efficient tools for early detection thus represents the most promising strategy to improve prognosis of lung cancer (Mulshine, J.L. Nat. Rev. Cancer 3, 65-73 (2003)).

Numerous screening approaches have been tested over the last decades, including chest X-ray, sputum cytology, analysis of molecular markers and, more recently, annual spiral computed tomography (Bremnes, R.M., et al., C. Lung Cancer 49:1-12 (2005); (Henschke, C.I., et al., N. Engl. J. Med. 355:1763-1771 (2006)); (Bach, P.B. et al., D.C. Chest 123:72-82 (2003)). However, wide-spread application of these methods is encumbered by either limitations in sensitivity or specificity (sputum tests, chest x-ray) or feasibility and cost issues (spiral computed tomography). Recently, Spira and colleagues demonstrated the high power of gene expression profiling to detect lung cancer in surrogate tissue using bronchoscopically obtained epithelial cells derived from patients with early clinical symptoms (Spira, A., et al., Nature Medicine (2007)).

Nevertheless, an early diagnosis of lung cancer is still desirable.

### Short Description of the Invention

It was now found that a particular blood-derived transcriptional signature is suitable to discriminate patients with lung cancer from non-affected smokers.

When applied to blood samples from a prospective population-based cancer study, this signature accurately predicted the occurrence of lung cancer in smokers two years before the onset of clinical symptoms.

The invention thus provides
(1) method for the prognosis or early detection of lung cancer which comprises
   (a) determining the expression rates of at least 25 of the indicator RNAs represented by the nucleic acid sequences of SEQ ID NOs:2n (wherein n is an integer from 1 to 74) in a bodily fluid of the individual to be tested, and
   (b) comparing the obtained expression rates with standard values of the respective expression rates, wherein a deviation of the expression rate of at least 30% of the indicator RNAs is indicative for lung cancer or the prognosis of lung cancer;
(2) a preferred embodiment of the method of (1) above, wherein the determination is effected by a hybridization with probes specific for the nucleic acid sequences of SEQ ID NOs:2n (wherein n is an integer from 1 to 74), preferably said probes comprising the nucleic acid sequences of SEQ ID NOs:2n-1 (wherein n is an integer from 1 to 74);
(3) a hybridization (micro)array carrying probes for at least 25 indicator RNAs as defined in (2) above;
(4) a kit for the prognosis or detection of lung cancer according to the method of (1) or (2) above which comprises means for the determination of the expression rate of the indicator proteins as defined in(1) or (2) above or a (micro)array as defined in (3) above.

The (micro)array and kit of the invention are suitable as a screening tool to enable early diagnosis of lung cancer at a curable stage.

### Short Description of the Figures

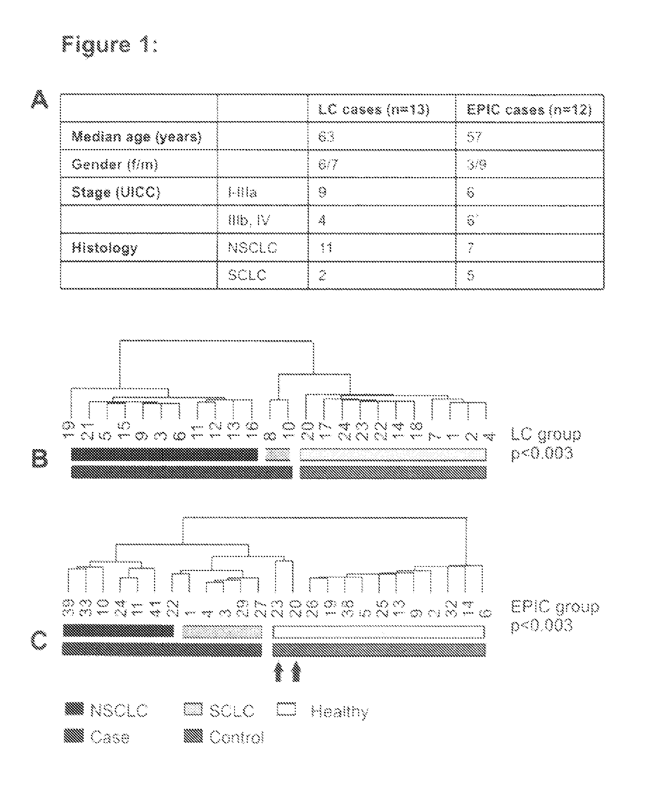

Figure 1: Patient characteristics and hierarchical clustering of gene expression data. (a) Patients with the diagnosis of lung cancer (*prevalent LC cases*) were recruited in local hospitals. Individuals developing lung cancer within two years after blood sample asservation were recruited in the EPIC trial (*incident EPIC cases*) (* = in one case no data on stage available). (b,c) Expression levels in blood samples were quantile normalized and differentially expressed genes were detected using an ANOVA based filter (ANOVA p<0.003 for difference in histology). We performed hierarchical clustering (distance metric: 1-correlation; linkage method: centroid) to group samples. A significant cluster for cases and controls was demonstrated (p<0.001) in prevalent lung cancer (b) and incident lung cancer (c).

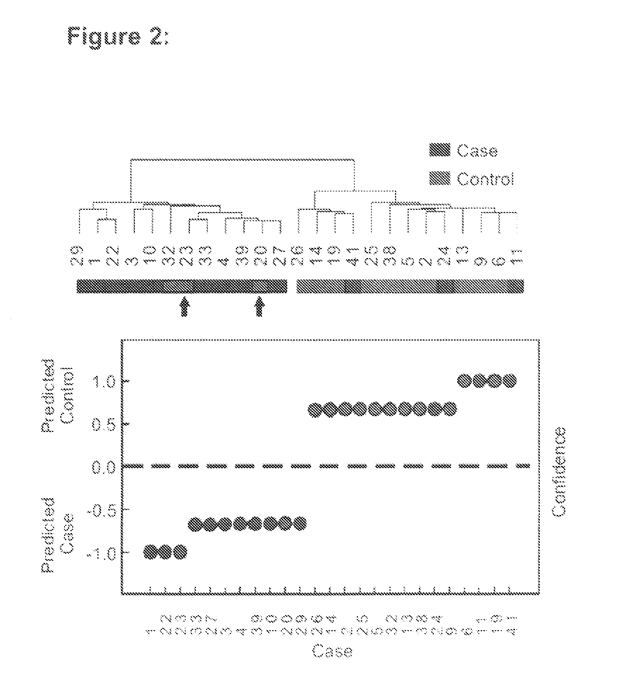

Figure 2: Prediction of future clinical manifestation of lung cancer in asymptomatic smokers: We defined an "RNA-fingerprint" for lung cancer in peripheral blood using an ANOVA based filter (ANOVA p<0.003 for difference in histology) in blood samples from patients with clinical manifest lung cancer. (a) We performed hierarchical clustering using this "RNA-fingerprint" with the samples from individuals developing lung cancer within two years after blood withdrawal. We observed significant clusters for cases and controls (p<0.01). 19/25 samples were correctly grouped. Of the 6 misclassified samples two (20 and 23) had also been misgrouped in the prior analysis (figure 1c) (b) Using a KNN algorithm we built a predictor for the occurrence of lung cancer within this "RNA-fingerprint". We used a 65 feature predictor to predict the clinical onset of lung cancer in the EPIC cohort. The prediction accuracy was 80%.

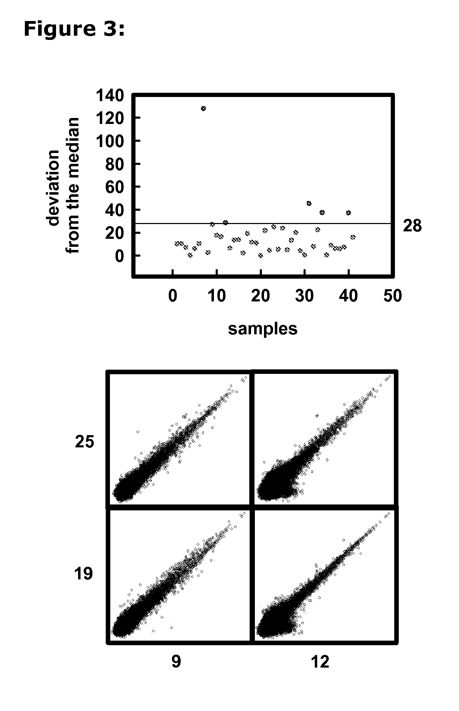

Fig. 3: Quality control of expression arrays. Upper panel: First the median expression value for each array and for the whole group of arrays was calculated. The difference between the median expression value for each array and the median overall expression value is plotted. High quality arrays are represented in red, poor quality in blue. Lower panel: Expression values from each array were plotted against all other arrays. In high quality arrays a symmetric cloud around the diagonal (lower left) was detected. In poor quality arrays asymmetric clouds were observed (lower right).

### Detailed description of the Invention

As transcriptional changes in peripheral blood were thought to be an early event in lung cancer development and might therefore be suitable to predict lung cancer before its clinical onset, a lung cancer associated "RNA-fingerprint" (Bild, A.H., et al. Nature 439:353-357 (2006)) was generated from peripheral blood samples obtained at diagnosis from 13 smokers with clinically manifest lung cancer (*LC cases*). Eleven of these 13 patients had been diagnosed with non-small cell lung cancer (NSCLC); the other 2 patients had a diagnosis of small-cell lung cancer (SCLC). Eleven samples from lung cancer-free smokers were used as controls (*LC controls*) (Fig. 1a and Table 2). As a first step differentially expressed genes between SCLC, NSCLC and controls in the group with clinically manifest lung cancer (*LC group*) were determined using an ANOVA based filter (p<0.003). These genes were consecutively used for hierarchical clustering. Using this method, a significant discrimination between the three different groups (NSCLC, SCLC and controls; p<0.0001) (Fig. 1b) was achieved.

Next, differentially expressed genes in blood samples before clinical onset of lung cancer in healthy smokers recruited in the European Prospective Investigation into Cancer and Nutrition (EPIC) were determined. This population-based cohort study prospectively investigates the natural history of more than 500,000 individuals from 10 European countries with 25,540 participants in the Heidelberg cohort. As part of this study, blood samples were routinely obtained from all individuals when entering the study. Within the EPIC-Heidelberg cohort 12 currently smoking individuals were identified, who had developed either NSCLC (n=7) or SCLC (n=5) within 24 months (median 14 months) post sampling (*EPIC cases*) with samples suitable for experimental evaluation (Table 2). These frequencies reflect the incidence of lung cancer among smokers within the Heidelberg cohort and are consistent with the incidence of lung cancer in Germany. Thirteen active smokers lacking a diagnosis of lung cancer within the total observation period following blood sampling were selected from the Heidelberg cohort based on matching of gender, age and smoking behaviour (*EPIC controls*) (Table 2). Differential gene expression in the EPIC cohort was assessed using the same ANOVA based filtering criteria (p < 0.003). This set of genes robustly separated NSCLC, SCLC and controls within the EPIC group (NSCLC / SCLC p<0.05; case / control p<0.01) with a misclassification of two samples (20 and 23) (Fig. 1c).

When seeking for the predictive potential of the "RNA fingerprint" identified in the prevalent lung cancer cases, genes differentially expressed between NSCLC, SCLC and controls in clinical manifest lung cancer (*LC group*) were analyzed in order to evaluate whether the expression rate of said genes could be used to predict the clinical onset of lung cancer in the EPIC samples. First we used hierarchical clustering to group samples and detected significant clusters for cases and controls (p=0.01). For a sample clustered in the case group the odds ratio (OR) for developing lung cancer was estimated to be 10 [95% CI: 1.6 to 63]. We next built a predictor of lung cancer development by applying a K-nearest neighbor algorithm to the "RNA-fingerprint" (differentially expressed genes in the group with clinical manifest lung cancer (*LC group*)) and validated this predictor using leave-one-out-cross-validation (Table 3). Finally, when applying the resulting 65-feature predictor to the EPIC group, 80% of cases and controls were accurately classified (Fig. 2b); yielding a sensitivity of 75% and a specificity of 85%. The estimated Odds Ratio for developing lung cancer in the *case group* was 17 [95% CI: 2.2 to 121] (Fig. 2b). A class permutation analysis revealed that these findings were statistically significant (p=0.003).

In summary, by applying a blood-based lung cancer 'RNA-fingerprint' to blood samples obtained from incident lung cancer cases in the EPIC Heidelberg cohort, we were able to identify smokers who developed clinical symptoms of lung cancer within 2 years post blood sampling. These findings suggest that early tumor growth induces significant and measurable transcriptional changes in blood cells. We speculate that these changes are the result of a biological amplification of the 'cancer signal' by the immune system (Wang, X., et al. N. Engl. J. Med. 353:1224-1235 (2005)). Our findings imply that a blood-based gene-expression signature could be used for identification of patients at high risk for the onset of lung cancer that could be followed more stringently, e.g., by means of spiral computed tomography (Henschke, C.I., et al., N. Engl. J. Med. 355:1763-1771 (2006)). Such an integrated approach may afford early diagnosis of lung cancer at a curable stage of the disease.

Aspects (1) and (2) of the invention pertains to a method for the prognosis or detection of lung cancer (hereinafter shortly referred to as "method of the invention"), which comprises (a) determining the expression rates of at least 25 of the indicator RNAs represented by the nucleic acid sequences of SEQ ID NOs:2n (wherein n is an integer from 1 to 74), i.e. are selected from SEQ ID NOs:2, 4, 6, 8, 10 ...74, in a bodily fluid of the individual to be tested, and
(b) comparing the obtained expression rates with standard values of the respective expression rates, wherein a deviation of the expression rate of at least 30% of the indicator RNAs is indicative for lung cancer or the prognosis of lung cancer.

The method of the invention is particularly suitable for early diagnosis of lung cancer.

A preferred embodiment of the method of the invention comprises determining the expression rates of at least 35, preferably at least 50, most preferably at least 70 of the indicator RNAs.

A further preferred embodiment of the method of the invention comprises determining the expression rate of at least the following indicator RNASs: SEQ ID NO: 25, 131, 17, 113, 13, 133, 43, 79, 53, 91, 103, 99, 1, 145, 5, 23, 101, 69, 73 and 141.

In still a further preferred embodiment of the method of the invention a deviation of the expression rate of at least 50%, preferably of at least 70% of the indicator RNAs is indicative for lung cancer or prognosis of lung cancer.

A "deviation of the expression rate" according to the method of the invention includes a decrease or an increase of the expression rate depending on the type of RNA as can be seen from Table 1, and a decrease or increase of at least 10% relative to the respective standard value is considered a significant event, i.e. a deviation of the expression rate.

It is particularly preferred that 30%, preferably 50% of the indicator RNAs, which are indicative with a decreased expression rate as shown in Table 1, show a decreased expression rate, and/or 30%, preferably 50% of the indicator RNAs, which are indicative with an increased expression rate as shown in Table 1, show an increased expression rate.

**Table 1: Indicator RNAs with increased (up) or decreased (down) expression rate.**

| SEQ ID NO: | probe set | fold change | up/down |
|---|---|---|---|
| 25 | GI_21361309-S | -1,63 | up |
| 131 | GI_5803026-S | -1,54 | up |
| 17 | GI_15147247-S | -1,52 | up |
| 113 | GI_4503938-S | -1,52 | up |
| 13 | GI_13899296-S | -1,46 | up |
| 133 | GI_5803097-S | -1,32 | up |
| 43 | GI_24797159-S | -1,31 | up |
| 79 | GI_34932495-S | -1,3 | up |
| 53 | GI_30149001-S | -1,29 | up |
| 91 | GI_40254449-S | -1,24 | up |
| 103 | GI-41281452-S | -1,24 | up |
| 99 | GI_40288190-S | -1,23 | up |
| 1 | GI_10863876-S | -1,21 | up |
| 57 | GI_30911100-S | -1,19 | up |
| 117 | GI_4507176-S | -1,18 | up |
| 37 | GI_23110986-A | -1,17 | up |
| 65 | GI_34147320-S | -1,16 | up |
| 27 | GI_21389370-S | -1,15 | up |
| 127 | GI_5730062-S | -1,15 | up |
| 129 | GI_5730064-S | -1,15 | up |
| 121 | GI_45580743-I | -1,14 | up |
| 59 | GI_32484980-S | -1,13 | up |
| 83 | GI_38016929-A | -1,13 | up |
| 139 | GI_7661597-S | -1,13 | up |
| 95 | GI_40255066-S | -1,11 | up |
| 71 | GI_34147626-S | -1,1 | up |
| 97 | GI_40255142-S | -1,1 | up |
| 85 | GI_38157980-1 | -1,09 | up |
| 7 | GI_11038673-1 | -1,06 | up |
| 9 | GI_11038675-A | -1,06 | up |
| 115 | GI-4504010-S | -1,05 | up |
| 137 | GI_7019566-S | -1,05 | up |
| 49 | GI_27894318-A | -1,03 | up |
| 81 | GI_37541012-S | -1,02 | up |
| 35 | GI_22749236-S | -1 | up |
| 19 | GI_18641377-S | 1 | down |
| 31 | GI_22202620-S | 1,01 | down |
| 15 | GI_14589867-A | 1,02 | down |
| 87 | GI_38195085-S | 1,02 | down |
| 51 | GI_28302128-S | 1,05 | down |
| 33 | GI_22748768-S | 1,06 | down |
| 75 | GI_34147709-S | 1,06 | down |
| 93 | GI 40254458-S | 1,06 | down |
| 125 | GI_5453622-S | 1,06 | down |
| 123 | GI_4885562-S | 1,08 | down |
| 143 | GI_7706086-I | 1,08 | down |
| 29 | GI_22027643-S | 1,09 | down |
| 147 | hmm25673-S | 1,09 | down |
| 47 | GI_27886683-S | 1,1 | down |
| 55 | GI_30425405-S | 1,1 | down |
| 61 | GI_33598961-S | 1,1 | down |
| 89 | GI_38201699-A | 1,1 | down |
| 77 | GI_34304319-S | 1,11 | down |
| 119 | GI_45243546-1 | 1,11 | down |
| 11 | GI_13376659-S | 1,12 | down |
| 39 | GI_23111059-S | 1,12 | down |
| 109 | GI_4502650-S | 1,14 | down |
| 63 | GI_33859792-S | 1,15 | down |
| 107 | GI_42544181-I | 1,15 | down |
| 45 | GI_26787992-S | 1,16 | down |
| 41 | GI_24308268-S | 1,17 | down |
| 67 | GI_34147598-S | 1,17 | down |
| 111 | GI_4502662-S | 1,18 | down |
| 3 | GI_11024711-S | 1,19 | down |
| 21 | GI_19924140-A | 1,2 | down |
| 105 | GI_41393588-S | 1,2 | down |
| 135 | GI_6633803-S | 1,2 | down |
| 145 | GI_8923658-S | 1,2 | down |
| 5 | GI_11038671-A | 1,21 | down |
| 23 | GI_20149601-S | 1,21 | down |
| 101 | GI 40807470-S | 1,21 | down |
| 69 | GI_34147611-S | 1,22 | down |
| 73 | GI_34147705-S | 1,22 | down |
| 141 | GI_7705420-S | 1,31 | down |

According to the method of the invention, the standard values of the expression rates are obtainable from a set of healthy patients (e.g. as a mean value) or via a standard mixture of the indicator RNAs. The latter may be generated according to the results from standard values of healthy patients.

According to the method of the invention, a bodily fluid includes blood, urine, sputum, saliva etc. Particular preferred is blood.

Further, in the method of the invention any RNA determination method for the determination of the expression rate available to the skilled person is applicable. A suitable method for the determination of the expression rate are RNA hybridization assays, particularly solid phase hybridization (micro)arrays.

Aspect (2) of the invention utilizes such hybridization assay with suitable probes for the indicator RNAs. It is preferred that said probes comprise 15 to 150, preferably 30 to 70 consecutive nucleotides of the nucleic acid sequences of SEQ ID NOs:2n (wherein n is an integer from 1 to 74). Alternatively the probes may comprise the nucleic acid sequences of SEQ ID NOs:2n-1 (wherein n is an integer from 1 to 74), i.e. are selected from SEQ ID NOs: 1, 3, 5, 7, 9...73, preferably the probes are consisting of the nucleic acid sequences of SEQ ID NOs:2n-1 (wherein n is an integer from 1 to 74).

Aspect (3) of the invention pertains to hybridization (micro)array for the prognosis or detection of lung cancer carrying probes for at least 25 indicator RNAs, wherein said probes are as defined hereinbefore.

Aspect (4) of the invention pertains to a kit for the prognosis or detection of lung cancer according to the method of the invention, which comprises means for the determination of the expression rate of the indicator proteins as defined hereinbefore.

In particular the may comprise probes for said at least 25 indicator RNAs or a (micro)array of aspect (3) of the invention.

The kit may comprise further components suitable for performing the method of the invention, notably buffer, nucleotide solutions, markers/labels, standard solutions (including a standard mixture of the indicator RNAs as defined hereinbefore) etc.

The invention is described in more detail in the following examples which are however not to be construed as limiting the invention.

### Examples

### Materials and Methods

Recruitment of incident lung cancer cases in the EPIC-Heidelberg cohort: The European Prospective Investigation into Cancer and Nutrition (EPIC) is one of the largest epidemiological studies of diet and health. From 1993 to 1999 EPIC recruited 520.000 individuals aged over 20 years in 10 European countries. In Germany, 2 study centers (Heidelberg and Potsdam) contributed to EPIC. From all participants blood samples were collected at recruitment (Riboli, E., et al. Public health nutrition 5, 1113-1124 (2002)). Follow-up is still active and based on follow-up questionnaires, health insurance records and cancer and pathology registries. The participants of the EPIC Heidelberg cohort were drawn from a random sample of the general population of the Heidelberg area. All individuals entering the trial were healthy. 25540 individuals were recruited into this cohort between 1994 and 1998. A total of 21 incident lung cancer cases with a histologically proven diagnosis of lung cancer (according to ICD-10 (C34)) within the first two years of follow-up were identified within the EPIC-Heidelberg cohort. The incidence of lung cancer in the EPIC-Heidelberg cohort within these two years reflects the lung cancer incidence in Germany (men: EPIC Heidelberg 58/100,000, Germany 61.2/100,000; women: EPIC Heidelberg 26/100,000, Germany 20.8/100.000; Ferlay, J., et al. Ann Oncol 18, 581-592 (2007)). We excluded 1 case from the further analysis due to histology (neuroendocrine tumor). From the 20 individuals with non-small cell lung cancer (NSCLC) or small cell lung cancer (SCLC) 6 were not current smokers (1 never-smoker and 5 ex-smokers) and thus excluded from the analysis. Two further cases were excluded due to RNA of minor quality (EPIC-12 and EPIC-34). Twelve samples from current smokers with lung cancer (*EPIC cases*) with sufficient amounts of RNA in a quality passing our standard quality control filter were included for further analysis. The number of samples of never-smokers or ex-smokers within the EPIC Heidelberg cohort is too small to establish a distinct expression signature for this group. We did not conduct a joint expression analysis of smokers and non-smokers due to pronounced changes in RNA expression induced by smoking itself (unpublished results). *EPIC controls* were matched for age, gender and smoking behaviour (never-smoker, ex-smoker, smoker). All controls were free of lung cancer for the whole observation period (mean observation time 8.6 years). All individuals gave informed consent and the study was approved by the local ethics committee.

### Recruitment of prevalent lung cancer cases in hospital-based patients:

Prevalent lung cancer samples (*LC cases*) were recruited in the University Hospital K61n and the Lung Clinic Merheim, K61n, after informed consent and approval by the local ethics committee. In this cohort controls were defined as individuals without clinical manifest lung cancer at the time of blood asservation (*LC controls*). All individuals gave informed consent and the study was approved by the local ethics committee. All individuals were smokers RNA extraction and array hybridization: We used snap frozen PBMC enriched blood (~ 300 µl) from the EPIC study for RNA extraction. Blood samples were directly thawed in 5ml of TRI Reagent BD (Molecular Research Center, Inc, USA). RNA was extracted according to the manufactures instruction and stored in 70% ethanol until finalization of preparation and dilution in H₂O. For further RNA purification we applied Qiagen MinElute columns (Qiagen, Germany). By this method we extracted 960ng RNA (mean; range 120 - 2500ng). One sample contained no RNA. The OD ratio 260/280 was 2.01 in mean (range 1.76-2.06). For the *LC-group*, we drew 2.5 ml blood directly into PAXgene vials providing stabilization of the gene expression profile. Samples were rested over night at room temperature and then stored at -80°C until further preparation. RNA was extracted according to the manufactures instruction (Qiagen, Hilden, Germany). Biotin labeled cRNA preparation was performed using the Ambion® Illumina RNA amplification kit (Ambion, UK). Quality was controlled using a PCR based system (Zander, T. et al., J Med Biol Res 39, 589-593 (2006)). 1.5 µg biotin labeled cRNA was hybridized to Sentrix® whole genome bead chips 6 x 2 (Illumina, USA) and scanned on the Illumina® BeadStation 500x (Debey, S., et al. Genomics 87, 653-664 (2006)). For data collection, we used Illumina® BeadStudio 1.4.0.2 software.

Bioinformatic tools: Primary expression data were extracted using BeadStudio software (Illumina, USA). Quantile normalization was performed using R-software (http://www.r-project.org/) (Boston, USA).

To validate our predictor for future onset of lung cancer we performed a permutation analysis. For the permutation analysis the classes of samples in the group of prevalent lung cancer and controls were randomly assigned leading to new "randomized" datasets. For each randomised data set we generated a new "RNA fingerprint" based on differentially expressed genes (ANOVA p=0.003) in the group of prevalent lung cancer (*LC group*). We then used this new "RNA-fingerprint" to predict samples from individuals with future onset of lung cancer (*EPIC group*). The odds ratio was calculated without inclusion of covariables (Bland, J.M. & Altman, BMJ, Clinical research ed 320, 1468 (2000)).

Quality control of microarrays: Quality control was crucial for samples of the group of *EPIC cases and controls*, that were stored as whole blood and frozen without RNA stabilization. We performed quality control by visual inspection of the distribution of raw expression values. In addition, the absolute deviation of each array from the overall median was determined. In short, the median expression value for each array was calculated. Next the median of these median expression values (overall median) was taken and the deviation of each array median from the overall median was determined (analogous to probe outlier detection used by Affymetrix before expression value calculation (Affymetrix. http: www.affymetrix.com support/technical/whitepapers/sadd whitepaper,pdf(2002)). When plotting the deviations (Fig. 3 upper panel) 4 samples (7, 31, 34, 40) showed large deviations from the median (37.2 to 127.9), while two samples (9 &12) had very similar deviations (27.1 respectively 28.5). We therefore applied a further quality control and constructed pairwise scatterplots of expression values from all arrays. For data derived from an array of good quality a high correlation of expression values is expected leading to a cloud of dots along the diagonal. When analysing sample 12, systematic differences of low expression values could be detected (Fig. 3, lower right panel, shown are two exemplified plots). This was also the case when plotting samples 7, 31, 34 and 40 (data not shown). Sample 9, on the other hand exhibited a good correlation to all other arrays (Fig. 3, lower left panel). Therefore, samples 7, 12, 31, 34 and 40 were excluded from further analysis and a cut-off value of 28 for deviation from the overall median was selected as exclusion criterion.

Description of the generation of the gene-list: Oligos were selected as described in the paper and supplements. Briefly, RNA was extracted from PBMC enriched frozen blood samples for the incident cases and from PAXgene stabilized blood samples for the prevalent samples by the technique as described in the supplement. cRNA was generated and labelled and arrays were hybridized as described in supplemental methods. Primary expression data were extracted using Bead-Studio software (Illumina). Further primary expression data were quantile normalized. Low quality samples were extracted by measuring the deviation from the median expression value. The cut-off value was set at 28. High quality samples were used for further analysis. Then transcripts which are significantly different between NSCLC, SCLC and controls were identified by ANOVA test with a p-value of <0.003 in samples from patients with prevalent lung cancer compared to controls. The transcripts matching these criteria were selected by the dCHIP software. In a next step only these selected transcripts are used to classify cases and controls within a group of samples with incident lung cancer and controls. In a following step those Oligos specific for the transcripts matching the above mentioned criteria and further being identical on the Illumina platform WG6 VS1 and WG6 VS2 were identified by BLAST analysis. WG6 VS1 is no longer available and reproduction of results was performed on WG6 VS2. These Oligos were then blasted against the human genome and the RefSeq sequence with the highest homology was taken and the FASTA sequence for the respective transcript is given.

For a test this implies that the expression of the transcripts within the list have to be measured in peripheral blood. The complex expression pattern of these transcripts can be then used to identify cases as it is significantly different.

Characteristics of the prevalent hospital-based lung cancer cases and controls (LC group): For each blood sample in the (prevalent) group of *LC controls and cases* gender, smoking behaviour, age, histology and clinical stage of lung cancer at diagnosis is given. All blood samples from cases derive from patients with known diagnosis of lung cancer are summarized in the following Table 2.

**Table 2. Lung cancer cases (na=not applicable; NSCLC=non-small cell lung cancer; SCLC=small cell lung cancer):**

| Pseudonym | Case / control - status 1=case 0=control | Gender 1=male 2=female | Smoking status 0=never-smoker 1=ex-smoker 2=active smoker | Age (years) | Histology 0=normal 1=NSCLC 2=SCLC | UICC stage |
|---|---|---|---|---|---|---|
| LC-1 | 0 | 1 | 1 | 71 | 0 | na |
| LC-2 | 0 | 1 | 1 | 61 | 0 | na |
| LC-3 | 1 | 1 | 2 | 69 | 1 | Ib |
| LC-4 | 0 | 2 | 2 | 62 | 0 | na |
| LC-5 | 1 | 1 | 2 | 63 | 1 | IIIa |
| LC-6 | 1 | 2 | 2 | 71 | 1 | IIb |
| LC-7 | 0 | 1 | 1 | 48 | 0 | na |
| LC-8 | 1 | 1 | 2 | 68 | 2 | IV |
| LC-9 | 1 | 1 | 2 | 69 | 1 | IV |
| LC-10 | 1 | 2 | 2 | 52 | 2 | Ib |
| LC-11 | 1 | 1 | 2 | 73 | 1 | IIIA |
| LC-12 | 1 | 1 | 2 | 53 | 1 | IV |
| LC-13 | 1 | 2 | 2 | 62 | 1 | IIb |
| LC-14 | 0 | 2 | 2 | 53 | 0 | na |
| LC-15 | 1 | 2 | 2 | 67 | 1 | I |
| LC-16 | 1 | 1 | 1 | 50 | 1 | Ia |
| LC-17 | 0 | 1 | 1 | 58 | 0 | na |
| LC-18 | 0 | 2 | 2 | 70 | 0 | na |
| LC-19 | 1 | 2 | 2 | 56 | 1 | IV |
| LC-20 | 0 | 1 | 2 | 72 | 0 | na |
| LC-21 | 1 | 2 | 1 | 48 | 1 | IV |
| LC-22 | 0 | 2 | 1 | 50 | 0 | na |
| LC-23 | 0 | 2 | 2 | 67 | 0 | na |
| LC-24 | 0 | 1 | 1 | 54 | 0 | na |

Characteristics of the incident lung cancer cases and controls in the EPIC cohort (EPIC-group): For each blood sample from the EPIC-Heidelberg cohort (incident cases and controls) gender, smoking behaviour, age, histology, clinical stage of lung cancer at diagnosis and time between blood sampling and clinical manifestation of lung cancer is given in Table 3 below.

**Table 3. details about lung cancer cases (na=not applicable; NSCLC=non-small cell lung cancer; SCLC=small cell lung cancer):**

| Pseudonym | Case / control - status | Gender | Smoking status | Age (years) | Histology | UICC stage | Time (Months between blood sampling and clinical diagnosis) |
|---|---|---|---|---|---|---|---|
| | 1=case 0=control | 1=male 2=female | 0=never-smoker 1=ex-smoker 2=active smoker | | 0=normal 1=NSCLC 2=SCLC 3=neuroendocrine tumor | | |
| EPIC-01 | 1 | 2 | 2 | 63 | 2 | IIIb | 8 |
| EPIC-02 | 0 | 1 | 2 | 60 | 0 | na | na |
| EPIC-03 | 1 | 1 | 2 | 62 | 2 | IIIa | 2 |
| EPIC-04 | 1 | 1 | 2 | 43 | 2 | IV | 18 |
| EPIC-05 | 0 | 2 | 2 | 64 | 0 | na | na |
| EPIC-06 | 0 | 1 | 2 | 42 | 0 | na | na |
| EPIC-07 | 0 | 1 | 1 | 59 | 0 | na | na |
| EPIC-08 | 1 | 1 | 1 | 57 | 1 | Ib | 22 |
| EPIC-09 | 0 | 1 | 2 | 54 | 0 | na | na |
| EPIC-10 | 1 | 1 | 2 | 52 | 1 | IV | 17 |
| EPIC-11 | 1 | 1 | 2 | 64 | 1 | IIIa | 11 |
| EPIC-12 | 1 | 1 | 2 | 42 | 1 | IIIb | 8 |
| EPIC-13 | 0 | 1 | 2 | 60 | 0 | na | na |
| EPIC-14 | 0 | 1 | 2 | 41 | 0 | na | na |
| EPIC-15 | 1 | 2 | 1 | 63 | 1 | IV | 2 |
| EPIC-16 | 0 | 2 | 0 | 50 | 0 | na | na |
| EPIC-17 | 0 | 2 | 1 | 60 | 0 | na | na |
| EPIC-18 | 1 | 2 | 0 | 53 | 1 | IIIb | 13 |
| EPIC-19 | 0 | 1 | 2 | 54 | 0 | na | na |
| EPIC-20 | 0 | 1 | 2 | 61 | 0 | na | na |
| EPIC-21 | 0 | 2 | 1 | 52 | 0 | na | na |
| EPIC-22 | 1 | 1 | 2 | 63 | 1 | IIb | 5 |
| EPIC-23 | 0 | 1 | 2 | 62 | 0 | na | na |
| EPIC-24 | 1 | 2 | 2 | 59 | 1 | Ib | 15 |
| EPIC-25 | 0 | 1 | 2 | 60 | 0 | na | na |
| EPIC-26 | 0 | 2 | 2 | 55 | 0 | na | na |
| EPIC-27 | 1 | 1 | 2 | 60 | 2 | IV | 18 |
| EPIC-28 | 1 | 2 | 1 | 51 | 1 | IV | 24 |
| EPIC-29 | 1 | 1 | 2 | 52 | 2 | IV | 24 |
| EPIC-30 | 1 | 1 | 1 | 57 | 1 | IV | 14 |
| EPIC-31 | 0 | 1 | 2 | 62 | 0 | na | na |
| EPIC-32 | 0 | 1 | 2 | 58 | 0 | na | na |
| EPIC-33 | 1 | 1 | 2 | 64 | 1 | IIb | 20 |
| EPIC-34 | 1 | 1 | 2 | 62 | 2 | IIIb | 18 |
| EPIC-35 | 1 | 1 | 2 | 55 | 3 | IIIb | 6 |
| EPIC-36 | 1 | 2 | 1 | 64 | 2 | IV | 13 |
| EPIC-37 | 0 | 2 | 1 | 63 | 0 | na | na |
| EPIC-38 | 0 | 2 | 2 | 37 | 0 | na | na |
| EPIC-39 | 1 | 2 | 2 | 37 | 1 | Ia | 11 |
| EPIC-40 | 0 | 1 | 2 | 48 | 0 | na | na |
| EPIC-41 | 1 | 1 | 2 | 46 | 1 | no data | 18 |
| EPIC-42 | 0 | 1 | 1 | 55 | 0 | na | na |

Characteristics of the predictive genes identified in the LC group: Using an ANOVA test (3 groups: NSCLC, SCLC and controls) 151 differentially expressed transcripts were identified (p-value < 0.003) in the *LC group*. We used this set of genes as the lung cancer specific "RNA fingerprint" (Bild, A.H., et al. Nature 439, 353-357 (2006)). To predict incident lung cancer cases within the EPIC group we built a predictor using a KNN algorithm (GenePattern). For validation of this predictor a leave-one-out-cross-validation was performed. The results are summarized in the attached Table 4. In detail, the probe names of the transcripts used for the predictor, the gene symbol (if available) and the accession number (if available) are given. Furthermore, the p-value determined in the former ANOVA test is given. The right column indicates the number of arrays for which the respective probe was used as a predictive feature.

**Table 4.**

| | Probe | Accession | Gene symbol | p-value ANOVA actual cases | Number of arrays (EPIC group) predicted with this probe |
|---|---|---|---|---|---|
| 1 | Hmm19606-S | NA | NA | 0.00250552 | 25 |
| 2 | GI_42657212-S | NA | NA | 0.00163634 | 25 |
| 3 | SI_21389370-S | NM_144590 | ANKRD22 | 0.00287859 | 25 |
| 4 | GI_20149601-S | NM_012229 | NT5C2 | 0.0012678 | 25 |
| 5 | GI_42659468-S | NA | NA | 0.00065651 | 25 |
| 6 | GI_38195085-S | NM_022098 | LOC63929 | 0.000705223 | 25 |
| 7 | Hs.463913-S | NA | NA | 0.00139197 | 25 |
| 8 | GI_40807470-S | NM_003594 | TTF2 | 0.00121638 | 25 |
| 9 | GI_21314710-S | NM_024048 | MGC3020 | 0.00295729 | 25 |
| 10 | Hs.443817-S | NA | NA | 0.000614819 | 25 |
| 11 | GI_22202620-S | NM_000846 | GSTA2 | 0.000854216 | 25 |
| 12 | GI_40255066-S | NM_144682 | FLJ31952 | 2.53E-06 | 25 |
| 13 | GI_11038673-I | NM_000626 | CD79B | 0.00121602 | 25 |
| 14 | GI_7705420-S | NM_016371 | HSD17B7 | 0.00212517 | 25 |
| 15 | Hs.179641-S | NA | NA | 0.00101283 | 25 |
| 16 | Hs.475119-S | NA | NA | 0.00154416 | 25 |
| 17 | GI_4507176-S | NM_003121 | SPIB | 0.00107239 | 25 |
| 18 | GI_34304319-S | NM_024026 | MRP63 | 0.000945413 | 25 |
| 19 | GI_34147626-S | NM_023926 | ZNF447 | 0.0013713 | 25 |
| 20 | GI-13376659-S | NM_025097 | FLJ21106 | 0.000480451 | 25 |
| 21 | Hs.503501-S | NA | NA | 0.000509971 | 25 |
| 22 | GI_42544181-I | NM_001721 | BMX | 0.00261564 | 25 |
| 23 | GI_5453713-S | NM_001011513 | PDLIM5 | 0.00286142 | 25 |
| 24 | GI_38157980-I | NM_004223 | UBE2L6 | 0.00143946 | 25 |
| 25 | GI_11038671-A | NM_001783 | CD79A | 2.30E-05 | 25 |
| 26 | hmm28413-S | NA | NA | 0.00284309 | 25 |
| 27 | GI_4508040-S | NM_003430 | ZNF91 | 0.00125313 | 25 |
| 28 | GI_8923658-S | NM_017946 | FKBP14 | 0.0023116 | 25 |
| 29 | GI_7706086-I | NM_016552 | ANKMY1 | 0.000808884 | 25 |
| 30 | GI_41393588-S | NM_024769 | ASAM | 0.000139857 | 25 |
| 31 | Hs.473021-S | NA | NA | 0.00294036 | 25 |
| 32 | Ski_7662439-S | NM_014960 | KIAA1001 | 0.00165399 | 25 |
| 33 | GI_28302128-S | | HBB | 0.00107959 | 25 |
| 34 | GI_34147611-S | NM_024670 | SUV39H2 | 0.00220831 | 25 |
| 35 | hmm25043-S | NA | NA | 0.00132864 | 25 |
| 36 | GI_27886525-I | NM_006599 | NFAT5 | 0.0016897 | 25 |
| 37 | GI_34147705-S | NM_145042 | MGC16703 | 0.00122549 | 25 |
| 38 | GI_33859792-S | NM_173494 | CXorf41 | 0.00175359 | 25 |
| 39 | GI_34147320-S | NM_005533 | IFI35 | 0.000249605 | 25 |
| 40 | GI_19924114-I | NM_002877 | RAD51L1 | 0.00161613 | 25 |
| 41 | Hs.387255-S | NA | NA | 0.00184399 | 24 |
| 42 | GI_5730062-S | NM_006542 | SPHAR | 0.00270009 | 24 |
| 43 | Hs.459511-S | NA | NA | 0.00100187 | 24 |
| 44 | Hs.504779-S | NA | NA | 0.00145517 | 24 |
| 45 | GI_14589867-S | NM_001716 | BLR1 | 0.00031141 | 24 |
| 46 | Ski 26787992-S | NM_006873 | SBLF | 0.00275942 | 24 |
| 47 | GI_41281452-S | NM_014720 | SLK | 0.00201966 | 24 |
| 48 | GI_42658659-S | NA | NA | 0.000888823 | 24 |
| 49 | GI_6633803-S | | HBD | 0.00204048 | 24 |
| 50 | GI_24797159-S | NM_000593 | TAP1 | 0.000651189 | 24 |
| 51 | Hs.487038-S | NA | NA | 0.000595039 | 23 |
| 52 | Hs.455680-S | NA | NA | 0.00107714 | 23 |
| 53 | hmm26256-S | NA | NA | 0.00249655 | 22 |
| 54 | Hs.105147-S | NA | NA | 0.00236704 | 20 |
| 55 | Hs.439591-S | NA | NA | 0.000420733 | 20 |
| 56 | GI-45331210-S | NM_033206 | FLJ00060 | 0.000601029 | 19 |
| 57 | hmm3576-S | NA | NA | 0.00115777 | 17 |
| 58 | Hs.41391-S | NA | NA | 6.25E-05 | 17 |
| 59 | hmm30337-S | NA | NA | 0.00160364 | 16 |
| 60 | GI_23111059-S | NM_005850 | SF3B4 | 0.0021919 | 15 |
| 61 | Hs.458163-S | NA | NA | 0.00289446 | 14 |
| 62 | Hs.278665-S | NA | NA | 0.0013819 | 13 |
| 63 | GI_40254458-S | NM_001884 | HAPLN1 | 0.00224952 | 13 |
| 64 | GI_32484980-S | NM_017935 | BANK1 | 0.00235537 | 12 |
| 65 | GI_38257138-S | NM_002735 | PRKAR1B | 0.000945009 | 11 |
| 66 | hmm30387-S | NA | NA | 0.000683955 | 11 |
| 67 | GI_31341794-I | NM_030945 | C1QTNF3 | 0.00138828 | 10 |
| 68 | GI_7661597-S | NM_015535 | DNAPTP6 | 0.000126427 | 10 |
| 69 | GI_24308268-S | NM_021218 | C9orf80 | 0.000945579 | 8 |
| 70 | GI_4502650-S | NM_001771 | CD22 | 0.000317009 | 8 |
| 71 | GI_38016929-A | NM_003733 | OASL | 0.000794278 | 6 |
| 72 | Hs.371362-S | NA | NA | 0.00285103 | 6 |
| 73 | GI_40255142-S | NM_024581 | C6orf60 | 0.00213972 | 5 |
| 74 | GI_4502662-S | NM_001774 | CD37 | 0.00168784 | 5 |
| 75 | GI_5453622-S | NM_006314 | CNKSR1 | 0.000579393 | 4 |
| 76 | GI_45243546-I | NM_001107 | ACYP1 | 0.00136892 | 4 |
| 77 | GI_40288190-S | NM_017523 | BIRC4BP | 8.06E-05 | 4 |
| 78 | SI_7019566-S | NM_013378 | VPREB3 | 2.12E-05 | 4 |
| 79 | GI_34932495-S | NM_007047 | BTN3A2 | 0.00299365 | 3 |
| 80 | Hs.148277-S | NA | NA | 0.00278483 | 3 |
| 81 | GI_19924140-A | NM_004737 | LARGE | 0.00299301 | 3 |
| 82 | Hs.474081-S | NA | NA | 0.00171741 | 3 |
| 83 | hmm27707-S | NA | NA | 0.00182347 | 3 |
| 84 | GI_30911100-S | NM_015055 | SWAP70 | 0.000599461 | 3 |
| 85 | Hs.465277-S | NA | NA | 0.00173828 | 2 |
| 86 | hmm34655-S | NA | NA | 0.00104155 | 2 |
| 87 | GI_4506958-S | NM_003035 | SIL | 0.00185767 | 2 |
| 88 | GI_18641377-S | NM_002120 | HLA-DOB | 1.38E-05 | 1 |
| 89 | hmm21486-S | NA | NA | 0.00188132 | 1 |
| 90 | GI_11038675-A | NM_000626 | CD79B | 0.00235909 | 1 |
| 91 | hmm32554-S | NA | NA | 0.0019331 | 1 |
| 92 | Hs.485542-S | NA | NA | 0.000347975 | 1 |
| 93 | GI_27894318-A | NM_000577 | IL1RN | 0.00157415 | 1 |
| 94 | GI_11415027-S | NM_021966 | TCL1A | 7.98E-06 | 1 |
| 95 | GI_5803097-S | NM_006766 | MYST3 | 0.00249811 | 1 |
| 96 | Hs.494077-S | NA | NA | 0.00140988 | 1 |
| 97 | GI_11024711-S | NM_017533 | MYH4 | 0.00203687 | 1 |
| 98 | GI_38327546-I | NM_014499 | P2RY10 | 0.00211655 | 1 |
| 99 | Hs.464703-S | NA | NA | 0.000836461 | 1 |
| 100 | hmm22395-S | NA | NA | 0.00167487 | 1 |
| 101 | GI_34147598-S | NM_002002 | FCER2 | 0.000153367 | 1 |
| 102 | Hs.441650-S | NA | NA | 0.00225655 | 1 |
| 102 | Hs.443763-S | NA | NA | 0.000320059 | 1 |
| 103 | hmm19912-S | NA | NA | 0.00259816 | 1 |
| 104 | Hs.436487-S | NA | NA | 0.00230149 | 1 |
| 105 | GI_4885562-S | NM_005400 | PRKCE | 0.000525153 | 1 |
| 106 | GI_14456711-S | | HBA1 | 0.00107959 | 1 |
| 107 | hmm33317-S | NA | NA | 0.00231044 | 1 |

Blood Collection and storage in straws: Blood samples from the EPIC cohort were not taken explicitly for RNA analysis. No stabilization was performed. Blood samples were taken and centrifuged in straws. PBMC enriched fractions (5mm) of the straw were taken for further analysis.

Blood Collection using PAX gene system: The PAXgene system was used for prevalent lung cancer samples. It allows standardized and easy blood collection based on convenient BD Vacutainer™ technology. The PAXgene tube contains a proprietary reagent that immediately stabilizes intracellular RNA for 3 days at room temperature (18-25°C) and 5 days at 2-8°C (Rainen, L., et al., Clin Chem 48, 1883-1890 (2002)). The here described protocol is extracted from the manufacturer's instructions.
- Ensure that the PAXgene Blood RNA Tube is at room temperature (18°C-25°C) prior to use and properly labeled with patient identification.
- If the PAXgene Blood RNA Tube is the only tube to be drawn, a small amount of blood should be drawn into a "Discard Tube" prior to drawing blood into the PAXgene Blood RNA Tube. Otherwise, the PAXgene Blood RNA Tube should be the last tube drawn in the phlebotomy procedure.
- Using a BD Vacutainer® Safety-Lok™ Blood Collection Set, collect blood into the PAXgene Blood RNA Tube holding the PAXgene Blood RNA Tube vertically, below the blood donor's arm, during blood collection.
- Allow at least 10 seconds for a complete blood draw to take place. Ensure that the blood has stopped flowing into the tube before removing the tube from the holder.
- Gently invert the PAXgene Blood RNA Tube 8 to 10 times.
- Store the PAXgene Blood RNA Tube upright at room temperature (18°C-25°C) for at least 2h. After 24h proceed to RNA isolation or store at -20°C or -80°C for long-term storage.
- To freeze PAXgene™Blood RNA Tubes stand them upright in a wire rack. Do not freeze tubes upright in a Styrofoam tray as this may cause the tubes to crack. Tubes can be stored at -20°C and below. If tubes are to be frozen at temperatures below -20°C, freeze the tubes first at -20°C for 24 hours, then transfer them to -70°C or -80°C.
- To thaw PAXgene™ Blood RNA Tubes, place them in a wire rack at ambient temperature (18-22°C) for approximately two hours. Do not thaw PAXgene™ Blood RNA Tubes at temperatures above 22°C. One suggestion is to remove the tubes from the freezer the afternoon before processing, thaw overnight, and process tubes the next day. After thawing, carefully invert the tubes 10 times before starting RNA preparation.

### RNA preparation after native asservation

Homogenization: Homogenize blood samples in TRI Reagent (5 ml/5mm straw) by directly thawing sample in TriReagent. Rapidely homogenize by mechanically pipetting samples several times. Thorough pipetting is important and coagulation of samples must be avoided

Phase separation: Store the homogenate for 5 minutes at room temperature to permit the complete dissociation of nucleoprotein complexes. Next, supplement the homogenate with 0.2 ml chloroform per 1 ml of TRI Reagent, cover the samples tightly and shake vigorously for 15 seconds. Store the resulting mixture at room temperature for 2-15 minutes and centrifuge at 12,000 g for 15 minutes at 4 C. Following centrifugation, the mixture separates into a lower red phenol-chloroform phase, interphase and the colorless upper aqueous phase. RNA remains exclusively in the aqueous phase whereas DNA and proteins are in the interphase and organic phase. The volume of the aqueous phase is about 60% of the volume of TRI Reagent used for homogenization. Chloroform used for phase separation should not contain isoamyl alcohol or any other additive.

RNA Prcipitation: Transfer the aqueous phase to a fresh. Precipitate RNA from the aqueous phase by mixing with isopropanol. Use 0.5 ml of isopropanol per 1 ml of TRI Reagent used for the initial homogenization. Store samples at room temperature for 5-10 minutes and centrifuge at 12,000 g for 8 minutes at 4 - 25 C. RNA precipitate (often invisible before centrifugation) forms a gel-like or white pellet on the side and bottom of the tube.

RNA wash: Remove the supernatant and wash the RNA pellet ( by vortexing) with 70% ethanol and subsequent centrifugation at 7,500 g for 5 minutes at 4 - 25 C. Add at least 1 ml of 70% ethanol per 1 ml TRI Reagent used for the initial homogenization.Samples were stored in 70% ethanol prior further clean-up.

RNA solubilization: Remove the ethanol wash and briefly air-dry the RNA pellet for 3 - 5 min. Ad the desired amount of RNAse free water.

RNA Clean-up using RNeasev MinElute kit (Oiagen): Adjust the sample to a volume of 100 µl with RNase-free water. Add _ 350 µl Buffer RLT, and mix well. If starting with an RNA pellet, be sure that the pellet is dissolved in the RNase-free water (supplied) before adding Buffer RLT.

Add 250 µl of 96-100% ethanol to the diluted RNA, and mix well by pipetting. Do not centrifuge. Proceed immediately to nex step.

Transfer the sample (700 µl) to an RNeasy MinElute spin column placed in a 2 ml collection tube (supplied). Close the lid gently, and centrifuge for 15 s at 8000 x g. Discard the flow-through.

Place the RNeasy MinElute spin column in a new 2 ml collection tube (supplied). Add 500 µl Buffer RPE to the spin column. Close the lid gently, and centrifuge for 15 s at 8000 x g to wash the spin column membrane. Discard the flow-through. Note: Buffer RPE is supplied as a concentrate. Ensure that ethanol is added to Buffer RPE before use.

Add 500 µl of 80% ethanol to the RNeasy MinElute spin column. Close the lid gently, and centrifuge for 2 min at 8000 x g to wash the spin column membrane. Discard the flow-through and collection tube.

Place the RNeasy MinElute spin column in a new 2 ml collection tube (supplied). Open the lid of the spin column, and centrifuge at full speed for 5 min. Discard the flow-through and collection tube. It is important to dry the spin column membrane since residual ethanol may interfere with downstream reactions.

Place the RNeasy MinElute spin column in a new 1.5 ml collection tube (supplied). Add 14 µl RNase-free water directly to the center of the spin column membrane. Close the lid gently, and centrifuge for 1 min at full speed to elute the RNA.

RNA preparation after PAXgene asservation: The PAXgene Blood RNA Kit allows the isolation of total RNA from 2.5 ml human whole blood collected in the PAXgene Blood RNA Tube. The here described protocol is extracted from the manufacturer's instructions with slight modifications.

Centrifuge the PAXgene Blood RNA Tube for 10 minutes at 3000-5000 x g at room temperature using a swing out rotor. Important: The rotor must contain tube adapters for round-bottom tubes. If other types of tube adapter are used, the tubes may break during centrifugation.

Remove the supernatant by decanting or pipetting. Add 5 ml RNase-free water to the pellet, and close the tube using a fresh secondary Hemogard closure. If the supernatant is decanted, take care not to disturb the pellet, and dry the rim of the tube with a clean paper towel.

Vortex until the pellet is visibly dissolved, and centrifuge for 10 minutes at 3000-5000 x g using a swing-out rotor. Remove and discard the entire supernatant. Small debris remaining in the supernatant after vortexing but before centrifugation will not affect the procedure. Important: Incomplete removal of the supernatant will inhibit lysis and dilute the lysate, and therefore affect the conditions for binding RNA to the PAXgene membrane.

Thoroughly resuspend the pellet in 360 µl Buffer BR1 by vortexing.

Pipet the sample into a 1.5 ml microcentrifuge tube. Add 300 µl Buffer BR2 and 40 µl proteinase K. Mix by vortexing for 5 seconds, and incubate for 15 minutes at 55°C using a shaker-incubator at 1400 rpm. After incubation, set the temperature of the shaker-incubator to 65°C (for step 20). Important: Do not mix Buffer BR2 and proteinase K together before adding them to the sample. See Note 1.

Centrifuge for 5 min at maximum speed in a microcentrifuge. Transfer the supernatant to a fresh 2 ml microcentrifuge tube. A minimum g-force of 10,000 x g is required. Transfer of small debris remaining in the supernatant after centrifugation at full speed will not affect the procedure. See Note 2.

Add 350 µl abs. ethanol. Mix by vortexing, and centrifuge briefly (1-2 seconds at 500-1000 x g) to remove drops from the inside of the tube lid. Important: The length of the centrifugation must not exceed 1-2 seconds, as this may result in pelleting of nucleic acids and reduced yields of total RNA.

Pipet 700 µl sample into the PAXgene RNA spin column (red) placed in a 2 ml processing tube, and centrifuge for 1 minute at 8000-20,000 x g. Place the spin column in a new 2 ml processing tube, and discard the old processing tube containing flow-through.

Pipet the remaining sample into the PAXgene RNA spin column, and centrifuge for 1 minute at 8000-20,000 x g. Place the spin column in a new 2 ml processing tube, and discard the old processing tube containing flow-through. Important: Carefully pipet the sample into the spin column and visually check that the sample is completely transferred to the spin column. See Note 3.

Pipet 350 µl Buffer BR3 into the PAXgene RNA spin column. Centrifuge for 1 minute at 8000-20,000 x g. Place the spin column in a new 2 ml processing tube, and discard the old processing tube containing flow-through.

Add 10 µl DNase I stock solution to 70 µl Buffer RDD in a 1.5 ml microcentrifuge tube. Mix by gently flicking the tube, and centrifuge briefly to collect residual liquid from the sides of the tube. If processing, for example, 10 samples, add 110 µl DNase I stock solution to 770 µl Buffer RDD. Important: DNase I is especially sensitive to physical denaturation. Mixing should only be carried out by gently flicking the tube. Do not vortex.

Pipet the DNase I incubation mix (80 µl) directly onto the PAXgene RNA spin column membrane, and place on the benchtop (20-30°C) for 15 minutes. Important: Ensure that the DNase I incubation mix is placed directly onto the membrane. DNase digestion will be incomplete if part of the mix is applied to and remains on the walls or the O-ring of the spin column.

Pipet 350 µl Buffer BR3 into the PAXgene RNA spin column, and centrifuge for 1 minute at 8000-20,000 x g. Place the spin column in a new 2 ml processing tube, and discard the old processing tube containing flow-through.

Pipet 500 µl Buffer BR4 to the PAXgene RNA spin column, and centrifuge for 1 minute at 8000-20,000 x g. Place the spin column in a new 2 ml processing tube, and discard the old processing tube containing flow-through. Important: Buffer BR4 is supplied as a concentrate. Ensure that ethanol is added to Buffer BR4 before use.

Add another 500 µl Buffer BR4 to the PAXgene RNA spin column. Centrifuge for 3 minutes at 8000-20,000 x g.

Discard the tube containing the flow-through, and place the PAXgene RNA spin column in a new 2 ml processing tube. Centrifuge for 1 minute at 8000-20,000 x g.

Discard the tube containing the flow-through. Place the PAXgene RNA spin column in a 1.5 ml microcentrifuge tube, and pipet 40 µl RNAse-free water directly onto the PAXgene RNA spin column membrane. Centrifuge for 1 minute at 8000-20,000 x g to elute the RNA. It is important to wet the entire membrane with H₂O in order to achieve maximum elution efficiency.

Repeat the elution step as described, using the same 40 µl RNAse-free water and the same microcentrifuge tube.

Incubate the eluate for 5 minutes at 65°C in the shaker-incubator without shaking. After incubation, chill immediately on ice. Important: This incubation at 65°C denatures the RNA for downstream applications. Do not exceed the incubation time or temperature.

If the RNA samples will not be used immediately, store at -20°C or -70°C. Since the RNA remains denatured after repeated freezing and thawing, it is not necessary to repeat the incubation at 65°C.

### Quantification and determination of quality of total RNA:

The concentration of RNA should be determined by measuring the absorbance at 260 nm (A260) in a spectrophotometer. To ensure significance, readings should be greater than 0.15. An absorbance of 1 unit at 260 nm corresponds to 44 µg of RNA per ml (A260 = 1, c = 44 µg/ml). This relation is valid only for measurements in 10 mM Tris-Cl, pH 7.5. Therefore, if it is necessary to dilute the RNA sample, this should be done in 10 mM Tris·Cl.

The ratio of the readings at 260 nm and 280 nm (A260/A280) provides an estimate of the purity of RNA with respect to contaminants that absorb in the UV, such as protein. However, the A260/A280 ratio is influenced considerably by pH. Lower pH results in a lower A260/A280 ratio and reduced sensitivity to protein contamination. For accurate values, we recommend measuring absorbance in 10 mM Tris·Cl, pH 7.5. Pure RNA has an A260/A280 ratio of 1.9-2.1 in 10 mM Tris·Cl, pH 7.5. Always calibrate the spectrophotometer with the same solution.

The integrity and size distribution of total RNA purified using the PAXgene Blood RNA System can be checked by denaturing agarose gel electrophoresis (1% w/v) and ethidium bromide staining. The respective ribosomal bands should appear sharp on the stained gel. 28S ribosomal RNA (5 kb) bands should be present with an intensity approximately twice that of the 18S RNA (1.9 kb) band. If the ribosomal bands in a given lane are not sharp, but appear as a smear of smaller sized RNAs, it is likely that the RNA sample suffered major degradation during preparation. Only RNA samples of good purity and integrity should be used for further microarray analysis.

cDNA and cRNA synthesis using Illumina TotalPrep RNA Amplification Kit: RNA amplification has become the standard method for preparing RNA samples for array analysis (Kacharmina, J.E., et al., Methods Enzymol 303, 3-18 (1999); 3. Pabon, C., et al., Biotechniques 31, 874-879 (2001)). The Illumina® TotalPrep RNA Amplification Kit is a complete system for generating biotinylated, amplified RNA for hybridization with Illumina Sentrix® arrays. It is based on the RNA amplification protocol developed in the laboratory of James Eberwine Van Gelder, R.N., et al., Proc Natl Acad Sci U S A 87, 1663-1667 (1990)). The procedure consists of reverse transcription with an oligo(dT) primer bearing a T7 promoter using Array-Script™ a reverse transcriptase (RT) engineered to produce higher yields of first strand cDNA than wild type enzymes. ArrayScript catalyzes the synthesis of virtually full-length cDNA. The cDNA then undergoes second strand synthesis and clean-up to become a template for in vitro transcription with T7 RNA Polymerase where hundreds to thousands of biotinylated, antisense RNA copies are synthesized of each mRNA in a sample. The labeled cRNA produced with the kit was developed for hybridization with Illumina arrays. The here described protocol is according to the manufacturer's instructions.

### Reverse Transcription to Synthesize First Strand cDNA:

- Place a maximum volume of 11 µl of total RNA (100 ng) into a nonstick, sterile, RNase-free, 0.2 ml microcentrifuge tube.
- Add Nuclease-free Water as necessary to bring all samples to 11 µl.
- At room temperature, prepare Reverse Transcription Master Mix in a nuclease-free tube. Assemble enough to synthesize first strand cDNA from all the RNA samples in the experiment

Reverse Transcription Master Mix (for a single 20 µl reaction)
1 µl T7 Oligo(dT) Primer (17-21dT)
2 µl 10X First Strand Buffer
4 µl dNTP Mix
1 µl RNase Inhibitor
1 µl ArrayScript

- Mix well by gently vortexing. Centrifuge briefly (~5 sec) to collect the Reverse Transcription Master Mix at the bottom of the tube and place on ice.
- Transfer 9 µl of Reverse Transcription Master Mix to each RNA sample. Mix thoroughly by pipetting up and down 2-3 times, then flicking the tube 3-4 times, and centrifuge briefly to collect the reaction in the bottom of the tube.
- Place the samples in a 42°C incubator. Incubate reactions for 2h at 42°C
- After the incubation, centrifuge briefly (~5 sec) to collect the reaction mixture at the bottom of the tube. Place the tubes on ice and immediately proceed Second Strand cDNA Synthesis.

### Second Strand cDNA Synthesis:

- On ice, prepare a Second Strand Master Mix in a nuclease-free tube in the order listed below. Assemble enough to synthesize second strand cDNA from all the samples in the experiment. Assemble the Second Strand Master Mix on ice in the order shown:

Second Strand Master Mix (for a single 100 µl reaction)
63 µl Nuclease-free Water
10 µl 10X Second Strand Buffer
4 µl dNTP Mix (2mM dATP, dTTP, dCTP and dGTP)
2 µl DNA Polymerase
1 µl RNase H

- Mix well by gently vortexing. Centrifuge briefly (~5 sec) to collect the mixture at the bottom of the tube and place on ice.
- Transfer 80 µl of Second Strand Master Mix to each sample. Mix thoroughly by pipetting up and down 2-3 times, then flicking the tube 3-4 times, and centrifuge briefly to collect the reaction in the bottom of the tube.
- Place the tubes in a 16°C thermal cycler. It is important to cool the thermal cycler block to 16°C before adding the reaction tubes because subjecting the reactions to temperatures >16°C will compromise cRNA yield. Incubate 2h in a 16°C thermal cycler.
- After the 2h incubation at 16°C, place the reactions on ice and proceed to cDNA Purification (below), or immediately freeze reactions at -20°C. Do not leave the reactions on ice for more than 1 h.
- This is a potential overnight stopping point (at -20°C), but it is better to complete the cDNA purification before stopping.

### cDNA Purification

- Preheat Nuclease-free Water to 50-55°C
- Check the cDNA Binding Buffer for precipitation before using it. If a precipitate is visible, redissolve it by warming the solution to 37°C for up to 10 min and vortexing vigorously. Cool to room temp before use.
- Add 250 µl of cDNA Binding Buffer to each sample, and mix thoroughly by pipetting up and down 2-3 times, then flicking the tube 3-4 times. Follow up with a quick spin to collect the reaction mixture in the bottom of the tube.

Proceed quickly to the next step.
- Check that the cDNA Filter Cartridge is firmly seated in its wash tube (supplied).
- Pipet the cDNA sample/cDNA Binding Buffer onto the center of the cDNA Filter Cartridge.
- Centrifuge for ~1 min at 10,000 x g, or until the mixture is through the filter. Discard the flow-through and replace the cDNA Filter Cartridge in the wash tube.
- Apply 500 µl Wash Buffer to each cDNA Filter Cartridge.
- Centrifuge for ∼1 min at 10,000 X g, or until all the Wash Buffer is through the filter.
- Discard the flow-through and spin the cDNA Filter Cartridge for an additional minute to remove trace amounts of Wash Buffer.
- Transfer cDNA Filter Cartridge to a cDNA Elution Tube.
- Elute cDNA with a total of 19 µl 50-55°C Nuclease-free Water. It is important to use Nuclease-free Water that is at 50-55°C for the cDNA elution.

Colder water will be less efficient at eluting the cDNA, and using hotter water (≥ 58°C) may result in reduced cRNA yield.
- Apply 10 µl of Nuclease-free Water (preheated to 50-55°C) to the center of the filter in the cDNA Filter Cartridge.
- Leave at room temperature for 2 min and then centrifuge for ~1.5 min at 10,000 X g, or until all the Nuclease-free Water is through the filter.
- Apply a second aliquot of 9 µl preheated Nuclease-free Water and centrifuge for 2 min. The double-stranded cDNA will now be in the eluate (~17.5 µl.)
- Proceed directly to section In Vitro Transcription to Synthesize cRNA, or place the cDNA at -20°C.

In Vitro Transcription to Synthesize CRNA:
- At room temperature, prepare an IVT Master Mix by adding the following reagents to a nuclease-free microcentrifuge tube in the order listed below.

Assemble enough to synthesize cRNA from all the samples in the experiment.
IVT Master Mix for a single 25 µl reaction
2.5 µl T7 10X Reaction Buffer
2.5 µl T7 Enzyme Mix
2.5 µl Biotin-NTP Mix

- Mix well by gently vortexing. Centrifuge briefly (~5 sec) to collect the IVT Master Mix at the bottom of the tube and place on ice.
- Transfer 7.5 µl of IVT Master Mix to each cDNA sample (volume ~17.5 µl). If less than 17.5 µl are eluted fill up with RNAse-free water to reach volume of 17.5 µl. Mix thoroughly by pipetting up and down 2-3 times, then flicking the tube 3-4 times, and centrifuge briefly to collect the reaction mixture in the bottom of the tube. Once assembled, place the tubes at 37°C.
- Incubate reactions for 16h. It is important to maintain a constant 37°C incubation temperature. It is recommended to incubate in a hybridization oven because it is extremely important that condensation does not form inside the tubes; his would change the reagent concentrations and reduce yield.

Alternatively you can use a thermal cycler with heated lid temperature set to 40°C.
- Stop the reaction by adding 75 µl Nuclease-free Water to each cRNA sample to bring the final volume to 100 µl. Mix thoroughly by gentle vortexing.

Proceed to the cRNA purification step (below) or store at -20°C.

### cRNA Purification:

- Preheat Nuclease-free Water to 50-60°C
- Assemble cRNA Filter Cartridges and tubes and add 350 µl of cRNA Binding Buffer to each cRNA sample. Proceed to the next step immediately.
- Add 250 µl of 100% ethanol to each cRNA sample, and mix by pipetting the mixture up and down 3 times. Do NOT vortex to mix and do NOT centrifuge.
- Pipet each sample mixture from step 3 onto the center of the filter in the cRNA Filter Cartridge.
- Centrifuge for ~1 min at 10,000 X g. Continue until the mixture has passed through the filter.
- Discard the flow-through and replace the cRNA Filter Cartridge back into the cRNA Collection Tube.
- Apply 650 µl Wash Buffer to each cRNA Filter Cartridge.
- Centrifuge for ~1 min at 10,000 x g, or until all the Wash Buffer is through the filter.
- Discard the flow-through and spin the cRNA Filter Cartridge for an additional ~1 min to remove trace amounts of Wash Buffer.
- Transfer Filter Cartridge(s) to a fresh cRNA Collection Tube.
- To the center of the filter, add 100 µl Nuclease-free Water (preheated to 50-60°C). Leave at room temperature for 2 min and then centrifuge for ~1.5 min at 10,000 x g, or until the Nuclease-free Water is through the filter.
- The cRNA will now be in the cRNA Collection Tube in ~100 µl of Nuclease-free Water.

### Quantification and determination of total CRNA:

The concentration of cRNA is determined by OD measurement.

The size distribution of cRNA can be checked by denaturing agarose gel electrophoresis (1% w/v) and ethidium bromide staining.

Hybridization: The BeadStation 500X system uses a "direct hybridization" assay, whereby gene-specific probes are used to detect labeled RNAs. Each bead in the array contains a 50-mer, sequence-specific oligo probe synthesized in-house using Illumina's Oligator™ technology. The most consistent results are achieved by hybridizing equivalent amounts of cRNA on each array. An appropriate volume of cRNA from each sample is aliquoted into the hybridization tube(s). The here described protocol is according to the manufacturer's instructions and is applicable only to 6-sample Beadchips with InteIliHyb Seal.
- Preheat the oven (with rocking platform) to 58°C.
- Prepare cRNA samples (dried down in a vacuum centrifuge, if necessary to achieve required concentration). To 1.5 µg cRNA, add RNase-free water up to 10 µL and mix. Leave at room temp for 10 minutes to resuspend cRNA.
- Place the GEX-HYB and GEX-HCB tubes in the 58°C oven for 10 minutes to dissolve any salts that may have precipitated in storage. Inspect the solution;

if any salts remain undissolved, incubate at 58°C for another 10 minutes. After allowing to cool to room temperature, mix thoroughly before using.
- Add 20 µl GEX-HYB to each cRNA sample.
- Place Illumina Hyb Chamber Gaskets into BeadChip Hyb Chamber.
- Dispense 200 µl GEX-HCB into each of the two humidifying buffer reservoirs in each Hyb Chamber. Only add buffer to chambers that will be used.
- Seal Hyb Chamber with lid and keep on bench at room temperature (~22°C) until ready to load BeadChips into Hyb Chamber.
- Remove all BeadChips from their packages.
- Holding BeadChip by coverseal tab with tweezers using powder-free gloved hands, slide BeadChip into Hyb Chamber Insert such that the barcode lines up with barcode symbol on the Insert.
- Preheat the assay sample at 65°C for 5 minutes.
- Briefly vortex, then briefly centrifuge to collect the liquid in the bottom of the tube. Allow sample to cool to room temperature before using. Pipet sample immediately after cooling to room temp.
- Load Hyb Chamber Inserts containing BeadChips into the Hyb Chamber with barcode on BeadChip aligned to barcode symbol on the Hyb Chamber.
- Dispense 30 µL assay sample onto the large sample port of each array.
- Seal lid onto the Hyb Chamber carefully to avoid dislodging the Hyb Chamber Insert(s).
- Incubate for 16-20h at 58°C with rocker speed at 5.
- Prepare 1X High-Temp Wash buffer (add 50 ml 10X stock to 450 ml RNAse-free water).
- Place waterbath insert into heat block, and add 500 ml prepared 1X High-Temp Wash buffer.
- Set heat block temp to 55°C and pre-warm High-Temp Wash buffer to that temperature.
- Close heat block lid and leave overnight.

### Washing and Scanning:

- Make Wash E1BC solution (add 7 ml E1BC buffer to 2.5 I RNase-free water).
- Pre-warm Block E1 buffer (4 ml/chip) to room temperature.
- Prepare Block E1 buffer (2 ml/chip) with streptavidin-Cy3 (2 µL of 1 mg/ml stock per chip). Use a single conical tube for all BeadChips. Store in dark until detection step.
- Remove Hyb Chamber from oven and disassemble.
- Remove coverseal from the BeadChip with Beadchip submerged in ~ 1 I E1BC-Buffer (use glass dish for this step). Using tweezers or powder-free gloved hands, place the BeadChip into the slide rack submerged in the staining dish containing 250 ml Wash E1BC solution.
- Repeat disassembly and placement in E1BC solution for all BeadChips.
- Using the slide rack handle, transfer the rack into the Hybex Waterbath insert containing High-Temp Wash buffer.
- Incubate static for 10 minutes with the Hybex lid closed.
- During the 10-minute High-Temp Wash buffer incubation, add fresh 250 ml Wash E1BC solution to a clean staining dish.
- After the 10-minute High-Temp Wash buffer incubation is complete, immediately transfer the slide rack into the staining dish containing E1BC.
- Briefly agitate using rack, then shake on orbital shaker for 5 minutes at the highest speed possible without allowing solution to splash out of dish.
- Transfer rack to a clean staining dish containing 250 ml 100% Ethanol (use fresh from Ethanol source bottle).
- Briefly agitate using rack handle, then shake on orbital shaker for 10 minutes.
- Transfer rack to a clean staining dish containing fresh 250 ml Wash E1BC solution.
- Briefly agitate using rack handle, then shake on orbital shaker for 2 minutes.
- Pipette 4 ml Block E1 buffer into the Wash Tray(s).
- Transfer the BeadChip, face up into BeadChip Wash Tray(s) on rocker.
- Rock at medium speed for 10 minutes.
- Pipette 2 ml Block E1 buffer + streptavidin-Cy3 into fresh Wash Tray(s).
- Transfer the BeadChip, face up into Wash Tray(s) on rocker.
- Place cover on tray and rock at medium speed for 10 minutes.
- Add 250 ml of Wash E1BC solution to a clean staining dish.
- Transfer the BeadChip to the slide rack submerged in the staining dish.
- Briefly agitate using rack, and then shake at room temp on orbital shaker for 5 minutes with Beadchips protected from light.
- Prepare centrifuge with plateholders, paper towels and balance rack. Set speed to 275 rcf.
- Transfer rack of BeadChips from staining dish to centrifuge and spin at room temperature for 4 min.
- Store dry chips in the dark until scanned.
- BeadChips are imaged using the Illumina BeadArray Reader, a two-channel, 0.8 µm resolution confocal laser scanner. The BeadArray Reader, using SentrixScan menu-driven software, automatically scans up to three different BeadChips or a SAM, hands-free, at one or at two wavelengths and creates an image file for each channel.

### Sequence Listing, Free Text

SEQ ID NOs:1/2 gi|10863876|ref|NM_021105.1| Homo sapiens phospholipid scramblase 1 (PLSCR1), probe and coding sequence.
SEQ ID NOs:3/4 gi|110611902|ref|NM_017533.2| Homo sapiens myosin, heavy chain 4, skeletal muscle (MYH4), probe and coding sequence.
SEQ ID NOs:S/6 gi|90193587|ref|NM_001783.3| Homo sapiens CD79a molecule, immunoglobulin-associated alpha (CD79A), transcript variant 1, probe and coding sequence.
SEQ ID NOs:7/8 gi|90193589|ref|NM_000626.2| Homo sapiens CD79b molecule, immunoglobulin-associated beta (CD79B), transcript variant 1, probe and coding sequence.
SEQ ID NOs:9/10 gi|90193589|ref|NM_000626.2| Homo sapiens CD79b molecule, immunoglobulin-associated beta (CD79B), transcript variant 1, probe and coding sequence.
SEQ ID NOs:11/12 gi|89363029|ref|NM_001039717.1| Homo sapiens chromosome 4 open reading frame 29 (C4orf29), probe and coding sequence.
SEQ ID NOs:13/14 gi|13899296|ref|NM_031458.1| Homo sapiens poly (ADP-ribose) polymerase family, member 9 (PARP9), probe and coding sequence.
SEQIDNOs:15/16 gi|166091436|ref|NM_001716.3| Homo sapiens chemokine (C-X-C motif) receptor 5 (CXCR5), transcript variant 1, probe and coding sequence.
SEQ ID NOs:17/18 gi|50428916|ref|NM_001002264.1| Homo sapiens epithelial stromal interaction 1 (breast) (EPSTI1), transcript variant 1, probe and coding sequence.
SEQ ID NOs:19/20 gi|118402587|ref|NM_002120.3| Homo sapiens major histocompatibility complex, class II, DO beta (HLA-DOB), probe and coding sequence.
SEQ ID NOs:21/22 gi|59853527|ref|NM_004737.3| Homo sapiens like-glycosyltransferase (LARGE), transcript variant 1, probe and coding sequence.
SEQ ID NOs:23/24 gi|20149601|ref|NM_012229.2| Homo sapiens 5'-nucleotidase, cytosolic II (NT5C2), probe and coding sequence.
SEQ ID NOs:25/26 gi|166706910|ref|NM_006417.4| Homo sapiens interferon-induced protein 44 (IFI44), probe and coding sequence.
SEQ ID NOs:27/28 gi|54091031|ref|NM_144590.2| Homo sapiens ankyrin repeat domain 22 (ANKRD22), probe and coding sequence.
SEQ ID NOs:29/30 gi|22027643|ref|NM_002257.2| Homo sapiens kallikrein 1 (KLK1), probe and coding sequence.
SEQ ID NOs:31/32 gi|22202620|ref|NM_000846.3| Homo sapiens glutathione S-transferase A2 (GSTA2), probe and coding sequence.
SEQ ID NOs:33/34 gi|22748768|ref|NM_152355.1| Homo sapiens zinc finger protein 441 (ZNF441), probe and coding sequence.
SEQ ID NOs:35/36 gi|122937216|ref|NM_001080411.1| Homo sapiens zinc finger protein 433 (ZNF433), probe and coding sequence.
SEQ ID NOs:37/38 gi|68348721|ref|NM_021950.3| Homo sapiens membrane-spanning 4-domains, subfamily A, member 1 (MS4A1), transcript variant 3, probe and coding sequence.
SEQ ID NOs:39/40 gi|23111059|ref|NM_005850.3| Homo sapiens splicing factor 3b, subunit 4, 49kDa (SF3B4), probe and coding sequence.
SEQ ID NOs:41/42 gi|24308268|ref|NM_021218.1| Homo sapiens chromosome 9 open reading frame 80 (C9orf80), probe and coding sequence.
SEQ ID NOs:43/44 gi|53759115|ref|NM_000593.5| Homo sapiens transporter 1, ATP-binding cassette, sub-family B (MDR/TAP) (TAP1), probe and coding sequence.
SEQ ID NOs:45/46 gi|26787992|ref|NM_006873.2| Homo sapiens stonin 1 (STON1), probe and coding sequence.
SEQ ID NOs:47/48 gi|78190479|ref|NM_001034837.1| Homo sapiens Kv channel interacting protein 1 (KCNIP1), transcript variant 1, probe and coding sequence.
SEQ ID NOs:49/50 gi|27894318|ref|NM_173842.1| Homo sapiens interleukin 1 receptor antagonist (IL1RN), transcript variant 1, probe and coding sequence.
SEQ ID NOs:51/52 gi|28302128|ref|NM_000518.4| Homo sapiens hemoglobin, beta (HBB), probe and coding sequence.
SEQ ID NOs:53/54 gi|117606369|ref|NM_207315.2| Homo sapiens cytidine monophosphate (UMP-CMP) kinase 2, mitochondrial (CMPK2), nuclear gene encoding mitochondrial protein, probe and coding sequence.
SEQ ID NOs:55/56 gi|50263054|ref|NM_178483.2| Homo sapiens chromosome 20 open reading frame 79 (C20orf79), probe and coding sequence.
SEQ ID NOs:57/58 gi|93102363|ref|NM_015055.2| Homo sapiens SWAP-70 protein (SWAP70), probe and coding sequence.
SEQ ID NOs:59/60 gi|144953890|ref|NM_017935.3| Homo sapiens B-cell scaffold protein with ankyrin repeats 1 (BANK1), transcript variant 1, probe and coding sequence.
SEQ ID NOs:61/62 gi|33598961|ref|NM_004443.3| Homo sapiens EPH receptor B3 (EPHB3), probe and coding sequence.
SEQ ID NOs:63/64 gi|33859792|ref|NM_173494.1| Homo sapiens chromosome X open reading frame 41 (CXorf41), probe and coding sequence.
SEQ ID NOs:65/66 gi34147320|ref|NM_005533.2| Homo sapiens interferon-induced protein 35 (IFI35), probe and coding sequence.
SEQ ID NOs:67/68 gi|34147598|ref|NM_002002.3| Homo sapiens Fc fragment of IgE, low affinity II, receptor for (CD23) (FCER2), probe and coding sequence.
SEQ ID NOs:69/70 gi|34147611|ref|NM_024670.3| Homo sapiens suppressor of variegation 3-9 homolog 2 (Drosophila) (SUV39H2), probe and coding sequence.
SEQ ID NOs:71/72 gi|34147626|ref|NM_023926.3| Homo sapiens zinc finger and SCAN domain containing 18 (ZSCAN18), probe and coding sequence.
SEQ ID NOs:73/74 gi|153070786|ref|NR_003608.1| Homo sapiens tubulin, alpha pseudogene (MGC16703) on chromosome, probe and coding sequence.
SEQ ID NOs:75/76 gi|153252109|ref|NM_145051.3| Homo sapiens ring finger protein 183 (RNF183), probe and coding sequence.
SEQ ID NOs:77/78 gi|46370097|ref|NM_024026.4| Homo sapiens mitochondrial ribosomal protein 63 (MRP63), nuclear gene encoding mitochondrial protein, probe and coding sequence.
SEQ ID NOs:79/80 gi|76781490|ref|NM_007047.3| Homo sapiens butyrophilin, subfamily 3, member A2 (BTN3A2), probe and coding sequence.
SEQ ID NOs:81/82 gi|44771200|ref|NM_030628.1| Homo sapiens integrator complex subunit 5 (INTS5), probe and coding sequence.
SEQ ID NOs:83/84 gi|38016929|ref|NM_198213.11 Homo sapiens 2'-5'-oligoadenylate synthetase-like (OASL), transcript variant 2, probe and coding sequence.
SEQ ID NOs:85/86 gi|38157980|ref|NM_004223.3| Homo sapiens ubiquitin-conjugating enzyme E2L 6 (UBE2L6), transcript variant 1, probe and coding sequence.
SEQ ID NOs:87/88 gi|38195085|ref|NM_022098.21 Homo sapiens X-prolyl aminopeptidase (aminopeptidase P) 3, putative (XPNPEP3), probe and coding sequence.
SEQ ID NOs:89/90 gi|109633030|ref|NM_198253.2| Homo sapiens telomerase reverse transcriptase (TERT), transcript variant 1, probe and coding sequence.
SEQ ID NOs:91/92 gi|150010588|ref|NM_003641.3| Homo sapiens interferon induced transmembrane protein 1 (9-27) (IFITM1), probe and coding sequence.
SEQ ID NOs:93/94 gi|40254458|ref|NM_001884.2| Homo sapiens hyaluronan and proteoglycan link protein 1 (HAPLN1), probe and coding sequence.
SEQ ID NOs:95/96 gi|142357995|ref|NM_144682.4| Homo sapiens schlafen family member 13 (SLFN13), probe and coding sequence.
SEQ ID NOs:97/98 gi|154350243|ref|NM_001100411.1| Homo sapiens chromosome 6 open reading frame 60 (C6orf60), transcript variant 2, probe and coding sequence.
SEQ ID NOs:99/100 gi|40288190ref|NM_017523.2| Homo sapiens XIAP associated factor 1 (XAF1), transcript variant 1, probe and coding sequence.
SEQ ID NOs:101/102 gi|40807470|ref|NM_003594.3| Homo sapiens transcription termination factor, RNA polymerase II (TTF2), probe and coding sequence.
SEQ ID NOs:103/104 gi|41281452|ref|NM_014720.2| Homo sapiens STE20-like kinase (yeast) (SLK), probe and coding sequence.
SEQ ID NOs:105/106 gi|41393588|ref|NM_024769.2| Homo sapiens adipocyte-specific adhesion molecule (ASAM), probe and coding sequence.
SEQ ID NOs:107/108 gi|156523233|refl|NM_001721.6| Homo sapiens BMX non-receptor tyrosine kinase (BMX), transcript variant 2, probe and coding sequence.
SEQ ID NOs:109/110 gi|157168354|ref|NM_001771.21 Homo sapiens CD22 molecule (CD22), probe and coding sequence.
SEQ ID NOs:111/112 gi|91807109|ref|NM_001040031.1| Homo sapiens CD37 molecule (CD37), transcript variant 2, probe and coding sequence.
SEQ ID NOs:113/114 gi|166706902|ref|NM_002053.2| Homo sapiens guanylate binding protein 1, interferon-inducible, 67kDa (GBP1), probe and coding sequence.
SEQ ID NOs:115/116 gi|53759142|ref|NM_002061.2| Homo sapiens glutamate-cysteine ligase, modifier subunit (GCLM), probe and coding sequence.
SEQ ID NOs:117/118 gi|61888835|ref|NM_003121.2| Homo sapiens Spi-B transcription factor (Spi-1/PU.1 related) (SPIB), probe and coding sequence.
SEQ ID NOs:119/120 gi|45243546|ref|NM_203488.1| Homo sapiens acylphosphatase 1, erythrocyte (common) type (ACYP1), transcript variant 2, probe and coding sequence.
SEQ ID NOs:121/122 gi|62912451|ref|NM_016824.3| Homo sapiens adducin 3 (gamma) (ADD3), transcript variant 1, probe and coding sequence.
SEQ ID NOs:123/124 gi|47157326|ref|NM_005400.2| Homo sapiens protein kinase C, epsilon (PRKCE), probe and coding sequence.
SEQ ID NOs:125/126 gi|5453622|ref|NM_006314.| Homo sapiens connector enhancer of kinase suppressor of Ras 1 (CNKSR1), probe and coding sequence.
SEQ ID NOs:127/128 gi|156713456|ref|NM_006542.3| Homo sapiens S-phase response (cyclin-related) (SPHAR), probe and coding sequence.
SEQ ID NOs:129/130 gi|112293284|ref|NM_006717.2| Homo sapiens spindlin 1 (SPIN1), probe and coding sequence.
SEQ ID NOs:131/132 gi|166706908|ref|NM_006820.2| Homo sapiens interferon-induced protein 44-like (IFI44L), probe and coding sequence.
SEQ ID NOs:133/134 gi|150378462|ref|NM_001099412.1| Homo sapiens MYST histone acetyltransferase (monocytic leukemia) 3 (MYST3), transcript variant 1, probe and coding sequence.
SEQ ID NOs:135/136 gi|62865863|ref|NM_000519.3| Homo sapiens hemoglobin, delta (HBD), probe and coding sequence.
SEQ ID NOs:137/138 gi|62865863|ref|NM_000519.3| Homo sapiens hemoglobin, delta (HBD), probe and coding sequence.
SEQ ID NOs:139/140 gi|154426252|ref|NM_001100422.1| Homo sapiens viral DNA polymerase-transactivated protein 6 (LOC26010), transcript variant 2, probe and coding sequence.
SEQ ID NOs:141/142 gi|50593017|refNM_016371.21 Homo sapiens hydroxysteroid (17-beta) dehydrogenase 7 (HSD17B7), probe and coding sequence.
SEQ ID NOs:143/144 gi|75750528|ref|NM_016552.2| Homo sapiens ankyrin repeat and MYND domain containing 1 (ANKMY1), transcript variant 1, probe and coding sequence.
SEQ ID NOs:145/146 gi|51593093|ref|NM_017946.2| Homo sapiens FK506 binding protein 14, 22 kDa (FKBP14), probe and coding sequence.
SEQ ID NOs:147/148 gi|169160852|ref|XR_037068.1| PREDICTED: Homo sapiens misc_RNA (LOC646970), probe and coding sequence.

## Claims

1. A method for the prognosis or early detection of lung cancer which comprises
(a) determining the expression rates of at least 25 of the indicator RNAs represented by the nucleic acid sequences of SEQ ID NOs:2n (wherein n is an integer from 1 to 74) in a bodily fluid of the individual to be tested, and
(b) comparing the obtained expression rates with standard values of the respective expression rates, wherein a deviation of the expression rate of at least 30% of the indicator RNAs is indicative for lung cancer or the prognosis of lung cancer.

2. The method of claim 1, wherein
(i) the method is suitable for early diagnosis of lung cancer; and/or
(ii) the method comprises determining the expression rates of at least 35, preferably at least 50, most preferably at least 70 of the indicator RNAs; and/or
(iii) the method comprises determining the expression rate of at least the following indicator RNASs: SEQ ID NO: 25, 131, 17, 113, 13, 133, 43, 79, 53, 91, 103, 99, 1, 145, 5, 23, 101, 69, 73 and 141; and/or
(iv) a deviation of the expression rate of at least 50%, preferably of at least 70% of the indicator RNAs is indicative for lung cancer or prognosis of lung cancer.

3. The method of claim 1 or 2, wherein
(i) the expression rates of the indicator RNAs are decreased or increased as shown in Table 1 and a decrease or increase of at least 10% relative to the respective standard value is considered a significant event; and/or
(ii) 30%, preferably 50% of the indicator RNAs, which are indicative with a decreased expression rate as shown in Table 1, show a decreased expression rate, and/or 30%, preferably 50% of the indicator RNAs, which are indicative with an increased expression rate as shown in Table 1, show an increased expression rate; and/or
(iii) the standard values of the expression rates are obtainable from a set of healthy patients or a standard mixture of the indicator RNAs.

4. The method of any of claims 1 to 3, wherein the bodily fluid is selected from blood, urine, sputum, saliva etc., preferably the bodily fluid is blood.

5. The method of any of claims 1 to 4, wherein the determination of the expression rate is effected by an RNA hybridization assays, preferably by a solid phase hybridization (micro)array.

6. The method of claim 5, wherein the determination is effected by a hybridization with probes specific for the nucleic acid sequences of SEQ ID NOs:2n (wherein n is an integer from 1 to 74).

7. The method of claim 6, wherein said probes
(i) comprise 15 to 150, preferably 30 to 70 consecutive nucleotides of the nucleic acid sequences of SEQ ID NOs:2n (wherein n is an integer from 1 to 74); or
(ii) the probes comprise the nucleic acid sequences of SEQ ID NOs:2n-1 (wherein n is an integer from 1 to 74), preferably the probes are consisting of the nucleic acid sequences of SEQ ID NOs:2n-1 (wherein n is an integer from 1 to 74).

8. A hybridization (micro)array for the prognosis or detection of lung cancer carrying probes for at least 25 indicator RNAs as defined in claim 6 or 7.

9. A kit for the prognosis or detection of lung cancer according to the method of claims 1 to 7, which comprises means for the determination of the expression rate of the indicator proteins as defined in claims 1 to 7.

10. The kit of claim 7, which comprises probes as defined in claim 6 or 7 or a (micro)array as defined in claim 8.

11. The kit of claim 9 or 10, which further comprises standard mixture of the indicator RNAs.


SEQUENCE LISTING

<110>  Universitaet zu Koeln
       DKFZ Deutsches Krebsforschungszentrum, Stiftung des Oeffentlichen Rechts

<120>  A Method for Lung Cancer Early Detection and Prognosis

<130>  072888ep

<160>  148

<170>  PatentIn version 3.3

<210>  1
<211>  50
<212>  DNA
<213>  homo sapiens

<400>  1
gccaggaaca aaaatcagga gtgtggtagt ggattagtga aagtctcctc          50


<210>  2
<211>  2077
<212>  DNA
<213>  homo sapiens

<400>  2
ccgagcgcca gcgcggggaa ccgggaaaag gaaaccgtgt tgtgtacgta agattcagga    60

aacgaaacca ggagccgcgg gtgttggcgc aaaggttact cccagaccct tttccggctg   120

acttctgaga aggttgcgca cagctgtgcc cggcagtcta gaggcgcaga agaggaagcc   180

atcgcctggc cccggctctc tggaccttgt ctcgctcggg agcggaaaca gcggcagcca   240

gagaactgtt ttaatcatgg acaaacaaaa ctcacagatg aatgcttctc acccggaaac   300

aaacttgcca gttgggtatc ctcctcagta tccaccgaca gcattccaag gacctccagg   360

atatagtggc taccctgggc cccaggtcag ctacccaccc ccaccagccg gccattcagg   420

tcctggccca gctggctttc ctgtcccaaa tcagccagtg tataatcagc cagtatataa   480

tcagccagtt ggagctgcag gggtaccatg gatgccagcg ccacagcctc cattaaactg   540

tccacctgga ttagaatatt taagtcagat agatcagata ctgattcatc agcaaattga   600

acttctggaa gttttaacag gttttgaaac taataacaaa tatgaaatta agaacagctt   660

tggacagagg gtttactttg cagcggaaga tactgattgc tgtacccgaa attgctgtgg   720

gccatctaga ccttttacct tgaggattat tgataatatg ggtcaagaag tcataactct   780

ggagagacca ctaagatgta gcagctgttg ttgtccctgc tgccttcagg agatagaaat   840

ccaagctcct cctggtgtac caataggtta tgttattcag acttggcacc catgtctacc   900

aaagtttaca attcaaaatg agaaaagaga ggatgtacta aaaataagtg gtccatgtgt   960

tgtgtgcagc tgttgtggag atgttgattt tgagattaaa tctcttgatg aacagtgtgt  1020

ggttggcaaa atttccaagc actggactgg aattttgaga gaggcattta cagacgctga  1080

taactttgga atccagttcc ctttagacct tgatgttaaa atgaaagctg taatgattgg 1140

tgcctgtttc ctcattgact tcatgttttt tgaaagcact ggcagccagg aacaaaaatc 1200

aggagtgtgg tagtggatta gtgaaagtct cctcaggaaa tctgaagtct gtatattgat 1260

tgagactatc taaactcata cctgtatgaa ttaagctgta aggcctgtag ctctggttgt 1320

atacttttgc ttttcaaatt atagtttatc ttctgtataa ctgatttata aaggtttttg 1380

tacatttttt aatactcatt gtcaatttga gaaaaaggac atatgagttt ttgcatttat 1440

taatgaaact tcctttgaaa aactgctttg aattatgatc tctgattcat tgtccatttt 1500

actaccaaat attaactaag gccttattaa tttttatata aattatatct tgtcctatta 1560

aatctagtta caatttattt catgcataag agctaatgtt attttgcaaa tgccatatat 1620

tcaaaaaagc tcaaagataa ttttctttac tattatgttc aaataatatt caatatgcat 1680

attatcttta aaaagttaaa tgttttttta atcttcaaga aatcatgcta cacttaactt 1740

ctcctagaag ctaatctata ccataatatt ttcatattca caagatatta aattaccaat 1800

tttcaaatta ttgttagtaa agaacaaaat gattctctcc caaagaaaga cacattttaa 1860

atactccttc actctaaaac tctggtatta taacttttga aagttaatat ttctacatga 1920

aatgtttagc tcttacactc tatccttcct agaaaatggt aattgagatt actcagatat 1980

taattaaata caatatcata tatatattca cagagtataa acctaaataa tgatctatta 2040

gattcaaata tttgaaataa aaacttgatt tttttgt 2077

<210> 3
<211> 50
<212> DNA
<213> homo sapiens

<400> 3
ggccagggtg agagagcttg aaagtgaggt ggaaagtgaa cagaagcaca 50

<210> 4
<211> 6016
<212> DNA
<213> homo sapiens

<400> 4
atccttcctc aaaattcttg aagtagttgt ctgctttgag cctgccacct tcttcatctg 60

ataatacaag aggtatacct agtccagtac tgccatcaat aacctgcagc catgagttct 120

gactctgaga tggccatttt tggggaggct gctcctttcc tccgaaagtc tgaaaaggag 180

cgaattgaag ctcagaacaa gccttttgat gccaagacat cagtctttgt ggtggaccct 240

aaggagtcct acgtgaaagc aatagtgcag agcagggaag gggggaaggt gacagccaag 300

accgaagctg gagctactgt aactgtgaaa gaagaccaag tcttctccat gaaccctccc 360

aaatatgaca agatcgagga catggccatg atgactcacc tgcatgagcc tgctgtgctg 420
tataacctca aagagcgtta cgcagcctgg atgatctaca cctactcggg cctcttctgt 480
gtcaccgtca acccctacaa gtggctgccg gtgtacaacc ctgaggtggt gacagcctac 540
cgaggcaaaa agcgccagga ggccccaccc catatcttct ccatctctga caatgcctat 600
cagttcatgc taactgatcg tgaaaaccag tcaatcttga ttactggaga atctggtgca 660
gggaagactg tgaacacgaa gcgtgtcatc cagtactttg caacaattgc agttactgga 720
gagaagaaaa aagaggaacc tgcctctggc aaaatgcagg ggacccttga agatcaaatc 780
atcagtgcta accccctact ggaagccttc ggcaatgcca agaccgtgag gaatgacaac 840
tcctctcgct ttggtaaatt catcaggatc cattttggtg ccacaggcaa actggcttct 900
gcagatattg aaacatatct gctagagaag tcccgagtta cttttcagct aaaggctgaa 960
agaagctacc acatatttta tcaaatcctg tccaataaga aaccagagct cattgaaatg 1020
cttctgatca ccaccaaccc atatgacttc gcatttgtca gccaagggga aattactgtg 1080
cccagcattg atgaccagga agagctgatg gccacagata gtgctgtgga catcctgggt 1140
ttcactgctg atgaaaaggt ggccatttac aagctcactg gagccgtgat gcattatggg 1200
aacatgaaat tcaagcaaaa gcaaagggaa gagcaggcag agccagatgg cacggaagtt 1260
gctgacaaag ctgcttatct gacaagtctg aactctgctg acctgctcaa atctctctgc 1320
tatcccagag tcaaggtcgg caatgagttc gtaaccaaag gccagactgt gcagcaggtg 1380
tacaatgcag tgggtgctct ggccaaagcc atctacgaga agatgttcct gtggatggtc 1440
acccgcatca accagcagct ggacaccaag cagcccaggc agtacttcat cggggtcttg 1500
gacattgctg gctttgagat ctttgatttc aacagcctgg agcagctgtg catcaacttc 1560
accaacgaga aactgcaaca gtttttcaac caccacatgt tcgtgctgga gcaggaagag 1620
tacaagaagg aaggcatcga gtgggagttc attgacttcg ggatggacct ggctgcctgc 1680
atcgagctca tcgagaagcc tatgggcatc ttctccatcc tagaagagga gtgcatgttc 1740
cccaaggcaa cagacacctc cttcaagaac aagctgtatg aacaacatct tggaaaatcc 1800
aacaacttcc agaagcccaa gcctgccaaa ggcaagcctg aggctcactt ctcactggtg 1860
cactatgccg gcaccgtgga ctacaacatc gccggctggc tggacaaaaa caaggacccc 1920
ctgaatgaga ctgtggtggg gctgtaccag aagtctgcaa tgaagactct ggctttcctc 1980
ttctctgggg cacaaactgc tgaagcagag ggtggtggtg gaaagaaagg tggcaaaaag 2040
aagggttctt ctttccagac agtgtcagct cttttcaggg agaatttgaa taagctgatg 2100
accaacttga ggagcactca cccccacttt gtgcggtgca tcatccccaa tgaaactaaa 2160
actcctggtg ccatggagca tgagcttgtc ctgcatcagc tgaggtgtaa cggtgtgctg 2220
gaaggcatcc gcatctgcag gaaaggcttc ccaagcagaa tcctttatgc agacttcaaa 2280

cagagataca aggttctaaa tgcgagtgct atcccagagg gtcagttcat tgacagcaag 2340

aaggcttctg agaaacttct agggtctatt gaaattgacc acacccagta caaattcggt 2400

cataccaagg ttttcttcaa agctggcctg ctgggaactc tagaggaaat gcgagatgaa 2460

aagctagctc aactcatcac gcgcactcaa gccatatgca gagggttcct gatgagagtg 2520

gagttcagaa agatgatgga gaggagagag tccatcttct gcattcagta caacatccgt 2580

gctttcatga atgtgaagca ctggccctgg atgaagctgt atttcaagat caagcccctc 2640

ctcaagagtg cagagacaga gaaggagatg gccaacatga aggaagaatt tgagaaaacc 2700

aaagaagagc tggctaagac agaggcaaaa aggaaagaac tagaagaaaa gatggtgacg 2760

ctaatgcaag agaaaaatga cttacaactc caagttcaag ctgaagcaga tgccttggct 2820

gatgcagagg aaagatgtga tcagttgatt aaaaccaaaa tccaacttga ggccaaaatc 2880

aaagaggtaa ctgaaagagc tgaggatgag gaagagatca atgctgagct gacagccaag 2940

aagaggaaac tggaggatga atgttcagag ctcaagaaag acattgatga ccttgagctg 3000

acactggcca aggttgagaa ggagaaacat gccacagaga acaaggtgaa aaacctcaca 3060

gaagagatgg caggtctgga tgaaaccatt gctaagctga ccaaggagaa gaaggctctc 3120

caggaggccc accagcagac cctggatgac ctgcagatgg aggaggacaa agtcaacacc 3180

ctgaccaaag ctaaaaccaa gctagaacag caagtggacg atcttgaagg atctctggaa 3240

caagaaaaga aactttgcat ggacttagaa agagccaaga gaaaactgga gggtgaccta 3300

aaattggccc aagaatccac aatggataca gaaaatgaca aacagcaact taatgagaaa 3360

ctcaaaaaga aagagtttga aatgagcaat ctgcaaggca agattgaaga tgaacaagcc 3420

cttgcaatac agctacaaaa gaagatcaaa gaattacagg cccgcattga ggagctggag 3480

gaggaaatcg aggcagagcg ggcctcccgg gccaaagcag agaagcagcg ctctgacctc 3540

tcccgggagc tggaggagat cagtgagagg ctggaagaag ccggtggggc cacttcagcc 3600

cagattgaga tgaacaagaa gcgggaggct gagttccaga aaatgcgcag ggacctggaa 3660

gagtccaccc tgcagcacga agccacggca gctgctcttc ggaagaagca cgcagatagt 3720

gtggctgagc ttggggagca gattgacagc cttcagcggg tcaagcagaa gctggagaag 3780

gaaaagagtg agctgaagat ggagatcaat gaccttgcta gtaacatgga gactgtctcc 3840

aaagccaagg caaactttga gaaaatgtgc cgcaccctag aggaccagct tagtgaaata 3900

aaaacaaagg aagaagagca acaacgctta ataaatgagt tgtcagccca gaaggcacgt 3960

ttacacacag aatcaggtga gttttcacga cagctagatg aaaaagatgc tatggtttct 4020

cagctatccc gaggcaaaca agcatttaca caacagattg aagaattaaa gaggcagcta 4080

gaagaggaga ctaaggccaa gagcactctg gcccatgccc tgcagtcagc ccgccatgac 4140

tgtgacctgc tgcgggaaca gtatgaggag gagcaggaag ccaaggctga gctgcagagg 4200

ggaatgtcca aggccaacag tgaggttgcc cagtggagga ccaagtacga gacggacgcc 4260

atccagcgca cagaggagct ggaggaggcc aagaagaagc tagcccagcg tctgcaggat 4320

gcagaagaac atgtagaagc tgtgaattcc aaatgtgctt ctcttgaaaa gacaaagcag 4380

aggctacaga atgaagtaga ggacctcatg attgatgtgg aacgatctaa tgctgcctgc 4440

atagctctcg ataagaagca aagaaacttt gacaaggttc tggcagaatg gaaacagaag 4500

tatgaggaaa ctcaggctga acttgaggcc tcccagaagg agtcgcgttc tctcagcact 4560

gagctgttca aggtgaagaa tgcctacgag gaatccctgg atcatcttga aactctaaag 4620

cgagagaata agaacttaca acaggagatt tctgacctga cagagcaaat tgcagagggt 4680

ggaaagcata tccatgaact ggagaaagta aagaaacaac ttgatcatga gaagagtgaa 4740

ctacagactt ccctagagga agcagaggca tctcttgagc atgaagaagg caaaattctt 4800

cgcattcaac ttgagctaaa tcaggtgaaa tctgagattg accgaaaaat tgctgaaaaa 4860

gatgaagaac tcgatcagct aaagaggaac catctcagag ttgtggagtc aatgcagagt 4920

acactggatg ctgagatcag gagcagaaat gatgctctga ggatcaagaa gaagatggag 4980

ggagatctta atgaaatgga aatccagctg aaccatgcca accgccaggc tgctgaggca 5040

ctaaggaatc ttagaaacac acaaggaata ctgaaggaca ctcagctaca tttggatgat 5100

gccatcagag gccaagatga ccttaaggaa caactggcaa tggttgagcg cagagctaac 5160

ctgatgcagg ctgaagttga agagctcagg gcatccctgg aacggactga gagaggcagg 5220

aaaatggcag agcaagagct tctggatgcc agtgaacgtg tgcaacttct gcacactcag 5280

aacaccagcc tgatcaacac caagaagaag ctggaaacag acatttccca aatccaggga 5340

gagatggagg acatcgtcca ggaagcccgc aatgcagagg agaaggccaa gaaggccatc 5400

actgatgctg ccatgatggc tgaggagctg aagaaggaac aggacaccag cgcccacctg 5460

gagcggatga agaagaacat ggagcagacc gtgaaggatc tgcagctccg tctggatgag 5520

gctgagcagc tggcgctgaa gggtgggaag aagcagatcc agaaactgga ggccagggtg 5580

agagagcttg aaagtgaggt ggaaagtgaa cagaagcaca atgttgaggc tgtcaagggt 5640

cttcgcaaac atgagagaag agtgaaggaa ctcacttacc agactgagga ggaccgcaag 5700

aatattctca ggctgcagga cttggtggac aaattgcaaa ccaaagtcaa agcttacaag 5760

agacaagctg aagaggctga ggaacaatcc aatgtcaacc ttgccaagtt ccgcaagctc 5820

cagcacgagc tggaggaggc caaggaacgg gctgacattg ctgagtccca agtcaacaag 5880

ctgagagtga agagtcggga ggttcacaca aaagtcataa gtgaagagta attcattcta 5940

atgaaagaaa atgtgaccaa agaaatgcac gaaatgtgaa gttctttgtc actgtcctgt 6000

atatcaagga aataaa 6016

<210>  5
<211>  50
<212>  DNA
<213>  homo sapiens

<400>  5
gggagctgat tgtagcagcc tcgttagtgt caccccctcc tccctgatct          50


<210>  6
<211>  1258
<212>  DNA
<213>  homo sapiens

<400>  6
   tccactcaca gcctgaagca tacccggcag gggctgtccc caggcccaac aagcaaaggg     60
   cccagtagcg agggccactg gagcccatct ccggggggct gggcaggaag tagggtgggg    120
   tttggggtag ggatctggta ccctgggact gctgcaactc aaactaacca acccactggg    180
   agaagatgcc tgggggtcca ggagtcctcc aagctctgcc tgccaccatc ttcctcctct    240
   tcctgctgtc tgctgtctac ctgggccctg ggtgccaggc cctgtggatg cacaaggtcc    300
   cagcatcatt gatggtgagc ctgggggaag acgcccactt ccaatgcccg cacaatagca    360
   gcaacaacgc caacgtcacc tggtggcgcg tcctccatgg caactacacg tggccccctg    420
   agttcttggg cccgggcgag gaccccaatg gtacgctgat catccagaat gtgaacaaga    480
   gccatggggg catatacgtg tgccgggtcc aggagggcaa cgagtcatac cagcagtcct    540
   gcggcaccta cctccgcgtg cgccagccgc cccccaggcc cttcctggac atgggggagg    600
   gcaccaagaa ccgaatcatc acagccgagg ggatcatcct cctgttctgc gcggtggtgc    660
   ctgggacgct gctgctgttc aggaaacgat ggcagaacga gaagctcggg ttggatgccg    720
   gggatgaata tgaagatgaa aacctttatg aaggcctgaa cctggacgac tgctccatgt    780
   atgaggacat ctcccggggc ctccagggca cctaccagga tgtgggcagc ctcaacatag    840
   gagatgtcca gctggagaag ccgtgacacc cctactcctg ccaggctgcc cccgcctgct    900
   gtgcacccag ctccagtgtc tcagctcact tccctgggac attctccttt cagcccttct    960
   gggggcttcc ttagtcatat tcccccagtg gggggtggga gggtaacctc actcttctcc   1020
   aggccaggcc tccttggact cccctggggg tgtcccactc ttcttccctc taaactgccc   1080
   cacctcctaa cctaatcccc ccgccccgct gcctttccca ggctcccctc accccagcgg   1140
   gtaatgagcc cttaatcgct gcctctaggg gagctgattg tagcagcctc gttagtgtca   1200
   ccccctcctc cctgatctgt cagggccact tagtgataat aaattcttcc caactgca      1258


<210>  7
<211>  50
<212>  DNA

<213>  homo sapiens

<400>  7
gagtcccaga acgaatctct cgccaccctc accatccaag gcatccggtt          50


<210>  8
<211>  1300
<212>  DNA
<213>  homo sapiens

<400>  8
ctgcagccgg tgcagttaca cgttttcctc caaggagcct cggacgttgt cacgggtttg      60

gggtcgggga cagagcggtg accatggcca ggctggcgtt gtctcctgtg cccagccact     120

ggatggtggc gttgctgctg ctgctctcag ctgagccagt accagcagcc agatcggagg     180

accggtaccg gaatcccaaa ggtagtgctt gttcgcggat ctggcagagc ccacgtttca     240

tagccaggaa acggggcttc acggtgaaaa tgcactgcta catgaacagc gcctccggca     300

atgtgagctg gctctggaag caggagatgg acgagaatcc ccagcagctg aagctggaaa     360

agggccgcat ggaagagtcc cagaacgaat ctctcgccac cctcaccatc caaggcatcc     420

ggtttgagga caatggcatc tacttctgtc agcagaagtg caacaacacc tcggaggtct     480

accagggctg cggcacagag ctgcgagtca tgggattcag caccttggca cagctgaagc     540

agaggaacac gctgaaggat ggtatcatca tgatccagac gctgctgatc atcctcttca     600

tcatcgtgcc tatcttcctg ctgctggaca aggatgacag caaggctggc atggaggaag     660

atcacaccta cgagggcctg gacattgacc agacagccac ctatgaggac atagtgacgc     720

tgcggacagg ggaagtgaag tggtctgtag gtgagcaccc aggccaggag tgagagccag     780

gtcgccccat gacctgggtg caggctccct ggcctcagtg actgcttcgg agctgcctgg     840

ctcatggccc aacccctttc ctggaccccc cagctggcct ctgaagctgg cccaccagag     900

ctgccatttg tctccagccc ctggtcccca gctcttgcca aagggcctgg agtagaagga     960

caacagggca gcaacttgga gggagttctc tggggatgga cgggacccag ccttctgggg    1020

gtgctatgag gtgatccgtc cccacacatg ggatggggga ggcagagact ggtccagagc    1080

ccgcaaatgg actcggagcc gagggcctcc cagcagagct tgggaagggc catggaccca    1140

actgggcccc agaagagcca caggaacatc attcctctcc cgcaaccact cccaccccag    1200

ggaggccctg gcctccagtg ccttcccccg tggaataaac ggtgtgtcct gagaaaccac    1260

aaaaaaaaaa aaaaaaaaaa aaaaaaaaaa aaaaaaaaaa                          1300


<210>  9
<211>  50
<212>  DNA
<213>  homo sapiens

<400>  9

cttgccaaag ggcctggagt agaaggacaa cagggcagca acttggaggg 50

<210> 10
<211> 1300
<212> DNA
<213> homo sapiens

<400> 10
ctgcagccgg tgcagttaca cgttttcctc caaggagcct cggacgttgt cacgggtttg 60

gggtcgggga cagagcggtg accatggcca ggctggcgtt gtctcctgtg cccagccact 120

ggatggtggc gttgctgctg ctgctctcag ctgagccagt accagcagcc agatcggagg 180

accggtaccg gaatcccaaa ggtagtgctt gttcgcggat ctggcagagc ccacgtttca 240

tagccaggaa acggggcttc acggtgaaaa tgcactgcta catgaacagc gcctccggca 300

atgtgagctg gctctggaag caggagatgg acgagaatcc ccagcagctg aagctggaaa 360

agggccgcat ggaagagtcc cagaacgaat ctctcgccac cctcaccatc caaggcatcc 420

ggtttgagga caatggcatc tacttctgtc agcagaagtg caacaacacc tcggaggtct 480

accagggctg cggcacagag ctgcgagtca tgggattcag caccttggca cagctgaagc 540

agaggaacac gctgaaggat ggtatcatca tgatccagac gctgctgatc atcctcttca 600

tcatcgtgcc tatcttcctg ctgctggaca aggatgacag caaggctggc atggaggaag 660

atcacaccta cgagggcctg gacattgacc agacagccac ctatgaggac atagtgacgc 720

tgcggacagg ggaagtgaag tggtctgtag gtgagcaccc aggccaggag tgagagccag 780

gtcgccccat gacctgggtg caggctccct ggcctcagtg actgcttcgg agctgcctgg 840

ctcatggccc aacccctttc ctggaccccc cagctggcct ctgaagctgg cccaccagag 900

ctgccatttg tctccagccc ctggtcccca gctcttgcca aagggcctgg agtagaagga 960

caacagggca gcaacttgga gggagttctc tggggatgga cgggacccag ccttctgggg 1020

gtgctatgag gtgatccgtc cccacacatg ggatggggga ggcagagact ggtccagagc 1080

ccgcaaatgg actcggagcc gagggcctcc cagcagagct tgggaagggc catggaccca 1140

actgggcccc agaagagcca caggaacatc attcctctcc cgcaaccact cccaccccag 1200

ggaggccctg gcctccagtg ccttcccccg tggaataaac ggtgtgtcct gagaaaccac 1260

aaaaaaaaaa aaaaaaaaaa aaaaaaaaaa aaaaaaaaaa 1300

<210> 11
<211> 50
<212> DNA
<213> homo sapiens

<400> 11
aaaaactttt atgctggctg ggcgcggtgg cttacgcctg taatcccagc 50

<210>  12
<211>  2200
<212>  DNA
<213>  homo sapiens

<400>  12
agcgcagggc gaaaggaggc tgtgtcctca gcctcctccc tcggcacgcg cctcccatcc      60

tgacggggac tgagagccca ggagagctcg gggcgtgtcc aaactgccgc gccgccctgc     120

tccgggtttg tcttcctccc tccgtttctc cttccgctgt atctagcatt tcggttcctg     180

gaaaggttac cggagctgcg attggggctg ggaccaaagt tgagtcctgg aaagggagcc     240

tggagagcgg cggtgctggg aggcccggcc agctcgatcg caggcttcca cctggcggcc     300

aatgcttgtt tgctactgat gggtgtgagc aagttagata ttctataccg gagacttctc     360

ctaacaaaac tttttatcag aggatgggga aggccagaag atctcaaaag actctttgaa     420

ttcagaaaga tgattggaaa tcgggaaaga tgccagaatc tggtttcaag cgattatcca     480

gtacacattg ataagattga agagcaatca gattgtaaga tcttagatgg acactttgtt     540

tcccccatgg ctcactatgt gcctgatatc atgccaattg aatctgttat tgcaaggttc     600

caatttattg tgcctaaaga atggaacagc aaatatagac ctgtatgcat tcatcttgct     660

ggaacaggag atcatcatta ctggaggcga cgaacactaa tggcccgtcc tatgattaaa     720

gaagcccgaa tggcttcttt attgttagaa aacccttatt atggctgcag gaaacccaag     780

gaccaagtaa ggtccagctt aaaaaatgtg tccgaccttt ttgtgatggg aggagctctt     840

gttttagaat ctgcagctct cttgcactgg ctagagaggg aaggttacgg ccctttagga     900

atgactggaa tatccatggg aggacacatg gcttccttag cggtatccaa ctggcctaag     960

cccatgccat tgattccatg cctgtcttgg tccacagcat ctggggtctt cactacgaca    1020

gattctttca aaatgggaca agagtttgtg aaacacttca ctagcagtgc agacaagcta    1080

actaacctta atctggtttc cagaacttta aatttagata tatcaaacca agttgtatcc    1140

caaaaacctg ctgactgcca taattctagc aaaacatctg tcagtgcgac atcagaagga    1200

ctcttattgc aagatacctc taagatgaag cgcttcaatc aaacactttc aaccaacaaa    1260

agtggttata caagtcgcaa ccctcagtca taccacctac ttagtaaaga acaaagcaga    1320

aacagtcttc ggaaagagtc tttaatattt atgaaaggag tcatggatga atgtactcat    1380

gtagcaaatt tctcagtccc agttgatcca agcctcatta tagtggttca agccaaagaa    1440

gatgcctata ttccacgaac aggagttcga agtttacaag aaatttggcc tggttgtgaa    1500

atccgatact tagaaggggg tcatattagt gcttatcttt ttaaacaagg actcttcagg    1560

taagacggct ggtctaaaca tgtattcgtt catttgttgt ttgttttctg gattttttgc    1620

ttcaacaaat gtaaaagatc acatattttt atagtctgtt tcaatctttg aattccctcc    1680

tcacctccca caaatctcac agagttactt ttccctcttt cctagaattt gatcttctta    1740

ttttcttcta gagaactaag catgatgcat attataggca tgctttatcg tgtggctttc 1800

ccctcttcaa atattaatgg ccaggtattt taggatgtga gaaggaagat catggcttgt 1860

taaagttgct ttggtgcatc aagagttttt tcactctatt ctgatgtttt ttcagaatgg 1920

cctgaacatc tgtggaataa tttagcaatg tttttcagtt agcaagtgaa tattgaacct 1980

taaataagaa gtgcttttaa tgtgaacaac cttatatgac tccactattt tagttagaac 2040

ttataaaaac ttttatgctg gctgggcgcg gtggcttacg cctgtaatcc cagcactttg 2100

ggaggcggag gcgggtgtat cacgaggtca ggagatcaag gccatcctgg ctaacacggt 2160

gaaaccccgt ctctagtaaa aatacaaaaa aaaaaaaaaa 2200

<210> 13
<211> 50
<212> DNA
<213> homo sapiens

<400> 13
cccatgggtt atctgtatgg actgactggg ttattgaaag gactagccac 50

<210> 14
<211> 3243
<212> DNA
<213> homo sapiens

<400> 14
gggcttcgtg ttcctgggtg ctgaccgtgc actccccgcc gcccgaggac ttagagctct 60

ggaagtagct ctccagcttc cttcgtactc gggggccgga cttgtacacc cgcacgagga 120

gcggggacgg cgggcgcaga agtgggccac catatctgga aactacagtc tatgctttga 180

agcgcaaaag ggaataaaca tttaaagact cccccgggga cctggaggat ggacttttcc 240

atggtggccg gagcagcagc ttacaatgaa aaatcaggta ggattacctc gctctcactc 300

ttgtttcaga aagtctttgc tcagatcttt cctcagtgga gaaaggggaa tacagaagaa 360

tgtctcccct acaagtgctc agagactggt gctcttggag aaaactatag ttggcaaatt 420

cccattaacc acaatgactt caaaatttta aaaaataatg agcgtcagct gtgtgaagtc 480

ctccagaata agtttggctg tatctctacc ctggtctctc cagttcagga aggcaacagc 540

aaatctctgc aagtgttcag aaaaatgctg actcctagga tagagttatc agtctggaaa 600

gatgacctca ccacacatgc tgttgatgct gtggtgaatg cagccaatga agatcttctg 660

catgggggag gcctggccct ggccctggta aaagctggtg gatttgaaat ccaagaagag 720

agcaaacagt ttgttgccag atatggtaaa gtgtcagctg gtgagatagc tgtcacggga 780

gcagggaggc ttccctgcaa acagatcatc catgctgttg ggcctcggtg gatggaatgg 840

gataaacagg gatgtactgg aaagctgcag agggccattg taagtattct gaattatgtc 900

atctataaaa atactcacat taagacagta gcaattccag ccttgagctc tgggattttt 960

cagttccctc tgaatttgtg tacaaagact attgtagaga ctatccgggt tagtttgcaa 1020

gggaagccaa tgatgagtaa tttgaaagaa attcacctgg tgagcaatga ggaccctact 1080

gttgctgcct ttaaagctgc ttcagaattc atcctaggga agagtgagct gggacaagaa 1140

accacccctt ctttcaatgc aatggtcgtg aacaacctga ccctccagat tgtccagggc 1200

cacattgaat ggcagacggc agatgtaatt gttaattctg taaacccaca tgatattaca 1260

gttggacctg tggcaaagtc aattctacaa caagcaggag ttgaaatgaa atcggaattt 1320

cttgccacaa aggctaaaca gtttcaacgg tcccagttgg tactggtcac aaaaggattt 1380

aacttgttct gtaaatatat ataccatgta ctgtggcatt cagaatttcc taaacctcag 1440

atattaaaac atgcaatgaa ggagtgtttg gaaaaatgca ttgagcaaaa tataacttcc 1500

atttcctttc ctgcccttgg gactggaaac atggaaataa agaaggaaac agcagcagag 1560

attttgtttg atgaagtttt aacatttgcc aaagaccatg taaaacacca gttaactgta 1620

aaatttgtga tctttccaac agatttggag atatataagg ctttcagttc tgaaatggca 1680

aagaggtcca agatgctgag tttgaacaat tacagtgtcc cccagtcaac cagagaggag 1740

aaaagagaaa atgggcttga agctagatct cctgccatca atctgatggg attcaacgtg 1800

gaagagatgt gtgaggccca cgcatggatc caaagaatcc tgagtctcca gaaccaccac 1860

atcattgaga ataatcatat tctgtacctt gggagaaagg aacatgacat tttgtctcag 1920

cttcagaaaa cttcaagtgt ctccatcaca gaaattatca gcccaggaag gacagagtta 1980

gagattgaag gagcccgggc tgacctcatt gaggtggtta tgaacattga agatatgctt 2040

tgtaaagtac aggaggaaat ggcaaggaaa aaggagcgag gcctttggcg ctcgttagga 2100

cagtggacta ttcagcaaca aaaaacccaa gacgaaatga aagaaaatat catatttctg 2160

aaatgtcctg tgcctccaac tcaagagctt ctagatcaaa agaaacagtt tgaaaaatgt 2220

ggtttgcagg ttctaaaggt ggagaagata gacaatgagg tccttatggc tgcctttcaa 2280

agaaagaaga aaatgatgga agaaaaactg cacaggcaac ctgtgagcca taggctgttt 2340

cagcaagtcc cataccagtt ctgcaatgtg gtatgcagag ttggctttca aagaatgtac 2400

tcgacacctt gcgatccaaa atacggagct ggcatatact tcaccaagaa cctcaaaaac 2460

ctggcagaga aggccaagaa aatctctgct gcagataagc tgatctatgt gtttgaggct 2520

gaagtactca caggcttctt ctgccaggga catccgttaa atattgttcc cccaccactg 2580

agtcctggag ctatagatgg tcatgacagt gtggttgaca atgtctccag ccctgaaacc 2640

tttgttattt ttagtggcat gcaggctata cctcagtatt tgtggacatg cacccaggaa 2700

tatgtacagt cacaagatta ctcatcagga ccaatgagac cctttgcaca gcatccttgg 2760

aggggattcg caagtggcag ccctgttgat taatctctac atcattttaa cagctggtat 2820

ggccttacct tgggtgaact aaccaaataa tgaccatcga tggctcaaag agtggcttga 2880

atatatccca tgggttatct gtatggactg actgggttat tgaaaggact agccacatac 2940

tagcatctta gtgcctttat ctgtctttat gtcttggggt tggggtaggt agataccaaa 3000

tgaaacactt tcaggacctt ccttcctctt gcagttgttc tttaatctcc tttactagag 3060

gagataaata ttttgcatat aatgaagaaa tttttctagt atataacgca ggccttttat 3120

tttctaaaat gatgatagta taaaaatgtt aggataacag aatgatttta gattttccag 3180

agaatattat aaagtgcttt aggtatgaaa ataaatcatc tttgtctgat taaaaaaaaa 3240

aaa 3243

<210> 15
<211> 50
<212> DNA
<213> homo sapiens

<400> 15
gtgagagtgt cttcacggca ggacaacgag gaagccctaa gacgtccctt 50

<210> 16
<211> 2828
<212> DNA
<213> homo sapiens

<400> 16
tcatttgctt aaatttgcag ctgacggctg ccacctctct agaggcacct ggcggggagc 60

ctctcaacat aagacagtga ccagtctggt gactcacagc cggcacagcc atgaactacc 120

cgctaacgct ggaaatggac ctcgagaacc tggaggacct gttctgggaa ctggacagat 180

tggacaacta taacgacacc tccctggtgg aaaatcatct ctgccctgcc acagaggggc 240

ccctcatggc ctccttcaag gccgtgttcg tgcccgtggc ctacagcctc atcttcctcc 300

tgggcgtgat cggcaacgtc ctggtgctgg tgatcctgga gcggcaccgg cagacacgca 360

gttccacgga gaccttcctg ttccacctgg ccgtggccga cctcctgctg gtcttcatct 420

tgccctttgc cgtggccgag ggctctgtgg gctgggtcct ggggaccttc ctctgcaaaa 480

ctgtgattgc cctgcacaaa gtcaacttct actgcagcag cctgctcctg gcctgcatcg 540

ccgtggaccg ctacctggcc attgtccacg ccgtccatgc ctaccgccac cgccgcctcc 600

tctccatcca catcacctgt gggaccatct ggctggtggg cttcctcctt gccttgccag 660

agattctctt cgccaaagtc agccaaggcc atcacaacaa ctccctgcca cgttgcacct 720

tctcccaaga gaaccaagca gaaacgcatg cctggttcac ctcccgattc ctctaccatg 780

tggcgggatt cctgctgccc atgctggtga tgggctggtg ctacgtgggg gtagtgcaca 840

ggttgcgcca ggcccagcgg cgccctcagc ggcagaaggc agtcagggtg gccatcctgg 900

tgacaagcat cttcttcctc tgctggtcac cctaccacat cgtcatcttc ctggacaccc 960
tggcgaggct gaaggccgtg gacaatacct gcaagctgaa tggctctctc cccgtggcca 1020
tcaccatgtg tgagttcctg ggcctggccc actgctgcct caaccccatg ctctacactt 1080
tcgccggcgt gaagttccgc agtgacctgt cgcggctcct gacgaagctg ggctgtaccg 1140
gccctgcctc cctgtgccag ctcttcccta gctggcgcag gagcagtctc tctgagtcag 1200
agaatgccac ctctctcacc acgttctagg tcccagtgtc cccttttatt gctgcttttc 1260
cttggggcag gcagtgatgc tggatgctcc ttccaacagg agctgggatc ctaagggctc 1320
accgtggcta agagtgtcct aggagtatcc tcatttgggg tagctagagg aaccaacccc 1380
catttctaga acatccctgc cagctcttct gccggccctg gggctaggct ggagcccagg 1440
gagcggaaag cagctcaaag gcacagtgaa ggctgtcctt acccatctgc acccccctgg 1500
gctgagagaa cctcacgcac ctcccatcct aatcatccaa tgctcaagaa acaacttcta 1560
cttctgccct tgccaacgga gagcgcctgc ccctcccaga acacactcca tcagcttagg 1620
ggctgctgac ctccacagct tcccctctct cctcctgccc acctgtcaaa caaagccaga 1680
agctgagcac caggggatga gtggaggtta aggctgagga aaggccagct ggcagcagag 1740
tgtggccttc ggacaactca gtccctaaaa acacagacat tctgccaggc ccccaagcct 1800
gcagtcatct tgaccaagca ggaagctcag actggttgag ttcaggtagc tgcccctggc 1860
tctgaccgaa acagcgctgg gtccacccca tgtcaccgga tcctgggtgg tctgcaggca 1920
gggctgactc taggtgccct tggaggccag ccagtgacct gaggaagcgt gaaggccgag 1980
aagcaagaaa gaaacccgac agagggaaga aaagagcttt cttcccgaac cccaaggagg 2040
gagatggatc aatcaaaccc ggcggtcccc tccgccaggc gagatggggt ggggtggaga 2100
actcctaggg tggctgggtc caggggatgg gaggttgtgg gcattgatgg ggaaggaggc 2160
tggcttgtcc cctcctcact cccttcccat aagctataga cccgaggaaa ctcagagtcg 2220
gaacggagaa aggtggactg gaaggggccc gtgggagtca tctcaaccat cccctccgtg 2280
gcatcacctt aggcagggaa gtgtaagaaa cacactgagg cagggaagtc cccaggcccc 2340
aggaagccgt gccctgcccc cgtgaggatg tcactcagat ggaaccgcag gaagctgctc 2400
cgtgcttgtt tgctcacctg ggtgtgggga ggcccgtccg gcagttctgg gtgctcccta 2460
ccacctcccc agcctttgat caggtgggga gtcagggacc cctgcccttg tcccactcaa 2520
gccaagcagc caagctcctt gggaggcccc actggggaaa taacagctgt ggctcacgtg 2580
agagtgtctt cacggcagga caacgaggaa gccctaagac gtcccttttt tctctgagta 2640
tctcctcgca agctgggtaa tcgatggggg agtctgaagc agatgcaaag aggcaagagg 2700
ctggattttg aattttcttt ttaataaaaa ggcacctata aaacaggtca atacagtaca 2760
ggcagcacag agacccccgg aacaagccta aaaattgttt caaaataaaa accaagaaga 2820

tgtcttca                                                          2828

<210>  17
<211>  50
<212>  DNA
<213>  homo sapiens

<400>  17
gggagtcact tgatgctttc aggttaatca gagctatggg tgctacaggc              50

<210>  18
<211>  1594
<212>  DNA
<213>  homo sapiens

<400>  18
atcttgagac tcgctaagcg tcccagccgc atccctcccg cagcgacggc ggcccgggac      60

ccgcgggctg tgaaccatga acacccgcaa tagagtggtg aactccgggc tcggcgcctc     120

ccctgcctcc cgcccgaccc gggatcccca ggacccttct gggcggcaag gggagctgag     180

ccccgtggaa gaccagagag agggtttgga ggcagcccct aagggccctt cgcgggagag     240

cgtcgtgcac gcgggccaga ggcgcacaag tgcatacacc ttgatagcac caaatataaa     300

ccggagaaat gagatacaaa gaattgcgga gcaggagctg gccaacctgg agaagtggaa     360

ggagcagaac agagctaaac cggttcacct ggtgcccaga cggctaggtg gaagccagtc     420

agaaactgaa gtcagacaga aacaacaact ccagctgatg caatctaaat acaagcaaaa     480

gctaaaaaga gaagaatctg taagaatcaa gaaggaagct gaagaagctg aactccaaaa     540

aatgaaggca attcagagag agaagagcaa taaactggag gagaaaaaaa gacttcaaga     600

aaaccttaga agagaagcat ttagagagca tcagcaatac aaaaccgctg agttcttgag     660

caaactgaac acagaatcgc cagacagaag tgcctgtcaa agtgctgttt gtggcccaca     720

atcctcaaca tggaaacttc ctatcctgcc tagggatcac agctgggcca gaagctgggc     780

ttacagagat tctctaaagg cagaagaaaa cagaaaattg caaaagatga aggatgaaca     840

acatcaaaag agtgaattac tggaactgaa acggcagcag caagagcaag aaagagccaa     900

aatccaccag actgaacaca ggagggtaaa taatgctttt ctggaccgac tccaaggcaa     960

aagtcaacca ggtggcctcg agcaatctgg aggctgttgg aatatgaata gcggtaacag    1020

ctggggttct ctattagttt tttcgaggca cctaagggta tatgagaaaa tattgactcc    1080

tatctggcct tcatcaactg acctcgaaaa gcctcatgag atgctttttc ttaatgtgat    1140

tttgttcagc ctcactgttt ttaccttaat ttcaactgcc cacacacttg accgtgcagt    1200

caggagtgac tggcttctcc ttgtcctcat ttatgcatgt ttggaggagc tgattcctga    1260

actcatattt aatctctact gccagggaaa tgctacatta tttttctaat tggaagtata    1320

attagagtga tgttggtagg gtagaaaaag agggagtcac ttgatgcttt caggttaatc 1380

agagctatgg gtgctacagg cttgtctttc taagtgacat attcttatct aattctcaga 1440

tcaggttttg aaagctttgg gggtcttttt agattttaat ccctactttc tttatggtac 1500

aaatatgtac aaaagaaaaa ggtcttatat tcttttacac aaatttataa ataaattttg 1560

aactccttct gtaaaaaaaa aaaaaaaaaa aaaa 1594

<210> 19
<211> 50
<212> DNA
<213> homo sapiens

<400> 19
ggcattgtcc agaaactccc ctgagacctt actccttcca gccccaaatc 50

<210> 20
<211> 1388
<212> DNA
<213> homo sapiens

<400> 20
gatttatact cttaatgggt actttctgac tgaattttat gagctcattc tgaagaggct 60

gacgatttta ctatctcatt tttttccttt ctccagaatg ggttctgggt gggtcccctg 120

ggtggtggct ctgctagtga atctgacccg actggattcc tccatgactc aaggcacaga 180

ctctccagaa gattttgtga ttcaggcaaa ggctgactgt tacttcacca acgggacaga 240

aaaggtgcag tttgtggtca gattcatctt taacttggag gagtatgtac gtttcgacag 300

tgatgtgggg atgtttgtgg cattgaccaa gctggggcag ccagatgctg agcagtggaa 360

cagccggctg gatctcttgg agaggagcag acaggccgtg gatggggtct gtagacacaa 420

ctacaggctg ggcgcaccct tcactgtggg gagaaaagtg caaccagagg tgacagtgta 480

cccagagagg accccactcc tgcaccagca taatctgctg cactgctctg tgacaggctt 540

ctatccaggg gatatcaaga tcaagtggtt cctgaatggg caggaggaga gagctggggt 600

catgtccact ggccctatca ggaatggaga ctggaccttt cagactgtgg tgatgctaga 660

aatgactcct gaacttggac atgtctacac ctgccttgtc gatcactcca gcctgctgag 720

ccctgtttct gtggagtgga gagctcagtc tgaatattct tggagaaaga tgctgagtgg 780

cattgcagcc ttcctacttg ggctaatctt ccttctggtg ggaatcgtca tccagctaag 840

ggctcagaaa ggatatgtga ggacgcagat gtctggtaat gaggtctcaa gagctgttct 900

gctccctcag tcatgctaag gtcctcactg aagcttctct ctctggagcc tgaagtagtg 960

atgagtagtc tgggccctgg gtgaggtaaa ggacattcat gaggtcaatg ttctgggaat 1020

aactctcttc cctgatcctt ggaggagccc gaactgattc tggagctctg tgttctgaga 1080

tcatgcatct cccacccatc tgcccttctc ccttctacgt gtacatcatt aatccccatt 1140

gccaagggca ttgtccagaa actcccctga gaccttactc cttccagccc caaatcattt 1200

acttttctgt ggtccagccc tactcctata agtcatgatc tccaaagctt tctgtcttcc 1260

aactgcagtc tccacagtct tcagaagaca aatgctcagg tagtcactgt ttccttttca 1320

ctgtttttaa aaacctttta ttgtcaaata aaatggagat acaaaaaatg taaaaaaaaa 1380

aaaaaaaa 1388

<210> 21
<211> 50
<212> DNA
<213> homo sapiens

<400> 21
tgccctaccc tctgctgggc tgtttgtatt gagttgcaat gtgaccagcg 50

<210> 22
<211> 4194
<212> DNA
<213> homo sapiens

<400> 22
   gcggccgcgc tcgtctcgcc gggctgttcg cgggcaggcc ctgccctgaa gggacgaatc 60
   ggcttggagc gcgggaggtg gagtcggccc cggcggtcgc tccctggacc caacccgagg 120
   ctgacccagg cccctgccca tgcggggcgc ccctggctcg gaagagtccc ccgggccggg 180
   agcagctcca ggcagcggcc ccggaggaag aggaagaagg gacagtgctc agcttggggg 240
   acccggaccc tcgccgcggc atttggagcc gggggcagtc ccgaactctg tgcttggcac 300
   cgccgctccg agtagggcag cgcctgccgg gaccctgacc cggaccccct gcgcctcgta 360
   ggcggcggcg ccgccgcgcc accctgttct tccgtgtctc cctctgcctg gcggcagtca 420
   cggccaagaa cctacattaa gaaacgcatt cagatgcatc agccgtttgt aaagcacctg 480
   tcgtgagcca gagagtatta tgagggaggc cgaggacttc atgctccgga cagagaaacg 540
   gcgctgggat tagggattgc cacttctgag aggatgctgg gaatctgcag ggggagacgg 600
   aaattcttgg ctgcctcgtt gagtcttctc tgcatcccag ccatcacctg gatttacctg 660
   ttttctggga gcttcgaaga tggaaagccc gtgtctctgt caccgctgga gtcccaggca 720
   cacagcccca ggtacacggc ctccagccag cgggagcgcg agagcctgga ggtgcgcatg 780
   cgcgaggtgg aggaggagaa ccgcgccctc cgcaggcagc tcagcctggc ccagggccga 840
   gccccatccc atcgccgagg caaccactcc aagacctact ccatggagga gggcactgga 900
   gacagcgaga accttcgggc tggcatcgtg gcaggcaaca gctccgagtg tgggcagcag 960
   ccggtcgtgg agaaatgcga gacaatccac gttgctattg tctgcgccgg atacaatgcc 1020
   agccgggatg tcgtcaccct ggtcaaatcc gtcctgttcc atagacggaa ccctctgcac 1080
   ttccacctta ttgctgactc cattgcggag cagatcctgg ccacgctctt ccagacctgg 1140
   atggtgcccg ctgtgcgtgt ggacttctac aatgcagacg agctcaagtc tgaagtttcc 1200
   tggatcccca ataaacatta ctctgggatt tatggtctga tgaagcttgt cctgaccaag 1260
   actcttcctg ccaacctgga gagagtcatc gtccttgaca cggatatcac ctttgccact 1320
   gacattgcag agctgtgggc tgtgttccac aagttcaaag gtcagcaagt cctgggcttg 1380
   gtggagaacc agagtgactg gtaccttgga aacctgtgga aaaatcaccg cccatggcca 1440
   gcccttggaa gaggctacaa cacaggggtg atcctgttac ttctggataa gctgcggaag 1500
   atgaaatggg agcagatgtg gaggctgacc gcagagaggg agctcatggg catgctctct 1560
   acatccttag ctgaccagga tattttcaat gccgtcatca aacaaaaccc cttccttgtg 1620
   taccagctcc cctgcttctg gaatgtgcag ctgtcagacc acacccgctc cgagcagtgc 1680
   tacagagacg tgtctgatct aaaggtcatt cactggaact cccccaagaa gctccgggtg 1740
   aagaacaagc atgtggagtt ttttcgcaac ctctacctga ccttcctgga gtatgacggc 1800
   aatcttctga ggcgggaact gtttggctgc cccagtgagg ctgatgtcaa cagtgaaaac 1860
   ctccagaagc agctgtctga gctggacgag gacgacctgt gctatgagtt ccggcgagag 1920
   cgcttcactg tccaccgcac ccacctgtac ttcctgcact acgagtatga gcctgcagca 1980
   gacagcacgg acgtcaccct ggtcgctcag ctgtccatgg acaggctcca gatgctggag 2040
   gccatctgca agcactggga ggggcccatc agcctggccc tctacctgtc agacgccgag 2100
   gcccagcagt tcctccgcta cgcacagggc tctgaggtgc ttatgagccg ccacaacgtg 2160
   ggctaccaca tcgtgtacaa ggagggccag ttctaccccg tgaacctgct gcgcaacgtg 2220
   gccatgaagc acatcagcac tccctacatg ttcctgtctg acattgactt cctgcccatg 2280
   tatgggctct atgagtacct caggaagtct gtcatccagc tcgatcttgc caacaccaag 2340
   aaagcaatga ttgtccccgc gttcgagaca ctgcgctacc ggctgtcctt ccccaagtca 2400
   aaagcggagt tgctgtcaat gctggacatg gggaccctct tcacattcag gtaccacgtc 2460
   tggacgaaag gccacgcacc cacaaacttc gccaagtggc ggaccgccac cacgccttac 2520
   cgggttgagt gggaggccga ttttgagccg tatgttgttg tgagacgtga ctgcccggag 2580
   tacgaccgga ggtttgtagg ctttggctgg aacaaagtgg ctcatatcat ggagctggat 2640
   gtgcaggagt atgagttcat tgtgctgccc aacgcctaca tgatccacat gcctcatgcc 2700
   cccagcttcg acattaccaa gttccgttcc aacaagcaat accgcatctg tctcaaaacc 2760
   ctcaaggaag agtttcagca ggacatgtcc cgccgctacg gctttgctgc cctgaaatat 2820
   ctcacagccg agaacaacag ctagcaccaa gaagcccacc actaggggga gacatgctgt 2880
   aggggaagtg ccactcgctg tttgggcccc ggccttcaaa ttcaaaattg agccatgctt 2940
   tttcggtttg tttttattta tctctttggc ccagccaagc tgccctcact acagagacct 3000
   tggacaagga tccagccagt ccctctctgc cccacaaccc tgcattccca gaggttagct 3060
   atgcagccca cctagatgag tctcttcaag aatgggaaat caaggggtga cagggagtaa 3120
   aagggttatc atcttactgc aaagccacaa gatcagggca gggctttagg atgttctgga 3180
   tgctttttaa taattatgct tcccatcata actggggaga aagggaagtc agggttctag 3240
   gggttattcg tcccaggaaa tagaagtgaa attgtcttta ttaagtgaaa actttcccct 3300
   ttgccctgca atgtagctgg gcattcaaac ggagggcaaa ccgatgatct aaaccaacca 3360
   cttggaaaaa cccaatgggg acattgtaac cagagggtcc tggaggtggg gttgatgggt 3420
   ttccttatcc ccaaagtcac tcctgttttg ttttgttttt ctttgggggt tttgtttatt 3480
   tttggggctg gcaatccaaa atagaaaatc tgatcctttg aggctctaaa ggaaaatcag 3540
   ctgcctctac caaccaccct ctatcagcag tggcccagga aggaggtcaa gcatcttcgg 3600
   ccgatattta aacatgggca gcttccttca ggatgatcac cgaggctccc gtgactttga 3660
   actccctact ctccagaatc caggggctat agcgatgggg actgcggaat tacgagggct 3720
   ggctgtttta caccggtcac attttctatt ggcagtgact gattcatggg aaagggcttt 3780
   gaaggaacta cttcagtgca cacacaaggt acgaacctct caggcctttc gaagaacttt 3840
   cataattcat gaaagcccag ttctgaagat tcacgtatcc atctggagac ctacaggaag 3900
   aaagtgattg ggttcctctg gttcttgcct gcttcactgt ggatgggaag aggtgacaac 3960
   ctcagtctcc ctttgggacc tgtccaaggg taggcaacca ccttcacctt cacacagatt 4020
   gaggagacac tggacttttt acccattttc tttaatcttc aatattaata ttgtgtttac 4080
   attgatgaga acaagagtta atgccctacc ctctgctggg ctgtttgtat tgagttgcaa 4140
   tgtgaccagc gaaagctgca tttaataaat gaaagtacag actgtttctt cccc 4194

<210> 23
<211> 50
<212> DNA
<213> homo sapiens

<400> 23
acctgggcgt ggtggtgcat gcctgtaatc ccatcatcta ctcaggaggc 50

<210> 24
<211> 3364
<212> DNA
<213> homo sapiens

<400> 24
gattgttgcc gtgcgctgga gccgagtttc gctctgctgc ccaggctgga gtacagtgat 60

gtgatctcct gacgtcaagt gatccattcg cctcggcctc ccaaagttct gggattacaa 120

gtaatacaaa atacagttaa taaaatgtca acctcctgga gtgatcggtt acagaatgca 180

gcagatatgc ctgctaacat ggataagcat gccctgaaaa agtatcgtcg agaagcctat 240

catcgggtgt ttgtgaaccg aagtttagca atggaaaaga taaagtgttt tggttttgat 300

atggattata cccttgctgt gtacaagtcc ccagagtatg agtcccttgg ttttgagctt 360

actgtggaga gattagtttc tattggctat ccccaggagt tgctcagctt tgcttatgat 420

tctacattcc ctaccagggg acttgtcttt gacacactgt atggaaatct tttgaaagtc 480

gatgcctatg gaaacctctt ggtctgtgca catggattta actttataag gggaccagaa 540

actagagaac agtatccaaa taaatttatc cagcgagatg atactgaaag attttacatt 600

ctgaacacac tattcaacct accagagacc tacctgttgg cctgcctagt agattttttt 660

actaattgtc ccagatatac cagttgtgaa acaggattta aagatgggga cctcttcatg 720

tcctaccgga gtatgttcca ggatgtaaga gatgctgttg actgggttca ttacaagggc 780

tcccttaagg aaaagacagt tgaaaatctt gagaagtatg tagtcaaaga tggaaaactg 840

cctttgcttc tgagccggat gaaggaagta gggaaagtat ttcttgctac caacagtgac 900

tataaatata cagataaaat tatgacttac ctgtttgact tcccacatgg ccccaagcct 960

gggagctccc atcgaccatg gcagtcctac tttgacttga tcttggtgga tgcacggaaa 1020

ccactctttt ttggagaagg cacagtactg cgtcaggtgg atactaaaac tggcaagctg 1080

aaaattggta cctacacagg gcccctacag catggtatcg tctactcagg aggttcttct 1140

gatacgatct gtgacctgtt gggagccaag ggaaaagaca ttttgtatat tggagatcac 1200

atttttgggg acattttaaa atcaaagaaa cggcaagggt ggcgaacttt tttggtgatt 1260

cctgaactcg cacaggagct acatgtctgg actgacaaga gttcactttt cgaagaactt 1320

cagagcttgg atattttctt ggctgaactc tacaagcatc ttgacagcag tagcaatgag 1380

cgtccagaca tcagttccat ccagagacgt attaagaaag taactcatga catggacatg 1440

tgctatggga tgatgggaag cctgtttcgc agtggctccc ggcagaccct ttttgccagt 1500

caagtgatgc gttatgctga cctctatgca gcatctttca tcaacctgct gtattaccct 1560

ttcagctacc tcttcagggc tgcccatgtc ttgatgcctc atgaatcaac ggtggagcac 1620

acacacgtag atatcaatga gatggagtct cctcttgcca cccggaaccg cacatcagtg 1680

gatttcaaag acactgacta caagcggcac cagctgacac ggtcaattag tgagattaaa 1740

cctcccaacc tcttcccact ggccccccag gaaattacac actgccatga cgaagatgat 1800

gatgaagagg aggaggagga ggaagaataa ggaggaaaac caaaacccca agcacccatt 1860

aaacaagtcc tggcaggact cacaggaaca aacgaggtcc ctgttagggt tctactcggg 1920

ggagggaggg ggctccatga aaggtacgtc tgaaaagttt ctgaagattt tattatcata 1980

gatacttgtt ttggttttgt gtatctgtac tctctgcaga tggtccaaaa ttgtaatgga 2040

gtctgtatta gaagaaaata agggtaaaat caggctgaac tgcatgtata tggctccact 2100

gtggcttgtg acacttttaa aatcatccgt atgtcagtgt atctggatac acgaggaaaa 2160

ggaaagagtc tcagagtgga acaaagagtg ggaagaggtg atctgtaatg ttacaaattg 2220

tgctattact ccaaggtcca acttttccag tgcattacat ggtattgtat atcagtggag 2280

aaatgtatta tttccatgat caaatgtagt ctctgttaag gtcaagtttt cttttataag 2340

cctttaattc atcctcagtg actctggcaa ggctgcttct ctatcactgg ctttgcacag 2400

aagtatgctc tacttgcgtt gctttagggc aggattctat tttgagggaa aagacagtat 2460

ccttattacc ttttgtttgt ttaatagcac aaatgcttat ttgttatcca aaaacaacct 2520

ccttcttatc tgtgataaat ctatagaaag aatttagctg caagtggaca aaggaacaag 2580

cccccagaaa agaaagggaa gaactgcctt cttatactac agaacatgca ttagtgtggg 2640

ctatatagct gtggctcatg ctacccaatt ccagatttct ttgtcctcta agagttgatt 2700

gctgtatatt aaaattgaac atcagaggat gggaagaggg ctctgtaagc cagaacctta 2760

ctaaagtaga gggcacaatc agtgtgaata aattcacttc agaatctcaa gtcaaggcca 2820

ggcacggcgg ctcacgcctg taatcccagc actttgggag gccgagacag gcggatcacc 2880

tgaggtcggg agttcgagac cagccttacc aacatggaga aaccccatct ctactaaaaa 2940

tacaaaatta cctgggcgtg gtggtgcatg cctgtaatcc catcatctac tcaggaggct 3000

gaggcaggag aattgcttga acccaggagg cggaggttgc agtgagccag gattgtgcca 3060

ttgcactcca gcctgggcaa caagaacaaa actccatctc aaaaaataaa aatcccaatc 3120

ccaagtcgaa atcacctctt gttttaaaca agaatgaatc attactgtgt atgttagggt 3180

attaaaactg tttcaccagt acagtgaaag ttgtttcaac attttaaaca aacagtggtt 3240

atagactctt tctttaacca ttgtatattt tcttccattc ttgtcattgg tcaatagggg 3300

agggtagatt agctgctcca gaattcaata aagtgtaata tttctaaaaa aaaaaaaaaa 3360

aaaa 3364

<210> 25
<211> 50
<212> DNA
<213> homo sapiens

<400> 25
ctcggtggtt agcaattatt cctctgagtg ggagctggac cctgtaaagg 50

<210> 26
<211> 1742
<212> DNA
<213> homo sapiens

<400> 26
tctttgaagc ttcaaggctg ctgaataatt tccttctccc attttgtgcc tgcctagcta 60

tccagacaga gcagctaccc tcagctctag ctgatactac agacagtaca acagatcaag 120

aagtatggca gtgacaactc gtttgacatg gttgcacgaa aagatcctgc aaaatcattt 180

tggagggaag cggcttagcc ttctctataa gggtagtgtc catggattcc gtaatggagt 240

tttgcttgac agatgttgta atcaagggcc tactctaaca gtgatttata gtgaagatca 300

tattattgga gcatatgcag aagagagtta ccaggaagga aagtatgctt ccatcatcct 360

ttttgcactt caagatacta aaatttcaga atggaaacta ggactatgta caccagaaac 420

actgttttgt tgtgatgtta caaaatataa ctccccaact aatttccaga tagatggaag 480

aaatagaaaa gtgattatgg acttaaagac aatggaaaat cttggacttg ctcaaaattg 540

tactatctct attcaggatt atgaagtttt tcgatgcgaa gattcactgg atgaaagaaa 600

gataaaaggg gtcattgagc tcaggaagag cttactgtct gccttgagaa cttatgaacc 660

atatggatcc ctggttcaac aaatacgaat tctgctgctg ggtccaattg gagctgggaa 720

gtccagcttt ttcaactcag tgaggtctgt tttccaaggg catgtaacgc atcaggcttt 780

ggtgggcact aatacaactg ggatatctga gaagtatagg acatactcta ttagagacgg 840

gaaagatggc aaatacctgc cgtttattct gtgtgactca ctggggctga gtgagaaaga 900

aggcggcctg tgcagggatg acatattcta tatcttgaac ggtaacattc gtgatagata 960

ccagtttaat cccatggaat caatcaaatt aaatcatcat gactacattg attccccatc 1020

gctgaaggac agaattcatt gtgtggcatt tgtatttgat gccagctcta ttcaatactt 1080

ctcctctcag atgatagtaa agatcaaaag aattcgaagg gagttggtaa acgctggtgt 1140

ggtacatgtg gctttgctca ctcatgtgga tagcatggat ttgattacaa aaggtgacct 1200

tatagaaata gagagatgtg agcctgtgag gtccaagcta gaggaagtcc aaagaaaact 1260

tggatttgct ctttctgaca tctcggtggt tagcaattat tcctctgagt gggagctgga 1320

ccctgtaaag gatgttctaa ttctttctgc tctgagacga atgctatggg ctgcagatga 1380

cttcttagag gatttgcctt ttgagcaaat agggaatcta agggaggaaa ttatcaactg 1440

tgcacaagga aaaaaataga tatgtgaaag gttcacgtaa atttcctcac atcacagaag 1500

attaaaattc agaaaggaga aaacacagac caaagagaag tatctaagac caaagggatg 1560

tgttttatta atgtctagga tgaagaaatg catagaacat tgtagtactt gtaaataact 1620

agaaataaca tgatttagtc ataattgtga aaaataataa taatttttct tggatttatg 1680

ttctgtatct gtgaaaaaat aaatttctta taaaactcgg gtctaaaaaa aaaaaaaaaa 1740

aa 1742

<210> 27
<211> 50
<212> DNA
<213> homo sapiens

<400>  27
ccagtctctt atccctctgc tcttggaagc ccgtgcagac cccacaataa          50


<210>  28
<211>  3993
<212>  DNA
<213>  homo sapiens

<400>  28
aatgtaagaa cttttcttcc tcccttaact ttgcttcctt ctttcctgca tgttaccact     60

ggcagagcaa atatgactca gaaaccggct cctcagggtt gtaacattag atgatacagg    120

cttgggtcgt tacacatgac accagtgcct ttgtttcatt gggctgggct ctctggaagg    180

tgtgctgctg cctgagctgc tggaaaagca ctgacaggtg tttgctagaa aagcactcct    240

ggagcttgcc accagcttgg acttctaggg actttcctct cagccaggaa ggattttgat    300

attcatcaga aatacctcca gaagattcaa ggagctgtag aggtgaagta agcctgtgaa    360

ggaccagcat gggaatccta tactctgagc ccatctgcca agcagcctat cagaatgact    420

ttggacaagt gtggcggtgg gtgaaagaag acagcagcta tgccaacgtt caagatggct    480

ttaatggaga cacgcccctg atctgtgctt gcaggcgagg gcatgtgaga atcgtttcct    540

tccttttaag aagaaatgct aatgtcaacc tcaaaaacca gaaagagaga acctgcttgc    600

attatgctgt gaagaaaaaa tttaccttca ttgattatct actaattatc ctcttaatgc    660

ctgttctgct tattgggtat ttcctcatgg tatcaaagac aaagcagaat gaggctcttg    720

tacgaatgct acttgatgct ggcgtcgaag ttaatgctac agattgttat ggctgtaccg    780

cattacatta tgcctgtgaa atgaaaaacc agtctcttat ccctctgctc ttggaagccc    840

gtgcagaccc cacaataaag aataagcatg gtgagagctc actggatatt gcacggagat    900

taaaattttc ccagattgaa ttaatgctaa ggaaagcatt gtaatccttg tgaccacacc    960

gatggagata cagaaaaagt taacgactgg attctatctt cattttagac ttttggtctg   1020

tgggccattt aacctggatg ccaccatttt atggggataa tgatgcttac catggttaat   1080

gttttggaag agctttttat ttatagcatt gtttactcag tcaagttcac catggccgta   1140

atccttctaa gggaaacact aaagttgttg tagtctccac ttcagtcaga aactgatgtt   1200

tcagctaggc acagtggtac atgcctgtaa tcccagctac ttgggaggct gaggtgggag   1260

gatcacttga actcaggagt ttgagagcag ccagggcaac acagcgagac cctgtctcaa   1320

aaaaaaaaaa aaaaaaaaaa gccctggtgt tccaaactca gtctttcctg aagaagagga   1380

tctgagttat cttctgaaac agcgttctcc cttcccagtt gtatcactct tataaaaaga   1440

ctgtccagtc tatgtcatgc cctaggagac aaactgttcc tcccagcccc ctttgagtat   1500

tgagcagaag aatcaaatta ttaaatacgt atgtttgtac agaatggtat ttgtgtatgt   1560

gtgtgggctt agagattcac aagtaaatat tcctttggtg aaggaatttc aataaaaaca 1620
tctatcaagt gtcagcggtg agtgtgttta caccacagaa attggcaaat tgacaaatca 1680
gagtttgttt ttgttttttt gtttttactt ttccataaag ttcgtttacc agcataccac 1740
tagagatttc ggtttacaaa taaaagccat cttggtttga gcaagactat gcaactatga 1800
aaatgttcgt ttaaaaaaat cttcatgatc cttttgtaaa tacaaggtgg ttgccaagct 1860
tgttagtttt gtttatttta ttgatagatg taaaatatta ttgtaactta tttggataaa 1920
gttcttcaaa agaaacagag ctatacaatg aggtaggatc tggattattt gtctaagtga 1980
gagattgcga atatcaaaat atctgtctca cttcttctgt gaatgacaca gagtagaaat 2040
aaattcactt taaaaatatg actgaatttt gaaaatcaag actgaatctc acatagctgc 2100
agacaggaac taagccagcc tctttgtatg tggtaacaag tacagtataa gaatgaaaga 2160
tttaccatcc ttgaaagctc taatgaaaat caaatccagc aatatatatt caactgtgta 2220
caggatttaa gaaacttatt ttatgaagga agtaatagtg tgtagatata gattctgaag 2280
tctttaaacg tgtcttaata aattaagatt cactggcatt gagctgagct accaggtgac 2340
ccttggggac aaaaaaccca cacaagtgaa tttcacacac cagtatacct tcaacaatat 2400
acttttgaca cacacaaacc tttgatttgg tttcagagat tttgcaaaat agtaccaatg 2460
taatttacaa ctgtcatctt tgaaattgtg taaaagtgga ataattttct gaagaaataa 2520
atcatggttt gtcaatgagt tgcagagact gtctgacatt aactttgtca agattaaagg 2580
ataaagtata tgacaatttg tttcatcatg ctcatgacat tatgcaattt tctccctagc 2640
ttttaatttt tggaggcaga aaattgagcc agaaattttt agtcattagg tctcctagca 2700
acaagctgta aaccttccaa caagcttgga ctagaatcta gacactgaaa tgcacataca 2760
tgctttatgt aatgcagaat gcatttattg gagaactcat aaacatccta taaaattttc 2820
ttccctgaga tgcaactata aaacttggcc ttattctgag aatgcttaac atagatttca 2880
tccatactgt aacactgatt ttgttgttgt tgtccttaaa gcagctcagc ttcctgaggt 2940
agtgttatgt ctctgtggca acaaggtgaa aatgtctagc ttattttgtc aaagtcaaca 3000
ataatccaca gactccagac ctcaatatct gtcccaattt gccattttac tttagtgctc 3060
caaaaatatg gcttatagaa aaaacaatag gtgttttaaa gagatttacc tgaatgatat 3120
agagaatgtc tagatatttt ctggctatca ggtaaaacct acccttcaag atggtagaat 3180
atataatagc atacaaaacc tctatttacc taataagtac tttaatttac agaaaaaaaa 3240
tgtaaatgta agtgtcggat ttagtgccaa gtgcagggaa tctgaaaaat gtatactagg 3300
tctctgctct ccgtaattct gccttcatgg gtcctagccc catccctcag gaggttgtcc 3360
taagatcgtc agtgtcagat gcttcacaat acggcctcac accgtccctg ggaaaggttg 3420
gtctcctcct gctgcatcag atggatgatt tcattgtaca tacggtgagg agcatccaaa 3480

ccccagatga aatccacgtg agcccattca ggaatattct tatggtagat gaggttggtc 3540

acctcagaga gcagcatttt cacgtcttct ggatttgaaa gccagtcctg acctcctgtc 3600

cacattgctg tagggaccgt catatctctg actctgtacc ttacaggagt tggctagaga 3660

aaaggaatag ttcttaactc taggtaacat ttggactttc aggctcataa tttatgtttc 3720

aaatagacat aataaacatg ccatctgttg tggtgaaggg tacatgggtg ttagagccac 3780

acaactctgt taagaatttc tgttcccgcc cttactttaa ggtaaaatta cttaacatta 3840

ttgaacctca gtttcttctt ctgtgactgg ggataatatc tgtaataact tgctagatca 3900

aatgacaaaa cacataaaaa catgtaatgc cttgtatttc ttttttcttc ctattaaata 3960

ttttgtaaat aaattgtttt taaaaaaaaa aaa 3993

<210> 29
<211> 50
<212> DNA
<213> homo sapiens

<400> 29
cgccgtcaga gtgctgtctt atgtgaagtg gatcgaggac accatagcgg 50

<210> 30
<211> 874
<212> DNA
<213> homo sapiens

<400> 30
agttcctcca cctgctggcc cctggacacc tctgtcacca tgtggttcct ggttctgtgc 60

ctcgccctgt ccctgggggg gactggtgct gcgcccccga ttcagtcccg gattgtggga 120

ggctgggagt gtgagcagca ttcccagccc tggcaggcgg ctctgtacca tttcagcact 180

ttccagtgtg ggggcatcct ggtgcaccgc cagtgggtgc tcacagctgc tcattgcatc 240

agcgacaatt accagctctg gctgggtcgc cacaacttgt ttgacgacga aaacacagcc 300

cagtttgttc atgtcagtga gagcttccca caccctggct tcaacatgag cctcctggag 360

aaccacaccc gccaagcaga cgaggactac agccacgacc tcatgctgct ccgcctgaca 420

gagcctgctg ataccatcac agatgctgtg aaggtcgtgg agttgcccac cgaggaaccc 480

gaagtgggga gcacctgttt ggcttccggc tggggcagca tcgaaccaga gaatttctca 540

tttccagatg atctccagtg tgtggacctc aaaatcctgc ctaatgatga gtgcaaaaaa 600

gcccacgtcc agaaggtgac agacttcatg ctgtgtgtcg gacacctgga aggtggcaaa 660

gacacctgtg tgggtgattc aggggggccg ctgatgtgtg atggtgtgct ccaaggtgtc 720

acatcatggg gctacgtccc ttgtggcacc cccaataagc cttctgtcgc cgtcagagtg 780

ctgtcttatg tgaagtggat cgaggacacc atagcggaga actcctgaac gcccagccct 840

gtcccctacc cccagtaaaa tcaaatgtgc atcc                                874

<210>  31
<211>  50
<212>  DNA
<213>  homo sapiens

<400>  31
gccctttact caacctgagg aacaagatgc caagcttgcc ttgatccaag                50

<210>  32
<211>  976
<212>  DNA
<213>  homo sapiens

<400>  32
tattgagagg aacaaagagc ttataaatac attaggacct ggaattcggt tgtccagcca     60

caaaggtgac agcatttaac aaagcttaga gaaacctcca ggagactgct accatggcag    120

agaagcccaa gctccactac tccaatatac ggggcagaat ggagtccatc cggtggctcc    180

tggctgcagc tggagtagag tttgaagaga aatttataaa atctgcagaa gatttggaca    240

agttaagaaa tgatggatat ttgatgttcc agcaagtgcc aatggttgag attgatggga    300

tgaagctggt gcagaccaga gccattctca actacattgc cagcaaatac aacctctatg    360

ggaaagacat aaaggagaaa gccctgattg atatgtatat agaaggtata gcagatttgg    420

gtgaaatgat ccttcttctg ccctttactc aacctgagga acaagatgcc aagcttgcct    480

tgatccaaga gaaaacaaaa aatcgctact tccctgcctt tgaaaaagtc ttaaagagcc    540

acggacaaga ctaccttgtt ggcaacaagc tgagccgggc tgacattcac ctggtggaac    600

ttctctacta cgtggaagag cttgactcta gccttatttc cagcttccct ctgctgaagg    660

ccctgaaaac cagaatcagt aacctgccca cagtgaagaa gtttctacag cctggcagcc    720

caaggaagcc tcccatggat gagaaatctt tagaagaatc aaggaagatt ttcaggtttt    780

aataaaccag ccatagaggt caagaacatg caagaccagt attctaaagt tttgcaacaa    840

ttaagtgctt tacctaagtg ttgattgtgc ctgttgtgaa gctaatgaac tctttcaaat    900

tatatgctaa ttaaataata caactcctat tcacccactt agttaaaatt gatttcttct    960

cattaggatc tgatgt                                                    976

<210>  33
<211>  50
<212>  DNA
<213>  homo sapiens

<400>  33
gtcttctcta gtttctgagg tgaattgatt ccagcaccct caggaatacc                50

<210>  34

<211>  2829
<212>  DNA
<213>  homo sapiens

<400>  34
actgagagac gccctggaac gtctgtggca gcttctgtct cgctgggacc cgcactgaca      60

gcgggaggca gagggaggaa cctggacgcc ggaagccggg aaatggactc agtggcattt     120

gaggatgtgg ctataaactt cacctgtgag gagtgggctt tgctgggtcc atcacagaag     180

agtctctaca gagatgtgat gcaggaaacc atcagaaacc tggactgtat agactgaata     240

agaggagggg ttggtcttgc tgtctcaaga gtagcagaga ctgaagaaat tcatatgaat     300

gatgatggca aaatcatgat atagaagaag atcagtacaa agatctcaga agaaatctaa     360

gatgtcatat ggtagagaga gcctgtgaaa ttaaagataa tagtcaatgt ggaggaccct     420

ttacccagac tcaagacagt attgtgaacg agaaaatacc tggagtagat ccatgggaaa     480

gcagtgagtg tacagacgtc ctcatgggtc gttcatctct taattgctac attagagttg     540

acagtgaaca caaaccatat gagtatcaag aatatggaga gaagccatat acacatacac     600

aatgtgggac agctttcagt tatcagccct gctttcaaat acatgaaaga cctcagcatg     660

gaaagaaact ctatgattgt aaggaatgtg caagcttcag ttctcttgaa aaccttcaaa     720

gacacatggc agcacaccat ggagatggac ctcgtatatg taagttgtgt ggaaacgcct     780

ttatttggcc tagtttattt catatgctta gaagaactca cactgaagag aaaccatatg     840

aatatgagca gtgttctaca gcgtttcctg cttatagttc cactctaaga catgaaagaa     900

cacacagtgg agagaaaccc tatcaatgta aacaatgtgg gaaagccttc agttgttcct     960

gttacactca actatatgaa aggactcaca ctggagaaca atcctatgaa tgtaagcaat    1020

gtgggaaagc attttatcat cttggaagct ttcaaagaca catgatagtg cacactggag    1080

atgggcctca taaatgtaag atatgtggaa aaggctttct ttctcccagt tcagttcgaa    1140

gacataaaag aactcacact ggagagaaac cgtatgaatg taagtattgt gggaaagcat    1200

tctctgattg cacaggtttt cgaagacaca tgataacgca cactggagat ggacctcata    1260

aatgcaaggt atgtgggaaa gcctttgatt ctcccagttt atgtcgaagg catgaaacag    1320

ctcatactgg ggagaaaccc tataaatgtg aatgtgggaa agcctttagt gatttctatt    1380

actttcgaaa tcatgaaact actcacactg gagagaagcc atataaatgt aaacaatgtg    1440

gaaaagcctt catttgttgc acttaccttc aaatacatga aagaattcac actggggaga    1500

gaccctataa atgtaaacaa tgcggaaaag ccttcaggtc ttccaattac attcgagtac    1560

atgaaaagac tcacactgga gaaaagccct atgaatgtaa gcagtgtgga aaagcacttt    1620

ctcatctgaa aagctttcag agacacatga taatgcacac tggagatgga cctcataaat    1680

gtaagatatg tgggaaaagc tttgattctc ccagttcatt tcgaagacat gaaagaattc    1740

acactgggga gagaccctat aagtgtaaac tatgtgggaa aggcttcagg tcttccagtt 1800

acattcaact acatgaaagg actcacactg gagagaaacc ctatggttgt cagcaatgtg 1860

ggaaagcatt atctgatctc tcaagctttc gaagacacat gataacacat actggaaatg 1920

gacctcataa atgtaagata tgtgggaaag gctttgatta tcccagttca gtgcaaagac 1980

atgaaagaac tcacactgga gagaaaccct atgaatgcaa ggaatgtggt aaagccttca 2040

gtcattcaag ttacttacga atacacgaaa gagttcatac tggagagaag ccgtataaat 2100

gtaaggaatg tgggaaacca ttccattgtc ccagtgcctt tcataaacat gaaaggaccc 2160

acagtatgga gaaaccctat aagtgtaaag aatgtgggga agcatttcat tgtatcagtt 2220

cctttcataa acatgaaatg actcactaga gaaaacccct atgagtgttg aacatgtgag 2280

aaagccttaa gtactttcag cttcttacaa atacataaga gactcacact gaaaaaagtt 2340

gtatgaatat aaaaatatac taaaaccttc catttttttc agtacctttt gaaaacatga 2400

ccaaactcat acttcaaaaa tatatatata atgaatgtga ggaatatgaa aaaactttct 2460

ttctctagca aactttcaaa ggtggttatt ataacatact agcaatggac cttactactg 2520

taaggaatgt agaaatgcat ttactagtcc taactcattt cagttacatg aaagatttca 2580

aactgcagag aaccactatg aatgtaaaca atgtggtaaa gcatttagta gcttcagttt 2640

cttttgaata cagtcttctc tagtttctga ggtgaattga ttccagcacc ctcaggaata 2700

cctatattca gagatattca agtccctgat attaaaaaca gcatatttgc atataatcta 2760

ggcacatttt tctgtatatc ttaaatcatt tctagattac ttataataca caatgcattg 2820

taaattctg 2829

<210> 35
<211> 50
<212> DNA
<213> homo sapiens

<400> 35
aagcagtgtg ggaagtcttt tggatgtgcc tcgcgacttc aaatgcatgg 50

<210> 36
<211> 2321
<212> DNA
<213> homo sapiens

<400> 36
gtccatcggg gagggcgaag gccctgctct cctcggttcc cggctccagg cggcgagctg 60

aggttgggag cctggctttc ccctccgaga gggttcaggt gcctctgcca tagcttctgt 120

cgcctgtgct gtgacccgca ctggtcgtgg gagtcacctg aaaggcaaga aatgatgttt 180

caggattcag tggcctttga ggatgtggct gtgaccttca cccaagagga gtgggctttg 240

ctggatcctt cccagaaaaa tctctgtaga gatgtgatgc aagaaacctt caggaacctg 300

gcctctatag ggaaaaaatg gaaaccccag aacatatatg tagagtacga aaatctaagg 360

agaaacctaa gaattgtggg agagagactc tttgaaagta aagaaggtca tcagcatgga 420

gaaattttga cccaggttcc agatgacatg ctgaagaaaa caactactgg agtaaaatca 480

tgcgaaagca gtgtgtatgg agaagtaggc agtgctcatt catctcttaa taggcacatc 540

agagatgaca ctggacacaa ggcatatgag tatcaagaat atggacagaa accatataaa 600

tgtaaatact gtaaaaaacc tttcaactgt ctctcctctg ttcagacaca tgaaagggct 660

catagtggaa ggaaactcta tgtttgtgag gaatgcggaa aaacatttat ttcccattca 720

aaccttcaaa gacacaggat aatgcaccgt ggagatggac cttataagtg taaattttgt 780

gggaaagcct tgatgtttct cagtttgtat cttatccaca aacgaactca cactggagag 840

aaaccatatc aatgtaaaca gtgtggtaaa gcctttagtc attctagtag ccttcgaata 900

catgaaagaa ctcacactgg ggagaagcct tataaatgta atgaatgtgg gaaagcattc 960

catagttcca catgccttca tgctcataaa agaactcaca ctggggagaa gccatatgaa 1020

tgtaaacagt gtgggaaagc cttcagctct tcccattcct ttcaaataca tgaaagaact 1080

cacacggggg agaagccata tgaatgtaag gaatgtggaa aagcattcaa gtgtcccagt 1140

tctgttcgca gacatgaaag aacccactct aggaaaaaac cctatgaatg taaacattgt 1200

gggaaagtat tatcttatct taccagcttt caaaaccact tgggaatgca cactggagag 1260

atatctcata aatgtaagat atgtgggaaa gccttttatt ctcccagttc acttcaaaca 1320

catgaaaaaa ctcacactgg agagaaaccc tataaatgca accaatgtgg taaagccttt 1380

aattcttcca gttccttccg atatcatgaa agaactcaca ctggagagaa accttacgag 1440

tgtaagcaat gtgggaaagc cttcagatct gcctcactcc ttcaaacaca tggtaggact 1500

cacacgggag agaaacccta tgcatgtaag gaatgtggaa aaccatttag taatttctct 1560

ttctttcaaa tacatgaaag gatgcacaga gaagagaagc cgtatgaatg taagggttat 1620

gggaaaacat tcagtttgcc cagtttattt catagacatg aaaggactca cactggagga 1680

aaaacctatg aatgcaagca gtgtggcaga tccttcaact gttcgagctc ctttcgatat 1740

catggaagga ctcacactgg agagaaaccc tatgaatgca agcaatgtgg aaaagccttc 1800

agatctgcct cacagcttca aattcatgga aggactcaca ctggagagaa accttatgaa 1860

tgtaagcagt gtgggaaagc ctttggatct gcctcacacc ttcaaatgca tggaaggact 1920

cacactggag agaaacccta tgaatgtaag cagtgtggga agtcttttgg atgtgcctcg 1980

cgacttcaaa tgcatggaag gactcacact ggagagaaac cgtataaatg taagcaatgt 2040

gggaaagctt ttggatgtcc ctcaaacctt cgaaggcatg gaaggactca cactggagag 2100

aaaccctata aatgtaacca atgtggtaaa gtctttagat gttcttcaca acttcaagtg 2160

catggaaggg ctcactgcat agacacccca taaccccagg ctttaggagg ctgaggtggg 2220

gggactgctt aagcccagga gttcaagacc agcctgggca acatagtgag actctgttat 2280

aaaaattaaa taaataaata aaaagacttc cagttggtgg t 2321

<210> 37
<211> 50
<212> DNA
<213> homo sapiens

<400> 37
gagttaccac accccatgag ggaagctcta aatagccaac acccatctgt 50

<210> 38
<211> 3331
<212> DNA
<213> homo sapiens

<400> 38
   gtctatcagc gatttcatct tcaggcctgg actacaccac tcaccctccc agtgtgcttg 60
   agaaacaaac tgcacccact gaactccgca gctagcatcc aaatcagccc ttgagatttg 120
   aggccttgga gactcaggag ttttgagagc aaaatgacaa cacccagaaa ttcagtaaat 180
   gggactttcc cggcagagcc aatgaaaggc cctattgcta tgcaatctgg tccaaaacca 240
   ctcttcagga ggatgtcttc actggtgggc cccacgcaaa gcttcttcat gagggaatct 300
   aagactttgg gggctgtcca gattatgaat gggctcttcc acattgccct gggggtcttt 360
   ctgatgatcc cagcagggat ctatgcaccc atctgtgtga ctgtgtggta ccctctctgg 420
   ggaggcatta tgtatattat ttccggatca ctcctggcag caacggagaa aaactccagg 480
   aagtgtttgg tcaaaggaaa aatgataatg aattcattga gcctctttgc tgccatttct 540
   ggaatgattc tttcaatcat ggacatactt aatattaaaa tttcccattt tttaaaaatg 600
   gagagtctga attttattag agctcacaca ccatatatta acatatacaa ctgtgaacca 660
   gctaatccct ctgagaaaaa ctccccatct acccaatact gttacagcat acaatctctg 720
   ttcttgggca ttttgtcagt gatgctgatc tttgccttct tccaggaact tgtaatagct 780
   ggcatcgttg agaatgaatg gaaaagaacg tgctccagac ccaaatctaa catagttctc 840
   ctgtcagcag aagaaaaaaa agaacagact attgaaataa aagaagaagt ggttgggcta 900
   actgaaacat cttcccaacc aaagaatgaa gaagacattg aaattattcc aatccaagaa 960
   gaggaagaag aagaaacaga gacgaacttt ccagaacctc cccaagatca ggaatcctca 1020
   ccaatagaaa atgacagctc tccttaagtg atttcttctg ttttctgttt ccttttttaa 1080
   acattagtgt tcatagcttc caagagacat gctgactttc atttcttgag gtactctgca 1140
   catacgcacc acatctctat ctggcctttg catggagtga ccatagctcc ttctctctta 1200
   cattgaatgt agagaatgta gccattgtag cagcttgtgt tgtcacgctt cttcttttga 1260
   gcaactttct tacactgaag aaaggcagaa tgagtgcttc agaatgtgat ttcctactaa 1320
   cctgttcctt ggataggctt tttagtatag tatttttttt tgtcattttc tccatcaaca 1380
   accagggaga ctgcacctga tggaaaagat atatgactgc ttcatgacat tcctaaacta 1440
   tctttttttt attccacatc tacgtttttg gtggagtccc ttttgcatca ttgttttaag 1500
   gatgataaaa aaaaataaca actagggaca atacagaacc cattccattt atctttctac 1560
   agggctgaca ttgtggcaca ttcttagagt taccacaccc catgagggaa gctctaaata 1620
   gccaacaccc atctgttttt tgtaaaaaca gcatagctta tacatggaca tgtctctgcc 1680
   ttaacttttc ctaactccca ctctaggcta ttgtttgcat gtctacctac ttttagccat 1740
   tatgcgagaa aagaaaaaaa tgaccataga aaatgccacc atgaggtgcc caaatttcaa 1800
   ataataatta acatttagtt atatttataa tttccagatg acaaagtatt tcatcaaata 1860
   acttcatttg atgttccatg atcaagaaag aatccctatc tctattttac aagtaattca 1920
   aagaggccaa ataacttgta aacaagaaaa ggtaacttgt caacagtcat aactagtaat 1980
   tatgagagcc ttgtttcata accaggtctt cttactcaaa tcctgtgatg tttgaaataa 2040
   ccaaattgtc tctccaatgt ctgcataaac tgtgagagcc aagtcaacag cttttatcaa 2100
   gaatttactc tctgaccagc aataaacaag cactgagaga cacagagagc cagattcaga 2160
   ttttacccat ggggataaaa agactcagac tttcaccaca tttggaaaac tacttgcatc 2220
   ataaatatat aataactggt agtttatatg aagcagacac taagtgctat agacactctc 2280
   agaatatcat acttggaaac aatgtaatta aaatgccgaa tctgagtcaa cagctgccct 2340
   acttttcaat tcagatatac tagtacctta cctagaaata atgttaacct agggtgaagt 2400
   cactataatc tgtagtctat tatttgggca tttgctacat gatgagtgct gccagattgt 2460
   ggcaggtaaa gagacaatgt aatttgcact ccctatgata tttctacatt tttagcgacc 2520
   actagtggaa gacattcccc aaaattagaa aaaaaggaga tagaagattt ctgtctatgt 2580
   aaagttctca aaatttgttc taaattaata aaactatctt tgtgttcttt tctgcaacag 2640
   atgattccaa catgggtgtt tgtctattct tctttactct tgaaacatta gaccatggga 2700
   ggctcttaca gccttgagtt gatatttata caacccaaat ctaggtttga acggtgaggt 2760
   gtcaggtcat caaatattca tgtctatata gtcttacaca ggttctcaaa aaaaatgttc 2820
   atgggatagg tcattgataa tggattcctt attctgagaa ctccagacga ctgaaatata 2880
   tgagagaagg aaaaggacat agtaggagca ggcctgagaa aaaaatgaaa gtcagaaatc 2940
   tttaaaaaaa tacaagatct tatttctatc ttattttttc tcctcttctg aaatatatat 3000
   gaggattcct ctccaaaccc atggtttctc taagaatttt gagtcatttg tatgacctca 3060
   aataattagt tttagctgac ctcacataac tccttataat aggagacatc tttaatgtct 3120
   gctattaaag aaggatgaaa attcctatga ccttctcccc gattatccct ttggcaatat 3180
   agagtcaaat aataacattg accaatagta aacatgcttt gccaagaagt agaagatata 3240
   ttctctagcc ttagtttttc ctcccaattt gcatttttgt aaaaataatg ttgtatccac 3300
   aaaggaaata aactttaaaa acccaagtgc a 3331

<210> 39
<211> 50
<212> DNA
<213> homo sapiens

<400> 39
cgaggcccac ttcgaggccc tctccctcag taaattcaca ttttccttcc 50

<210> 40
<211> 1570
<212> DNA
<213> homo sapiens

<400> 40
   tgaggtcaga aggcggaacc gctgctggga gacggcggga tctctttcgc catggctgcc 60
   gggccgatct ccgagcggaa tcaggatgcc actgtgtacg tggggggcct ggatgagaag 120
   gttagtgaac cgctgctgtg ggaactgttt ctccaggctg gaccagtagt caacacccac 180
   atgccaaagg atagagtcac tggccagcac caaggctatg gctttgtgga attcttgagt 240
   gaggaagatg ctgactatgc cattaagatc atgaacatga tcaaactcta tgggaagcca 300
   atacgggtga acaaagcatc agctcacaac aaaaacctgg atgtaggggc caacattttc 360
   attgggaacc tggaccctga gattgatgag aagttgcttt atgatacttt cagcgccttt 420
   ggggtcatct tacaaacccc caaaattatg cgggaccctg acacaggcaa ctccaaaggt 480
   tatgccttta ttaattttgc ttcatttgat gcttcggatg cagcaattga agccatgaat 540
   gggcagtacc tctgtaaccg tcctatcacc gtatcttatg ccttcaagaa ggactccaag 600
   ggtgagcgcc atggctcagc agccgaacga cttctggcag ctcagaaccc gctctcccag 660
   gctgatcgcc ctcatcagct gtttgcagat gcacctcctc caccctctgc tcccaatcct 720
   gtggtatcat cattggggtc tgggcttcct ccaccaggca tgcctcctcc tggctccttc 780
   ccacccccag tgccacctcc tggagccctc ccacctggga tacccccagc catgccccca 840
   ccacctatgc ctcctggggc tgcaggacat ggccccccat cggcaggaac ccccagggca 900
   ggacatcctg gtcatggaca ctcacatcct cacccattcc caccgggtgg gatgccccat 960
   ccagggatgt ctcagatgca gcttgcacac catggccctc atggcttagg acatccccac 1020
   gctggacccc caggctctgg gggccagcca ccgccccgac caccacctgg aatgcctcat 1080
   cctggacctc ctccaatggg catgcccccc cgagggcctc cattcggatc tcccatgggt 1140
   cacccaggtc ctatgcctcc gcatggtatg cgtggacctc ctccactgat gcccccccat 1200
   ggatacactg gccctccacg acccccaccc tatggctacc agcgggggcc tctccctcca 1260
   cccagaccca ctccccggcc accagttccc cctcgaggcc cacttcgagg ccctctccct 1320
   cagtaaattc acattttcct tcctcctgtt acattttccc aatatctttt ctattccttg 1380
   gaccaatcag agatgctgta gctccttggg gcaaaggtac taatcccttt cagcaccccc 1440
   actccattcc cctttttaat gtaacttttt ccacaggagg tatttctttt ttatgttggt 1500
   cctgagtatt ttgcaaatgc acagagaaaa taaaactaaa ctccttgtta aaaaaaaaaa 1560
   aaaaaaaaaa 1570

<210> 41
<211> 50
<212> DNA
<213> homo sapiens

<400> 41
agaatcgctt gaacctggga ggcaggggtt gcagtgagct gagacggtgt 50

<210> 42
<211> 1532
<212> DNA
<213> homo sapiens

<400> 42
agcgcagcgg gggcgggaag gttgtagtgc cgcgagttga gctcctcttg cctaagtggt 60

cgcgccccct ttaagagcag cgattgtaag gagaggcggt cccggtgtcc tcgggtccca 120

ggtgattgtg aagtgctgac caattgccac tggacatact tgaaacaaaa taggaaaatg 180

gcagcaaact cttcaggaca aggttttcaa aacaaaaata gagttgcaat cttggcagaa 240

ctggacaaag agaaaagaaa actacttatg cagaaccagt cttcaacaaa tcatcctgga 300

gctagcattg cactctcgag accctctctt aataaggact tccgggatca cgctgagcag 360

cagcatattg cagcccaaca gaaggcagct ttgcagcatg ctcatgcaca ttcatctgga 420

tacttcatca ctcaagactc tgcatttggg aaccttattc ttcctgtttt acctcgcctt 480

gacccagaat gaagaaaaca tttgcgatgg aaaagtgact ttgtaatatc aaatgccaaa 540

gctactatca ttcagtgcta catgaactgt gactttaaga attttggtga actttgatat 600

tttttgtttg tctgaaagaa aggaatgtgt aagtgaaagc tgaaagaaga ataaccagga 660

tgatgagagc tgtggaagct gtatcgtcca aggaattgat tatgtaccgt gactgtaact 720

tttttgtaat gctgtttaac tctcaatcag actgtgaact ggatggtcac gaagtcattc 780

cccaactcct agcaagtttg actgaatata tcatgtccac agtagatttt caagaatcat 840

ttatagtact taactttaaa gaaacaaggc tgcttttaaa aaatgaacta ataggcttaa 900

atcaattgca tccatatttg ctgtttatag gattgctatc agtatacctt ttgcgtttat 960

agtcaacatg tatcatcctg aaatattctt tctggactta taactacttc cccctttttc 1020

actttaaaac aaacctcaag aataaattac taaccagtct taaccatctt ttataaacat 1080

atgctcttat aaatgttgtg actagatgca attaaaaata atagggaatg tggtaggttt 1140

ttaatttgta catcctctta tttagtgtta ccacataaat gatgagtttg tgtggttctg 1200

ttttccattt ttgttctaac tgaaaacttt ttggctgggc acggtgcctc atgcctgtaa 1260

tcccagcact ttgggaggcc aaggcgggca gatcacttga gatcaggagt ttgagaccag 1320

cctggccaac atggtgaaac cctgtctcta ctaaaagtat aaaaaattag ccatgtgtgg 1380

tggcacacgc ctgtaatccc agctactcag gaggctgagg caggagaatc gcttgaacct 1440

gggaggcagg ggttgcagtg agctgagacg gtgtcactgc actccagcct gggtgacagt 1500

gagtctttgt ctcaaagaaa aaaaaaaaaa aa 1532

<210> 43
<211> 50
<212> DNA
<213> homo sapiens

<400> 43
ccttactgta acggagttta gagccagggc tgatgctttg gtgtggccag 50

<210> 44
<211> 2974
<212> DNA
<213> homo sapiens

<400> 44
gtgtgcgtga tggagaaaat tgggcaccag ggctgctccc gagattctca gatctgattt 60

ccacgcttgc taccaaaata gtctgggcag gccacttttg gaagtaggcg ttatctagtg 120

agcaggcggc cgctttcgat ttcgctttcc cctaaatggc tgagcttctc gccagcgcag 180

gatcagcctg ttcctgggac tttccgagag ccccgccctc gttccctccc ccagccgcca 240

gtaggggagg actcggcggt acccggagct tcaggcccca ccggggcgcg gagagtccca 300

ggcccggccg ggaccgggac ggcgtccgag tgccaatggc tagctctagg tgtcccgctc 360

cccgcgggtg ccgctgcctc cccggagctt ctctcgcatg gctggggaca gtactgctac 420

ttctcgccga ctgggtgctg ctccggaccg cgctgccccg catattctcc ctgctggtgc 480

ccaccgcgct gccactgctc cgggtctggg cggtgggcct gagccgctgg gccgtgctct 540

ggctgggggc ctgcggggtc ctcagggcaa cggttggctc caagagcgaa aacgcaggtg 600

cccagggctg gctggctgct ttgaagccat tagctgcggc actgggcttg gccctgccgg 660

gacttgcctt gttccgagag ctgatctcat ggggagcccc cgggtccgcg gatagcacca 720

ggctactgca ctggggaagt caccctaccg ccttcgttgt cagttatgca gcggcactgc 780

ccgcagcagc cctgtggcac aaactcggga gcctctgggt gcccggcggt cagggcggct 840

ctggaaaccc tgtgcgtcgg cttctaggct gcctgggctc ggagacgcgc cgcctctcgc 900

tgttcctggt cctggtggtc ctctcctctc ttggggagat ggccattcca ttctttacgg 960

gccgcctcac tgactggatt ctacaagatg gctcagccga taccttcact cgaaacttaa 1020

ctctcatgtc cattctcacc atagccagtg cagtgctgga gttcgtgggt gacgggatct 1080

ataacaacac catgggccac gtgcacagcc acttgcaggg agaggtgttt ggggctgtcc 1140

tgcgccagga gacggagttt ttccaacaga accagacagg taacatcatg tctcgggtaa 1200

cagaggacac gtccaccctg agtgattctc tgagtgagaa tctgagctta tttctgtggt 1260

acctggtgcg aggcctatgt ctcttgggga tcatgctctg gggatcagtg tccctcacca 1320

tggtcaccct gatcaccctg cctctgcttt tccttctgcc caagaaggtg ggaaaatggt 1380

accagttgct ggaagtgcag gtgcgggaat ctctggcaaa gtccagccag gtggccattg 1440

aggctctgtc ggccatgcct acagttcgaa gctttgccaa cgaggagggc gaagcccaga 1500

agtttaggga aaagctgcaa gaaataaaga cactcaacca gaaggaggct gtggcctatg 1560

cagtcaactc ctggaccact agtatttcag gtatgctgct gaaagtggga atcctctaca 1620

ttggtgggca gctggtgacc agtggggctg taagcagtgg gaaccttgtc acatttgttc 1680

tctaccagat gcagttcacc caggctgtgg aggtactgct ctccatctac cccagagtac 1740

agaaggctgt gggctcctca gagaaaatat ttgagtacct ggaccgcacc cctcgctgcc 1800

cacccagtgg tctgttgact cccttacact tggagggcct tgtccagttc caagatgtct 1860

cctttgccta cccaaaccgc ccagatgtct tagtgctaca ggggctgaca ttcaccctac 1920

gccctggcga ggtgacggcg ctggtgggac ccaatgggtc tgggaagagc acagtggctg 1980

ccctgctgca gaatctgtac cagcccaccg ggggacagct gctgttggat gggaagcccc 2040

ttccccaata tgagcaccgc tacctgcaca ggcaggtggc tgcagtggga caagagccac 2100

aggtatttgg aagaagtctt caagaaaata ttgcctatgg cctgacccag aagccaacta 2160

tggaggaaat cacagctgct gcagtaaagt ctgggcccca tagtttcatc tctggactcc 2220

ctcagggcta tgacacagag gtagacgagg ctgggagcca gctgtcaggg ggtcagcgac 2280

aggcagtggc gttggcccga gcattgatcc ggaaaccgtg tgtacttatc ctggatgatg 2340

ccaccagtgc cctggatgca aacagccagt tacaggtgga gcagctcctg tacgaaagcc 2400

ctgagcggta ctcccgctca gtgcttctca tcacccagca cctcagcctg gtggagcagg 2460

ctgaccacat cctctttctg gaaggaggcg ctatccggga gggggaaccc caccagcagc 2520

tcatggagaa aaaggggtgc tactgggcca tggtgcaggc tcctgcagat gctccagaat 2580

gaaagccttc tcagacctgc gcactccatc tccctccctt ttcttctctc tgtggtggag 2640

aaccacagct gcagagtagg cagctgcctc caggatgagt tacttgaaat ttgccttgag 2700

tgtgttacct cctttccaag ctcctcgtga taatgcagac ttcctggagt acaaacacag 2760

gatttgtaat tccttactgt aacggagttt agagccaggg ctgatgcttt ggtgtggcca 2820

gcactctgaa actgagaaat gttcagaatg tacggaaaga tgatcagcta ttttcaacat 2880

aactgaaggc atatgctggc ccataaacac cctgtaggtt cttgatattt ataataaaat 2940

tggtgttttg taaaaaaaaa aaaaaaaaaa aaaa 2974

<210> 45
<211> 50
<212> DNA
<213> homo sapiens

<400> 45
gggtataatc atgactcaca aagatatgag gctgggtgtg gtggctcacg 50

<210> 46
<211> 5822
<212> DNA
<213> homo sapiens

<400> 46
gatcccaaag atgtgctcca caaatccagg caaatgggtc acctttgatg atgatcctgc 60

tgttcaatct tctcaaaagt caaagaattt tcctctggag aatcaaggtg tctgtagacc 120

aaatggactg aagctgaacc ttcctggcct cagggaattt cccagtggat cttcctccac 180

cagcagcact cctctctcct cccccattgt agatttttat ttcagtccag gacctccaag 240

taactctcct ctttctacac ctaccaaaga cttcccaggt tttcctggca tccccaaagc 300

agggactcat gtgctttatc ctattccaga atcatcttca gacagcccac tcgcaatatc 360

aggaggagaa tcttccttac tgcctaccag accaacatgt ttatcccatg ccttgttacc 420

cagtgaccac tcatgtacac atccaactcc caaagtaggt cttccagatg aagttaatcc 480

tcaacaggct gaaagcctag gattccaaag tgatgatctc ccccagtttc agtattttcg 540

agaggactgt gctttttcaa gtccattttg gaaagatgaa ggcagtgatt cccatttcac 600

ccttgaccca ccaggaagca aaaagatgtt ctcatcaaga aacaaggaga tgcctattga 660

ccaaaaaagc ctaaataagt gttcactcaa ctatatctgt gagaagcttg aacatctcca 720

gtcagctgag aaccaagact cacttagaag tttgtctatg cactgtctat gtgctgaaga 780

aaatgcctct tcctttgtcc cccacacact cttcaggagt cagccaaaat ccggatggtc 840

tttcatgctg agaattcctg agaagaagaa tatgatgtct tcccggcaat ggggaccaat 900

ttttctgaaa gttttgcctg gaggaatttt gcagatgtat tatgaacagg gattagaaaa 960

accatttaaa gagatacagc ttgatccata ttgtaggctt tctgaaccca aggttgagaa 1020

cttcagtgta gcaggaaaaa tccacactgt gaagattgaa catgtgtctt acacagaaaa 1080

aaggaaatac cattctaaga cagaagtagt tcatgaacct gacatagagc agatgctgaa 1140

gttggggtcc acatcgtacc atgacttcct tgactttctg actactgtgg aggaggagct 1200
gatgaagttg ccagctgttt caaaaccaaa aaagaactac gaggagcaag aaatttcctt 1260
ggaaattgtg gacaactttt ggggtaaagt cacaaaagaa ggaaaatttg ttgaaagtgc 1320
tgtgataact caaatttatt gcctctgctt tgtgaatggg aacctggaat gctttttaac 1380
cttgaatgac cttgagttgc cgaagcgaga tgaatcctat tatgagaagg actcagaaaa 1440
aaaggggatt gatattcttg actaccattt tcataagtgt gtgaatgtac aagaatttga 1500
gcaatcaaga atcattaagt ttgtacctct ggatgcctgc cggtttgagc tgatgcgttt 1560
caagactttg tataatgggg ataatcttcc cttttccttg aagtctgtag tggttgtcca 1620
gggagcatac gtggaacttc aggcttttgt caacatggcc tcattggcgc agaggtcatc 1680
ctatgctggt tccttaaggt cctgtgacaa tataaggata cactttcctg tcccatcgca 1740
gtggatcaag gccctttgga ccatgaacct ccagaggcag aagtctctga aagctaaaat 1800
gaaccgccga gcatgtctgg ggagtttaca ggaacttgaa tctgaacctg tcattcaagt 1860
cactgtgggg tcagcaaaat atgagagtgc ctaccaggca gtggtatgga agatagatcg 1920
gcttccagac aaaaattcaa gtctagatca tccccattgt ctgtcataca aattagagct 1980
tggatcagac caagaaattc cctctgattg gtatccattt gctactgttc agttttccgt 2040
gcctgacacc tgtgcctcaa ggacagaggt caggtctctg ggagtggaga gtgatgtcca 2100
gccacagaaa catgttcagc agcgagcttg ctacaacatc caggttgaaa tagaaaagaa 2160
gtggattaaa atcgatggag aagacccaga taaaattggt gactgcataa ctcagtagga 2220
gtagcaagag tttatgatga cagcccactt gtcaaatatg taattcaccg aaaccacacc 2280
aagtcctgct actgtagagt ggaaatgact tctgaatagc ggttttagga caggtctgat 2340
ggctgtgttt agagaagttt agacctaaaa ccgaacaatc tgtatttttt gcttttcatg 2400
tgtttttgtc ctaggggttc gatctaaaat gtttctataa ttcgtgtgat gttttgcttc 2460
ctattgaaac tcaaaggcac tgttactcgt tgtgtgaccc cgcagccagt atgatttttg 2520
attactcagt ggctgactgt tttgctctct ggattactga ggtgccgtct tcatttcttc 2580
ccatctcttc ttgctgctta gtgtctgtac tagagggtaa gggaatcaaa ggagacataa 2640
accaaaatag ttaattttcc ctctttcttg cctctgaaat gtggctaggt gtagaatgat 2700
gttgaaacca caggctaaaa tgtagatctg gaagtatctg tgcttttgaa ttacttattt 2760
acctgtcctc ttaccgttgg taaataataa ttggctatat ttggtacctg tctctctcct 2820
actgtattgt cattttcaaa atgtgtttgt tttctggcgc gtgctgcaaa gaagctattt 2880
ctgttgtcct acatatttta gtataaatca ctaagacata tttcctttca cttggcagga 2940
ttcctttcaa aatggaatct gagtattaga cactagttaa catatttgtg tatattaaat 3000

atgaattttt aaaatttata aatactattt tccaaaagta cagactctaa ggacatattt 3060

tgataaagta ttatttgtaa tgaacacaag cctctcttga gtagaagtct aaatcacatt 3120

atgattattt tatactagtt ctgctatcat gctgttttat gctaattctg cttattttag 3180

agtatttttc attaaaaggt gagcaaagtg aaagatactt ggtattttac ccagatttct 3240

aagtggcaac atttttattc ttcagagtca ggtaaatgac tataatagtt tggtttctaa 3300

tgaaaagtca atagcattag cagttataat tctgtttata atttcctttc agcatttaca 3360

gtgaaaagtg agaattttaa aatttatgaa atctatattc caggtattgt ttttagtctg 3420

aaaaacaaac tcccccatgt ggtattaata taccttcaat ttatgttgag tcttaaacat 3480

aattaggaga tatttttctc tatttgctag atttgttttg agtcaaaact gatttagtgt 3540

tcttccaaac aacatttatg tgttggccta cagagtttat ttcatgtgtt ttttttaata 3600

tttaatatta tattacattc attgaaatct gttcaaaaac agattaagac aaacatttat 3660

gatggtctgt atcaatcagc agattttatt gcttttcatt attttactgt aaaggcaaag 3720

aatgcaatag gtgatggttg gttgaaagga attgttattg ctgtttttat tttttacttt 3780

ttttgagaca gagtctcact ctgttgccca gcctggagtg cagtgtgtca tcttggctca 3840

ctgcaacctc cgcctcctgg gttcaaggga ttctcctgcc tcagcctcca actagctggg 3900

attacaggca caagccacca tgcctggcta aaattctttt gtatttttac tacagacgga 3960

gtttccccat gttggccggg ctagtctcga actcctgacc tcaggtgatc caccagcctc 4020

ggcctcccaa agttctggga ttaaatgcgt gagccaccat gcccggccgc tattgctctt 4080

tttaacttca tttgatgcct tgcttataat atcatatgct tgaggctcac tgttgatgta 4140

gagtagggca aatctgtgtg tgtatgtcat taaaaaaatt ctaccatctt tctttatcat 4200

ctggtgtggg cgcactctac agtgacttca gtctgctcag aacgaatgtg gaggccggcc 4260

gaaactgatg ctgcccacag tcccagtgaa gttaggtggg ttaattactg ccattccttt 4320

ctaagtgtgt tttatggcat cctgcccaca aagaactcac agtctgatta ggatgaactt 4380

aatgactatt tacacctcaa aatatattca gcaaagggtt cacttagttg cccctcatgc 4440

ttcacaggtt gactagtatc tgtgggtacc tccctgccca ggttattcac tcacaagcca 4500

ccgggccttc aggacatctc aagattcagt cttgacaata taatagcaaa agctggacta 4560

aagcccaaat ggattgtctg tggcaatgca gaagctgata gcattaacag cagggtgtta 4620

cagcataatt gttaattcgc acatctatta ggatccttaa atattcatta cttaatgtta 4680

aattaacatt ctgtgtaggg aggggaggct tattcaattc ttctgacctc agaactggca 4740

gaaggtcaga tgtgactaca gaactctagg tgaaaaatca ggtagggttc aaattaagta 4800

gaactgccct tgccggaata gtgatcttca aaaaaccctt gcttttaggg gagggaagcg 4860

gggaaggaaa ggaatgaggg aggaaatatt cctttctagc attcattttg cttagatcac 4920

tccattgtga gtttgaccat tttggagtca aatgagcaga cttccaagga gttgaccagt 4980

ttgtgactag tctggtcacc tttccagtta caggatcata ttaactgtgt aaatgaattc 5040

atgggtagat gatttgtgca gatctgaatt taagaacatt tcctttttct gtgggaatca 5100

caagagttta tccactaaaa aaagatatgt aaaaaagata ctttccagca cataaataaa 5160

ggctggaatt ttacaacctg atgtatatat tagacagttc taggatgtta gttcccttca 5220

ttccagagct atgcttgtga tacagcccct tttcttataa agtcagttag aaggacttcc 5280

ttaacaatga ctattataat gtcttactta aaatacagtt ttgtattctg tcaatgcaaa 5340

tataagacag gttgagcctt aatcatgtaa caaaatattt tgtagattac atattgattt 5400

ttcaaaaatt aaaaatgtat ttcaaactat tcttactgtg ctgtgaattt atatcttctt 5460

ctgcaagacc tggggctatt tttttttctt aaggtatgaa atcaatttca ataaccttaa 5520

acaattgacc ctctgtcagg catatgtcag gctccactct taaggagtca gtgaccatga 5580

cctctttgca tatcctggaa aaatgacatc atcaaagtat agttgataat gttataaagg 5640

atgattttaa aaatagggta taatcatgac tcacaaagat atgaggctgg gtgtggtggc 5700

tcacgcctgt aatcccagct ctttgggagg ctgaagcggg tggatcatga ggtcagaaga 5760

tcaagaccat actggctaac acggtgaaac cccgtctcta ctaaaaatac aaaaaaaaaa 5820

aa 5822

<210> 47
<211> 50
<212> DNA
<213> homo sapiens

<400> 47
agccattgcc cagtggtcca tatctccacc acatcccctg cttgagccca 50

<210> 48
<211> 2053
<212> DNA
<213> homo sapiens

<400> 48
caggcagcag gcagcaggcg ggcgcgctgt ggctccgcgc cgcgcggtcc gggctctgtt 60

cattcatgat tggtactcgg ccctccgaga cccagcccga gcgcagggag gggagccgag 120

tgtgcggcag gaggggcggg cggacggcgg ctcccgcacc gcacgcggcg ctggctcggc 180

agcctcggcc gggcggccgc tctggccccg tgtccagtgc caggcaggct tcagggcacc 240

gtcctcggcc ctgggcgagg gaaccgccgg gccgggtcct cgcgcgggga agcggttccg 300

aaggctcgcg gggagcggct agccctgagt ccctgcatgt gcggggctga agaaggaagc 360

cagaagcctc ctagcctcgc ctccacgctt gctgaatacc aagctgcagg cgagctgccg 420

ggcgcttttc tctcctccaa ttcagagtag acaaaccacg ggatttcttt tccagggtag 480

gggaggggcc gggcccgggg tcccaactcg cactcaagtc ttcgctgcca tgggggccgt 540

catgggcacc ttctcatctc tgcaaaccaa acaaaggcga ccctcgaaag acatcgcctg 600

gtggtattac cagtatcaga gagataagat tgaagatgag ctggagatga ccatggtttg 660

ccatcggccc gagggactgg agcagctcga ggcccagacc aacttcacca agagggagct 720

gcaggtcctt tatcgaggct tcaaaaatga gtgccccagt ggtgtggtca acgaagacac 780

attcaagcag atctatgctc agtttttccc tcatggagat gccagcacgt atgcccatta 840

cctcttcaat gccttcgaca ccactcagac aggctccgtg aagttcgagg actttgtaac 900

cgctctgtcg attttattga gaggaactgt ccacgagaaa ctaaggtgga catttaattt 960

gtatgacatc aacaaggacg gatacataaa caaagaggag atgatggaca ttgtcaaagc 1020

catctatgac atgatgggga aatacacata tcctgtgctc aaagaggaca ctccaaggca 1080

gcatgtggac gtcttcttcc agaaaatgga caaaaataaa gatggcatcg taactttaga 1140

tgaatttctt gaatcatgtc aggaggacga caacatcatg aggtctctcc agctgtttca 1200

aaatgtcatg taactggtga cactcagcca ttcagctctc agagacattg tactaaacaa 1260

ccaccttaac accctgatct gcccttgttc tgattttaca caccaactct tgggacagaa 1320

acacctttta cactttggaa gaattctctg ctgaagactt tctatggaac ccagcatcat 1380

gtggctcagt ctctgattgc caactcttcc tctttcttct tcttgagaga gacaagatga 1440

aatttgagtt tgttttggaa gcatgctcat ctcctcacac tgctgcccta tggaaggtcc 1500

ctctgcttaa gcttaaacag tagtgcacaa aatatgctgc ttacgtgccc ccagcccact 1560

gcctccaagt caggcagacc ttggtgaatc tggaagcaag aggacctgag ccagatgcac 1620

accatctctg atggcctccc aaaccaatgt gcctgtttct cttcctttgg tgggaagaat 1680

gagagttatc cagaacaatt aggatctgtc atgaccagat tgggagagcc agcacctaac 1740

atatgtggga taggactgaa ttattaagca tgatattgtc tgatgaccca aactgcccat 1800

gtcatttgtt tccagaaacg aggaccaata attctctcac actggcattt gtgctggtag 1860

tacaagtcct ttaatatgtc caggaaggga gccattgccc agtggtccat atctccacca 1920

catcccctgc ttgagcccag cgctgcatgt ccctcccaag aagtccagaa tgcctgcaaa 1980

ttgctgtaat tttataccat gttctaacca ataaacagaa ctatttctta cactctcaaa 2040

aaaaaaaaaa aaa 2053

<210> 49
<211> 50
<212> DNA
<213> homo sapiens

<400> 49

gcacctagcc tcgctcttgg caggtactca gcgaatgaat gctgtatatg          50

<210>  50
<211>  1760
<212>  DNA
<213>  homo sapiens

<400>  50
   atttctttat aaaccacaac tctgggcccg caatggcagt ccactgcctt gctgcagtca      60
   cagaatggaa atctgcagag gcctccgcag tcacctaatc actctcctcc tcttcctgtt     120
   ccattcagag acgatctgcc gaccctctgg gagaaaatcc agcaagatgc aagccttcag     180
   aatctgggat gttaaccaga agaccttcta tctgaggaac aaccaactag ttgctggata     240
   cttgcaagga ccaaatgtca atttagaaga aaagatagat gtggtaccca ttgagcctca     300
   tgctctgttc ttgggaatcc atggagggaa gatgtgcctg tcctgtgtca agtctggtga     360
   tgagaccaga ctccagctgg aggcagttaa catcactgac ctgagcgaga acagaaagca     420
   ggacaagcgc ttcgccttca tccgctcaga cagcggcccc accaccagtt ttgagtctgc     480
   cgcctgcccc ggttggttcc tctgcacagc gatggaagct gaccagcccg tcagcctcac     540
   caatatgcct gacgaaggcg tcatggtcac caaattctac ttccaggagg acgagtagta     600
   ctgcccaggc ctgcctgttc ccattcttgc atggcaagga ctgcagggac tgccagtccc     660
   cctgccccag ggctcccggc tatgggggca ctgaggacca gccattgagg ggtggaccct     720
   cagaaggcgt cacaagaacc tggtcacagg actctgcctc ctcttcaact gaccagcctc     780
   catgctgcct ccagaatggt ctttctaatg tgtgaatcag agcacagcag cccctgcaca     840
   aagcccttcc atgtcgcctc tgcattcagg atcaaacccc gaccacctgc ccaacctgct     900
   ctcctcttgc cactgcctct tcctccctca ttccaccttc ccatgccctg gatccatcag     960
   gccacttgat gacccccaac caagtggctc ccacaccctg ttttacaaaa aagaaaagac    1020
   cagtccatga gggaggtttt taagggtttg tggaaaatga aaattaggat ttcatgattt    1080
   ttttttttca gtccccgtga aggagagccc ttcatttgga gattatgttc tttcggggag    1140
   aggctgagga cttaaaatat tcctgcattt gtgaaatgat ggtgaaagta agtggtagct    1200
   tttcccttct ttttcttctt tttttgtgat gtcccaactt gtaaaaatta aaagttatgg    1260
   tactatgtta gccccataat tttttttttc cttttaaaac acttccataa tctggactcc    1320
   tctgtccagg cactgctgcc cagcctccaa gctccatctc cactccagat tttttacagc    1380
   tgcctgcagt actttacctc ctatcagaag tttctcagct cccaaggctc tgagcaaatg    1440
   tggctcctgg gggttctttc ttcctctgct gaaggaataa attgctcctt gacattgtag    1500
   agcttctggc acttggagac ttgtatgaaa gatggctgtg cctctgcctg tctcccccac    1560
   cgggctggga gctctgcaga gcaggaaaca tgactcgtat atgtctcagg tccctgcagg    1620
   gccaagcacc tagcctcgct cttggcaggt actcagcgaa tgaatgctgt atatgttggg 1680
   tgcaaagttc cctacttcct gtgacttcag ctctgtttta caataaaatc ttgaaaatgc 1740
   ctaaaaaaaa aaaaaaaaaa 1760

<210> 51
<211> 50
<212> DNA
<213> homo sapiens

<400> 51
ggctaatgcc ctggcccaca agtatcacta agctcgcttt cttgctgtcc 50

<210> 52
<211> 626
<212> DNA
<213> homo sapiens

<400> 52
acatttgctt ctgacacaac tgtgttcact agcaacctca aacagacacc atggtgcatc 60

tgactcctga ggagaagtct gccgttactg ccctgtgggg caaggtgaac gtggatgaag 120

ttggtggtga ggccctgggc aggctgctgg tggtctaccc ttggacccag aggttctttg 180

agtcctttgg ggatctgtcc actcctgatg ctgttatggg caaccctaag gtgaaggctc 240

atggcaagaa agtgctcggt gcctttagtg atggcctggc tcacctggac aacctcaagg 300

gcacctttgc cacactgagt gagctgcact gtgacaagct gcacgtggat cctgagaact 360

tcaggctcct gggcaacgtg ctggtctgtg tgctggccca tcactttggc aaagaattca 420

ccccaccagt gcaggctgcc tatcagaaag tggtggctgg tgtggctaat gccctggccc 480

acaagtatca ctaagctcgc tttcttgctg tccaatttct attaaaggtt cctttgttcc 540

ctaagtccaa ctactaaact gggggatatt atgaagggcc ttgagcatct ggattctgcc 600

taataaaaaa catttatttt cattgc 626

<210> 53
<211> 50
<212> DNA
<213> homo sapiens

<400> 53
gctgagtgag gggaaattta tagagctgtg tgactttagg gagtaggttg 50

<210> 54
<211> 3009
<212> DNA
<213> homo sapiens

<400> 54
gtttcccgct ggcgcctggc tccctccggg tttcgtttcc cgccggcgcc tggctcccgc 60

caggtttcgt ttccgaggcg gggccgaggg cggcgtcgct gaggcgccca tggccttcgc 120

ccgccggctc ctgcgcgggc cactgtcggg gccgctgctc gggcggcgcg gggtctgcgc 180

tggggccatg gctccgccgc gccgcttcgt cctggagctt cccgactgca ccctggctca 240

cttcgcccta ggcgccgacg cccccggcga cgcagacgcc cccgaccccc gcctggcggc 300

gctgctgggg cccccggagc gcagctactc gctgtgcgtg cccgtgaccc cggacgccgg 360

ctgcggggcc cgggtccggg cggcgcggct gcaccagcgc ctgctgcacc agctgcgccg 420

cggccccttc cagcggtgcc agctgctcag gctgctctgc tactgcccgg gcggccaggc 480

cggcggcgca cagcaaggct tcctgctgcg cgaccccctg gatgaccctg acacccggca 540

agcgctgctc gagctgctgg gcgcctgtca ggaggcacca cgcccgcact tgggcgagtt 600

cgaggccgac ccgcgcggcc agctgtggca gcgcctctgg gaggtgcaag acggcaggcg 660

gctgcaggtg ggctgcgcac aggtcgtgcc cgtcccggag cccccgctgc acccggtggt 720

gccagacttg cccagttccg tggtcttccc ggaccgggaa gccgcccggg ccgttttgga 780

ggagtgtacc tcctttattc ctgaagcccg ggcagtgctt gacctggtcg accagtgccc 840

aaaacagatc cagaaaggaa agttccaggt tgttgccatc gaaggactgg atgccacggg 900

taaaaccacg gtgacccagt cagtggcaga ttcacttaag gctgtcctct taaagtcacc 960

accctcttgc attggccagt ggaggaagat ctttgatgat gaaccaacta tcattagaag 1020

agctttttac tctttgggca attatattgt ggcctccgaa atagctaaag aatctgccaa 1080

atctcctgtg attgtagaca ggtactggca cagcacggcc acctatgcca tagccactga 1140

ggtgagtggg ggtctccagc acctgccccc agcccatcac cctgtgtacc agtggccaga 1200

ggacctgctc aaacctgacc ttatcctgct gctcactgtg agtcctgagg agaggttgca 1260

gaggctgcag ggccggggca tggagaagac cagggaagaa gcagaacttg aggccaacag 1320

tgtgtttcgt caaaaggtag aaatgtccta ccagcggatg gagaatcctg gctgccatgt 1380

ggttgatgcc agcccctcca gagaaaaggt cctgcagacg gtattaagcc taatccagaa 1440

tagttttagt gaaccgtagt tactctggcc aggtgccacg tctaactaga ttagatgttg 1500

tttgaaacat ctacatccac catttgttat gcagtgttcc caaatttctg ttctacaagc 1560

atgttgtgtg gcagaaaact ggagaccagg catcttaatt ttacttcagc catcgtaccc 1620

tcttctgact gatggacccg tcatcacaaa ggtccctctc atcatgttcc agtgagaggc 1680

cagcgattgc tttcttcctg gcatagtaaa cattttcttg gaacatatgt ttcacttaat 1740

cactaccaaa tatctggaag acctgtctta ctcagacagc accaggtgta cagaagcagc 1800

agacaagatc ttccagatca gcagggagac cccggagcct ctgcttctcc tacactggca 1860

tgctgatgag atcgtgacat gcccacattg gcttcttcca catctggttg cactcgtcat 1920

gatgggctcg ctgcatctcc ctcagtccca aattctagag ccaagtgttc ctgcagaggc 1980

tgtctatgtg tcctggctgc ccaaggacac tcctgcagag ccatttttgg gtaaggaaca 2040

cttacaaaga aggcattgat cttgtgtctg aggctcagag cccttttgat aggcttctga 2100

gtcatatata aagacattca agccaagatg ctccaactgc aaatatacca accttctctg 2160

aattatattt tgcttattta tatttctttt ctttttttct aaagtatggc tctgaataga 2220

atgcacattt tccattgaac tggatgcatt tcatttagcc aatccagtaa tttatttata 2280

ttaatctata cataatatgt ttcctcagca taggagctat gattcattaa ttaaaagtgg 2340

agtcaaaacg ctaaatgcaa tgtttgttgt gtattttcat tacacaaact taatttgtct 2400

tgttaaataa gtacagtgga tcttggagtg ggatttcttg gtaaattatc ttgcacttga 2460

atgtctcatg attacatatg aaatcgcttt gacatatctt tagacagaaa aaagtagctg 2520

agtgaggggg aaattataga gctgtgtgac tttagggagt aggttgaacc aggtgattac 2580

ctaaaattcc ttccagttca aaggcagata aatctgtaaa ttattttatc ctatctacca 2640

tttcttaaga agacattact ccaaaataat taaatttaag gctttatcag gtctgcatat 2700

agaatcttaa attctaataa agtttcatgt taatgtcata ggatttttaa aagagctata 2760

ggtaatttct atataatatg tgtatattaa aatgtaattg atttcagttg aaagtatttt 2820

aaagctgata aatagcatta gggttctttg caatgtggta tctagctgta ttattggttt 2880

tatttacttt aaacattttg aaaagcttat actggcagcc tagaaaaaca aacaattaat 2940

gtatctttat gtccctggca catgaataaa ctttgctgtg gtttactaaa aaaaaaaaaa 3000

aaaaaaaaa 3009

<210> 55
<211> 50
<212> DNA
<213> homo sapiens

<400> 55
ggcaaggttc tgcttagctg gaagctggaa agggttttca aagactgggc 50

<210> 56
<211> 677
<212> DNA
<213> homo sapiens

<400> 56
ctttgatgtc acaatctgac ctgtgggtat taatctggtc acagctgctc acagactaga 60

tggcaaggtc caggggacta gcacaggaag atgtggaaga gaagtgacca tcaacccaag 120

atcaaagcag aggatggacc tctggtgggc cagttcgagg ttctgggttc agttccagaa 180

cctgccatgc cacatcctct agagctgtca gaatttgaga gcttcccagt gtttcaggac 240

attaggcttc acatcaggga agtgggagct caattggtca agaaagtcaa tgccgtcttt 300

cagctggaca tcaccaaaaa tgggaagacc attttgcggt ggaccattga tctgaagaat 360

ggttctgggg acatgtatcc gggacctgcc aggctcccag cagacactgt ctttacaatc 420

ccggagtctg tctttatgga gctggttttg ggcaaaatga acccgcagaa ggctttcctt 480

gccggaaagt tcaaagtgag tggcaaggtt ctgcttagct ggaagctgga aagggttttc 540

aaagactggg ctaaatttta agcatgcaaa aataccaagg aagaacttct cttcgatgat 600

aatgtcgagt ggaaatcatg cttaatctga agattaaagt aaaaagtatc caatattttt 660

actcaaaaaa aaaaaaa 677

<210> 57
<211> 50
<212> DNA
<213> homo sapiens

<400> 57
aatgaggaca gagaacctca ggtgttctta tgctagtgct tgctgagtgc 50

<210> 58
<211> 4848
<212> DNA
<213> homo sapiens

<400> 58
gactgcgcgg cgggctgtgg ctgcggaggt tgaggggcgt ccgaggcgcg gaggggctgg 60

ctgggcagga ggggttggcg gggcagcagg gccgcggcca tggggagctt gaaggaggag 120

ctgctcaaag ccatctggca cgccttcacc gcactcgacc aggaccacag cggcaaggtc 180

tccaagtccc agctcaaggt cctttcccat aacctgtgca cggtgctgaa ggttcctcat 240

gacccagttg cccttgaaga gcacttcagg gatgatgatg agggtccagt gtccaaccag 300

ggctacatgc cttatttaaa caggttcatt ttggaaaagg tccaagacaa ctttgacaag 360

attgaattca ataggatgtg ttggaccctc tgtgtcaaaa aaaacctcac aaagaatccc 420

ctgctcatta cagaagaaga tgcatttaaa atatgggtta ttttcaactt tttatctgag 480

gacaagtatc cattaattat tgtgtcagaa gagattgaat acctgcttaa gaagcttaca 540

gaagctatgg gaggaggttg gcagcaagaa caatttgaac attataaaat caactttgat 600

gacagtaaaa atggcctttc tgcatgggaa cttattgagc ttattggaaa tggacagttt 660

agcaaaggca tggaccggca gactgtgtct atggcaatta atgaagtctt taatgaactt 720

atattagatg tgttaaagca gggttacatg atgaaaaagg gccacagacg gaaaaactgg 780

actgaaagat ggtttgtact aaaacccaac ataatttctt actatgtgag tgaggatctg 840

aaggataaga aaggagacat tctcttggat gaaaattgct gtgtagagtc cttgcctgac 900

aaagatggaa agaaatgcct ttttctcgta aaatgttttg ataagacttt tgaaatcagt 960

gcttcagata agaagaagaa acaggagtgg attcaagcca ttcattctac tattcatctg 1020

ttgaagctgg gcagccctcc accacacaaa gaagcccgcc agcgtcggaa agaactccgg 1080

aagaagcagc tggctgaaca agaggaactg gagcgacaaa tgaaggaact ccaggccgcc 1140

aacgaaagca agcagcagga gctggaggcc gtgcggaaga aactggagga agcagcatct 1200

cgtgcagcag aagaggaaaa gaaacgcctt cagactcaag tggaacttca ggccaggttc 1260

agcacagagc tggaaagaga gaagcttatc agacagcaga tggaagaaca ggttgctcaa 1320

aagtcctctg aactggaaca gtatttacag cgagtacggg agctggaaga catgtaccta 1380

aagctgcagg aggctcttga agatgagaga caggcccggc aagatgaaga gacagtgcgg 1440

aagcttcagg ccaggttgtt ggaggaagag tcttccaaga gggctgaact agaaaagtgg 1500

cacttggagc agcagcaggc cattcagaca accgaggcgg agaagcagga gttggagaat 1560

cagcgtgtcc tgaaggaaca ggccctgcag gaggccatgg agcagctgga gcagcttgag 1620

ttagaacgga agcaagcact tgagcagtac gaggaagtta aaaagaagct ggagatggca 1680

actaataaga ccaagagctg gaaggacaaa gtggcccatc atgaaggatt aattcgactg 1740

atagaaccag gttcaaagaa ccctcacctg atcactaact ggggacctgc agctttcact 1800

gaggcagaac ttgaagagag agagaagaac tggaaagaga aaaagaccac ggagtgactg 1860

agcttgctgg cagtcacgtc agttatgtag atactgcatg gcaggagagc tttacgctaa 1920

agacaaaaga aacagctttg ggggccgggc gtggtggctc acgcctgtaa tcccagcact 1980

ttgggaggcc gaggcgggtg gatcacctga ggtcaggagt tcaagagcag cctggccaac 2040

ctggtgaaac cctgtctcta ctaaaaatac aaaaaaaatt agctgagcgt ggtggcgggc 2100

gcctgtaatc ccagctactt gggaggctga ggcaggagaa tcacttgaac gtgggaggcg 2160

gaggttgcag cgagctgaga tcatgccgtt gtactccagc ttgggcaaca gagtgagact 2220

ccatctcaaa acaaaacaaa acaaaacaaa acaaaaaaac ccggctttgc tgcttttaac 2280

tcttcttcct tctgtgcctc tctaagtggg tcagtatcct aaggaagcct tcttatttat 2340

cttcctgcaa acaaggttta cctgaaaaga aaaaaaaagt caacattgtc aagctgtttg 2400

tttactcttt ctttgaaaac atcaccttct gaaatttgtc ttttagctct ctcagattct 2460

tccccaaatg aggcagggtg cagacagcac agtcagctct gcagagtttg gaggggctca 2520

ctgccactgg gtactcagaa cctctgtgga ctggatgtca gctctttcct ttggcagcgt 2580

gtttcctttt ccgagtatgt gctgttaaac tagattggcc ggttcgcttt ccatttcctg 2640

acacttgaca tggaatgcct ttgaccattg gtgctctgac agagaagtca tggagtcatt 2700

gccatttcct ggttgccctt ttggaatgtg atcctgttag tagaggtttt ctagcttcta 2760

ctaagatatt tctttcccta accatcatac acttggcatg tttcattccc atctcctttc 2820

ccctcacctt aaaggagact acccctttgc cccatattgt caacctaatt ttctctcgta 2880

ctctctctag tgaatgatgt gctaccaagt atatgccagg ctgtgagagg attatactga 2940

gtagtagaaa gaagctaatt tgaaataaaa attatttgta taattaagaa agcagattag 3000

atgcacatgg tcaacaggaa gttgactgta tgtctgctag ttagattcaa aacatcataa 3060

agatgatagc atgtcaatat attagcctag ccattatgtt agcctttgtt aggtgggcag 3120

cttttctgct ttttcccttc ctctgtggtg acaacggagg aaatatccaa cagaaatacg 3180

tctaacaggg aaattgggat catagtttat atgcatctga tttgaaagga gtattgagga 3240

aggttttcat atatgatcta tctttggatt aaaaagaaca tttatgaaat caagccttct 3300

aacactagtt ataattgaga agcaacagta actccgtgga cagcaatcaa gcttaaaatt 3360

gtaaataaat atggggataa ttcagttgtt gcaaaaaaag ggcagaattc agtagaataa 3420

agtccttttc tcttacaggt attaaatgag gacagagaac ctcaggtgtt cttatgctag 3480

tgcttgctga gtgcatacta agaaagcaat tccaaataga tgtatacatc tagagagagt 3540

ggtattagag attcagtgta tgtatttatt tacatgagag gaaactggaa tataatccca 3600

taaattatca ccttttatga ctggaaaata tttgccaatg aagaaatggt ctgtaggtat 3660

ttgtcttaag atttttggct gtttaataaa aatgtaactt taacggtttc ttatagttgc 3720

ctttataaag tgtattgtct aaaatatttt tgtatcatgt gcctttgaaa tttgacagct 3780

gatttgggtg ttggatttct gcccagccat ttatcagtat tatcatttta ttcagtagct 3840

ggcaggtgta ttagaccaac gagacttagg taaggaatgg aacctttcct gtggtttgac 3900

tgcacattac accagaagac tccagtatcc ctcattccag aatgaggaaa aagtattcta 3960

caaagaacct aatcacctct gtgaaatcta tgggatggaa acagtgtggc cttaggagtc 4020

aaatagtctc tgcatggtgg ggaggatcat gatggaatat gtgaatttct acttctagaa 4080

gttgtgaaat aggtcctgca cttttgcaga atgtccttct ttaaacctgg cttattccac 4140

agctgtagct gataacatga cctggggctt agctgctcta gccctgggtt cttggagacc 4200

tcacactgcc tggcccctgg ccatccacct aaggactgcc tgctttctgg tcacatgtgg 4260

accttgatac gactaagcgg ttacatatgt ggttgtgcaa aagctttctg tttaatgcat 4320

agtgttaccg atttacatct tggttttcag tggcactatg tctaggaggc aatatccttt 4380

taaacagtgc tttggctaag atagatactt gtgaatcaaa gatagcacag aaatgaacta 4440

agtatatccc atttggaatt atattttgat actatttaaa atggtttcac ctgttaaagg 4500

gccaacagaa ctcttggttt tacttttgta attactgtac agaaaatttc aagagtgttt 4560

gagtgcttgt catcaggtgt tttccttaat aagtagggat atgatcattt acaggaatta 4620

tatatgaaaa aagtttttga aatgtatttt tgtgatgtgc tatgttgagg ggaaaccaaa 4680

tatttatgat tttaaaacat tcgtatgaaa acattgtaca atgtaatatg ctcaactttc 4740

tcaatttttt gctaattttt ctaagataca ttaaaaatgt tttatatttt tttttaagta 4800

aaatggaccc agtaagaaaa ttaaaaatac cagaacatac actttaaa 4848

<210> 59
<211> 50
<212> DNA
<213> homo sapiens

<400> 59
gttaaatgcg gtgtagcaaa gttatggggt ctgcttgagg gcactaacct 50

<210> 60
<211> 3455
<212> DNA
<213> homo sapiens

<400> 60
aaaccaaagc ggccttttga gaagggatgt aagagcaggc ccctgagcag tttcacttcc 60

tctgggcgcc cagcagagcg gagccctggc cttgcgggca tgagccccgg ggctgcgttt 120

cctgagacct ggccgcagcc tccgcgggtg gcaagcgggc tggggagagc cgagggccaa 180

aggaagagaa aatcgcgggg agtctctggc cgggagagtc caggtagcgc tcggcgggca 240

gcagtgcgca ggcccctcgg cttcaaccgc cacaatgctg ccagcagcgc caggcaaggg 300

gcttgggagc ccggaccccg ccccctgcgg cccagcgccc ccaggaaata caaaagatat 360

aataatgata tatgaagaag atgctgagga atgggctctg tacttgacag aagtattttt 420

acatgttgtg aaaagggaag ccatcctgtt atatcgcttg gagaatttct cttttcggca 480

tttggagttg ctgaacttaa cgtcttacaa atgtaaactt ttgatattat caaatagcct 540

gcttagagac ctaactccaa agaaatgtca gtttctggaa aagatacttc attcaccaaa 600

aagtgtagtt actttgcttt gtggagtgaa gagttcagat cagctctatg aattactaaa 660

tatctctcaa agcagatggg agatctcaac tgaacaggaa cctgaagact acatctctgt 720

aatccagagt atcatattca aagattctga agactacttt gaggtcaaca ttccaacaga 780

cctacgagca aaacattctg gggaaataag tgagagaaag gaaattgaag aactatcaga 840

agcttcaaga aacaccatac cactagcagt ggtgcttccc actgaaattc catgtgagaa 900

tcctggtgaa atattcataa ttttgagaga tgaagtaatt ggtgatactg tagaggttga 960

atttacatca agtaataagc gcattagaac acggccagcc ctttggaata agaaagtctg 1020

gtgcatgaaa gctttagagt ttcctgctgg ttcagtccat gtcaatgtct actgtgatgg 1080

aatcgttaaa gctacaacca aaattaagta ctacccaaca gcaaaggcaa aggaatgcct 1140

attcagaatg gcagattcag gagagagttt gtgccagaat agcattgaag aacttgatgg 1200

tgtccttaca tccatattca aacatgagat accatattat gagttccagt ctcttcaaac 1260

tgaaatttgt tctcaaaaca aatatactca tttcaaagaa cttccaactc ttctccactg 1320

tgcagcaaaa tttggcttaa agaacctggc tattcatttg cttcaatgtt caggagcaac 1380

ctgggcatct aagatgaaaa atatggaggg ttcagacccc gcacatattg ctgaaaggca 1440

tggtcacaaa gaactcaaga aaatcttcga agacttttca atccaagaaa ttgacataaa 1500

taatgagcaa gaaaatgatt atgaagagga tattgcctca ttttccacat atattccttc 1560

cacacagaac ccagcatttc atcatgaaag caggaagaca tacgggcaga gtgcagatgg 1620

agctgaggca aatgaaatgg aaggggaagg aaaacagaat ggatcaggca tggagaccaa 1680

acacagccca ctagaggttg gcagtgagag ttctgaagac cagtatgatg acttgtatgt 1740

gttcattcct ggtgctgatc cagaaaataa ttcacaagag ccactcatga gcagcagacc 1800

tcctctcccc ccgccgcgac ctgtagctaa tgccttccaa ctggaaagac ctcacttcac 1860

cttaccaggg acaatggtgg aaggccaaat ggaaagaagt caaaactggg gtcatcctgg 1920

tgttagacaa gaaacaggag atgaacccaa aggagaaaaa gagaagaaag aagaggaaaa 1980

agagcaggag gaggaagaag acccatatac ttttgctgag attgatgaca gtgaatatga 2040

catgatattg gccaatctga gtataaagaa aaaaactggg agtcggtctt tcattataaa 2100

tagacctcct gcccccacac cccgacccac aagtatacct ccaaaagagg aaactacacc 2160

ttacatagct caagtgtttc aacaaaagac agccagaaga caatctgatg atgacaagtt 2220

ctgtggtctt cctaagaaac aagacagagc tcggatagag agtccagcct tttctactct 2280

caggggctgt ctaactgatg gtcaggaaga actcatcctc ctgcaggaga aagtaaagaa 2340

tgggaaaatg tctatggatg aagctctgga gaaatttaaa cactggcaga tgggaaaaag 2400

tggcctggaa atgattcagc aggagaaatt acgacaacta cgagactgca ttattgggaa 2460

aaggccagaa gaagaaaatg tctataataa actcaccatt gtgcaccatc caggtggtaa 2520

ggaaactgcc cacaatgaaa ataagtttta taatgtacac ttcagcaata agcttcctgc 2580

tcgaccccaa gttgaaaagg aatttggttt ctgttgcaag aaagatcatt aaagaaggtt 2640

attataatga aactcacgaa tctacggaca ttttgctttc agggtgaagc aagcttgaat 2700

ttggattgcc tgctctcttt aaagcgaatt catactatga cagcagaaac aaaacttcag 2760

atttcagaat ttgttattgg caaaatttat tctcattata cctgcttcat atgggtatat 2820

tactattaaa acagaatacc atagagtaat tgcattattt gaaaattctc tcattttaca 2880

atgcacttca ccaatgaaac agctaatttc cattttgaaa attaaaagaa aacagcacag 2940

agaagttaaa tgcggtgtag caaagttatg gggtctgctt gagggcacta acctcaacag 3000

attattcctc ctctccttag aataaccatg aaaatacaaa tttacttagc acatttttgc 3060

tttttaagta gctggttcat tttctgaatt tctcacattc agagttccag tcattattgt 3120

tacatcatgt ttgcagaaac cttgtcttat ttagtgtcta tttgcatata accctgaaaa 3180

cattattatt tgaaaacttt tctatatctc aaattaatat acattttcat aacctacctt 3240

tgtattaaga cttgcaattt tatcaatcta ttatttctta gaaacaattt actagcttag 3300

aatagaaagc aatgttatcg tcatataatt ttcatgtaca aatgccacaa ataaattgaa 3360

tgtttaaagc tatgtctgag tttttaaagt aaatttataa gaattagcca ataaaattgc 3420

ttctcggcct tttggctaag atcaaaaaaa aaaaa 3455

<210> 61
<211> 50
<212> DNA
<213> homo sapiens

<400> 61
ccagcttgca cctccagttt gcacagggat ttgttctggg ggctgagggc 50

<210> 62
<211> 4234
<212> DNA
<213> homo sapiens

<400> 62
cgtgagcggc gcagcaagat cccagctcgg accccggacg gcgcgcgccc ccgaagcccc 60

ggatcccagt cgggcccgca gctgaccgcc agattactgt gcatcccgaa tcacgaccac 120

ctgcaccctc ctgccccggc ccgcccccca agtcctcagg cacccagctc cccggcgccc 180

cggatcctcc tggaccggtc cgtccagatt cccgcgggac cgacctgtcc gcatccccag 240

gaccgccggg ctcggtgcac cgcctcggtc ccggagccgc ccgcctggat tgcattccct 300

cctctcctgg atctcctggg acccgacgcg agcctgcccc ggagcccgcc gagcgcaccc 360

tctctcgggt gcctgcagcc ccgccggcgc ggcccggccc ggcgcggccc ggctcggctc 420

ctagagctgc cacggccatg gccagagccc gcccgccgcc gccgccgtcg ccgccgccgg 480

ggcttctgcc gctgctccct ccgctgctgc tgctgccgct gctgctgctg cccgccggct 540

gccgggcgct ggaagagacc ctcatggaca caaaatgggt aacatctgag ttggcgtgga 600

catctcatcc agaaagtggg tgggaagagg tgagtggcta cgatgaggcc atgaatccca 660

tccgcacata ccaggtgtgt aatgtgcgcg agtcaagcca gaacaactgg cttcgcacgg 720

ggttcatctg gcggcgggat gtgcagcggg tctacgtgga gctcaagttc actgtgcgtg 780

actgcaacag catccccaac atccccggct cctgcaagga gaccttcaac ctcttctact 840

acgaggctga cagcgatgtg gcctcagcct cctccccctt ctggatggag aacccctacg 900

tgaaagtgga caccattgca cccgatgaga gcttctcgcg gctggatgcc ggccgtgtca 960

acaccaaggt gcgcagcttt gggccacttt ccaaggctgg cttctacctg gccttccagg 1020

accagggcgc ctgcatgtcg ctcatctccg tgcgcgcctt ctacaagaag tgtgcatcca 1080

ccaccgcagg cttcgcactc ttccccgaga ccctcactgg ggcggagccc acctcgctgg 1140

tcattgctcc tggcacctgc atccctaacg ccgtggaggt gtcggtgcca ctcaagctct 1200

actgcaacgg cgatggggag tggatggtgc ctgtgggtgc ctgcacctgt gccaccggcc 1260

atgagccagc tgccaaggag tcccagtgcc gcccctgtcc ccctgggagc tacaaggcga 1320

agcagggaga ggggccctgc ctcccatgtc cccccaacag ccgtaccacc tccccagccg 1380

ccagcatctg cacctgccac aataacttct accgtgcaga ctcggactct gcggacagtg 1440

cctgtaccac cgtgccatct ccaccccgag gtgtgatctc caatgtgaat gaaacctcac 1500

tgatcctcga gtggagtgag ccccgggacc tgggtggccg ggatgacctc ctgtacaatg 1560

tcatctgcaa gaagtgccat ggggctggag gggcctcagc ctgctcacgc tgtgatgaca 1620

acgtggagtt tgtgcctcgg cagctgggcc tgacggagcg ccgggtccac atcagccatc 1680

tgctggccca cacgcgctac acctttgagg tgcaggcggt caacggtgtc tcgggcaaga 1740

gccctctgcc gcctcgttat gcggccgtga atatcaccac aaaccaggct gccccgtctg 1800

aagtgcccac actacgcctg cacagcagct caggcagcag cctcacccta tcctgggcac 1860

ccccagagcg gcccaacgga gtcatcctgg actacgagat gaagtacttt gagaagagcg 1920

agggcatcgc ctccacagtg accagccaga tgaactccgt gcagctggac gggcttcggc 1980

ctgacgcccg ctatgtggtc caggtccgtg cccgcacagt agctggctat gggcagtaca 2040

gccgccctgc cgagtttgag accacaagtg agagaggctc tggggcccag cagctccagg 2100

agcagcttcc cctcatcgtg ggctccgcta cagctgggct tgtcttcgtg gtggctgtcg 2160

tggtcatcgc tatcgtctgc ctcaggaagc agcgacacgg ctctgattcg gagtacacgg 2220

agaagctgca gcagtacatt gctcctggaa tgaaggttta tattgaccct tttacctacg 2280

aggaccctaa tgaggctgtt cgggagtttg ccaaggagat cgacgtgtcc tgcgtcaaga 2340

tcgaggaggt gatcggagct ggggaatttg gggaagtgtg ccgtggtcga ctgaaacagc 2400

ctggccgccg agaggtgttt gtggccatca agacgctgaa ggtgggctac accgagaggc 2460

agcggcggga cttcctaagc gaggcctcca tcatgggtca gtttgatcac cccaatataa 2520

tccggctcga gggcgtggtc accaaaagtc ggccagttat gatcctcact gagttcatgg 2580

aaaactgcgc cctggactcc ttcctccggc tcaacgatgg gcagttcacg gtcatccagc 2640

tggtgggcat gttgcggggc attgctgccg gcatgaagta cctgtccgag atgaactatg 2700

tgcaccgcga cctggctgct cgcaacatcc ttgtcaacag caacctggtc tgcaaagtct 2760

cagactttgg cctctcccgc ttcctggagg atgacccctc cgatcctacc tacaccagtt 2820

ccctgggcgg gaagatcccc atccgctgga ctgccccaga ggccatagcc tatcggaagt 2880

tcacttctgc tagtgatgtc tggagctacg gaattgtcat gtgggaggtc atgagctatg 2940

gagagcgacc ctactgggac atgagcaacc aggatgtcat caatgccgtg gagcaggatt 3000

accggctgcc accacccatg gactgtccca cagcactgca ccagctcatg ctggactgct 3060

gggtgcggga ccggaacctc aggcccaaat tctcccagat tgtcaatacc ctggacaagc 3120

tcatccgcaa tgctgccagc ctcaaggtca ttgccagcgc tcagtctggc atgtcacagc 3180

ccctcctgga ccgcacggtc ccagattaca caaccttcac gacagttggt gattggctgg 3240

atgccatcaa gatggggcgg tacaaggaga gcttcgtcag tgcggggttt gcatcttttg 3300

acctggtggc ccagatgacg gcagaagacc tgctccgtat tggggtcacc ctggccggcc 3360

accagaagaa gatcctgagc agtatccagg acatgcggct gcagatgaac cagacgctgc 3420

ctgtgcaggt ctgacaccgg ctcccacggg gaccctgagg accgtgcagg gatgccaagc 3480

agccggctgg actttcggac tcttggactt ttggatgcct ggccttaggc tgtggcccag 3540

aagctggaag tttgggaaag gcccaagctg ggacttctcc aggcctgtgt tccctcccca 3600

ggaagtgcgc cccaaacctc ttcatattga agatggatta ggagaggggg tgatgacccc 3660

tccccaagcc cctcagggcc cagaccttcc tgctctccag caggggatcc ccacaacctc 3720

acacttgtct gttcttcagt gctggaggtc ctggcagggt caggctgggg taagccgggg 3780

ttccacaggg cccagccctg gcaggggtct ggccccccag gtaggcggag agcagtccct 3840

ccctcaggaa ctggaggagg ggactccagg aatggggaaa tgtgacacca ccatcctgaa 3900

gccagcttgc acctccagtt tgcacaggga tttgttctgg gggctgaggg ccctgtcccc 3960

acccccgccc ttggtgctgt cataaaaggg caggcagggg caggctgagg agttgccctt 4020

tgccccccag agactgactc tcagagccag agatgggatg tgtgagtgtg tgtgtgtgtg 4080

tgtgtgtgtg cgcgcgcgcg cgcgtgtgtg tgtgcacgca ctggcctgca cagagagcat 4140

gggtgagcgt gtaaaagctt ggccctgtgc cctacaatgg ggccagctgg gccgacagca 4200

gaataaaggc aataagatga aaaaaaaaaa aaaa 4234

<210> 63
<211> 50
<212> DNA
<213> homo sapiens

<400> 63
cctggcgtgg tggcaggtgc ctgtaatccc agctactctg tctcaaaaaa 50

<210> 64
<211> 1677
<212> DNA
<213> homo sapiens

<400> 64
cgccacgctc tgcacagtct attttcggag cctagccaga gacgggaaaa ctgaccaaga 60

taatggaatc tgaaaatatg gattctgaaa atatgaagac agaaaatatg gaatctcaaa 120

atgtagactt tgagagtgtt tcttcagtta cagctctgga agccctctct aagctactta 180

atcctgaaga agaggatgat tctgactatg gacagacaaa tggtttatct actattggag 240

ccatgggtcc tgggaatatt ggaccacccc aaatagaaga gctcaaagtc atccctgaaa 300

ccagcgagga aaataatgag gacatctgga attcagaaga gattccagaa ggagcagaat 360

atgatgatat gtgggatgtt agagaaatcc cagagtatga gattatattc agacagcagg 420

tgggaactga agatatattt ttagggttgt caaaaaagga ctcctcaaca ggttgttgca 480

gtgaactagt ggctaaaatt aaattgccaa atacaaaccc ttctgatatt caaattgata 540

tccaggaaac aatccttgac cttcgtactc ctcagaagaa gctgttgata actcttcctg 600

agctggtgga atgtaccagt gccaaagcat tctatatccc agagactgaa actcttgaaa 660

tcactatgac tatgaaaaga gagttagata ttgctaattt cttctgaaac tgcatgaaaa 720

agataaaaag tagtaaaatg gcattggtaa caattaaaaa actttgaaaa aaatgattgt 780

tttctatatt ctctttatta ttctgacata tttttgaaag ggacaaaatt cagttctagt 840

gatattgtga ccatttacag taatttctat tactctgtta ggaaatatgt ttccttggcc 900

aggtgcaatg gctcatgcct gtaatcccaa cactttggga ggccgaagca ggcggatcac 960

ttgaggtcag gagtttgaga ccagcctggt caacatggcg aaaccctgtc tctactaaaa 1020

atacgaaaat tagcctggcg tggtggcagg tgcctgtaat cccagctact ctgtctcaaa 1080

aaacaaacaa aaaaaggaaa tatgttttct tatgtaaata aactattcta tttttatagt 1140

gatagctgag tagaatcttt acaggttaaa aaattagaat atttcatggt caattacata 1200

attgaaatat gatcatgaaa tggttggtcc acttacttca gtagtctgaa aaacttatgc 1260

taattattta catttacact gtgatacaat cagttgcata gcaattggtc attattatta 1320

ccagagaatt ttatttggtt gctttttcat tgatttgagc aaatgattta cagtgttctc 1380

tgtttaggct ctcactgttg ttctggaata tacaggtaat aaatagtttg atgtaccata 1440

ctatcaacat atttacttga gtaaggaaca cttttgtgcg tacagtatta tttggcttct 1500

tcagttgtgc ttttaatgct tggatatagt taggttaaaa acatttttta tatgcactaa 1560

taattattag cattgccttt ttgggtactg tatttatatg tagtcaattg aaatccttga 1620

ctttaaaata aatttaaagt gtggcacatt taaaaaaaaa aaaaaaaaaa aaaaaaa 1677

<210> 65
<211> 50
<212> DNA
<213> homo sapiens

<400> 65
ccaaggttct cacactggcc tgggcttggg tgcccatata ggaggtctgt 50

<210> 66
<211> 1180
<212> DNA
<213> homo sapiens

<400>  66
   ctgaggtgta tttcgggtct tgctggggct gagagagacc acagcccttt ggggggtaca      60
   aacaagagtt cagttgctgt gaattctgcc actgtgccca gctctgaagc ctcagctctt     120
   gccaaacaga cccgagaccc atgtcagccc cactggatgc cgccctccac gcccttcagg     180
   aggagcaggc cagactcaag atgaggctgt gggacctgca gcagctgaga aaggagctcg     240
   gggactcccc caaagacaag gtcccatttt cagtgcccaa gatccccctg gtattccgag     300
   gacacaccca gcaggacccg gaagtgccta agtctttagt ttccaatttg cggatccact     360
   gccctctgct tgcgggctct gctctgatca cctttgatga ccccaaagtg gctgagcagg     420
   tgctgcaaca aaaggagcac acgatcaaca tggaggagtg ccggctgcgg gtgcaggtcc     480
   agcccttgga gctgcccatg gtcaccacca tccaggtgat ggtgtccagc cagttgagtg     540
   gccggagggt gttggtcact ggatttcctg ccagcctcag gctgagtgag gaggagctgc     600
   tggacaagct agagatcttc tttggcaaga ctaggaacgg aggtggcgat gtggacgttc     660
   gggagctact gccagggagt gtcatgctgg ggtttgctag ggatggagtg gctcagcgtc     720
   tgtgccaaat cggccagttc acagtgccac tgggtgggca gcaagtccct ctgagagtct     780
   ctccgtatgt gaatggggag atccagaagg ctgagatcag gtcgcagcca gttccccgct     840
   cggtactggt gctcaacatt cctgatatct tggatggccc ggagctgcat gacgtcctgg     900
   agatccactt ccagaagccc acccgcgggg gcggggaggt agaggccctg acagtcgtac     960
   cccaaggaca gcagggccta gcagtcttca cctctgagtc aggctagggg cctccccttc    1020
   tcatcctccc cacccccccg ccaaggttct cacactggcc tgggcttggg tgcccatata    1080
   ggaggtctgt atgttcacca acagtgcgga ggggtcacac attgcaaaac actgcccaga    1140
   acagtaaaaa gagcctgcat gccaaaaaaa aaaaaaaaaa                          1180


<210>  67
<211>  50
<212>  DNA
<213>  homo sapiens

<400>  67
cagctcaggt ggtgagtcct cctgtccagc ctgcatcaat aaaatggggc                50


<210>  68
<211>  1652
<212>  DNA
<213>  homo sapiens

<400>  68
ctgcttaaac ctctgtctct gacggtccct gccaatcgct ctggtcgacc ccaacacact      60

aggaggacag acacaggctc caaactccac taaccagagc tgtgattgtg cccgctgagt     120

ggactgcgtt gtcagggagt gagtgctcca tcatcgggag aatccaagca ggaccgccat     180

ggaggaaggt caatattcag agatcgagga gcttcccagg aggcggtgtt gcaggcgtgg 240

gactcagatc gtgctgctgg ggctggtgac cgccgctctg tgggctgggc tgctgactct 300

gcttctcctg tggcactggg acaccacaca gagtctaaaa cagctggaag agagggctgc 360

ccggaacgtc tctcaagttt ccaagaactt ggaaagccac cacggtgacc agatggcgca 420

gaaatcccag tccacgcaga tttcacagga actggaggaa cttcgagctg aacagcagag 480

attgaaatct caggacttgg agctgtcctg gaacctgaac gggcttcaag cagatctgag 540

cagcttcaag tcccaggaat tgaacgagag gaacgaagct tcagatttgc tggaaagact 600

ccgggaggag gtgacaaagc taaggatgga gttgcaggtg tccagcggct ttgtgtgcaa 660

cacgtgccct gaaaagtgga tcaatttcca acggaagtgc tactacttcg gcaagggcac 720

caagcagtgg gtccacgccc ggtatgcctg tgacgacatg gaagggcagc tggtcagcat 780

ccacagcccg gaggagcagg acttcctgac caagcatgcc agccacaccg gctcctggat 840

tggccttcgg aacttggacc tgaaggggga gtttatctgg gtggatggga gccacgtgga 900

ctacagcaac tgggctccag gggagcccac cagccggagc cagggcgagg actgcgtgat 960

gatgcggggc tccggtcgct ggaacgacgc cttctgcgac cgtaagctgg gcgcctgggt 1020

gtgcgaccgg ctggccacat gcacgccgcc agccagcgaa ggttccgcgg agtccatggg 1080

acctgattca agaccagacc ctgacggccg cctgcccacc ccctctgccc ctctccactc 1140

ttgagcatgg atacagccag gcccagagca agaccctgaa gacccccaac cacggcctaa 1200

aagcctcttt gtggctgaaa ggtccctgtg acattttctg ccacccaaac ggaggcagct 1260

gacacatctc ccgctcctct atggcccctg ccttcccagg agtacacccc aacagcaccc 1320

tctccagatg ggagtgcccc caacagcacc ctctccagat gagagtacac cccaacagca 1380

ccctctccag atgagagtac accccaacag caccctctcc agatgagagt acaccccaac 1440

agcaccctct ccagatgcag ccccatctcc tcagcacccc aggacctgag tatccccagc 1500

tcaggtggtg agtcctcctg tccagcctgc atcaataaaa tggggcagtg atggcctccc 1560

aaaaaaaaaa aaaaaaaaaa aaaaaaaaaa aaaaaaaaaa aaaaaaaaaa aaaaaaaaaa 1620

aaaaaaaaaa aaaaaaaaaa aaaaaaaaaa aa 1652

<210> 69
<211> 50
<212> DNA
<213> homo sapiens

<400> 69
tgaggtagga ggatcacgtg aggtcgggag ttcaagacca gcctggccat 50

<210> 70
<211> 3093

<212>  DNA
<213>  homo sapiens

<400>  70
cggggccgag gcgcgaggag gtgaggctgg agcgcggccc cctcgccttc cctgttccca      60

ggcaagctcc caaggcccgg gcggcggggc cgtcccgcgg gccagccaga tggcgacgtg     120

gcggttcccc gcccgccgcg accccaactc cgggacgcac gctgcggacg cctatcctcc     180

cccaggccgc tgacccgcct ccctgcccgg ccggctcccg ccgcggagga tatggaatat     240

tatcttgtaa aatggaaagg atggccagat tctacaaata cttgggaacc tttgcaaaat     300

ctgaagtgcc cgttactgct tcagcaattc tctaatgaca agcataatta tttatctcag     360

gtaaagaaag gcaaagcaat aactccaaaa gacaataaca aaactttgaa acctgccatt     420

gctgagtaca ttgtgaagaa ggctaaacaa aggatagctc tgcagagatg gcaagatgaa     480

ctcaacagaa gaaagaatca taaaggaatg atatttgttg aaaatactgt tgatttagag     540

ggcccacctt cagacttcta ttacattaac gaatacaaac cagctcctgg aatcagctta     600

gtcaatgaag ctacctttgg ttgttcatgc acagattgct tctttcaaaa atgttgtcct     660

gctgaagctg gagttctttt ggcttataat aaaaaccaac aaattaaaat cccacctggt     720

actcccatct atgaatgcaa ctcaaggtgt cagtgtggtc ctgattgtcc caataggatt     780

gtacaaaaag gcacacagta ttcgctttgc atctttcgaa ctagcaatgg acgtggctgg     840

ggtgtaaaga cccttgtgaa gattaaaaga atgagttttg tcatggaata tgttggagag     900

gtaatcacaa gtgaagaagc tgaaagacga ggacagttct atgacaacaa gggaatcacg     960

tatctctttg atctggacta tgagtctgat gaattcacag tggatgcggc tcgatacggc    1020

aatgtgtctc attttgtgaa tcacagctgt gacccaaatc ttcaggtgtt caatgttttc    1080

attgataacc tcgatactcg tcttccccga atagcattgt tttccacaag aaccataaat    1140

gctggagaag agctgacttt tgattatcaa atgaaaggtt ctggagatat atcttcagat    1200

tctattgacc acagcccagc caaaaagagg gtcagaacag tatgtaaatg tggagctgtg    1260

acttgcagag gttacctcaa ctgaactttt tcaggaaata gagctgatga ttataatatt    1320

tttttcctaa tgttaacatt tttaaaaata catatttggg actcttatta tcaaggttct    1380

acctatgtta atttacaatt catgtttcaa gacatttgcc aaatgtatta ccgatgcctc    1440

tgaaaagggg gtcactgggt ctcatagact gatatgaagt cgacatattt atagtgctta    1500

gagaccaaac taatggaagg cagactattt acagcttagt atatgtgtac ttaagtctat    1560

gtgaacagag aaatgcctcc cgtagtgttt gaaagcgtta agctgataat gtaattaaca    1620

actgctgaga gatcaaagat tcaacttgcc atacacctca aattcggaga aacagttaat    1680

ttgggcaaat ctacagttct gtttttgcta ctctattgtc attcctgttt aatactcact    1740

gtacttgtat ttgagacaaa taggtgatac tgaattttat actgttttct acttttccat    1800

taaaacattg gcacctcaat gataaagaaa tttaaggtat aaaattaaat gtaaaaatta 1860

atttcagctt catttcgtat ttcgaagcaa tctagactgt tgtgatgagt gtatgtctga 1920

acctgtaatt cttaaaagac ttcttaatct tctagaagaa aaatctccga agagctctct 1980

ctagaagtcc aaaatggcta gccattatgc ttctttgaaa ggacatgata atgggaccag 2040

gatggttttt tggagtacca agcaagggga atggagcact ttaagggcgc ctgttagtaa 2100

catgaattgg aaatctgtgt cgagtacctc tgatctaaac ggtaaaacaa gctgcctgga 2160

gagcagctgt acctaacaat actgtaatgt acattaacat tacagcctct caatttcagg 2220

caggtgtaac agttcctttc caccagattt aatattttta tacttcctgc aggttcttct 2280

taaaaagtaa tctatatttt tgaactgata cttgttttat acataaattt tttttagatg 2340

tgataaagct aaacttggcc aaagtgtgtg cctgaattat tagacctttt tattagtcaa 2400

cctacgaaga ctaaaataga atatattagt tttcaaggga gtgggaggct tccaacatag 2460

tattgaatct caggaaaaac tattctttca tgtctgattc tgagatttct aattgtgttg 2520

tgaaaatgat aaatgcagca aatctagctt tcagtattcc taatttttac ctaagctcat 2580

tgctccaggc tttgattacc taaaataagc ttggataaaa ttgaaccaac ttcaagaatg 2640

cagcacttct taatctttag ctctttcttg ggagaagcta gactttattc attatattgc 2700

tatgacaact tcactctttc ataatatata ggataaattg tttacatgat tggaccctca 2760

gattctgtta accaaaattg cagaatgggg ggccaggcct gtgtggtggc tcacacctgt 2820

gatcccagca ctttgggagg ctgaggtagg aggatcacgt gaggtcggga gttcaagacc 2880

agcctggcca tcatggtgaa accctgtctc tactgaaaat acaaaaatta gccgggcgtg 2940

gtggcacacg cctgtagtcc cagctactca ggaggctgag gcaggagaat cacttgaatt 3000

caggaggcgg aggttgcagt gagccaagat cataccactg cactgcagcc tgagtgacac 3060

agtaagactg tctccaaaaa aaaaaaaaaa aaa 3093

<210> 71
<211> 50
<212> DNA
<213> homo sapiens

<400> 71
cacagaaatg cttgcagcct aaggcagggt tttcagaccg tgggtcccag 50

<210> 72
<211> 2795
<212> DNA
<213> homo sapiens

<400> 72
   gggcggctgg gaccggcctg gggtagctgc gcagcaggtg ggtcggtccc ggggatgcag 60
   cgcgggggtc agagagcgac cccaacaggg tagacggtga ggacgcctca gtgggcagcg 120
   ttagggaaag gggccagggt gttcccgggg ttggggtgcg agcccccgct gtgcgtccag 180
   gaggccgagg gtctggcatc ccttcgcgtg gagtgcgggg caggggccgg gtccccccgg 240
   aggtggctga gatccagatc ggtgaggggc cctgcaggca ggtctgtggg ttctggcacc 300
   acctgagccc actgggcatc tggtcatccc tggcacctct cctttggagc caccttgtcc 360
   ctggctagac agtcacattt tccagtgccg ttttggaaag atgttgcctt tggagaaggc 420
   gtttgcctcc cccaggagct ccccagcccc gccggatctg cccacgccgg ggtcagcagc 480
   cggagtccag caggaagaac ccgagaccat ccctgagagg acccctgctg acctggagtt 540
   ctcccgcctg cgtttccggg aatttgtcta ccaggaggct gccgggcccc accagaccct 600
   ggcccggctg catgagctgt gccgccagtg gctgatgcct gaggcgcgct ccaaggagca 660
   gatgctggag ctgctggtgc tggagcagtt cctgggcatc ctgcctgata aggtccggcc 720
   ctgggtggtg gcacagtacc ctgagagctg caagaaggca gcctccctgg tggagggcct 780
   cgctgatgtc ctggaagagc cagggatgct gctgggctcc cctgcgggct catcctcaat 840
   tcttagcgat ggagtgtacg agaggcacat ggaccctctg ctgctaccag gcgagctcgc 900
   gagccccagc caggcccttg gagctgggga gatcccggca ccttctgaga caccctggct 960
   ttctccggac cccctgtttc tggaacagag gagggtcaga gaagcaaaga ccgaagagga 1020
   cggccctgcc aacaccgagc agaagctgaa gtcctttcca gaggaccctc agcacctggg 1080
   ggagtggggc cacctggacc ctgccgagga gaacctgaag agctaccgga agctgctcct 1140
   gtgggggtat cagctttccc agcctgacgc tgcctccagg ctggacactg aggaactccg 1200
   gttggtggaa agagatccac aaggaagcag cctcccagaa ggcgggaggc ggcaggagag 1260
   cgctgggtgc gcctgcgagg aggccgcccc cgcgggggtg ctgcctgagc tgcctacgga 1320
   ggcgccccct ggggacgccc ttgccgatcc cccgtcgggc accactgagg aggaggaaga 1380
   gcagcctggg aaggccccgg acccgcagga cccccaggac gcggagtccg actctgccac 1440
   cggatcgcag aggcagtccg tcatccagca gcctgccccg gacaggggca cggcgaaact 1500
   gggaaccaag aggccgcacc ccgaggatgg ggacgagcag agcctcgagg gcgtctctag 1560
   ctccggcgac agcgcagggc tggaggccgg gcagggccct ggggctgacg agccgggctt 1620
   gtcccgcggg aagccctatg cctgcggcga gtgcggggag gccttcgcgt ggctctcgca 1680
   cctgatggag caccacagca gccatggcgg ccggaagcgc tacgcctgtc agggctgctg 1740
   gaagaccttc cacttcagcc tggccctagc cgagcaccag aagacccacg agaaggagaa 1800
   aagctacgcg ctggggggcg cccgggcccc ccaaccgtcc acccgcgaag cccaggcggg 1860
   ggctagggcg ggcggtcccc cagagagcgt ggagggcgag gctccccccg cacccccaga 1920
   ggcgcagagg tgagccgctg tgctgtcccg ttccggaggg gccgctttgc cggccgtgaa 1980
   tcccagacga ggcattgggc ctttccacgc ccctgggtgg cggcttcctg tggtgtttgt 2040
   ggacgtcctc tgcctgtgcc ctgaatccgc tcctgaggct aagcgctccc aacgagaagg 2100
   gtccacggga agccctcacc tctgtaaaca caccctgggc cagcgctcgc atccgagggg 2160
   agccgccgga tgtggaagaa gactcggctt tcctgcagcc atttagtgcc gccccatgct 2220
   aggttatttg acattgtgca gtgtagagtt gccttaaagt gcgtgatctg ccagtgcttt 2280
   cttcaagtca cccttgcccc gattcctcct gtttgcgctc cccagggttg ctcaagtgga 2340
   aattttgtca gctgtttagc cttttcgtac ttggcgtgat gtcaacttca cttctaatct 2400
   gcaaaagcag aagctgtttc ctagtttacc tcgcgtgtgt ttacctatat ggagtagctc 2460
   gcagagatca cagaaatgct tgcagcctaa ggcagggttt tcagaccgtg ggtcccagcc 2520
   catttagtaa aatgggaaat caattagcaa gtggtcacca gcattacaca gcaatgaagc 2580
   agaataaagt aggccagaat gcatcatgta gtaaaggcaa atactgtttt gtgaaacttt 2640
   tcacccatac atctaaatgt gagaactggt tgcaatgtaa gacatttctt gctgggaagt 2700
   tgtgagcaaa ataagttgaa aacactaata aagatctgtc tgtctgagca aaggagacta 2760
   aactccttgg gctacataaa aaaaaaaaaa aaaaa 2795

<210> 73
<211> 50
<212> DNA
<213> homo sapiens

<400> 73
aaaaaattag ccgggtgtgg tggcagacac ctgtggtccc agctattcgg 50

<210> 74
<211> 1964
<212> DNA
<213> homo sapiens

<400> 74
gggtcgcgct ctaagtgagg cgccaagcgg tctccgcctc agggtctgag gctgcgaaag 60

gggcgtaacg atgagcggtt cctgccagag gtctggggag gataaaaagc aggaggaaga 120

ggcgacggcg gcctgtggac gtcttgcagg ggtccccgaa gccaagcagg gtcccaaggc 180

cgattcagac tccgacctag agacggaagg tgctcggggc aggggccagg cccgtctcct 240

ccccttgggg gcttctcccg caggggttgt gggaggtggg ctggcgccgc cgaggaggca 300

ggagacctct gtccagcagg gcacgtagcc ctctgcgggg cgtctccagc cccctgccct 360

ctcctcggcg ctcctggcgg catcaggaac tttaccatga gaaggcgctt ctagaaggag 420

gttaaaatgg aaaatcccgc cagacagtgc accaaaggct gtcccgccct gaaatgcaat 480

cagagctggg ttttcccaat ccacagatgt ggacctgttg gagaaccagc tgggagtggc 540

aggagcccag gccctctgtg ccgccctcac agtgaaccag gccatgcgga agatgcagct    600

gtcagggaat ggcctggagg agcaggcggc ccagcacctt gccgaactcc tgctggccca    660

cacagacctg aagtccctgg acctgagcta caaccagctg aatgaccaag caggcagatc    720

tgtgcatggg actgcagggc ttcctcatct tccacagctt tgggggcggc actggctctg    780

ggttcgtgtc tctgctcatg aagcggctct cggtggacta cgggaagaag tccaagctgg    840

agtttgccat ttgcccagcc ccccaggtct ccatggccat gacggagccc tacaactcca    900

tcctgaccac ctacacgacc ctggaacatt ctgactgtgc cttcatagtc gacagcaaag    960

ccacctatga catgtcagca caacctggac atcgagtgtc ccatgtacac caacctcagt   1020

cgtctgggca gatcgtgtcc tccatcacgg cctccctgtg attcgatggg gccctgaatg   1080

tgtacttgac agaattccaa accaacttgg tgccataccc ccacatccac ctccccctgg   1140

ccacctacgc cctggtcatc tcagccaaga aggcctacca tgagcagctg tccgtggcca   1200

agatcgccag tgcccgcttc gagccagcca atcagatggt caagtgtgac cctcaccatg   1260

gcaagtacat ggcctgctgc atgttgtaca gaggggatgt ggtcctgaaa gatgtcagtg   1320

cggctgtcgc caccatcaag agcaagcaca ccaaccagtt tgtggactgg tgtccgattg   1380

gatttaaggt aggactgggt gatgtggagt ccttgtgcca tcaggaagca ggagacctgc   1440

agaataatgc tgcccctgaa ggcccgcatc ctttatggag acaacccctt ttcacgtcag   1500

ctaggttgca aggataataa ttttagtaat ttatttccta ccgccttata ctttaccaaa   1560

tactttatgt tccctcatac cttaaccact accctttaaa gttcatgggg caatattacc   1620

agtatcttac actttagaaa acagaggctg aggctgggca cggtggctca tgcctgtaat   1680

cccagcactt tgggaggcca aggcaggcag atcacgaggt caggagattg agaccatcct   1740

ggctaacacg gtgaaacccc ggctctacta aaaaatacaa aaaattagcc gggtgtggtg   1800

gcagacacct gtggtcccag ctattcggga ggctgaggca ggaggatggc gtgaacccgg   1860

gaggcagagc ttgcagtgag tcgagattgc accactgcac tccagcctgg gcaacagagc   1920

gagactacat ctcaaaaaaa aaaaaaagaa agaaaaagaa aaaa                    1964

<210>  75
<211>  50
<212>  DNA
<213>  homo sapiens

<400>  75
atgtggagcg acctccgggt gtgagtgtgg cgtcatggaa gggcagagaa                50

<210>  76
<211>  1237
<212>  DNA
<213>  homo sapiens

<400>  76
cgattcaggg gagggagcaa ctggagcctc aggccctcca gagtagtctg cctgaccacc      60

ctggagccca cagaagccca ggacgtctcc cgcgaagcct ccccgtgtgt ggctgaggat     120

ggctgagcag cagggccggg agcttgaggc tgagtgcccc gtctgctgga accccttcaa     180

caacacgttc cataccccca aaatgctgga ttgctgccac tccttctgcg tggaatgtct     240

ggcccacctc agccttgtga ctccagcccg gcgccgcctg ctgtgcccac tctgtcgcca     300

gcccacagtg ctggcctcag ggcagcctgt cactgacttg cccacggaca ctgccatgct     360

cgccctgctc cgcctggagc cccaccatgt catcctggaa ggccatcagc tgtgcctcaa     420

ggaccagccc aagagccgct acttcctgcg ccagcctcaa gtctacacgc tggaccttgg     480

cccccagcct gggggccaga ctgggccgcc cccagacacg gcctctgcca ccgtgtctac     540

gcccatcctc atccccagcc accactcttt gagggagtgt ttccgcaacc ctcagttccg     600

catctttgcc tacctgatgg ccgtcatcct cagtgtcact ctgttgctca tattctccat     660

cttttggacc aagcagttcc tttggggtgt ggggtgagtg ctgttcccag acaagaaacc     720

aaaccttttt cggttgctgc tgggtatggt gactacggag cctcatttgg tattgtcttc     780

ctttgtagtg ttgtttattt tacaatccag ggattgttca ggccatgtgt ttgcttctgg     840

gaacaatttt aaaaaaaaac aaaaaaacga aaagcttgaa ggactgggag atgtggagcg     900

acctccgggt gtgagtgtgg cgtcatggaa gggcagagaa gcggttctga ccacagagct     960

ccacagcaag ttgtgccaaa gggctgcaca gtggtatcca ggaacctgac tagcccaaat    1020

agcaagttgc atttctcact ggagctgctt caaaatcagt gcatattttt ttgagttgct    1080

cttttactat gggttgctaa aaaaaaaaaa aaaattggga agtgagcttc aattctgtgg    1140

gtaaatgtgt gtttgtttct ctttgaatgt cttgccactg gttgcagtaa aagtgttctg    1200

tattcattaa aaaaaaaaaa aaaaaaaaaa aaaaaaa                             1237


<210>  77
<211>  50
<212>  DNA
<213>  homo sapiens

<400>  77
aattggacat ttaagcgaag taaaagaggc cgggtgcggt ggctcacgcc                50


<210>  78
<211>  2671
<212>  DNA
<213>  homo sapiens

<400>  78
gcagcacccg ccctgcgcct cggtggcagc cgtccacgcc gtcagtgcct ggcggttccc      60

gtgggacata ccgtccactc gccacgcccc tccgcccgcg gcgcacccca cgcaccgtgc     120

cctggccgcc cgcctcacgc accgctttcc tctccgtcca gcgctcgctg cagccgggcc 180

gtctcgcagt ccagcgtgct ggccagagac cgcagcttcc cgcagaagct ccggataggg 240

tccatgctga gcacagcggg gaaggactcc aggcgtacgc agaccccacc gctcagctca 300

cagcctcccg ccactagttt gaatctcggc gccggacgcg tcctcggctc tgcgcaggcg 360

cagtcctctc tcgcgagagc cgaagtctct cgccgaggcc ccaggagacc ccgcgagacg 420

ggtgcgctta cgccacggcg tctgctggcg gccgcggaga cgcagagtct tgagcagcgc 480

ggcaggcacc atgttcctga ctgcgctcct ctggcgcggc cgcattcccg gccgtcagtg 540

gatcgggaag caccggcggc cgcggttcgt gtcgttgcgc gccaagcaga acatgatccg 600

ccgcctggag atcgaggcgg agaaccatta ctggctgagc atgccctaca tgacccggga 660

gcaggagcgc ggccacgccg cggtgcgcag gagggaggcc ttcgaggcca taaaggcggc 720

cgccacttcc aagttccccc cgcatagatt cattgcggac cagctcgacc atctcaatgt 780

caccaagaaa tggtcctaat cctgagtcgt cacccttgga ttttatggat cacggagctg 840

accatcttta cctggtcctg gaactgaaaa actgtagctt gtgtgaaaat gagcctttgg 900

accagtcttt attaaaacaa acaaacatga gtagtctgca tatcgaatat ctagagctct 960

aaacccccaa tacttaaaag tctaattgct gtcctgtggt ttcattagtc tgataggaag 1020

ataggatttt cctcagtcac agatgatatt ttgaaggaaa gctgcaataa agccacaatg 1080

atttgaggtc tttgcttaag tatgagatac ttgatggggg ctttatcatg caacattagt 1140

ttgcttacct taagaattgc ccaaaaatga aagaaaatat gagcttttca gttaaacata 1200

ctcctaaaaa cattttccgg gattttacta ctaaaattgg acatttaagc gaagtaaaag 1260

aggccgggtg cggtggctca cgcctgtaat cccaacactt taggagtcca aggcgggcgg 1320

atcacttgag gtcaggagtt caagaccagc ttggccaagg tgataaaacc cgtctctact 1380

aatacaaaaa aaattagctg ggtatgttgg tgcaggcttg caatcccagc tacttgggac 1440

gctgaggtgg gaggatcgcc tgaacctcag aggcagaggt tgcaatgaag cggaggttgc 1500

agtgagccga gagcatgccg ctgcactcca gcctgggaga ctgcatctca aaaaagaaaa 1560

ataaacgaag taaaagatat ttatcagaac tgttacagga aggtgtattt tttagctatt 1620

ttggcagggt gctgtggtgc ttgcatgtag tcccagctat tcaggaatat tgcttgagtc 1680

cagcttgggc aacatagcga gaccctatct aaaataacaa taatatatgg ccaggcggga 1740

tggctcacgc ctgtaatccc agcactttgg gaggctgagg cgggcagatc acttgaggcc 1800

aggagttcaa gaccagccca accaacatgg tgaaaccctg tctccactga aaatacataa 1860

aattagccgg gtgcagtggt gaggcctgta atcccagcta ctcaggaggc tgaggcagga 1920

gaatcgcttg cacccaggag gtggaggctg cagtaagtcg agattgtgtc actgcactct 1980

agcctgggcg acagaaaaag aaaatttagg tggggaaaaa aatcagaatt gcgtctgtgg 2040

gaacaggaat aaaggagaaa ccactattga ctgggaagac acacaaagaa ctttgtcaaa 2100

gttgtatggc tgaggcccac acggtggctc acttctgtaa tcccatcact ttgggaggct 2160

taggcagaag gatcacctga gatcaggagt tccagaccag cctggccaac atggcgaaac 2220

cccatctcta ctaaaaatac aaaaattagc cgggcgtggt ggcttacgcc tgtaatccca 2280

gcactttggg aggccaaggt gggcggatca caaggtcagg agttcgagac cagcctggcc 2340

aatatggtga aaccccatct ctactaaaaa tataaaaatc agcaaaaaaa atttagccag 2400

gcttggtcat gcatgcctgt aatcctagct actgggaagg ctgaggaagg agaattgctt 2460

gaacccaggt ggtggaggtt gcagtgagct gagatcgtgc cactgctctc cagcctgcat 2520

gacgggagtg agacaccatc tcaaaaaata catataataa tataaataaa aatatctttt 2580

ttgaaaataa tttaatatac catgtaattt accagttgaa gatgttcgct ttcagaatat 2640

aataaaaata gtcaaaaaaa aaaaaaaaaa a 2671

<210> 79
<211> 50
<212> DNA
<213> homo sapiens

<400> 79
agctcacgag tggtcgagtg aagattgaaa attaacctct gagggccagc 50

<210> 80
<211> 3812
<212> DNA
<213> homo sapiens

<400> 80
aatcaaaaaa ctaattcttc caaagagcga ctcttactgt ttctcatggt gagaagacaa 60

tatttgcttt ctctttttcc tttcttccgg atgagaggct aagccataat agaaagaatg 120

gagaattatt gattgaccgt ctttattctg tgggctctga ttctccaatg ggaataccaa 180

gggatggttt tccatactgg aacccaaagg taaagacact caaggacaga catttttggc 240

agagcataga tgaaaatggc aagttccctg gctttccttc tgctcaactt tcatgtctcc 300

ctcctcttgg tccagctgct cactccttgc tcagctcagt ttctgtgctt tggaccctct 360

gggcccatcc tggccatggt gggtgaagac gctgatctgc cctgtcacct gttcccgacc 420

atgagtgcag agaccatgga gctgaagtgg gtaagttcca gcctaaggca ggtggtgaac 480

gtgtatgcag atggaaagga agtggaagac aggcagagtg caccgtatcg agggagaact 540

tcgattctgc gggatggcat cactgcaggg aaggctgctc tccgaataca caacgtcaca 600

gcctctgaca gtggaaagta cttgtgttat ttccaagatg gtgacttcta tgaaaaagcc 660

ctggtggagc tgaaggttgc agcactgggt tctaatcttc acgtcgaagt gaagggttat 720

gaggatggag ggatccatct ggagtgcagg tccaccggct ggtaccccca accccaaata 780
cagtggagca acgccaaggg agagaacatc ccagctgtgg aagcacctgt ggttgcagat 840
ggagtgggcc tatatgaagt agcagcatct gtgatcatga gaggcggctc cggggagggt 900
gtatcctgca tcatcagaaa ttccctcctc ggcctggaaa agacagccag catttccatc 960
gcagacccct tcttcaggag cgcccagccc tggatcgcag ccctggcagg gaccctgcct 1020
atcttgctgc tgcttctcgc cggagccagt tacttcttgt ggagacaaca gaaggaaata 1080
actgctctgt ccagtgagat agaaagtgag caagagatga aagaaatggg atatgctgca 1140
acagagcggg aaataagcct aagagagagc ctccaggagg aactcaagag gaaaaaaatc 1200
cagtacttga ctcgtggaga ggagtcttcg tccgatacca ataagtcagc ctgatgctct 1260
aatggaaaaa tggccctctt caagcctggt gaggaaatgc ttcagatgag gctccacctt 1320
gttaaataaa ttggatgtat ggaaaaatag actgcagaaa aggggaactc atttagctca 1380
cgagtggtcg agtgaagatt gaaaattaac ctctgagggc cagcacagca gctcatgcct 1440
gtaatcctag cactttggaa ggctgaggag ggcggatcac aaggtcagga gatcaagacc 1500
atcctggcta acacggtgaa accccgtctc tactaaaaat acaaaaaata aaaaattagc 1560
cgggcatggt gacgggcacc tgtagtccca gctactcggg aggctgaggc aggagaatgg 1620
catgaacccg gaaggcagag cttgcagtga gccgagatca cgccactgca ctccagcctg 1680
ggagacagag cgagactctg tctcaagaaa aaaaaaaaaa aaaaaaaaga aaagaaaatt 1740
aacctctgag tataaagcat cagtgggcag aatcaatgtg gggagggaaa caacaaaaat 1800
gtagaaagag gatccttgtt gcttcttggg gccgcatcag ggtattgggt taggcagata 1860
ctgaccttac tttcatttcc cctctggtca ctagacccct ggggctttca ccaatgacat 1920
tgatgagaga atcacattca gggcaggcta gggacacggg gttctggaag gacctcctca 1980
gcatggccca agccttgcat gctgtggctc ttaaatccag gaaaaatggc tgaccccatg 2040
gacacctcct caaactctct gcagcagatg taattctgta tccagacatg gcaaatgcca 2100
tcctccttgt ttctgaggac cagaggagtg tacagcgtgc tgaggagccc catgacctac 2160
cagacaaccc tgagagattt gaatggcgtt actgtgtgct tggctgtgaa agcttcatgt 2220
cagagagaca ctactgggag gtggaagtgg gggacagaaa agagtggcat attggggtat 2280
gtagtaagaa cgtggagagg aaaaaagttt gggtcaaaat gacaccggag aacggatact 2340
ggactatggg cctgactgat gggaataagt atcgggctct cactgagccc agaaccaacc 2400
tgaaacttcc tgagcctcct aggaaagtgg gggtcatcct ggactatgag actggacata 2460
tctcgttcta caatgccacg gatggatctc atatctacac atttctgcac gcctcttcct 2520
ctgagcctct gtatcctgta ttcagaattt tgaccttgga gcccactgcc ctgaccgttt 2580
gcccaatacc aaaagtagag agttcccccg atcccgacct agtgcctgat cattccctgg 2640

agataccact gaccccaggc ttagctaatg aaagtgggga gcctcaggct gaagtaacat 2700

ctctgcttct ccctgcccag cctggagcta agggtctcac cctccacaac agccagtcag 2760

aaccataaag ctacaggcac acactgaagc actttactga tattcattca attattccat 2820

aggacagttg tttgagtttg gtgccacctt attggcccct ttatacagat aaggaaactg 2880

gggtgtagaa aagtgtattg actttacaaa gcagacagga atagtgaaca acagagctgg 2940

gatctgaaca acaatgacta acattaatgg agaatttaaa acgttctgag tgctgtgtta 3000

tgagctttgg tgggtgtcac tcctttaatc ctcacaacac cctgtcaggt agtctcattt 3060

ggcaagtatg gaagcagagg cagggcaaca ttaagtagct tacataactc acacggtaat 3120

ttgtgcagtt gggagatgtt cagcttcagt ccctggccaa ttgcccgttc ttttccagcc 3180

tgatttttcc tgcatgggaa gagcccacat gtagccctga ggttcccttc ccaggacagc 3240

tccaggatcg agatcactgt gagtggttgt ggagttaaga cccctatgga ctccttccca 3300

gctgattatc agagccttag acccagcact ccttggattg gctctgcaga gtgtcttggt 3360

tgagagaata acgttgcagt tcccacaggg catgtgactt tgaaagagac tagaggccac 3420

actcagttaa taatggggca cagatgtgtt cccacccaac aaatgtgata agtgatcgtg 3480

cagccagagc cagccttcct tcagtcaagg tttccaggca gagcaaatac cctagagatt 3540

ctctgtaata ttggtaattt ggatgaagga agctagaaga attacaggga tgtttttaat 3600

cccactatgg actcagtctc ctggaaaagg atctgtccac tcctggtcat tggtggatgt 3660

taaacccata ttcctttcaa ctgctgcctg ctagggaaaa ctgctcctca ttatcatcac 3720

tattattgct caccactgta tcccctctac tgggcaagtg cttgtcaagt tctagttgtt 3780

caataaattt gttaataatg ctgactcttc aa 3812

<210> 81
<211> 50
<212> DNA
<213> homo sapiens

<400> 81
ggggacgtag ccttttcttg ctctggaagc ccagggaggt tgagcagtga 50

<210> 82
<211> 3300
<212> DNA
<213> homo sapiens

<400> 82
   atgcgccgcc tgcgctccgc cgctcgcgcc tcgggctcgg ctcgggctcc gggatgtccg 60
   cgctgtgcga ccctcccggg gccccagggc cacctgggcc tgccccggcc acccacggtc 120
   ccgcgcctct cagtgctcag gagctgtccc aggaaatcaa ggcttttctg actggcgtag 180
   accccattct gggccaccaa ctctcagccc gggaacatgc tcgctgtggt cttctcctgc 240
   tccgttcttt gccacctgct cgggctgctg tgcttgacca cttgagaggt gtctttgatg 300
   agagtgtccg ggcccacctg gctgccctgg atgaaacccc tgtggctggt ccacctcacc 360
   tccgtccacc tccaccctct catgtccctg ctggtggacc tggtctagag gatgtggttc 420
   aggaagtgca gcaggtgctg tctgagttta tccgggccaa cccaaaggcc tgggcacctg 480
   tgattagtgc atggtccatt gacctcatgg ggcaactgag cagcacgtac tcaggccagc 540
   accagcgtgt tccccacgct actggcgctc ttaatgaact gctacagctg tggatgggtt 600
   gtagggccac gcgtacatta atggacatct atgtgcagtg cctctcggct ctcattggta 660
   gctgcccaga tgcgtgtgtg gatgccttgc tggatacctc tgttcagcat tctccacact 720
   ttgactgggt tgtggcacat attggctcct cttttcctgg caccatcatt tcccgggttc 780
   tctcctgtgg ccttaaggac ttttgtgtcc atggtggggc tggaggtgga gctggcagta 840
   gtggtggaag ctcttctcag acccctctta cagacccctt ccctggatct cctgccattc 900
   ctgcggagaa acgggtgccc aagattgcct cagttgtagg catcctaggt cacctggcct 960
   cccgccacgg agatagcatc cgacgggagc tcctgcgaat gttccatgat agcctggcag 1020
   gggatctgga aggccgcagt ggggacccct cccttcaggc cacggttccg ttcctactgc 1080
   agctggcagt catgtcacca gctttgctgg gcactgtctc tggagagctt gtggattgcc 1140
   tcaagccccc agctgtgctg agccagctgc agcaacacct tcaaggattc ccccgagagg 1200
   agctggacaa catgttgaac ctggctgtgc acctggtgag ccaggcctct ggggcaggtg 1260
   cctaccgctt gctgcagttc ctggtggaca cagctatgcc tgcttcggtc attaccaccc 1320
   agggcctggc tgtgccagac accgtgcgtg aggcttgtga ccggctaatc cagctgctgc 1380
   tgctgcacct gcaaaaactg gttcatcacc ggggagggtc tcctggggaa ggggtgctag 1440
   gcccgccccc acctccccgc ttggtgccct ttttagatgc gctcaaaaac catgttggag 1500
   agctgtgtgg agagacgtta cgattggaac ggaagcgctt cctctggcag caccagctct 1560
   tgggcctgct gtctgtctat acccggccta gctgtggacc tgaggccttg ggccatctgc 1620
   tgagccgagc ccgaagccct gaagagttga gtttggccac ccagttatat gcagggctag 1680
   tggtcagcct ctctggcctc ctgcccctgg ctttccgaag ctgtctggct cgggtgcatg 1740
   cagggacatt acagcctccc ttcacggccc ggttcctgcg caacttggca ctgctagtag 1800
   ggtgggaaca gcagggtggc gagggccctg cagccctagg ggcgcacttt ggggaatctg 1860
   cctcagccca tctgtctgac ctggctcctc tcctgctaca tcctgaggag gaagtagctg 1920
   aagctgctgc ctctctcctg gccatttgtc cctttccttc tgaagcctta tccccctccc 1980
   agctcctggg actggtaagg gctggggtgc accgcttctt tgcctctctg aggctgcatg 2040
   gacccccagg tgtggcctca gcctgtcagc ttctcacccg cctgtctcag acatccccag 2100
   ctgggctcaa ggctgtcctg cagctgctgg ttgaaggagc cttacatcga ggcaacacag 2160
   aactgtttgg tgggcaagta gatggggaca atgagactct ctcagttgtt tcagcttctt 2220
   tggcttctgc ctccctgttg gacactaacc ggaggcacac tgcagctgtg ccaggtcctg 2280
   gagggatttg gtcagttttc catgctggag tcatcggccg tggcttaaag ccacccaagt 2340
   ttgtccagtc acgaaatcag caggaagtga tctataacac ccagagcctc ctcagcctcc 2400
   tggttcactg ctgcagtgcc ccagggggca ctgaatgtgg ggaatgctgg ggggcaccca 2460
   tcttgagtcc agaggcagcc aaagcagtgg cagtgacctt ggtggagagt gtgtgtcccg 2520
   atgcagctgg tgcagagctg gcctggcccc ccgaggaaca cgcccgggcc accgtggagc 2580
   gggatctccg cattggccgg cgcttccgcg aacagcccct gctctttgag ctgttaaagc 2640
   tggtagcagc tgcaccccca gccctgtgct actgttccgt gctgcttcgg gggctgctgg 2700
   ccgccctctt gggccattgg gaagcctctc gccaccctga cacgacccac tccccctggc 2760
   acctggaggc atcctgcacc ttagtggctg tcatggctga gggaagcctc ctgcctccgg 2820
   ccctgggtaa tatgcatgaa gtatttagcc aactggcacc tttcgaggtg cgtctgctgc 2880
   tgctcagtgt ctggggtttt ctccgggagc atgggccctt gcctcagaag ttcatcttcc 2940
   aatcagagcg gggtcgcttc attcgggact tctccaggga gggtggaggt gagggtggac 3000
   cccatctggc tgtgctgcac agtgtcctcc accgcaacat cgaccgccta ggtcttttct 3060
   ctggccgttt ccaggcacct tcaccgtcca ctctccttcg acaggggacg tagccttttc 3120
   ttgctctgga agcccaggga ggttgagcag tgagagaggg aagggactaa cgtgctccgg 3180
   aagggtggag gtttctcttc taagtccttg gtctaaagag cgctgtcact tttttctctc 3240
   ccactttttt ttttctaaat aaaatttgcc aacttgagaa aaccaaaaaa aaaaaaaaaa 3300

<210> 83
<211> 50
<212> DNA
<213> homo sapiens

<400> 83
gggaaggtcc caggtcttca ccagttttac aatgagttat cccaggccag 50

<210> 84
<211> 1584
<212> DNA
<213> homo sapiens

<400> 84
   acagagatgg cactgatgca ggaactgtat agcacaccag cctccaggct ggactccttc 60
   gtggctcagt ggctgcagcc ccaccgggag tggaaggaag aggtgctaga cgctgtgcgg 120
   accgtggagg agtttctgag gcaggagcat ttccagggga agcgtgggct ggaccaggat 180
   gtgcgggtgc tgaaggtagt caaggtgggc tccttcggga atggcacggt tctcaggagc 240
   accagagagg tggagctggt ggcgtttctg agctgtttcc acagcttcca ggaggcagcc 300
   aagcatcaca aagatgttct gaggctgata tggaaaacca tgtggcaaag ccaggacctg 360
   ctggacctcg ggctcgagga cctgaggatg gagcagagag tccccgatgc tctcgtcttc 420
   accatccaga ccagggggac tgcggagccc atcacggtca ccattgtgcc tgcctacaga 480
   gccctggggc cttctcttcc caactcccag ccaccccctg aggtctatgt gagcctgatc 540
   aaggcctgcg gtggtcctgg aaatttctgc ccatccttca gcgagctgca gagaaatttc 600
   gtgaaacatc ggccaactaa gctgaagagc ctcctgcgcc tggtgaaaca ctggtaccag 660
   caggcccatc atcctggatc cggccgaccc caccctcaac gtggcagaag ggtacagatg 720
   ggacatcgtt gctcagaggg cctcccagtg cctgaaacag gactgttgct atgacaacag 780
   ggagaacccc atctccagct ggaacgtgaa gagggcacga gacatccact tgacagtgga 840
   gcagaggggt tacccagatt tcaacctcat cgtgaaccct tatgagccca taaggaaggt 900
   taaagagaaa atccggagga ccaggggcta ctctggcctg cagcgtctgt ccttccaggt 960
   tcctggcagt gagaggcagc ttctcagcag caggtgctcc ttagccaaat atgggatctt 1020
   ctcccacact cacatctatc tgctggagac catcccctcc gagatccagg tcttcgtgaa 1080
   gaatcctgat ggtgggagct acgcctatgc catcaacccc aacagcttca tcctgggtct 1140
   gaagcagcag attgaagacc agcaggggct tcctaaaaag cagcagcagc tggaattcca 1200
   aggccaagtc ctgcaggact ggttgggtct ggggatctat ggcatccaag acagtgacac 1260
   tctcatcctc tcgaagaaga aaggagaggc tctgtttcca gccagttagt tttctctggg 1320
   agacttctct gtacatttct gccatgtact ccagaactca tcctgtcaat cactctgtcc 1380
   cattgtctac tgggaaggtc ccaggtcttc accagtttta caatgagtta tcccaggcca 1440
   gacgtggtag ctcacacctg taatcccaga actttgggag gccgaggtgg gaggagcgct 1500
   tgagccgagg agttcaagac cagcctgggt atcacaggga gaccccgtct ctacaaaata 1560
   aaaaaataat tcactgggaa aaaa 1584

<210> 85
<211> 50
<212> DNA
<213> homo sapiens

<400> 85
gggtgccaca cactcggtcc cgacatgatg gcgagcatgc gagtggtgaa 50

<210> 86
<211> 1260
<212> DNA
<213> homo sapiens

<400>  86
gggggtgggg tccccggggc ggggcggggc gcgctgtgtc gcgggtcgga gctcggtcct      60

gctggaggcc acgggtgcca cacactcggt cccgacatga tggcgagcat gcgagtggtg     120

aaggagctgg aggatcttca gaagaagcct cccccatacc tgcggaacct gtccagcgat     180

gatgccaatg tcctggtgtg gcacgctctc ctcctacccg accaacctcc ctaccacctg     240

aaagccttca acctgcgcat cagcttcccg ccggagtatc cgttcaagcc tcccatgatc     300

aaattcacaa ccaagatcta ccaccccaac gtggacgaga acggacagat ttgcctgccc     360

atcatcagca gtgagaactg gaagccttgc accaagactt gccaagtcct ggaggccctc     420

aatgtgctgg tgaatagacc gaatatcagg gagcccctgc ggatggacct cgctgacctg     480

ctgacacaga atccggagct gttcagaaag aatgccgaag agttcaccct ccgattcgga     540

gtggaccggc cctcctaact catgttctga ccctctgtgc actggatcct cggcatagcg     600

gacggacaca cctcatggac tgaggccaga gccccctgtg gcccattccc cattcatttt     660

tcccttctta ggttgttagt cattagtttg tgtgtgtgtg tggtggaggg aagggagcta     720

tgagtgtgtg tgttgtgtat ggactcactc ccaggttcac ctggccacag gtgcaccctt     780

cccacaccct ttacattccc cagagccaag ggagtttaag tttgcagtta caggccagtt     840

ctccagctct ccatcttaga gagacaggtc accttgcagg cctgcttgca ggaaatgaat     900

ccagcagcca actcgaatcc ccctagggct caggcactga gggcctgggg acagtggagc     960

atatgggtgg gagacagatg gagggtaccc tatttacaac tgagtcagcc aagccactga    1020

tgggaatata cagatttagg tgctaaaccg tttattttcc acggatgagt cacaatctga    1080

agaatcaaac ttccatcctg aaaatctata tgtttcaaaa ccacttgcca tcctgttaga    1140

ttgccagttc ctgggaccag gcctcagact gtgaagtata tatcctccag cattcagtcc    1200

agggggagcc acggaaacca tgttcttgct taagccatta aagtcagaga tgaattctgg    1260


<210>  87
<211>  50
<212>  DNA
<213>  homo sapiens

<400>  87
aaaaaattag ctgggcgtgg tggcggatgc ctgtagtccc agctactcag                50


<210>  88
<211>  3060
<212>  DNA
<213>  homo sapiens

<400>  88
gctggcccgg tttcctggcg acgcggccct gcaggcggtt gcgttccccg tcgttaccct      60

ctttctcttc ccgacgcgtg agttaggccg taatgccttg gctgctctca gcccccaagc     120

tggttcccgc tgtagcaaac gtccgcggcc tctcaggatg tatgttgtgt tcacagcgaa 180

ggtactccct tcagcctgtc ccagaaagga ggattccaaa ccgatactta ggccagccca 240

gcccctttac acacccacac ctcctcagac caggggaggt aactccagga ctatctcagg 300

tggaatatgc acttcgcaga cacaaactaa tgtctctgat ccagaaggaa gctcaagggc 360

agagtgggac agaccagaca gtggttgtgc tctccaaccc tacatactac atgagcaacg 420

atattcccta tactttccac caagacaaca atttcctgta cctatgtgga ttccaagagc 480

ctgatagcat tcttgtcctt cagagcctcc ctggcaaaca attaccatca cacaaagcca 540

tactttttgt gcctcggcga gatcccagtc gagaactttg ggatggtccg cgatctggca 600

ctgatggagc aatagctcta actggagtag acgaagccta tacgctagaa gaatttcaac 660

atcttctacc aaaaatgaaa gctgagacga acatggtttg gtatgactgg atgaggccct 720

cacatgcaca gcttcactct gactatatgc agcccctgac tgaggccaaa gccaagagca 780

agaacaaggt tcggggtgtt cagcagctga tacagcgcct ccggctgatc aagtctcctg 840

cagaaattga acgaatgcag attgctggga agctgacatc acaggctttc atagaaacca 900

tgttcaccag taaagcccct gtggaagaag cctttcttta tgctaagttt gaatttgaat 960

gccgggctcg tggcgcagac attttagcct atccacctgt ggtggctggt ggtaatcggt 1020

caaacacttt gcactatgtg aaaaataatc aactcatcaa ggatggggaa atggtgcttc 1080

tggatggagg ttgtgagtct tcctgctatg tgagtgacat cacacgtacg tggccagtca 1140

atggcaggtt caccgcacct caggcagaac tctatgaagc cgttctagag atccaaagag 1200

attgtttggc cctctgcttc cctgggacaa gcttggagaa catctacagc atgatgctga 1260

ccctgatagg acagaagctt aaagacttgg ggatcatgaa gaacattaag gaaaataatg 1320

ccttcaaggc tgctcgaaaa tactgtcctc atcatgttgg ccactacctc gggatggatg 1380

tccatgacac tccagacatg ccccgttccc tccctctgca gcctgggatg gtaatcacaa 1440

ttgagcccgg catttatatt ccagaggatg acaaagatgc cccagagaag tttcggggtc 1500

ttggtgtacg aattgaggat gatgtagtgg tgactcagga ctcacctctc atcctttctg 1560

cagactgtcc caaagagatg aatgacattg aacagatatg cagccaggct tcttgacctt 1620

cactgcggcc cacatgcacc tcaggttcaa aatgggtgtc ttctggcagc cctgcacgtg 1680

tgctttctga gtgtctctgt gtgtgcatta atatatgcat tccatttggg agcatagcag 1740

ctgtgtgaat gtatgtaatt gtgtgtgggg ggttttttgt tttaagtagt tagaagtctg 1800

ggaaaatgaa tttttgaata gtatgttact gcagctttgg taacattaat tctatagaat 1860

taatgatcag agcaagttta atttttaaac ataaaggtct tggttacaca tgtccatgca 1920

ttccagttaa cacatttaaa catatagaaa attcacttct tctttcaagt ccttcccttt 1980

ctatactttt gctgagatca acccaatatc ataagaagtt tgtgtgatgc ctgtattttt 2040

agctaattac caaggtctct gcctatgcaa aaattcatct ttcggtgatt gtgggcggcc 2100

aatacatacc ttctgtaact cctccctacc tattctagat cctgcatatc tactggagcc 2160

atatgagccc tcaggggcac tggtgtcctg cagtcttact agtcagccac agtccaggtt 2220

ccagcaacac tagctaccta ccaaacaaaa cacttctgag cttttcttgc catttggttg 2280

cctgggacct ggaaagtgtt tagattcttg ataaagatgg aaatggaaga gaaagaaaat 2340

tatatatgta tatactaaag atccagacat cagggctggg cacagcggtt ccgcctgtaa 2400

tcccagcact ttgggaggtc caggcaggtg gatctcttga gaccaggagg tcaagaccag 2460

cctggccaac gtggtgaaac actgtctcta ctaaaaatac aaaaatgagc tgggtgtgat 2520

gatacatgcc tgtaatcctg gctacttggg aggctgaggc atgagaattg cttgaacctg 2580

ggaggtggag gttgcagtga gctgagctca tgcgactgca ctccagcctg ggtgactgag 2640

caagactctg cctcaaaaaa acaaataaat aaataaaatc ctgacatcct gccatctttt 2700

ctcttcaatt taaagttgat ttcttaatga tgacttcaaa aattaataaa aagaaaagaa 2760

aaaaatgatt tctggccggg cgtggtggct caagcctgta atcccagcac tttgggaggc 2820

cgaggtgggc ggatcacgag gtcaggagat tgagacctcc tggctaacac ggtgaaaccc 2880

cgtctttact aaaaatacaa aaaaaattag ctgggcgtgg tggcggatgc ctgtagtccc 2940

agctactcag gaggctgagg caggagaatg gcgtgaaccc agaaggcaga gcttgcagtg 3000

agccaagatc gcgccactgc actccagcct gggtgacaga gcgagactcc gtctcaaaaa 3060

<210> 89
<211> 50
<212> DNA
<213> homo sapiens

<400> 89
caccatccag gtggagaccc tgagaaggac cctgggagct ctgggaattt 50

<210> 90
<211> 4018
<212> DNA
<213> homo sapiens

<400> 90
caggcagcgc tgcgtcctgc tgcgcacgtg ggaagccctg gccccggcca cccccgcgat 60

gccgcgcgct ccccgctgcc gagccgtgcg ctccctgctg cgcagccact accgcgaggt 120

gctgccgctg gccacgttcg tgcggcgcct ggggccccag ggctggcggc tggtgcagcg 180

cggggacccg gcggctttcc gcgcgctggt ggcccagtgc ctggtgtgcg tgccctggga 240

cgcacggccg ccccccgccg cccctccttc cgccaggtgc tcctgcctga aggagctggt 300

ggcccgagtg ctgcagaggc tgtgcgagcg cggcgcgaag aacgtgctgg ccttcggctt 360

cgcgctgctg gacgggcccc gcgggggccc ccccgaggcc ttcaccacca gcgtgcgcag 420
ctacctgccc aacacggtga ccgacgcact gcgggggagc ggggcgtggg ggctgctgct 480
gcgccgcgtg ggcgacgacg tgctggttca cctgctggca cgctgcgcgc tctttgtgct 540
ggtggctccc agctgcgcct accaggtgtg cgggccgccg ctgtaccagc tcggcgctgc 600
cactcaggcc cggcccccgc cacacgctag tggaccccga aggcgtctgg gatgcgaacg 660
ggcctggaac catagcgtca gggaggccgg ggtccccctg ggcctgccag ccccgggtgc 720
gaggaggcgc gggggcagtg ccagccgaag tctgccgttg cccaagaggc ccaggcgtgg 780
cgctgcccct gagccggagc ggacgcccgt tgggcagggg tcctgggccc acccgggcag 840
gacgcgtgga ccgagtgacc gtggtttctg tgtggtgtca cctgccagac ccgccgaaga 900
agccacctct ttggagggtg cgctctctgg cacgcgccac tcccacccat ccgtgggccg 960
ccagcaccac gcgggccccc catccacatc gcggccacca cgtccctggg acacgccttg 1020
tcccccggtg tacgccgaga ccaagcactt cctctactcc tcaggcgaca aggagcagct 1080
gcggccctcc ttcctactca gctctctgag gcccagcctg actggcgctc ggaggctcgt 1140
ggagaccatc tttctgggtt ccaggccctg gatgccaggg actccccgca ggttgccccg 1200
cctgccccag cgctactggc aaatgcggcc cctgtttctg gagctgcttg ggaaccacgc 1260
gcagtgcccc tacggggtgc tcctcaagac gcactgcccg ctgcgagctg cggtcacccc 1320
agcagccggt gtctgtgccc gggagaagcc ccagggctct gtggcggccc ccgaggagga 1380
ggacacagac ccccgtcgcc tggtgcagct gctccgccag cacagcagcc cctggcaggt 1440
gtacggcttc gtgcgggcct gcctgcgccg gctggtgccc ccaggcctct ggggctccag 1500
gcacaacgaa cgccgcttcc tcaggaacac caagaagttc atctccctgg ggaagcatgc 1560
caagctctcg ctgcaggagc tgacgtggaa gatgagcgtg cgggactgcg cttggctgcg 1620
caggagccca ggggttggct gtgttccggc cgcagagcac cgtctgcgtg aggagatcct 1680
ggccaagttc ctgcactggc tgatgagtgt gtacgtcgtc gagctgctca ggtctttctt 1740
ttatgtcacg gagaccacgt ttcaaaagaa caggctcttt ttctaccgga agagtgtctg 1800
gagcaagttg caaagcattg gaatcagaca gcacttgaag agggtgcagc tgcgggagct 1860
gtcggaagca gaggtcaggc agcatcggga agccaggccc gccctgctga cgtccagact 1920
ccgcttcatc cccaagcctg acgggctgcg gccgattgtg aacatggact acgtcgtggg 1980
agccagaacg ttccgcagag aaaagagggc cgagcgtctc acctcgaggg tgaaggcact 2040
gttcagcgtg ctcaactacg agcgggcgcg gcgccccggc ctcctgggcg cctctgtgct 2100
gggcctggac gatatccaca gggcctggcg caccttcgtg ctgcgtgtgc gggcccagga 2160
cccgccgcct gagctgtact ttgtcaaggt ggatgtgacg ggcgcgtacg acaccatccc 2220
ccaggacagg ctcacggagg tcatcgccag catcatcaaa ccccagaaca cgtactgcgt 2280

gcgtcggtat gccgtggtcc agaaggccgc ccatgggcac gtccgcaagg ccttcaagag 2340

ccacgtctct accttgacag acctccagcc gtacatgcga cagttcgtgg ctcacctgca 2400

ggagaccagc ccgctgaggg atgccgtcgt catcgagcag agctcctccc tgaatgaggc 2460

cagcagtggc ctcttcgacg tcttcctacg cttcatgtgc caccacgccg tgcgcatcag 2520

gggcaagtcc tacgtccagt gccaggggat cccgcagggc tccatcctct ccacgctgct 2580

ctgcagcctg tgctacggcg acatggagaa caagctgttt gcggggattc ggcgggacgg 2640

gctgctcctg cgtttggtgg atgatttctt gttggtgaca cctcacctca cccacgcgaa 2700

aaccttcctc aggaccctgg tccgaggtgt ccctgagtat ggctgcgtgg tgaacttgcg 2760

gaagacagtg gtgaacttcc ctgtagaaga cgaggccctg ggtggcacgg cttttgttca 2820

gatgccggcc cacggcctat tcccctggtg cggcctgctg ctggataccc ggaccctgga 2880

ggtgcagagc gactactcca gctatgcccg gacctccatc agagccagtc tcaccttcaa 2940

ccgcggcttc aaggctggga ggaacatgcg tcgcaaactc tttggggtct tgcggctgaa 3000

gtgtcacagc ctgtttctgg atttgcaggt gaacagcctc cagacggtgt gcaccaacat 3060

ctacaagatc ctcctgctgc aggcgtacag gtttcacgca tgtgtgctgc agctcccatt 3120

tcatcagcaa gtttggaaga accccacatt tttcctgcgc gtcatctctg acacggcctc 3180

cctctgctac tccatcctga aagccaagaa cgcagggatg tcgctggggg ccaagggcgc 3240

cgccggccct ctgccctccg aggccgtgca gtggctgtgc caccaagcat tcctgctcaa 3300

gctgactcga caccgtgtca cctacgtgcc actcctgggg tcactcagga cagcccagac 3360

gcagctgagt cggaagctcc cggggacgac gctgactgcc ctggaggccg cagccaaccc 3420

ggcactgccc tcagacttca agaccatcct ggactgatgg ccacccgccc acagccaggc 3480

cgagagcaga caccagcagc cctgtcacgc cgggctctac gtcccaggga gggaggggcg 3540

gcccacaccc aggcccgcac cgctgggagt ctgaggcctg agtgagtgtt tggccgaggc 3600

ctgcatgtcc ggctgaaggc tgagtgtccg gctgaggcct gagcgagtgt ccagccaagg 3660

gctgagtgtc cagcacacct gccgtcttca cttccccaca ggctggcgct cggctccacc 3720

ccagggccag cttttcctca ccaggagccc ggcttccact ccccacatag gaatagtcca 3780

tccccagatt cgccattgtt cacccctcgc cctgccctcc tttgccttcc acccccacca 3840

tccaggtgga gaccctgaga aggaccctgg gagctctggg aatttggagt gaccaaaggt 3900

gtgccctgta cacaggcgag gaccctgcac ctggatgggg gtccctgtgg gtcaaattgg 3960

ggggaggtgc tgtgggagta aaatactgaa tatatgagtt tttcagtttt gaaaaaaa 4018

<210> 91
<211> 50
<212> DNA

<213>  homo sapiens

<400>  91
caacctttgc actccactgt gcaatgctgg ccctgcacgc tggggctgtt          50


<210>  92
<211>  733
<212>  DNA
<213>  homo sapiens

<400>  92
aaacagcagg aaatagaaac ttaagagaaa tacacacttc tgagaaactg aaacgacagg     60

ggaaaggagg tctcactgag caccgtccca gcatccggac accacagcgg cccttcgctc    120

cacgcagaaa accacacttc tcaaaccttc actcaacact tccttcccca aagccagaag    180

atgcacaagg aggaacatga ggtggctgtg ctgggggcac cccccagcac catccttcca    240

aggtccaccg tgatcaacat ccacagcgag acctccgtgc ccgaccatgt cgtctggtcc    300

ctgttcaaca ccctcttctt gaactggtgc tgtctgggct tcatagcatt cgcctactcc    360

gtgaagtcta gggacaggaa gatggttggc gacgtgaccg gggcccaggc ctatgcctcc    420

accgccaagt gcctgaacat ctgggccctg attctgggca tcctcatgac cattggattc    480

atcctgttac tggtattcgg ctctgtgaca gtctaccata ttatgttaca gataatacag    540

gaaaaacggg gttactagta gccgcccata gcctgcaacc tttgcactcc actgtgcaat    600

gctggccctg cacgctgggg ctgttgcccc tgcccccttg gtcctgcccc tagatacagc    660

agtttatacc cacacacctg tctacagtgt cattcaataa agtgcacgtg cttgtgaaaa    720

aaaaaaaaaa aaa                                                       733


<210>  93
<211>  50
<212>  DNA
<213>  homo sapiens

<400>  93
gctgcctgga aaattctcgg atatgaccgc tgtgatgcgg gctggttggc          50


<210>  94
<211>  1759
<212>  DNA
<213>  homo sapiens

<400>  94
gtgaggagaa agagcgctac gttcacttga tctccagctt ccaacttaag cagaacttga     60

gagcatccga actcctggat ttcaggacaa gtgaagaaga ttctttgggc tataaagatg    120

aagagtctac ttcttctggt gctgatttca atctgctggg ctgatcatct ttcagacaac    180

tatactctgg atcatgacag agctattcac atccaagcag aaaatggccc ccatctactt    240

gtggaagcag agcaagccaa ggtgttttca cacagaggtg gcaatgttac actgccatgt    300

aaattttatc gagaccctac agcatttggc tcaggaatcc ataaaatccg aattaagtgg 360

accaagctaa cttcggatta cctcaaggaa gtggatgttt ttgtttccat gggataccac 420

aaaaaaacct atggaggcta ccagggtaga gtgtttctga agggaggcag tgatagtgat 480

gcttctctgg tcatcacaga cctcactctg gaagattatg ggagatataa gtgtgaggtg 540

attgaaggat tagaagatga tactgttgtg gtagcactgg acttacaagg tgtggtattc 600

ccttactttc cacgactggg gcgctacaat ctcaattttc acgaggcgca gcaggcgtgt 660

ctggaccagg atgctgtgat cgcctccttc gaccagctgt acgacgcctg gcggggcggg 720

ctggactggt gcaatgccgg ctggctcagt gatggctctg tgcaatatcc catcacaaag 780

cccagagagc cctgtggggg ccagaacaca gtgcccggag tcaggaacta cggattttgg 840

gataaagata aaagcagata tgatgttttc tgttttacat ccaatttcaa tggccgtttt 900

tactatctga tccaccccac caaactgacc tatgatgaag cggtgcaagc ttgtctcaat 960

gatggtgctc agattgcaaa agtgggccag atatttgctg cctggaaaat tctcggatat 1020

gaccgctgtg atgcgggctg gttggcggat ggcagcgtcc gctaccccat ctctaggcca 1080

agaaggcgct gcagtcctac tgaggctgca gtgcgcttcg tgggtttccc agataaaaag 1140

cataagctgt atggtgtcta ctgcttcaga gcatacaact gaatgtgccc ttagagcgca 1200

tcagttttaa agtcattaag aacatgtgaa aggtgttttt tttttccaat atgaactcat 1260

gcaagttacc aaaactgtga taaccctttt ttacttactg taaagagtca ttttcataag 1320

atcaattcat tgatttgttt tttgtaaagc tatcattcaa tatatattat aaattaatat 1380

aaatttaagg gaagctctat gtaaggagac ttagagccaa actgtttaag ctgtatcatc 1440

ccaacaaagt atcctttcat gaacggggca tgcaatagct taagaattgc taggattaaa 1500

ttaaggaaag taaagctact cagagcaaca ggttccacaa gcacaaactt tacacatttg 1560

tacaattttg aaatgcacta caataaacaa attagagcaa cacatttgaa atacaggctt 1620

ctttacataa actgagaggt tatacaaaac tcagtttcac aagggaacaa tctatacctt 1680

tctaaaagtt aatatttcaa gtctctaata ggcagaatat tttactcttt aaaatcctgc 1740

ctttctgacc aaaaaaaaa 1759

<210> 95
<211> 50
<212> DNA
<213> homo sapiens

<400> 95
agggcatggt ggtgcatgcc tgtatcccag ctacttggga ggctgaagca 50

<210> 96
<211> 8431

<212>  DNA
<213>  homo sapiens

<400>  96
gtcctggagc ttgacgcgct ggagcttgag aggtctttca ggactgattt tggtggaaga      60

agacagcagg cggtgggcat tcactgtgct ggtgggaagt ctggttacat taagaaagga     120

attctagcaa agttattctg taaatacaac agaatcagtt gcttttgtta aatatattgg     180

tgcaagttca acatggaggc aaatcactgc tccctgggtg tgtatccatc ttacccagac     240

ctggtcatcg atgtcggaga agtgactctg ggagaagaaa acagaaaaaa gctacagaaa     300

actcagagag accaagagag ggcgagagtt atacgggccg cgtgtgcttt attaaactca     360

ggaggaggag tgattcagat ggaaatggcc aacagggatg agcgtcccac agagatggga     420

ctggatttag aagaatcctt gagaaagctt attcagtatc cgtatttgca ggctttcttt     480

gagactaagc aacacggaag gtgtttttat atttttgtta aatcttggag tggtgatcct     540

ttccttaaag atggttcttt caattcccgc atttgcagcc ttagttcttc attatactgt     600

agatctggca cctctgtgct tcacatgaat tcaagacagg cattcgattt cctgaagacc     660

aaggaaagac agtccaaata taatctgatt aatgaagggt ctccacctag taaaattatg     720

aaagctgtat accagaacat atctgagtca aatcctgcat atgaagtttt ccaaactgac     780

actattgaat atggtgaaat cctatctttt cctgagtctc catccataga gtttaaacag     840

ttctctacaa aacatatcca acaatatgta gaaaatataa ttccagagta catctctgca     900

tttgcaaaca ctgagggagg ctatcttttt attggagtgg atgataagag taggaaagtc     960

ctgggatgtg ccaaagaaca ggttgaccct gactctttga aaaatgtaat tgcaagagca    1020

atttctaagt tgcccattgt tcatttttgc tcttcaaaac ctcgggtaga gtacagcacc    1080

aaaatcgtag aagtgttttg tgggaaagag ttgtatggct atctctgtgt gattaaagtg    1140

aaggcattct gttgtgtggt gttctcggaa gctcccaagt catggatggt gagggagaag    1200

tacatccgcc ccttgacaac tgaggaatgg gtagagaaaa tgatggacgc agatccagag    1260

tttcctccag actttgctga ggcctttgag tctcagttga gtctatctga cagtccttca    1320

ctttgcagac cagtgtattc taagaaaggt ctggaacaca aagctgatct acaacaacat    1380

ttatttccag ttccaccagg acatttggaa tgtactccag agtccctctg gaaggagctg    1440

tctttacagc atgaaggact aaaggagtta atacacaagc aaatgcgacc tttctcccag    1500

ggaattgtga tcctctctag aagctgggct gtggacctga acttgcagga gaagccagga    1560

gtcatctgtg atgctctgct gatagcacag aacagcaccc ccattctcta caccattctc    1620

agggagcagg atgcagaggg ccaggactac tgcactcgca ccgcctttac tttgaagcag    1680

aagctagtga acatgggggg ctacaccggg aaggtgtgtg tcagggccaa ggtcctctgc    1740

ctgagtcctg agagcagcgc agaggccttg gaggctgcag tgtctccgat ggattaccct    1800

gcgtcctata gccttgcagg cacccagcac atggaagccc tgctgcagtc cctcgtgatt 1860

gtcttactcg gcttcaggtc tctcttgagt gaccagctcg gctgtgaggt tttaaatctg 1920

ctcacagccc agcagtatga gatattctcc agaagcctcc gcaagaacag agagttgttt 1980

gtccacggct tacctggctc agggaagacc atcatggcca tgaagatcat ggagaagatc 2040

aggaatgtgt ttcactgtga ggcacacaga attctctacg tttgtgaaaa ccagcctctg 2100

aggaacttta tcagtgatag aaatatctgc cgagcagaga cccgggaaac tttcctaaga 2160

gaaaaatttg aacacattca acacatcgtc attgacgaag ctcagaattt ccgtactgaa 2220

gatggggact ggtataggaa ggcaaaaacc atcactcaga gagaaaagga ttgtccagga 2280

gttctctgga tctttctgga ctactttcag accagtcact tgggtcacag tggccttccc 2340

cctctctcag cacagtatcc aagagaagag ctcaccagag tagttcgcaa tgcagatgaa 2400

atagccgagt acatacaaca agaaatgcaa ctaattatag aaaatcctcc aattaatatc 2460

ccccatgggt atctggcaat tctcagtgaa gctaaatggg ttccaggtgt tccaggcaac 2520

acaaagatta ttaaaaactt tactttggag caaatagtga cctatgtggc agacacctgc 2580

aggtgcttct ttgaaagggg ctattctcca aaggatgttg ctgtgcttgt cagcaccgtg 2640

acagaagtgg agcagtatca gtctaagctc ttgaaagcaa tgaggaagaa aatggtggtg 2700

cagctcagtg atgcatgtga tatgttgggt gtgcacattg tgttggacag tgtccggcga 2760

ttctcaggcc tggaaaggag catagtgttt gggatccatc caaggacagc tgacccagct 2820

atcttaccca atattctgat ctgtctggct tccagggcaa aacagcacct atatattttt 2880

ctgtgaagtg actattagga agaactccaa accaaaatac tgtgtaaatg tctatgggtg 2940

acagtctgct gatggtagaa acctttcttt ttagttcaca agtcagttag agatttggac 3000

agagctgaca caaagagttt ggagctcccc catttctggc tctcctttca ggggttcctc 3060

ccccaactct tttcagcagt ggtggctgcc ctccattctg acccctgact cttgcagcca 3120

gaaagatggt ggttttctaa aggaacttta gctgtgctgc acaatgcaga cctgtgtctt 3180

gctctctggg taaaagccat aaaaataaga aactcagcct gtggcctttc ttccaaggct 3240

ggagttctcg agttctttta tgtgacttcg tgtagtttgt tgctttaaaa aatttgtcca 3300

gaattgtttt ctgcagaagc atggtctgtt aggagcttac aggccatagg agaagcagtt 3360

gtttcctgaa tttatctttg ctgtattcat ttagggcttg ggagagtccc aagataattc 3420

agtcactgtc agattaatca tttcggcaga acaaacaata ttgttatgat tatttaatcc 3480

ttaaaattgt gatctccaga gtttgttatc agaataaccc agaccaaggc ttaattgtaa 3540

tagtgaacat taatggtacc tttacagaga aattataggc caagagaaaa tgctggcttt 3600

cagtagaagt taatattaga aacccaaatc tggttctgaa agtgtgtatc agatgtacgg 3660

tgaacaaact tgggaaagat tttctttaaa aatcaatgag cgttggccag gcacggtggc 3720

tcacacctgt aatcccagct gtttgggagg ctgaggcagg tggatcacct gaggtcagga 3780

gttcaagacc agcctggcca acatggagaa accccatctc tactaaaaat acaaaaatta 3840

gcagggcatg gtggtgcatg cctgtatccc agctacttgg gaggctgaag catgagaatc 3900

acttgaatcc tggaggcaga ggttgcagtg agctgagatc atgtcactgt actccagcct 3960

gggcaacaga gtgagactgt ctcaaaaaga agaagaagaa aaaaaatgca gtgagtttat 4020

gttgttacat tgtaacatct cagcgtagct gtatgcatta ctctatagca taactactta 4080

agttttatgt cattcttgaa ctgtgatttt tttttctatt tgctttcata gtttggtggg 4140

ccattgttat ggactgaatg tttgtgtccc atcctcaccc ctaaattccc atgttgaagc 4200

cccaaccttc actgtggagc tggggctgct aaggaagtaa ttaaggttac acgaagtcac 4260

agtggagaat ctgatctgct aagactggtg tccttatagg gagagacccc ggagagcttg 4320

ttcccttcct ccctgtgcac acaaacaaga gggcgtggga gcacacagag agatggcagc 4380

cacctacaag ccaagaggag aagcctcaca atgaaccccc ccccccaccc tgctggcacc 4440

tatgtcttcg acttccagcc cccagactgt gagaaataaa tttctgttgg ttcagccatc 4500

cagtctctag tgttttgtta ttgcagcctg agaacacagc tgtatgatca ttcatcaaac 4560

taaaaacact gatacttaac aatgctaatg caattactta tttgcttttc agtctctaca 4620

aaacattcta aaacactaat ctaaatacta acaataaaat atttgcataa ctaatgaaaa 4680

ttaagaaacc acatgcccaa tattccattt ataagtaatc ctatttggct gatttccctc 4740

catattgtaa gttttgaact tttctgccag tctttctcct atttgtgtta actcatggaa 4800

ttccagttgt ctagccttga aattgtaggc tcttctaact tgagcaaaat ctgacagatc 4860

agcaaaataa ggagaaaaat atttcttctt tctttctggc tgcattaaat gagatacagc 4920

tcagcaccaa aaagctatct ttgtaaatgt tcttagtaaa ctccctggtc tttggagtta 4980

tttcagcctt tgagagtaat tctttaaaag tctaattctg actggttgaa tctctctata 5040

tgatttattg ccctagtaat atttgaaaca gtatcatatt ttaaaatata taaatattta 5100

tgaatttttg ttcagcccaa agaggattac tgacgctttc ttctttttcc atatgatgag 5160

gaaatgttta gcctatatat ttattgcatt tctgttttaa ataaaaaatt gagagtttgt 5220

gtttcattaa actgaaaaaa aaggttctgt aaaataaatg attaaatgga ggaaaaggag 5280

aagactgagg ccagcaactc catgatacag ccaaaaatga gccagtaaaa gattaagcaa 5340

gattaactgt aaattttttt tttttttttt ttttttaaag agaaaaggtc tcactgtgtt 5400

tcccaggctg gagggtagtg gtgtgatcat agttcactag agccctgaac tcctgggctc 5460

gggtgatcct cttgccttag cctcccattg ctgggattat aggcatgagc cactgtgtca 5520

ggcttaattg ggttttgatg ggtacctgcc tatctctgta atttggctct agtcatatag 5580

gacctgcttc attaaagatc tgaaaatcta tccagccatt gtaaaatgaa acaaaatgtt 5640

ttgaaaattt aaattagcct tttattttag ttcagacata tagaaaagtt gcaagcatag 5700

tacagaaagt tcctatatac cccacttagt gccctccttt ttaaaatttt tttaaaccta 5760

gcttatttcc tttaagattt ctgagaattc caggaatgct cacagatctg tccttgtagt 5820

ctcctgccac ttcttgctat ggagtgctgt accaaggtct ctgctagttt ctagttggct 5880

tgacataatg actttcctaa acttcttctg ctctgaatct caagcatgga aagcggtcac 5940

atcttttgtt tcacgaatta acagtcaaca cctggcccta cttttaacaa aacttagtaa 6000

agccttgagc atagtgaaag acaaatattt aaattatata ttagcaggag gctgaggtgg 6060

gaggatcata tgaggccagc ctgggcagca tagcatgacc ccccctcgaa gaagacaaca 6120

aaaaattatc tgggtgtggt ggtgtgctcc tgcagtccta gctattccag aggccgaggc 6180

gagaggatcg cttgattcca ggagttcaag gctgtagtta gcgataatcg tgtcactgaa 6240

tcctagcctg ggcaacagaa taagacccca tgtctaacaa aataataata aaacaatcaa 6300

aagtttaaaa ttagttatta aatgtcagtg tgcccagagt cctttgacca cagatgatcc 6360

tgatggcctt ctctcgcctt gtccctgtgc ctccttctac catttttctt cctgagattc 6420

aaaagtaagt agcggacagg gttcccattt accactaaat acttcagtgg gtgtttccta 6480

aaaggacatt tatagtacca tcatcaaaat cagtaaatta accctgatac tattatctaa 6540

tctgtagact ttattgaatt atatcagttg tgcctcaagt gtctttatgt ttttttctca 6600

aaagagaaaa agttctggtc caggatttga tctaagatca tgcattgtat tgagctttcc 6660

tttcttttaa gtctcattta atctggagat gttaatctgt ctttgtcata tctttgacac 6720

tattgaagag gactggccta ttattttgca gagtatctga ttttgggtca tttcatgtgc 6780

tctcatgatt agattcaggt tatgcatttt tggcatgaat tccatagaag ggctgttacg 6840

gttctcagtt cacggtatta gaaggcactt gatagtgatt agttccattc ctgatgatgt 6900

taactctgat catttggata aagtggcgtc tgccaggttt ccctactgtg cagttacaat 6960

ttgctttttg taattaataa agtgtgtgtg tgtgtgtgtg tggagggggg gtgtttttgc 7020

ttctgaacgc ataatatttg agattttttt ctaattaaaa agtaattatt caaagagttc 7080

agagaactga tactgatcac attctttgac cccacatgca aatacattat aaactagtaa 7140

taaaacggta actagaaaaa tgttcatatc ttttggaatt aaaaaagtgt ttaactcgtg 7200

gatcaaagca gaaattctgt aaccattgtc acagcaggtt ctaacaagca agtttaagga 7260

ggagagaatt tcagtcctag agaatttcag tcttattcaa atgctttcct agaaagagga 7320

aacagttcct aactaatttc atgaggctgg cataatcttg ataacaagtc tgataggtaa 7380

agtatgaaag gaaaattgca ggtcactcaa ttatcactca aatatctaca tgaaaaattc 7440

taaacaaaat tttagcaaac aaaatccaac aatttgtaaa agtgataata caccacgacc 7500

aaggtggttc aagtcaggaa ttcaacgttg attttggcgt taaaaaatca atcatcaggc 7560

ccagtgaggt ggctcactcc tgtaatccca gactttctgg aggctgaagg aggaaagatc 7620

cttcaggcca gaagtttgag tctgcactga gctgtgactg catcaccgca ctctagcctg 7680

ggtgccagtt tgagaccctg tctccaagaa aaaaaaatta atcagtgtaa ttcaccacag 7740

attaagagtt caaaagagaa aaatcacatg atcatctgaa tagaggaaaa agcttttgat 7800

aaagttcaac atccattcat aataaggact ctcagcaact tagaagtata aaaacacttt 7860

tggaaatcaa caaagggaat ttacagcatg aacaaacagc aagccagcat ggtggctcac 7920

acccgtaatc tcagcacttc cagaggccaa ggcaggagaa tcacttgagc ccaggggtta 7980

gagacaagcc ttggcaacat agcaagatcc tggctctaca aaaaataaaa taaaatgaaa 8040

taaaatttaa aaattagctg ggtttggtgg tgtgtgcctg tagtcccagc tactgggagg 8100

ctgaggtgag aggatcactg gagcccagga ggtgaaggct gcagtgggct aaaaacaacc 8160

agaacaaccc cccaaccccc tccccgcaaa acactataaa gaatagcaac acaaggttca 8220

atgtaaacct tgatatggtg ttttacctct gaggagaggc ttattgttaa tattctttag 8280

ccgtgtaagg ggttcacaga tgttcatact tttgttcttt ataccttata tgtattctat 8340

aaatattgtt tctactcaat agttaataaa aacataattt aaaagttcaa aaaaaaaaaa 8400

aaaaaaaaaa aaaaaaaaaa aaaaaaaaaa a 8431

<210> 97
<211> 50
<212> DNA
<213> homo sapiens

<400> 97
gcttctccag atccccagcg ccaggagtgg tttgcccggt acttcacatt 50

<210> 98
<211> 3519
<212> DNA
<213> homo sapiens

<400> 98
ttttcatttg tgcctgtgat tggctcagct ctgcgtgtga agagggaaaa ggcttccgtt 60

tatttagcat aatgatggac caaaacaaaa gaagaaatca tttcactcat gcttaggtaa 120

tatatgcttt aaacactaaa aatgatgagc atgaatctgc aattcaagcc ctcaaagatg 180

ctcatgaaga agaaattcaa caaattcttg ctgaaacaag agaaaaaata ttgcagtata 240

aaagcaaagt aacagaggag ctagacctta gaagaaagat tcaagtttta gaatcatcat 300

tagaagatca cataaaaatg aagcagcagg ctttgacaga atttgaagct tataagcaca 360

gagttgagga catgcaactt tgtgcagaag cccagcatgt ccaacgcata gtgaccatgt 420

ctagagaagt cgaagagatt agaaggaaat ttgaagaaaa attacggagc tttggacaac 480

ttcaagtaca gtttgaaaaa gacaaacgat tggcattgga agacttgcaa gctgctcaca 540

gacgggagat acaagagcta ttgaagtcac agcaggatca cagtgcctca gtaaataaag 600

gccaggaaaa ggcagaggaa ctacacagaa tggaggtgga gtccctaaac aaaatgcttg 660

aggagctaag acttgaacgg aagaaactaa ttgaggatta tgaaggcaag ttgaataaag 720

ctcagtcctt ttatgaacgt gagcttgata ctttgaaaag gtcacagctt tttacagcag 780

aaagcctaca ggccagcaaa gaaaaggaag ctgatcttag aaaagaattt cagggacaag 840

aagcaatttt acgaaaaact ataggaaaat taaagacaga gttacagatg gtacaggatg 900

aagctggaag tcttcttgac aaatgccaaa agcttcagac ggcacttgcc atagcagaga 960

acaatgttca ggttcttcaa aaacagcttg atgatgccaa ggagggagaa atggccctat 1020

taagcaagca caaagaagtg gaaagtgagc tagcagctgc cagagaacgt ttacaacagc 1080

aagcttcaga tcttgtcctc aaagctagtc atattggaat gcttcaagca actcaaatga 1140

cccaggaagt tacaattaaa gatttagaat cagaaaaatc gagagtcaat gagagattat 1200

ctcaacttga agaggaaaga gcttttttgc gaagcaaaac ccaaagtctg gatgaagagc 1260

agaagcaaca gattctagaa ctggagaaga aagtaaatga agcaaagaga actcagcaag 1320

aatattatga aagggaactt aaaaacctgc aaagtagatt ggaagaggag gtgactcaat 1380

taaacgaggc ccattctaag actttggaag aattagcttg gaagcaccat atggcaattg 1440

aagctgtcca cagtaatgca attagggata agaaaaaact gcaaatggat ttggaagaac 1500

aacataacaa agataaacta aacctggaag aggataaaaa tcagcttcaa caagagctag 1560

aaaacctaaa ggaagtactg gaagacaagt tgaatacagc caatcaagag attggccacc 1620

tccaagatat ggtaaggaaa agtgaacaag gtcttggctc tgcagaagga cttattgcta 1680

gtcttcagga ctcccaggaa aggcttcaga atgagcttga cttgactaaa gacagcctaa 1740

aggagaccaa ggatgctcta ttaaatgtgg agggtgagct agaacaagaa aggcaacagc 1800

atgaagaaac aattgctgcc atgaaagaag aagagaagct caaagtggac aaaatggccc 1860

atgacttaga aattaagtgg actgaaaatc ttagacaaga gtgttctaaa cttcgtgaag 1920

agttaaggct tcaacatgaa gaggataaga agtcagcaat gtctcaactt ttgcagttga 1980

aagatcgaga gaaaaatgca gcaagagatt catggcagaa gaaagtagaa gatctcttaa 2040

accagatttc cttgctgaaa cagaatctgg agatacagct ttcccagtct cagacttctt 2100

tgcaacaact gcaagcccag tttacgcaag aacgacagcg gcttacgcaa gagcttgaag 2160

aattagagga gcaacatcag caaagacaca aatcattaaa agaagcacat gtccttgcat 2220

ttcaaactat ggaagaggaa aaggaaaagg agcaaagagc tcttgaaaat catttacaac 2280

agaagcattc tgcagagctt caatcactaa aagatgcaca cagagagtca atggagggct 2340

tccggataga aatggaacag gaacttcaga ctcttcggtt tgaattagaa gatgaaggaa 2400

aggctatgct tgcttccttg cgctcagaac tcaaccatca acatgcagct gcaattgatt 2460

tgttacggca taatcatcat caagaattgg cagctgctaa aatggaatta gagagaagca 2520

tagacatcag cagaagacag agtaaggagc acatatgtag aattacagat ctacaagagg 2580

aattaagaca cagagagcat cacatctctg aattggataa ggaggttcag caccttcatg 2640

agaatataag tgccctaacc aaagaactgg aatttaaggg gaaagaaatt ctcagaatac 2700

gaagtgaatc taaccaacag ataagattag aagaaatgga agaaaaatat ctaatgagag 2760

aatcaaaacc agaagatata cagatgatta cagaattaaa agccatgctt acagaaagag 2820

accagatcat aaagaaacta attgaggata ataagtttta tcagctggaa ttagtcaatc 2880

gagaaactaa cttcaacaaa gtgtttaact caagtcctac tgttggtgtt attaatccat 2940

tggctaagca aaagaagaag aatgataaat caccaacaaa caggtttgtg agtgttccca 3000

atctaagtgc tctggaatct ggtggagtgg gcaatggaca tcctaaccgc ctggatccca 3060

ttcctaattc tccagtccac gatattgagt tcaacagcag caaaccactt ccacagccag 3120

tgccacctaa agggcccaag acatttttga gtcctgctca gagtgaagct tctccagtgg 3180

cttctccaga tccccagcgc caggagtggt ttgcccggta cttcacattc tgaaagaatt 3240

gtgttggcac agctctgtat agactgttac taagagcatg actttataca gattgttatg 3300

taaataggct ttcctatgtc aaacactgtg aatgagaaag tatttgtctc tccaacttga 3360

aaatgcactg tatttcctgt gatatttatt ggaatcattc tataaggtac tatattatgt 3420

gtgtaattat aactgttatt tttatttgag atggaagagt ctttaacctt tgtaattact 3480

gcataataaa ttttgttaga atcaaaaaaa aaaaaaaaa 3519

<210> 99
<211> 50
<212> DNA
<213> homo sapiens

<400> 99
cactggcatt cctgcctact tgctgtggtg gtcttgtgaa aggtgatggg 50

<210> 100
<211> 3638
<212> DNA
<213> homo sapiens

<400> 100
ggtagatgcg gctgtgacag cagcaaagaa tgacggccaa gggcgacagc aggggctggc 60

catgctgtaa aggggcttct tgggagggtc cagcctcagg aatcaagggg aactcctgag 120

ccgagaattc tgaagatctc ctccctccct gaagctgtgg gctgggccat cggaaaactt 180

tcagttttgt ttccttgcct gcaagaaacg aaactcaacc gaaagcctgc agagagcaga 240

acatggaagg agacttctcg gtgtgcagga actgtaaaag acatgtagtc tctgccaact 300

tcaccctcca tgaggcttac tgcctgcggt tcctggtcct gtgtccggag tgtgaggagc 360

ctgtccccaa ggaaaccatg gaggagcact gcaagcttga gcaccagcag gttgggtgta 420

cgatgtgtca gcagagcatg cagaagtcct cgctggagtt tcataaggcc aatgagtgcc 480

aggagcgccc tgttgagtgt aagttctgca aactggacat gcagctcagc aagctggagc 540

tccacgagtc ctactgtggc agccggacag agctctgcca aggctgtggc cagttcatca 600

tgcaccgcat gctcgcccag cacagagatg tctgtcgcag tgaacaggcc cagctcggga 660

aaggggaaag aatttcagct cctgaaaggg aaatctactg tcattattgc aaccaaatga 720

ttccagaaaa taagtatttc caccatatgg gtaaatgttg tccagactca gagtttaaga 780

aacactttcc tgttggaaat ccagaaattc ttccttcatc tcttccaagt caagctgctg 840

aaaatcaaac ttccacgatg gagaaagatg ttcgtccaaa gacaagaagt ataaacagat 900

ttcctcttca ttctgaaagt tcatcaaaga aagcaccaag aagcaaaaac aaaaccttgg 960

atccactttt gatgtcagag cccaagccca ggaccagctc ccctagagga gataaagcag 1020

cctatgacat tctgaggaga tgttctcagt gtggcatcct gcttcccctg ccgatcctaa 1080

atcaacatca ggagaaatgc cggtggttag cttcatcaaa aggaaaacaa gtgagaaatt 1140

tcagctagat ttggaaaagg aaaggtacta caaattcaaa agatttcact tttaacactg 1200

gcattcctgc ctacttgctg tggtggtctt gtgaaaggtg atgggtttta ttcgttgggc 1260

tttaaaagaa aaggtttggc agaactaaaa acaaaactca cgtatcatct caatagatac 1320

agaaaaggct tttgataaaa ttcaacttga cttcatgtta aaaaccctca acaaaccagg 1380

cgtcgaagga acatacctca aaataataag agccatctat gacaaaacca cagccaacat 1440

catactgaat gagcaaaagc tggagcatta ctcttgagaa gtagaacaag gcacttcagt 1500

cctattcaac atagtactgg aagtcctcgc cacagcaatc aggcaagaga aagaaataaa 1560

aggcaaccaa aaagaaagga agtcgaagta tctctgtttg cagacgatat gattctatat 1620

ctagaaaacc ccatgatctt ggcccaaaag ctcctagatc tgataaacaa cttcagctaa 1680

ctttcaggag acaaaatcaa tatacaaaat atggtagcat ttttatacac caacgacatc 1740

caagctgaga gccaaatcaa gaatgcaatc ctattcacaa ttgccacaaa aagaataaaa 1800

tacctaggaa tacagctaac cagggagatg aaagatctct acaacaaaaa ttacaaaaca 1860

ctgctgaaag aaatcagaga tgacacaaat ggaaaaacat tccatactta tggataggaa 1920

gaatcaatat tgttaaaatg gccatactac ccaaagcaat ttatagattc aatgctattc 1980

ctatcaaact accaataaca ttcttcacag aatcagaaaa aaaaagcatt aaaatttatt 2040

tgaaaccaaa aaagagccca aaaagccaaa gcaatcctaa gcaaaaagaa caaagctgga 2100

ggcatcgcat tacccaactt caaactatac tacagggcta cagtaaccaa aactgcatga 2160

tactggtaca aaagcatggt gctggtacaa aagcagacac atagatcaat ggaacagaat 2220

agagggccca gaaataaagc tacacaccta caaccatcta atctttgaca aagttgacaa 2280

aaatacgcaa tggggaaaga attccccatt cagtaagtgg tactgggata actagctagc 2340

catatgcaga ggattgaaac tgaaccactt ccttacacca tatgcaaaaa tcaactcaag 2400

atggattaaa gacttaaatg taaaacccca aactataaaa actctggaag ataacctagg 2460

caataccatt ctggacatag gaacggaaaa agatttcatg acaaagatcc caaaaataat 2520

tgtaacgaaa gcaaaaattg acaaatggga catgattaaa cagaattacc atttgactca 2580

gcaatcccat tattggttat atacccaaag gaatctaaat cattctgtca taaagacata 2640

tatacacaaa tgttcacggc agcactatac acaatcgcaa agtcagggaa tcaaactaaa 2700

tgtccatcag tggtagaaag gataaagaaa atgtggtggc agggagtggt ggctcatgtc 2760

tgtaatccca gcactttggg aggctgaggc gggtggttca cctgaggtca ggagtttgag 2820

accagcctgg ccaacatggc gaaactccgt ctccgctaaa aatacgaaaa ttagccaggc 2880

gtggtggcga gcacctgtca tcccagctac ttgggaggcc taggcgtgag aatcgcttga 2940

acctggaagg tggtggttgc agtgagccga gatcctgcca ctgcactcca gcctgggcaa 3000

ccaagcgaga ctctgcctta aaaaaaaaaa aaagaaaatg tggcacatat acaccatgga 3060

atactatgca gccataaaaa agaatgggat catgtcctgt gcagcaacgt ggatggagct 3120

ggaagccatt atcctaaatg aactcactca gaaacagaaa accaaatacc acatgttctc 3180

acttataagt agaagctaaa cattgagtac acatggatac aaagaaggga accgcagaca 3240

ctggggccta cctgaggtcg gagcatggaa ggagggtgag gatcaaaaaa ctacctatct 3300

ggtactatgc tttttatctg gatgatgaaa taatctgtac aacaaaccct ggtgacatgc 3360

aatttaccta tatagcaagc ctacacatgt gcccctgaac ctaaaaaaaa agttaaaaga 3420

aaaacgtttg gattattttc cctctttcga acaaagacat tggtttgccc aaggactaca 3480

aataaaccaa cgggaaaaaa gaaaggttcc agttttgtct gaaaattctg attaagcctc 3540

tgggccctac agcctggaga acctggagaa tcctacaccc acagaacccg gctttgtccc 3600

caaagaataa aaacacctct ctaaaaaaaa aaaaaaaa 3638

<210> 101
<211> 50
<212> DNA
<213> homo sapiens

<400> 101
agcttcagta aagttcaaaa agcgggccag gtgtggtggc tcatgcctgt 50

<210>  102
<211>  4860
<212>  DNA
<213>  homo sapiens

<400>  102
aacttggggg acccagcgaa atggaagaag ttaggtgtcc agagcacggg actttctgct      60

ttcttaagac cggcgtccgc gatggcccga ataaaggaaa gagcttctac gtgtgccggg     120

cagacacgtg cagcttcgtg cgggccaccg acattcctgt ttcccattgc ttattgcatg     180

aggactttgt ggtagagctt cagggtttgc ttctgccaca ggacaagaaa gaatacagat     240

tgttcttccg atgcattaga agtaaggcag aggggaaacg ctggtgtgga agtattccat     300

ggcaggatcc tgattccaaa gaacattctg tatccaataa gtctcagcat gcatctgaga     360

catttcatca ttcttccaac tggctgagaa atccattcaa ggtacttgac aagaatcaag     420

aaccagctct ctggaaacag ctcatcaaag gtgaaggtga ggaaaagaag gctgataaga     480

agcaaagaga aaagggagat cagcttttcg atcaaaagaa agaacagaag cctgaaatga     540

tggagaaaga cctctcatct ggcctggtac caaagaaaaa acaatctgta gttcaagaga     600

agaagcaaga agagggagca gagattcagt gtgaggcaga gactggaggc acacacaaaa     660

gagacttttc tgaaattaaa tctcaacagt gccaaggtaa tgagcttaca agaccatctg     720

catcttctca ggagaaatca agtggtaaga gtcaagatgt ccaaagagaa tcagaacctc     780

tgagagaaaa ggttacccag cttttgcctc aaaatgttca cagtcacaac tcaataagca     840

agccccagaa agggggaccc ctcaacaagg agtacacgaa ctgggaggct aaagaaacaa     900

aggcaaagga tggccctagc atacaggcca cccagaaaag cctgcctcag gggcatttcc      960

aagagcggcc ggagacccac agtgtgcctg ctcctggagg accagcggct caggctgcac    1020

cagcagcacc agggctttcc ctgggtgagg gccgtgaagc tgccacaagc agtgacgacg    1080

aggaggaaga tgatgttgtt tttgtttcct ctaagcctgg gagcccccta ctctttgact    1140

cgactctgga cttagagacg aaggaaaacc tccaattccc tgatcgaagt gtgcaaagaa    1200

aggtgtctcc tgcctcaggt gtttccaaga aggtagaacc ctcagaccca gtagcccggc    1260

gtgtctacct tacaacacaa ctgaaacaaa agaagagcac actggcatca gtgaacatcc    1320

aggctcttcc agacaagggt cagaagttga tcaaacaaat ccaggagctg gaggaagtac    1380

tcagtggtct taccctttcc ccagagcaag gaactaatga gaagagtaac agtcaagtac    1440

cacagcagag tcacttcacc aaaactacca ctggccctcc ccacctggtg cctccccaac    1500

cccttcctcg tcgtggtacc caacctgtgg gttctctaga actaaagtct gcctgccagg    1560

tgactgctgg aggatccagt cagtgctatc gaggccatac aaaccaagat cacgttcatg    1620

cagtgtggaa aatcacaagt gaagccatcg gtcaactgca tcgctcactt gagtcatgtc    1680

ctggtgagac ggttgtggca gaagatcccg ccgggctgaa ggtccctttg ctactacacc 1740

agaagcaggc attggcttgg ttactatggc gagaaagtca gaagccacaa ggaggaattc 1800

tggcagatga tatgggctta ggaaaaaccc tgacaatgat tgcgctcatc ctgacccaga 1860

agaatcaaga gaaaaaggaa gaaaaggaga aaagcacagc tttgacgtgg ctctccaaag 1920

atgactcttg tgactttact tcccatggaa cactaatcat ctgtcctgcc tccctgatcc 1980

atcattggaa aaatgaggtg gagaaacggg tgaacagcaa caaactaaga gtctatctct 2040

accatgggcc aaaccgggat tcacgtgcca gagtcctctc tacatatgac atcgtgatca 2100

ctacctatag cctcgtggcc aaggagattc ccacaaacaa gcaagaggca gagatcccag 2160

gtgcaaacct caatgtggag ggcacctcaa cacctttgct tcgaatagcc tgggctcgaa 2220

tcatattgga tgaagctcac aatgttaaga atccccgagt gcagacttcc atagctgtgt 2280

gtaagctaca agcctgtgcc cgttgggctg tcactggaac ccccattcaa aacaacttat 2340

tggatatgta ttcgctgctg aagtttctcc gttgctctcc atttgatgag ttcaatctgt 2400

ggaggagtca ggttgacaat ggctcaaaga aaggaggaga acggttaagt attttaacca 2460

agagcctttt gctgaggaga acaaaagacc agctggactc tactggcaga cctttggtga 2520

tactgcccca gcgtaaattt cagttgcacc atttaaagct ttctgaagat gaagagactg 2580

tttacaatgt gttttttgca agatcaaggt cagctctgca atcctatcta aaaagacatg 2640

aaagtagagg caaccaatct ggaagaagcc ctaataatcc attcagtaga gtggcactgg 2700

agtttgggtc tgaggagcct agacactcgg aggcagcaga ctcaccgaga tccagcaccg 2760

tccacatact gtcccagttg ctgagactcc gccagtgttg ctgtcatctt tctttactga 2820

agtcggccct ggatccaatg gaactgaagg gtgaaggtct tgtcctttcc ctggaagaac 2880

agctcagtgc tttgaccttg tcagaactcc gtgactcaga gccatcttcc actgtttccc 2940

ttaacggcac cttcttcaag atggagcttt ttgaaggcat gcgagagagc accaagattt 3000

catctctgtt ggcagaattg gaggcaattc aaagaaattc agcatcccaa aagagtgtca 3060

ttgtctctca gtggaccaac atgctgaaag ttgtagcatt gcacctgaag aagcatggac 3120

tgacttatgc caccatcgat ggctctgtca atcccaagca gagaatggac ttggtagagg 3180

catttaacca ctccagaggc cctcaggtaa tgctaatctc tctcttggcc ggaggtgttg 3240

gtctaaacct gactggagga aatcacctct ttcttttgga catgcactgg aatccatcac 3300

ttgaagatca agcttgtgac cgaatttacc gagtaggcca gcagaaagat gttgtcatac 3360

acagatttgt ttgtgaggga acagtagaag aaaagatctt acagctccaa gaaaaaaaga 3420

aagatttggc caaacaagtt ctatcagggt ctggagaatc tgtcaccaag ctcaccttgg 3480

ctgacctcag agtccttttt ggcatctaac ctcctgtgga taaggctcca gaatagcacc 3540

attgctggtt tgtattagga tctgggaata acaacctaac catgagcctt gaactctgtt 3600

ctttgcattt caatttcacc gtcaagcctt tcaccttcct caaaatgagg cataatctta 3660

tccccagaat tgaggagggg ttgtgttaac caattagtta accaattatg ttaataattt 3720

atttaatgag tggtatggaa accaattgat ttttttagat aaaagaatat gttttagagt 3780

tggatgattt tggaaaacaa ctctggcaga attgtatata agcttcagta aagttcaaaa 3840

agcgggccag gtgtggtggc tcatgcctgt aatcccgaca ctttgggagg ccaaggcagg 3900

aggatggctt gaagccagta gttcaagacc agcctgggca acatagtaag accccatctc 3960

tatgaagaat aaaaaaatga gctaggcgtg gtagtgcaca cctgtagtcc tagctacttg 4020

ggaggctgag gcaggaggat cccttgagtc caggaggttg aggctgcagt gagctgtgat 4080

tgtgccactg ttttccagcc tgggcaatac agtgagaccc tgtctcaaaa aaaggaaaaa 4140

aaagctcaaa aagttctgtg ttgcctttct cttcttagct gatgatggga gaaccagtta 4200

ctaccttctc taacacaggc tccatcctat ctcttcatct cttcttgcca gtgatgttgc 4260

acctgtaacc atctttttat gggtggagca gagagtcaat ccctgcagcc accctgcagc 4320

cagccatctc tgcagttctc tcagtgcagg cagttcttcc tctcaggctg aagatcaagg 4380

agatgctttg tacatgaaca gatgctgagt atctgttatc attgtattgt ttagtgtcag 4440

tgtatcattt agtatttgta tcactgtacc atttagtgtt gtatatcact cagtcattta 4500

atattggtta tcaccataca gtttaatatt tgtatcattt ggttcattgt atcgtttagt 4560

atttctatta taacatttct atttgtagta tttgtataat atttgtgtca tttttaatag 4620

tatttgtatt tttacaaatt tgcagtattt gtagcattta gtatcattgt ttcatttagt 4680

atttggtaca atctaataaa tactaatcca gtccaatact aatccaatcc aatactaata 4740

atactattgg tacaatccaa tactaataat actattggta caatccaata ctaataatac 4800

taacagtagt attgatacaa tctaataaat aatgtgtttg acagtgtagc agaaaaaaaa 4860

<210> 103
<211> 50
<212> DNA
<213> homo sapiens

<400> 103
tctgagacag cacatttgtg aatgatgctt gctgcctgcc attttcaacc 50

<210> 104
<211> 5629
<212> DNA
<213> homo sapiens

<400> 104
cttaagtgca aggaactctg tgttgggagg aaaaatgtcc ttcttcaatt tccgtaagat 60

cttcaagttg gggagcgaga agaagaagaa gcagtacgaa cacgtgaaga gggacctgaa 120

ccccgaagac ttttgggaga ttataggaga actgggcgac ggagcctttg ggaaagtgta 180
caaggcccag aataaagaga ccagtgtttt agctgctgca aaagtgattg acactaaatc 240
tgaagaagaa cttgaagatt acatggtaga gattgacata ttagcatctt gtgatcaccc 300
aaatatagtc aagcttctag atgccttcta ttatgagaac aatctttgga tcctcattga 360
attttgtgca ggtggagcag tagatgctgt gatgcttgaa cttgagagac cattaactga 420
gtcccaaata caagtagttt gcaagcagac tttagatgca ttgaactact tacatgataa 480
taagatcatc cacagagatc tgaaggctgg caacattctc tttaccttag atggagatat 540
caaattggcg gattttggag tatcagctaa aaacacgagg acaattcaaa gaagagattc 600
ctttattggt acaccatatt ggatggctcc tgaagtagtc atgtgtgaaa catctaagga 660
cagaccctat gactacaaag ctgatgtttg gtccctgggt atcactttaa tagaaatggc 720
tgagatagaa ccacctcatc atgaattaaa tccaatgcga gtgctgctaa aaatagcaaa 780
atctgagcca cctacattag cacagccatc cagatggtct tcaaatttta aggactttct 840
aaagaaatgc ttagaaaaga atgtggatgc caggtggact acatctcagc tgctgcagca 900
tccctttgtt actgttgatt ccaacaaacc catccgagaa ttgattgcag aggcgaaggc 960
tgaagtaaca gaagaagttg aagatggcaa agaggaagat gaagaggagg aaacagaaaa 1020
ttctctgcca atacctgcaa gtaagcgtgc atcttctgac cttagtatcg ccagctctga 1080
agaagataaa ctttcacaaa atgcttgtat tttggagtct gtctcagaaa aaacagaacg 1140
tagtaactct gaagataaac tcaacagcaa aattcttaat gaaaaaccca ccactgatga 1200
acctgaaaag gctgtggagg atattaatga acatattacc gatgctcagt tagaagcaat 1260
gactgaactc catgacagaa cagcagtaat caaggagaat gaaagagaga agaggcccaa 1320
gcttgaaaat ctgcctgaca cagaagacca agaaactgtg gacattaatt cagtcagtga 1380
aggaaaagag aataatataa tgataacctt agaaacaaat attgaacata atctaaaatc 1440
tgaggaagaa aaggatcagg aaaagcaaca gatgtttgaa aataagctta taaaatctga 1500
agaaattaaa gatactattt tgcaaacagt agatttagtt tctcaagaga ctggagaaaa 1560
agaggcaaat attcaggcag ttgatagtga agttgggctt acaaaggaag acacccaaga 1620
gaaattgggg gaagacgaca aaactcaaaa agatgtgatc agcaatacaa gtgatgtgat 1680
aggaacatgt gaggcagcag atgtggctca gaaagtggat gaagacagtg ctgaggatac 1740
gcagagtaat gatgggaaag aagtggtcga agtaggccag aaattaatta ataagcccat 1800
ggtgggtcct gaggctggtg gtactaagga agttcctatt aaagaaatag ttgaaatgaa 1860
tgaaatagaa gaaggtaaaa ataaggaaca agcaataaac agttcagaga acataatgga 1920
catcaatgag gaaccaggaa caactgaagg tgaagaaatc actgagtcaa gtagcactga 1980
agaaatggag gtcagaagtg tggtggctga tactgaccaa aaggctttag gaagtgaagt 2040

tcaggatgct tctaaagtca ctactcagat agataaagag aaaaaagaaa ttccagtgtc 2100
aattaaaaaa gagcctgaag ttactgtagt ttcacagccc actgaacctc agcctgttct 2160
aatacccagt attaatatca actctgacag tggagaaaat aaagaagaaa taggttcttt 2220
atcaaaaact gaaactattc tgccaccaga atctgagaat ccaaaggaaa atgataatga 2280
ttcaggcact ggttccactg ctgatactag cagtattgac ttgaatttat ccatctctag 2340
ctttctaagt aaaactaaag acagtggatc gatatcttta caagaaacaa gaagacaaaa 2400
gaaaacattg aagaaaacac gcaaatttat tgttgatggt gtagaagtga gtgtaacaac 2460
atcaaagata gttacagata gtgattccaa aactgaagaa ttgcggtttc ttagacgtca 2520
ggaacttcgg gaattaagat ttcttcagaa agaagagcaa agagcccaac aacagctcaa 2580
tagcaaacta cagcaacaac gagaacaaat tttccggcgc tttgagcagg aaatgatgag 2640
taaaaagcga caatatgacc aggaaattga gaatctagaa aaacagcaga aacagactat 2700
cgaacgcctg gaacaagagc acacaaatcg cttgcgagat gaagccaaac gcatcaaagg 2760
agaacaagag aaagagttgt ccaaatttca gaatatgctg aagaaccgaa agaaggaggt 2820
tataaatgaa gtggagaaag cacccaaaga gctgagaaaa gagctcatga aacgcaggaa 2880
agaggagctt gcacaaagcc agcatgctca ggaacaagag tttgttcaga aacaacagca 2940
agaattagat ggctctctga aaaagatcat ccagcagcag aaggcagagt tagctaatat 3000
tgagagagag tgcctgaata acaagcaaca gctcatgaga gctcgagaag ctgcaatttg 3060
ggagctcgaa gaacgacact tacaagaaaa acaccagctg ctcaaacagc agcttaaaga 3120
tcagtatttc atgcaaagac atcagctact taagcgccac gagaaggaaa cagagcaaat 3180
gcagcgttac aatcaaagac ttattgagga attgaaaaac agacagactc aagaaagagc 3240
aagactgccc aagattcagc gcagtgaagc caagactcga atggccatgt ttaagaagag 3300
tttgagaatt aactcaacag ccacaccaga tcaggaccgt gataaaatta aacagtttgc 3360
tgcacaagaa gaaaagaggc agaaaaatga gagaatggct cagcatcaga aacatgagaa 3420
tcaaatgcga gatcttcagt tgcagtgtga agccaatgtc cgcgaactgc atcagctgca 3480
gaatgaaaaa tgccacttgt tggttgagca tgagactcag aaactgaagg agttagatga 3540
ggaacatagc caagaattaa aggagtggag agagaaattg agacctagga aaaagacact 3600
ggaagaagag tttgccagga aactacagga acaggaagta ttctttaaaa tgactgggga 3660
gtctgaatgc cttaacccat caacacagag ccggatttcc aaattttatc ctattcccag 3720
cttgcattcc accggatcat aacaaaggga agcattctgt gcgtgggttt ggctctttca 3780
gtatgtcatt ctgttctcat cttctgccac agtctctcag atagctcatg aagacaatca 3840
cctgcctcac cttctaggtg ttttcctttt ttgttttttt tgttttgttt tgtttttaag 3900

caaagatgaa gggaaaacga actaagacag acgctaggcc atgttggcaa agtagcatct 3960

tggtgactaa ggtgactttg tatattcatc ttaaaaatta tgttctttag acactgctac 4020

ctgaaaactg ttggagaaat aatgtttaaa gttatttaag aaaaactgtt acatcactaa 4080

gtattaataa attcttctta cctgacgtaa cttctcaatg cctaaattct gtagttgaag 4140

ctctgctgca gagagttggg ataattttct tttggtggat cagctctcat aaaaaagcta 4200

tgatttgctc aaatatgctg ttgactcagt aaatgaatat atttttttct ttaaatagga 4260

acaacctctt ttaaaagaga aaaattattt cagtgatttg tcaaaacgaa ttacctcttt 4320

tggcatgagc taataattga gggtgctaat tttcttaaga tagtgcctaa aacactaaat 4380

ttcagtcaag tcgtaagtag gattttcttt ttgatcaaca gggacaaaaa catctttaga 4440

attaaaaaca tggttgtttt ggaatttttg cttctcttac cgtttgatag aaattttcat 4500

cctaaaatac atgtacaaag tttggaaaga tgaaaaaaag aggtagcttt tagattgcaa 4560

attggaaatg taaaactcat gaaatttaag caatataggt ttagctatct gtgtttattt 4620

tctaaaataa tacctgagct ggttaaatga tttctctcca tcttagctaa ttctgtttaa 4680

aactctgtca gaggcctgca ggctgtgagt tatatttata aatatatctt cagaaattaa 4740

tcttaaaaga ggcattagtt cagaatactt ttttaaaagt ttaaattaaa tatttaggca 4800

cgtcagaaat tacttttcct tattttgaaa tgaggctact tatgtcttgg ttttattttg 4860

ttccatgttt aaatcattca ctttgatttg agtgggaaaa gcctgaagcc tttatcatgt 4920

ggttgctggt gtgtgtaatt attaatgaaa tgttcactcc tagtccctta tgaggcttag 4980

aatttcaacc acgtgtcagg tcagacagta ttataaactg tactttgctg tctgagacag 5040

cacatttgtg aatgatgctt gctgcctgcc attttcaacc tattctctct taagagtgct 5100

aggtaccaaa ttgtgaaagt ttgttttcag ttatattact tttgaggctg gtgaaaaatt 5160

taaatgtaac tttgtgggaa cactgattca tatttagaaa atgtaaatgt ctgtagcact 5220

ttcttgcagt taatttgaaa actttggatg ctgaaccttg tttgtcagtg atttagatga 5280

tttaaaaatg catgtgtgat ttgaatttta taattgtttt gacaagcata atttacttgg 5340

acaacttcgt aggtagcctt aacttctggc caagtttgtt ttttatataa atatatatac 5400

atatatacat attatgtatg gttgtaaatt catacactta tcacatgaat gtgttactgt 5460

atacaaaact cttaatgctt tattctcaaa tgctgggttg aaaaatgttt tgaaagcctt 5520

ttaaaatata tatctttata aagtaatatt caggatgatg ataaaaattg tttatattgt 5580

tatgataaaa atgacagtat aatgttaaaa aaaaaaaaaa aaaaaaaaa 5629

<210> 105
<211> 50
<212> DNA
<213> homo sapiens

<400>  105
tcagttttct cttaatatgt caaggaaggt agccgggcat ggtgccaggc          50


<210>  106
<211>  2645
<212>  DNA
<213>  homo sapiens

<400>  106
aggcaaccat gtggttccag ctgaattttt tttttccctc tctctttctt cactcctttt      60

tctttccaaa cagggaaaag tgttccacga agcggtagcg cctttccgcc tcgcgttttc     120

ctccctgacc ctggtcccgg ctcccgtccg ggcgccagct ggtggggcga gcgccgggag     180

cccatctgcc cccaggggca cggggcgcgg ggccggctcc cgcccggcac atggctgcag     240

ccacctcgcg cgcaccccga ggcgccgcgc ccagctcgcc cgaggtccgt cggaggcgcc     300

cggccgcccc ggagccaagc agcagctgag cggggaagcg cccgcgtccg gggatcggga     360

tgtccctcct ccttctcctc ttgctagttt cctactatgt tggaaccttg gggactcaca     420

ctgagatcaa gagagtggca gaggaaaagg tcactttgcc ctgccaccat caactggggc     480

ttccagaaaa agacactctg gatattgaat ggctgctcac cgataatgaa gggaaccaaa     540

aagtggtgat cacttactcc agtcgtcatg tctacaataa cttgactgag gaacagaagg     600

gccgagtggc ctttgcttcc aatttcctgg caggagatgc ctccttgcag attgaacctc     660

tgaagcccag tgatgagggc cggtacacct gtaaggttaa gaattcaggg cgctacgtgt     720

ggagccatgt catcttaaaa gtcttagtga gaccatccaa gcccaagtgt gagttggaag     780

gagagctgac agaaggaagt gacctgactt tgcagtgtga gtcatcctct ggcacagagc     840

ccattgtgta ttactggcag cgaatccgag agaaagaggg agaggatgaa cgtctgcctc     900

ccaaatctag gattgactac aaccaccctg gacgagttct gctgcagaat cttaccatgt     960

cctactctgg actgtaccag tgcacagcag gcaacgaagc tgggaaggaa agctgtgtgg    1020

tgcgagtaac tgtacagtat gtacaaagca tcggcatggt tgcaggagca gtgacaggca    1080

tagtggctgg agccctgctg attttcctct tggtgtggct gctaatccga aggaaagaca    1140

aagaaagata tgaggaagaa gagagaccta atgaaattcg agaagatgct gaagctccaa    1200

aagcccgtct tgtgaaaccc agctcctctt cctcaggctc tcggagctca cgctctggtt    1260

cttcctccac tcgctccaca gcaaatagtg cctcacgcag ccagcggaca ctgtcaactg    1320

acgcagcacc ccagccaggg ctggccaccc aggcatacag cctagtgggg ccagaggtga    1380

gaggttctga accaaagaaa gtccaccatg ctaatctgac caaagcagaa accacaccca    1440

gcatgatccc cagccagagc agagccttcc aaacggtctg aattacaatg gacttgactc    1500

ccacgctttc ctaggagtca gggtctttgg actcttctcg tcattggagc tcaagtcacc    1560

agccacacaa ccagatgaga ggtcatctaa gtagcagtga gcattgcacg gaacagattc 1620

agatgagcat tttccttata caataccaaa caagcaaaag gatgtaagct gattcatctg 1680

taaaaaggca tcttattgtg cctttagacc agagtaaggg aaagcaggag tccaaatcta 1740

tttgttgacc aggacctgtg gtgagaaggt tggggaaagg tgaggtgaat atacctaaaa 1800

cttttaatgt gggatatttt gtatcagtgc tttgattcac aattttcaag aggaaatggg 1860

atgctgtttg taaattttct atgcatttct gcaaacttat tggattatta gttattcaga 1920

cagtcaagca gaacccacag ccttattaca cctgtctaca ccatgtactg agctaaccac 1980

ttctaagaaa ctccaaaaaa ggaaacatgt gtcttctatt ctgacttaac ttcatttgtc 2040

ataaggtttg gatattaatt tcaaggggag ttgaaatagt gggagatgga gaagagtgaa 2100

tgagtttctc ccactctata ctaatctcac tatttgtatt gagcccaaaa taactatgaa 2160

aggagacaaa aatttgtgac aaaggattgt gaagagcttt ccatcttcat gatgttatga 2220

ggattgttga caaacattag aaatatataa tggagcaatt gtggatttcc cctcaaatca 2280

gatgcctcta aggactttcc tgctagatat ttctggaagg agaaaataca acatgtcatt 2340

tatcaacgtc cttagaaaga attcttctag agaaaaaggg atctaggaat gctgaaagat 2400

tacccaacat accattatag tctcttcttt ctgagaaaat gtgaaaccag aattgcaaga 2460

ctgggtggac tagaaaggga gattagatca gttttctctt aatatgtcaa ggaaggtagc 2520

cgggcatggt gccaggcacc tgtaggaaaa tccagcaggt ggaggttgca gtgagccaag 2580

attatgccat tgcactccag cctgggtgac aaagcaagac tccatctcaa aaaaaaaaaa 2640

aaaaa 2645

<210> 107
<211> 50
<212> DNA
<213> homo sapiens

<400> 107
acaatttgct tctggaaaca ggacagccgg ggccgtgttc ctgcaacagc 50

<210> 108
<211> 2530
<212> DNA
<213> homo sapiens

<400> 108
gggaatatga gtgatggtgc ctcaaagcag taactttttg cttagagctt gagagtcaaa 60

gttaaggacc cacatgtata cttcggctct agcgagtcta aggatgataa tatggataca 120

aaatctattc tagaagaact tcttctcaaa agatcacagc aaaagaagaa aatgtcacca 180

aataattaca aagaacggct ttttgttttg accaaaacaa acctttccta ctatgaatat 240

gacaaaatga aaaggggcag cagaaaagga tccattgaaa ttaagaaaat cagatgtgtg 300

gagaaagtaa atctcgagga gcagacgcct gtagagagac agtacccatt tcagattgtc 360
tataaagatg ggcttctcta tgtctatgca tcaaatgaag agagccgaag tcagtggttg 420
aaagcattac aaaaagagat aaggggtaac ccccacctgc tggtcaagta ccatagtggg 480
ttcttcgtgg acgggaagtt cctgtgttgc cagcagagct gtaaagcagc cccaggatgt 540
accctctggg aagcatatgc taatctgcat actgcagtca atgaagagaa acacagagtt 600
cccaccttcc cagacagagt gctgaagata cctcgggcag ttcctgttct caaaatggat 660
gcaccatctt caagtaccac tctagcccaa tatgacaacg aatcaaagaa aaactatggc 720
tcccagccac catcttcaag taccagtcta gcgcaatatg acagcaactc aaagaaaatc 780
tatggctccc agccaaactt caacatgcag tatattccaa gggaagactt ccctgactgg 840
tggcaagtaa gaaaactgaa aagtagcagc agcagtgaag atgttgcaag cagtaaccaa 900
aaagaaagaa atgtgaatca caccacctca aagatttcat gggaattccc tgagtcaagt 960
tcatctgaag aagaggaaaa cctggatgat tatgactggt ttgctggtaa catctccaga 1020
tcacaatctg aacagttact cagacaaaag ggaaaagaag gagcatttat ggttagaaat 1080
tcgagccaag tgggaatgta cacagtgtcc ttatttagta aggctgtgaa tgataaaaaa 1140
ggaactgtca aacattacca cgtgcataca aatgctgaga acaaattata cctggcagaa 1200
aactactgtt ttgattccat tccaaagctt attcattatc atcaacacaa ttcagcaggc 1260
atgatcacac ggctccgcca ccctgtgtca acaaaggcca acaaggtccc cgactctgtg 1320
tccctgggaa atggaatctg ggaactgaaa agagaagaga ttaccttgtt gaaggagctg 1380
ggaagtggcc agtttggagt ggtccagctg ggcaagtgga aggggcagta tgatgttgct 1440
gttaagatga tcaaggaggg ctccatgtca gaagatgaat tctttcagga ggcccagact 1500
atgatgaaac tcagccatcc caagctggtt aaattctatg gagtgtgttc aaaggaatac 1560
cccatataca tagtgactga atatataagc aatggctgct tgctgaatta cctgaggagt 1620
cacggaaaag gacttgaacc ttcccagctc ttagaaatgt gctacgatgt ctgtgaaggc 1680
atggccttct tggagagtca ccaattcata caccgggact tggctgctcg taactgcttg 1740
gtggacagag atctctgtgt gaaagtatct gactttggaa tgacaaggta tgttcttgat 1800
gaccagtatg tcagttcagt cggaacaaag tttccagtca agtggtcagc tccagaggtg 1860
tttcattact tcaaatacag cagcaagtca gacgtatggg catttgggat cctgatgtgg 1920
gaggtgttca gcctggggaa gcagccctat gacttgtatg acaactccca ggtggttctg 1980
aaggtctccc agggccacag gctttaccgg ccccacctgg catcggacac catctaccag 2040
atcatgtaca gctgctggca cgagcttcca gaaaagcgtc ccacatttca gcaactcctg 2100
tcttccattg aaccacttcg ggaaaaagac aagcattgaa gaagaaatta ggagtgctga 2160

taagaatgaa tatagatgct ggccagcatt ttcattcatt ttaaggaaag tagcaaggca 2220

taatgtaatt tagctagttt ttaatagtgt tctctgtatt gtctattatt tagaaatgaa 2280

caaggcagga aacaaaagat tcccttgaaa tttagatcaa attagtaatt ttgtttatgc 2340

tgctcctgat ataacacttt ccagcctata gcagaagcac attttcagac tgcaatatag 2400

agactgtgtt catgtgtaaa gactgagcag aactgaaaaa ttacttattg gatattcatt 2460

cttttcttta tattgtcatt gtcacaacaa ttaaatatac taccaagtac agaaatgtgg 2520

aaaaaaaaaa 2530

<210> 109
<211> 50
<212> DNA
<213> homo sapiens

<400> 109
tacgtatcag cggccccttg actctgggga ctccgggttt gagatggaca 50

<210> 110
<211> 3293
<212> DNA
<213> homo sapiens

<400> 110
cttttgctct cagatgctgc cagggtccct gaagagggaa gacacgcgga aacaggcttg 60

cacccagaca cgacaccatg catctcctcg gcccctggct cctgctcctg gttctagaat 120

acttggcttt ctctgactca agtaaatggg tttttgagca ccctgaaacc ctctacgcct 180

gggagggggc ctgcgtctgg atcccctgca cctacagagc cctagatggt gacctggaaa 240

gcttcatcct gttccacaat cctgagtata acaagaacac ctcgaagttt gatgggacaa 300

gactctatga aagcacaaag gatgggaagg ttccttctga gcagaaaagg gtgcaattcc 360

tgggagacaa gaataagaac tgcacactga gtatccaccc ggtgcacctc aatgacagtg 420

gtcagctggg gctgaggatg gagtccaaga ctgagaaatg gatggaacga atacacctca 480

atgtctctga aaggcctttt ccacctcata tccagctccc tccagaaatt caagagtccc 540

aggaagtcac tctgacctgc ttgctgaatt tctcctgcta tgggtatccg atccaattgc 600

agtggctcct agaggggttt ccaatgaggc aggctgctgt cacctcgacc tccttgacca 660

tcaagtctgt cttcacccgg agcgagctca agttctcccc acagtggagt caccatggga 720

agattgtgac ctgccagctt caggatgcag atgggaagtt cctctccaat gacacggtgc 780

agctgaacgt gaagcacacc ccgaagttgg agatcaaggt cactcccagt gatgccatag 840

tgagggaggg ggactctgtg accatgacct gcgaggtcag cagcagcaac ccggagtaca 900

cgacggtatc ctggctcaag gatgggacct cgctgaagaa gcagaataca ttcacgctaa 960

acctgcgcga agtgaccaag gaccagagtg ggaagtactg ctgtcaggtc tccaatgacg 1020

tgggcccggg aaggtcggaa gaagtgttcc tgcaagtgca gtatgccccg gaaccttcca 1080

cggttcagat cctccactca ccggctgtgg agggaagtca agtcgagttt ctttgcatgt 1140

cactggccaa tcctcttcca acaaattaca cgtggtacca caatgggaaa gaaatgcagg 1200

gaaggacaga ggagaaagtc cacatcccaa agatcctccc ctggcacgct gggacttatt 1260

cctgtgtggc agaaaacatt cttggtactg gacagagggg cccgggagct gagctggatg 1320

tccagtatcc tcccaagaag gtgaccacag tgattcaaaa ccccatgccg attcgagaag 1380

gagacacagt gaccctttcc tgtaactaca attccagtaa ccccagtgtt acccggtatg 1440

aatggaaacc ccatggcgcc tgggaggagc catcgcttgg ggtgctgaag atccaaaacg 1500

ttggctggga caacacaacc atcgcctgcg cagcttgtaa tagttggtgc tcgtgggcct 1560

cccctgtcgc cctgaatgtc cagtatgccc cccgagacgt gagggtccgg aaaatcaagc 1620

ccctttccga gattcactct ggaaactcgg tcagcctcca atgtgacttc tcaagcagcc 1680

accccaaaga agtccagttc ttctgggaga aaaatggcag gcttctgggg aaagaaagcc 1740

agctgaattt tgactccatc tccccagaag atgctgggag ttacagctgc tgggtgaaca 1800

actccatagg acagacagcg tccaaggcct ggacacttga agtgctgtat gcacccagga 1860

ggctgcgtgt gtccatgagc ccgggggacc aagtgatgga ggggaagagt gcaaccctga 1920

cctgtgagag cgacgccaac cctcccgtct cccactacac ctggtttgac tggaataacc 1980

aaagcctccc ctaccacagc cagaagctga gattggagcc ggtgaaggtc cagcactcgg 2040

gtgcctactg gtgccagggg accaacagtg tgggcaaggg ccgttcgcct ctcagcaccc 2100

tcaccgtcta ctatagcccg gagaccatcg gcaggcgagt ggctgtggga ctcgggtcct 2160

gcctcgccat cctcatcctg gcaatctgtg ggctcaagct ccagcgacgt tggaagagga 2220

cacagagcca gcaggggctt caggagaatt ccagcggcca gagcttcttt gtgaggaata 2280

aaaaggttag aagggccccc ctctctgaag gcccccactc cctgggatgc tacaatccaa 2340

tgatggaaga tggcattagc tacaccaccc tgcgctttcc cgagatgaac ataccacgaa 2400

ctggagatgc agagtcctca gagatgcaga gacctccccc ggactgcgat gacacggtca 2460

cttattcagc attgcacaag cgccaagtgg gcgactatga gaacgtcatt ccagattttc 2520

cagaagatga ggggattcat tactcagagc tgatccagtt tggggtcggg gagcggcctc 2580

aggcacaaga aaatgtggac tatgtgatcc tcaaacattg acactggatg ggctgcagca 2640

gaggcactgg gggcagcggg ggccagggaa gtccccgagt ttccccagac accgccacat 2700

ggcttcctcc tgcgcgcatg tgcgcacaca cacacacaca cgcacacaca cacacacaca 2760

ctcactgcgg agaaccttgt gcctggctca gagccagtct ttttggtgag ggtaacccca 2820

aacctccaaa actcctgccc ctgttctctt ccactctcct tgctacccag aaatccatct 2880

aaatacctgc cctgacatgc acacctcccc ctgcccccac cacggccact ggccatctcc 2940

acccccagct gcttgtgtcc ctcctgggat ctgctcgtca tcatttttcc ttcccttctc 3000

catctctctg gccctctacc cctgatctga catccccact cacgaatatt atgcccagtt 3060

tctgcctctg agggaaagcc cagaaaagga cagaaacgaa gtagaaaggg gcccagtcct 3120

ggcctggctt ctcctttgga agtgaggcat tgcacgggga gacgtacgta tcagcggccc 3180

cttgactctg gggactccgg gtttgagatg gacacactgg tgtggattaa cctgccaggg 3240

agacagagct cacaataaaa atggctcaga tgccacttca aagaaaaaaa aaa 3293

<210> 111
<211> 50
<212> DNA
<213> homo sapiens

<400> 111
gctcgggttc atgacgctct cgatattcct gtgcagaaac ctggaccacg 50

<210> 112
<211> 1229
<212> DNA
<213> homo sapiens

<400> 112
ttcctttctc tctcagctct ccgtctctct ttctctctca gcctctttct ttctccctgt 60

ctcccccact gtcagcacct cttctgtgtg gtgagtggac cgcttacccc actaggtgaa 120

gatgtcagcc caggagagct gcctcagcct catcaagtcc tcggcagcct gatcttctgc 180

ttcggcatct ggatcctcat tgacaagacc agcttcgtgt cctttgtggg cttggccttc 240

gtgcctctgc agatctggtc caaagtcctg gccatctcag gaatcttcac catgggcatc 300

gccctcctgg gttgtgtggg ggccctcaag gagctccgct gcctcctggg cctgtatttt 360

gggatgctgc tgctcctgtt tgccacacag atcaccctgg gaatcctcat ctccactcag 420

cgggcccagc tggagcgaag cttgcgggac gtcgtagaga aaaccatcca aaagtacggc 480

accaaccccg aggagaccgc ggccgaggag agctgggact atgtgcagtt ccagctgcgc 540

tgctgcggct ggcactaccc gcaggactgg ttccaagtcc tcatcctgag aggtaacggg 600

tcggaggcgc accgcgtgcc ctgctcctgc tacaacttgt cggcgaccaa cgactccaca 660

atcctagata aggtgatctt gccccagctc agcaggcttg gacacctggc gcggtccaga 720

cacagtgcag acatctgcgc tgtccctgca gagagccaca tctaccgcga gggctgcgcg 780

cagggcctcc agaagtggct gcacaacaac cttatttcca tagtgggcat ttgcctgggc 840

gtcggcctac tcgagctcgg gttcatgacg ctctcgatat tcctgtgcag aaacctggac 900

cacgtctaca accggctcgc tcgataccgt taggccccgc cctccccaaa gtcccgcccc 960

gcccccgtca cgtgcgctgg gcacttccct gctgcctgta aatatttgtt taatccccag 1020

ttcgcctgga gccctccgcc ttcacattcc cctggggacc cacgtggctg cgtgcccctg 1080

ctgctgtcac ctctcccacg ggacctgggg ctttcgtcca cagcttcctg tccccatctg 1140

tcggcctacc accacccaca agattatttt tcacccaaac ctcaaataaa tcccctgcgt 1200

ttttggtaaa aaaaaaaaaa aaaaaaaaa 1229

<210> 113
<211> 50
<212> DNA
<213> homo sapiens

<400> 113
gtcccaatat caaggacaac caccctagct tcttagtgaa gacaatgtac 50

<210> 114
<211> 3050
<212> DNA
<213> homo sapiens

<400> 114
ggagtcagtg atttgaacga agtactttca gtttcatatt actctaaatc cattacaaat 60

ctgcttagct tctaaatatt tcatcaatga ggaaatccca gccctacaac ttcggaacag 120

tgaaatatta gtccagggat ccagtgagag acacagaagt gctagaagcc agtgctcgtg 180

aactaaggag aaaaagaaca gacaagggaa cagcctggac atggcatcag agatccacat 240

gacaggccca atgtgcctca ttgagaacac taatgggcga ctgatggcga atccagaagc 300

tctgaagatc ctttctgcca ttacacagcc tatggtggtg gtggcaattg tgggcctcta 360

ccgcacaggc aaatcctacc tgatgaacaa gctggctgga aagaaaaagg gcttctctct 420

gggctccacg gtgcagtctc acactaaagg aatctggatg tggtgtgtgc ccaccccaaa 480

gaagccaggc cacatcctag ttctgctgga caccgagggt ctgggagatg tagagaaggg 540

tgacaaccag aatgactcct ggatcttcgc cctggccgtc ctcctgagca gcaccttcgt 600

gtacaatagc ataggaacca tcaaccagca ggctatggac caactgtact atgtgacaga 660

gctgacacat agaatccgat caaaatcctc acctgatgag aatgagaatg aggttgagga 720

ttcagctgac tttgtgagct tcttcccaga ctttgtgtgg acactgagag atttctccct 780

ggacttggaa gcagatggac aacccctcac accagatgag tacctgacat actccctgaa 840

gctgaagaaa ggtaccagtc aaaaagatga aacttttaac ctgcccagac tctgtatccg 900

gaaattcttc ccaaagaaaa aatgctttgt ctttgatcgg cccgttcacc gcaggaagct 960

tgcccagctc gagaaactac aagatgaaga gctggacccc gaatttgtgc aacaagtagc 1020

agacttctgt tcctacatct ttagtaattc caaaactaaa actctttcag gaggcatcca 1080

ggtcaacggg cctcgtctag agagcctggt gctgacctac gtcaatgcca tcagcagtgg 1140

ggatctgccg tgcatggaga acgcagtcct ggccttggcc cagatagaga actcagctgc 1200

agtgcaaaag gctattgccc actatgaaca gcagatgggc cagaaggtgc agctgcccac 1260

agaaaccctc caggagctgc tggacctgca cagggacagt gagagagagg ccattgaagt 1320

cttcatcagg agttccttca aagatgtgga ccatctattt caaaaggagt tagcggccca 1380

gctagaaaaa aagcgggatg acttttgtaa acagaatcag gaagcatcat cagatcgttg 1440

ctcagcttta cttcaggtca ttttcagtcc tctagaagaa gaagtgaagg cgggaattta 1500

ttcgaaacca gggggctatc gtctctttgt tcagaagcta caagacctga agaaaaagta 1560

ctatgaggaa ccgaggaagg ggatacaggc tgaagagatt ctgcagacat acttgaaatc 1620

caaggagtct atgactgatg caattctcca gacagaccag actctcacag aaaaagaaaa 1680

ggagattgaa gtggaacgtg tgaaagctga gtctgcacag gcttcagcaa aaatgttgca 1740

ggaaatgcaa agaaagaatg agcagatgat ggaacagaag gagaggagtt atcaggaaca 1800

cttgaaacaa ctgactgaga agatggagaa cgacagggtc cagttgctga aagagcaaga 1860

gaggaccctc gctcttaaac ttcaggaaca ggagcaacta ctaaaagagg gatttcaaaa 1920

agaaagcaga ataatgaaaa atgagataca ggatctccag acgaaaatga gacgacgaaa 1980

ggcatgtacc ataagctaaa gaccagagcc ttcctgtcac ccctaaccaa ggcataattg 2040

aaacaatttt agaatttgga acaagcgtca ctacatttga taataattag atcttgcatc 2100

ataacaccaa aagtttataa aggcatgtgg tacaatgatc aaaatcatgt tttttcttaa 2160

aaaaaaaaaa agactgtaaa ttgtgcaaca aagatgcatt tacctctgta tcaactcagg 2220

aaatctcata agctggtacc actcaggaga agtttattct tccagatgac cagcagtaga 2280

caaatggata ctgagcagag tcttaggtaa aagtcttggg aaatatttgg gcattggtct 2340

ggccaagtct acaatgtccc aatatcaagg acaaccaccc tagcttctta gtgaagacaa 2400

tgtacagtta tccgttagat caagactaca cggtctatga gcaataatgt gatttctgga 2460

cattgcccat gtataatcct cactgatgat ttcaagctaa agcaaaccac cttatacaga 2520

gatctagaat ctctttatgt tctccagagg aaggtggaag aaaccatggg caggagtagg 2580

aattgagtga taaacaattg ggctaatgaa gaaaacttct cttattgttc agttcatcca 2640

gattataact tcaatgggac actttagacc attagacaat tgacactgga ttaaacaaat 2700

tcacataatg ccaaatacac aatgtattta tagcaacgta taatttgcaa agatggactt 2760

taaaagatgc tgtgtaacta aactgaaata attcaattac ttattattta gaatgttaaa 2820

gcttatgata gtcttttcta actcttaaca ctcatacttg aaaactttct gagtttcccc 2880

agaagagaat atgggatttt ttttgacatt tttgactcat ttaataatgc tcttgtgttt 2940

acctagtata tgtagacttt gtcttatgtg tgaaaagtcc taggaaagtg gttgatgttt 3000

cttatagcaa ttaaaaatta tttttgaact gaaaatacaa tgtatttcac 3050

<210>  115
<211>  50
<212>  DNA
<213>  homo sapiens

<400>  115
aagcattcct gacattcaag cgcacgagtg ggtgccgctg tggctactgc          50


<210>  116
<211>  3074
<212>  DNA
<213>  homo sapiens

<400>  116
attgtcttcc aggaaacagc tccctcagtt tggaatcagc tctcccgctg cggccgcagt     60

agccggagcc ggagccgcag ccaccggtgc cttcctttcc cgccgccgcc cagccgccgt    120

ccggcctccc tcgggcccga gcgcagacca ggctccagcc gcgcggcgcc ggcagcctcg    180

cgctccctct cgggtctctc tcgggcctcg ggcaccgcgt cctgtggggc ggccgcctgc    240

ctgcccgccc gcccgcagcc ccttcgctgc gcggcccctg ggcggccgct gccatgggca    300

ccgacagccg cgcggccaag gcgctcctgg cgcgggcccg caccctgcac ctgcagacgg    360

ggaacctgct gaactggggc cgcctgcgga agaagtgccc gtccacgcac agcgaggagc    420

ttcatgattg tatccaaaaa accttgaatg aatggagttc ccaaatcaac ccagatttgg    480

tcagggagtt tccagatgtc ttggaatgca ctgtatctca tgcagtagaa aagataaatc    540

ctgatgaaag agaagaaatg aaagtttctg caaaactgtt cattgtagaa tcaaactctt    600

catcatcaac tagaagtgca gttgacatgg cctgttcagt ccttggagtt gcacagctgg    660

attctgtgat cattgcttca cctcctattg aagatggagt taatctttcc ttggagcatt    720

tacagcctta ctgggaggaa ttagaaaact tagttcagag caaaaagatt gttgccatag    780

gtacctctga tctagacaaa acacagttgg aacagctgta tcagtgggca caggtaaaac    840

caaatagtaa ccaagttaat cttgcctcct gctgtgtgat gccaccagat ttgactgcat    900

ttgctaaaca atttgacata cagctgttga ctcacaatga tccaaaagaa ctgctttctg    960

aagcaagttt ccaagaagct cttcaggaaa gcattcctga cattcaagcg cacgagtggg   1020

tgccgctgtg gctactgcgg tattcggtca ttgtgaaaag tagaggaatt atcaaatcaa   1080

aaggctacat tttacaagct aaaagaaggg gttcttaact gacttaggag cataacttac   1140

ctgtaatttc cttcaatatg agagaaaatt gagatgtgta aaaatctagt tactgcctgt   1200

aaatggtgtc attgaggcag atattctttc gtcatatttg acagtatgtt gtctgtcaag   1260

ttttaaatac ttatcttgcc tccatatcaa tccattctca tgaacctctg tattgctttc   1320

cttaaactat tgttttctaa ttgaaattgt ctataaagaa aatacttgca atatattttt   1380

cctttatttt tatgactaat ataaatcaag aaaatttgtt gttagatata ttttggccta 1440

ggtatcaggg taatgtatat acatattttt tatttccaaa aaaaattcat taattgcttc 1500

ttaactctta ttataaccaa gcaatttaat tacaattgtt aaaactgaaa tactggaaga 1560

agatattttt cctgtcattg atgagatata tcagagtaac tggagtagct gggatttact 1620

agtagtgtaa ataaaattca ctcttcaata catgaatgga aacttaaatt tttttttatg 1680

tgtccttgct tatagtttag ctgtaataat ttaaccttgt attcttgtgc catattctgt 1740

ctttttatta cttataaaga caaaccaaag taaatctgaa aggagactag aagctttgaa 1800

attattgttt gggggtttta taaaagcaac tactgtcacc tccatccaga ttcttttaaa 1860

ttattgatcc atccatagta tatattgcta ctcattcaag aatcctcaat aagtattgag 1920

tatttaccat atgttgggat actgtgggct ctggagagag gagggggcaa tagagctagg 1980

aattaagaat cagttgagta aaatgtgtaa tatttattcc ccattaataa ctgactagga 2040

aggactaaaa gccagaaagg ggatgaaaaa aaaatcctta attcagggcc gacattatct 2100

acttaaacaa ctttgagata tggtcttaat tattttaaag cagaataata taattgaaag 2160

tttatagcta aaagagacta tataggtcat ttagtataat tcttcattag tttacgaacc 2220

acaaaattgc aaataaataa gctatgaact ttgatgtaca ctataaatct ccttaattct 2280

ataaatttgt gtctgtaacc tgaatagttt gaaaacttct ttaaaaatct cttgtatttc 2340

atccgggcgc agtggctcac acctgtaatc ccagcacttt gggaggccga ggtgggcaga 2400

tcacgaggtc aggagtttga gaccagcctg accaacatgg taaaacccca tctctactaa 2460

aatacaaaaa ttggctgggc gtggtggcac tcgcctgtaa tctcagctac ttgggaggct 2520

gaggcaggag aatcgcttga acccgggagg cggaggttac agtgagccga gatcacatca 2580

ctgcactcca gcctgggcga cagagcgaga ctccatctca aaaaaaaaaa aaaactcttg 2640

tatctcaata tttttaaacc acaggcctaa ataaaactaa ttttgctcaa gttttctcaa 2700

cctagggaaa aagaactatg gttccatatt caaaataaat attatagacc cttttcctaa 2760

gtaggatttt gtggtttact gattgggtaa tttgatcatt aaaattatgt gaaatctgcc 2820

cgggcacacc tcatgcctgt aatcccagca ctctgggagg ccaaggcaga tgatcacctg 2880

aggtcaggag ttctagacca gcctggctaa catggtgaaa ccctgtatct gctaaaaata 2940

caaaaattag ccaggcgtgg tggcgggctc ctgtaatccc agctactttg gaggcgaggc 3000

acgagaatcg cttgaacctg ggaggcggag tttgcagtga gccgagatca cgccattgca 3060

ctccagcctg ggcg 3074

<210> 117
<211> 50
<212> DNA
<213> homo sapiens

<400>  117
tctcagatct cttcgcccat ctatggctgt gttgtcacat ctgtcccctc          50

<210>  118
<211>  1467
<212>  DNA
<213>  homo sapiens

<400>  118
caccatgctc gccctggagg ctgcacagct cgacgggcca cacttcagct gtctgtaccc     60

agatggcgtc ttctatgacc tggacagctg caagcattcc agctaccctg attcagaggg    120

ggctcctgac tccctgtggg actggactgt ggccccacct gtcccagcca ccccctatga    180

agccttcgac ccggcagcag ccgcttttag ccacccccag gctgcccagc tctgctacga    240

accccccacc tacagccctg cagggaacct cgaactggcc cccagcctgg aggccccggg    300

gcctggcctc cccgcatacc ccacggagaa cttcgctagc cagaccctgg ttcccccggc    360

atatgccccg taccccagcc ctgtgctatc agaggaggaa gacttaccgt tggacagccc    420

tgccctggag gtctcggaca gcgagtcgga tgaggccctc gtggctggcc ccgaggggaa    480

gggatccgag gcagggactc gcaagaagct gcgcctgtac cagttcctgc tggggctact    540

gacgcgcggg gacatgcgtg agtgcgtgtg gtgggtggag ccaggcgccg gcgtcttcca    600

gttctcctcc aagcacaagg aactcctggc gcgccgctgg ggccagcaga aggggaaccg    660

caagcgcatg acctaccaga agctggcgcg cgccctccga aactacgcca agaccggcga    720

gatccgcaag gtcaagcgca agctcaccta ccagttcgac agcgcgctgc tgcctgcagt    780

ccgccgggcc tgagcacacc cgaggctccc acctgcggag ccgctggggg acctcacgtc    840

ccagccagga tccccctgga agaaaaaggg cgtccccaca ctctaggtga taggacttac    900

gcatccccac cttttggggt aaggggagtg ctgccctgcc ataatcccca agcccagccc    960

gggcctgtct gggattcccc acttgtgcct ggggtccctc tgggatttct ttgtcatgta   1020

cagactccct gggatcctca tgttttgggt gacaggacct atggaccact atactcgggg   1080

aggcagggta gcagtgcttc cagagtccca agagcttctc tgggattttc ttgtgatatc   1140

tgattcccca gtgaggcctg ggaccttttt aagatcgctg tgtgtctgta aaccctgaat   1200

ctcatctggg gtgggggccc tgctggcaac cctgagccct gtccaaggtt ccctcttgtc   1260

agatctgaga tttcctagtt atgtctgggg ccctctggga gctgttatca tctcagatct   1320

cttcgcccat ctatggctgt gttgtcacat ctgtcccctc atttttgaga tcccccaatt   1380

ctctggaact attctgctgc ccctttttat gtgtctggag ttccccaatc acatctaggg   1440

ctcctccaaa aaaaaaaaaa aaaaaaa                                       1467

<210>  119

<211>  50
<212>  DNA
<213>  homo sapiens

<400>  119
tgctgaaact cacagcaaga gctgtgttcc agttagcttt gctaccagtt                50


<210>  120
<211>  700
<212>  DNA
<213>  homo sapiens

<400>  120
cggaggtttg cgcgccttgg tgagccgttg gcgtggtggt cccggagtga tcctggcagc      60

cggtggggaa gacaaggagg gtttgagcat ggcagaagga aacaccctga tatcagtgga     120

ttatgaaatt tttgggaagg tgcaaggggt gtttttccgt aagcatactc aggaaatgac     180

tgttgaaaac agaattgctg aaactcacag caagagctgt gttccagtta gctttgctac     240

cagttatgca ggctgagggt aaaaagctgg gattggtagg ctgggtccag aacactgacc     300

ggggcacagt gcaaggacaa ttgcaaggtc ccatctccaa ggtgcgtcat atgcaggaat     360

ggcttgaaac aagaggaagt cctaaatcac acatcgacaa agcaaacttc aacaatgaaa     420

aagtcatctt gaagttggat tactcagact tccaaattgt aaaataatgg cctgaattta     480

agttttctaa gataaactca gtggtttggt ttttattatt aatagagata gaactattgt     540

gtgttaatat tagcattagt caataagtta ttttaatgtc agatttttga atgttattat     600

atattacctg tatgatggaa ggattaccac tgtacacaaa tctaatcaat aaaaacgtta     660

gaaccttctg cttagagtac ttttaaaaaa aaaaaaaaaa                           700


<210>  121
<211>  50
<212>  DNA
<213>  homo sapiens

<400>  121
tgctgagcag gaattactct cagatgacgc ttcatctgtt tcacaaattc                50


<210>  122
<211>  4454
<212>  DNA
<213>  homo sapiens

<400>  122
gacgctcgag gggcgctcgg ctagtcccgc cagagcgcga gccgccagcc cgtaacggtc      60

gccagtgtga ggggcgggag ggaaagaaga ggggtttaaa ttagattttt taaaacacag     120

agcaagcgcc agaggcgtcg gcatcccagg tgtcgccgct tcctgctgca cagggctcgg     180

cgtacaggtc cctccctcct caagccccct ccccttctcc cgccctaccc tctggggctc     240

tgcggcgctt aagaggcggc cgcagcggcg gatccggcgg ctgctgcagc ccgggcggct     300

gccgagaagg agggagggga aacacaaagc cggctacgcg ctgcgagata acaagagtaa 360

tccacagact taaaacatga gctcagatgc cagccaaggc gtgattacca ctcctcctcc 420

tcccagcatg cctcacaaag agagatattt tgaccgcatc aatgaaaatg acccagaata 480

cattagggag aggaacatgt ctcctgatct acgacaagac ttcaacatga tggagcagag 540

gaaacgagtt actcagatcc tgcaaagtcc tgcctttcgg gaagacttgg aatgccttat 600

tcaagaacag atgaagaaag gccacaaccc aactggatta ctagcattac agcagattgc 660

agattacatc atggccaatt ctttctcggg tttttcttca cctcctctca gtcttggcat 720

ggtcacacct atcaatgacc ttcctggtgc agatacatcc tcatatgtga agggagaaaa 780

acttactcgc tgtaaacttg ccagcctgta cagacttgta gacttgtttg gatgggcaca 840

cctggcaaat acctatatct cagtaagaat aagtaaggag caagaccaca ttataataat 900

tcccagaggc ctatcttttt ctgaagctac agcctccaat ttggtgaaag tcaatataat 960

aggagaagtg gttgaccagg gaagtaccaa tttgaaaatt gaccatacag gattcagtcc 1020

ccatgctgca atctattcaa cacgtcctga tgttaagtgt gtgatacaca tccataccct 1080

tgcaacagca gctgtatcct ccatgaaatg tgggatcctt ccaatttctc aagagtctct 1140

tcttctggga gatgttgcct attatgacta ccaagggtca cttgaagaac aggaggagag 1200

aattcaactg cagaaggttc tgggaccaag ttgtaaggtg ctggtactca ggaatcatgg 1260

tgtggttgca cttggagaaa cattagagga ggcttttcat tatattttta atgtgcaact 1320

agcctgtgag attcaggtgc aggccctagc aggtgcaggt ggagtagaca atctccatgt 1380

actggacttt cagaagtata aagctttcac ttacactgta gcagcgtctg gtggaggagg 1440

tgtgaatatg ggttcccatc aaaaatggaa ggttggcgaa attgagtttg aagggcttat 1500

gaggactctg gacaacttgg ggtatagaac aggctatgct tacaggcatc ctctcattcg 1560

agagaagcct aggcacaaga gtgatgtgga aatcccagca actgtgactg ctttttcctt 1620

tgaagacgat acagtgccac tctctcctct caaatacatg gcacagaggc aacagcgtga 1680

aaaaacaaga tggctgaact caccaaatac ttacatgaaa gtgaatgtgc ctgaggagtc 1740

tcggaacgga gaaaccagtc cccgaaccaa aatcacgtgg atgaaagcag aagactcatc 1800

taaagttagt ggtggaacac ctatcaaaat tgaagatcca aatcagtttg ttcctttaaa 1860

cacaaacccg aatgaggtac tagaaaagag aaataagatt cgggaacaaa atcgatatga 1920

cttgaaaaca gcaggaccac aatctcagtt gcttgctgga attgttgtgg ataagccacc 1980

ttctactatg caatttgaag atgatgatca tggcccacca gctcctccta acccatttag 2040

tcatctcaca gaaggagaac ttgaagagta taagaggaca atcgaacgta aacaacaagg 2100

cctagaagat gctgagcagg aattactctc agatgacgct tcatctgttt cacaaattca 2160

gtctcaaact cagtcaccgc aaaatgtccc tgaaaaatta gaagaaaacc atgagctgtt 2220

ttccaagagc ttcatctcca tggaagtgcc tgtcatggta gtaaatggca aggatgatat 2280

gcatgatgtt gaagatgagc ttgctaagcg agtgagtagg ttaagcacaa gtacaaccat 2340

agaaaacatc gagattacta ttaagtctcc agagaaaatc gaagaagtcc tgtcacctga 2400

aggctcccct tcaaaatcgc catccaagaa aaagaagaaa ttccgcactc cttcttttct 2460

gaaaaagaac aaaaaaaagg agaaagttga ggcctaaata aagtcttttt ataattatta 2520

ttataacaat gtgacattgc acatctaaat accacattta agttgatcat taatatgcaa 2580

tggtagatca gattggggga tgtagcaaac tggactttaa gaactggaaa gaggttttac 2640

aaaagaaaaa ctttcagatt catctctcat tttatatgtc cagaaatggc tttgaatttt 2700

aagcaattac tagttttaat tagctctgcc ctcatgaagt attattataa ttcaccataa 2760

acagctatct gtctgaatta cttcaggcct tctccataat atctgttaga aagaaattgc 2820

cagtgagcaa gtgagaattt ttatttctca atacctgctt cacttgataa tcatattata 2880

attttttatc atgattattg actatatttt tggagtccca ttgtttcagt gggcattaac 2940

agaatgcttt aaaaacttct aagacaagaa tctatagcat tagtatacac tggcacataa 3000

ttttttaaaa agttttaaga aaagattcat ttggaatttt attcacagta taaaatttcc 3060

tcacctgaag taactttgtt tgccaaaaaa gttgttttaa taaactataa tttttgaaaa 3120

cttccttttt tattagttta gaaagcccct tatttttcaa caaaggggat tttgtacaca 3180

taacatgggt tatttagttt aactctggca aaaaaaaaaa aaaaaatttt gtatgttgat 3240

gtttgtatac cgttcagtat aaaagtgtcc taagcatatt agccaatctt ttcacagtag 3300

agcatactta aggctgcttg gtactgagta tacttaaata taactccaga atccagggac 3360

ttggtgttaa aacaggatta gagcatgtaa aggtacatct agattcatat ttgaatctta 3420

aactgtattt ttctcttagt attgctaatg agtaaagaaa agtctcataa ggtagccaaa 3480

tgaaaaagaa tgaaagggaa agtgaaaaat taaggggaca aaagatggga tgtgaaaaga 3540

agaattctag tttgatggtg actcatattc acgataggat acaaagtgtg atttgttgga 3600

aacatgtccc aaatttctaa aattctgctt ctctgccaaa agcaatgtct ttcttggttg 3660

atatttgagt tttaaaaggg tcaaatcttt ctaatttttt gtatctttag agggcagcac 3720

tagaagaaat cagcaggtct aatcccacca gtaagaaaac taccacttct tgatttttac 3780

agatttaaaa aaatcttttc agtgaccttt ctttttaatg taaatacaaa tttaaaccta 3840

ggcttaatat aggcgtttcc cctttcaccc aagtgatgtc acagttcgat gcaaaatcaa 3900

tgatccagaa tgatcgtggg taaaaataac tcaaagtgtt tcttaagggt gagttggcat 3960

gcaaaaaatt acattgatta cagtgtgttt tggagctggc tctgtttgtg tgcatatgat 4020

aatgcagagt tgagccagag cctggaaatg tcattctaga tctcactaac tactggaatc 4080

agtgttttaa tctcttggtg gaaactttca gttgcttaac tctctattgg aagatttttt 4140

taatgttcta catcatttat gttgtattac aatgtatgta gaaatagtaa cctgtgaact 4200

atgcttttcc ataacttttt aaaaatatat atatctaaat gaatgcaatg tgcataaata 4260

ttttttaaac ataacagtga actattgcac cttttgctaa tgcctctatt tacttgcttt 4320

ggcataaaga atgagccaat gaacctctgt gtcctgtgga aaaatgtata aatgttatct 4380

gatattgctc ttagatgtaa tgctaattaa tgttaaatca caaataaaca gtattttaaa 4440

tataaaaaaa aaaa 4454

<210> 123
<211> 50
<212> DNA
<213> homo sapiens

<400> 123
cccgggaaga gccggtactc acccttgtgg acgaagcaat tgtaaagcag 50

<210> 124
<211> 5537
<212> DNA
<213> homo sapiens

<400> 124
gaacccggcg aggaaataca tgcactggct gagaatcgcc cgcgccaggg cgcaacgcca 60

caaggtgtag ggagtgtgcg gggtggggcg aaaggggacc caagagtccc tgtggctcgg 120

agtgccgggc cgtcggttct tcattcctgc cctcggggca gacggagtga ccccggcccc 180

cactccccgc cccgaccatg gtagtgttca atggccttct taagatcaaa atctgcgagg 240

ccgtgagctt gaagcccaca gcctggtcgc tgcgccatgc ggtgggaccc cggccgcaga 300

ctttccttct cgacccctac attgccctca atgtggacga ctcgcgcatc ggccaaacgg 360

ccaccaagca gaagaccaac agcccggcct ggcacgacga gttcgtcacc gatgtgtgca 420

acggacgcaa gatcgagctg gctgtctttc acgatgcccc cataggctac gacgacttcg 480

tggccaactg caccatccag tttgaggagc tgctgcagaa cgggagccgc cacttcgagg 540

actggattga tctggagcca gaaggaagag tgtatgtgat catcgatctc tcagggtcgt 600

cgggtgaagc ccctaaagac aatgaagagc gtgtgttcag ggaacgcatg cggccgagga 660

agcggcaggg ggccgtcagg cgcagggtcc atcaggtcaa cggccacaag ttcatggcca 720

cctatcttcg gcagcccacc tactgctccc attgcagaga cttcatctgg ggtgtcatag 780

gaaagcaggg ataccagtgt caagtctgca cctgcgtggt ccacaagcgg tgccacgagc 840

tcataatcac aaagtgtgct gggttaaaga agcaggagac ccccgaccag gtgggctccc 900

agcggttcag cgtcaacatg ccccacaagt tcggtatcca caactacaag gtccctacct 960

tctgcgatca ctgtgggtcc ctgctctggg gactcttgcg gcagggtttg cagtgtaaag 1020
tctgcaaaat gaatgttcac cgtcgatgtg agaccaacgt ggctcccaac tgtggagtgg 1080
atgccagagg aatcgccaaa gtactggccg acctgggcgt taccccagac aaaatcacca 1140
acagcggcca gagaaggaaa aagctcattg ctggtgccga gtccccgcag cctgcttctg 1200
gaagctcacc atctgaggaa gatcgatcca agtcagcacc cacctcccct tgtgaccagg 1260
aaataaaaga acttgagaac aacattcgga aagccttgtc atttgacaac cgaggagagg 1320
agcaccgggc agcatcgtct cctgatggcc agctgatgag ccccggtgag aatggcgaag 1380
tccggcaagg ccaggccaag cgcctgggcc tggatgagtt caacttcatc aaggtgttgg 1440
gcaaaggcag ctttggcaag gtcatgttgg cagaactcaa gggcaaagat gaagtatatg 1500
ctgtgaaggt cttaaagaag gacgtcatcc ttcaggatga tgacgtggac tgcacaatga 1560
cagagaagag gattttggct ctggcacgga aacacccgta ccttacccaa ctctactgct 1620
gcttccagac caaggaccgc ctctttttcg tcatggaata tgtaaatggt ggagacctca 1680
tgtttcagat tcagcgctcc cgaaaattcg acgagcctcg ttcacggttc tatgctgcag 1740
aggtcacatc ggccctcatg ttcctccacc agcatggagt catctacagg gatttgaaac 1800
tggacaacat ccttctggat gcagaaggtc actgcaagct ggctgacttc gggatgtgca 1860
aggaagggat tctgaatggt gtgacgacca ccacgttctg tgggactcct gactacatag 1920
ctcctgagat cctgcaggag ttggagtatg gcccctccgt ggactggtgg gccctggggg 1980
tgctgatgta cgagatgatg gctggacagc ctccctttga ggccgacaat gaggacgacc 2040
tatttgagtc catcctccat gacgacgtgc tgtacccagt ctggctcagc aaggaggctg 2100
tcagcatctt gaaagctttc atgacgaaga atccccacaa gcgcctgggc tgtgtggcat 2160
cgcagaatgg cgaggacgcc atcaagcagc acccattctt caaagagatt gactgggtgc 2220
tcctggagca gaagaagatc aagccaccct tcaaaccacg cattaaaacc aaaagagacg 2280
tcaataattt tgaccaagac tttacccggg aagagccggt actcaccctt gtggacgaag 2340
caattgtaaa gcagatcaac caggaggaat tcaaaggttt ctcctacttt ggtgaagacc 2400
tgatgccctg agagcccact gcagttggac tttgccgatg ctgcaagaag gggtgcagag 2460
aagactcctg tgttggagac actcagcagg tcttgaacta cttctcctcc tcggagcccc 2520
agtcccatgt ccactgtcta tttattgcat tcccttgccc caggccacct cctccccctc 2580
ccacctggtg accagaaggc gctctcggtt cttgtctcac cagtaatgca gactcattgg 2640
gtcagcaatt agctgtatac actgccgtgt ttggaccatt ggcaagcctg gttccactcc 2700
tcaggggctc ctggcagtga agcaacttca gttcttttac tgcaaagaac agaaaaaaga 2760
aagaaagcaa acaagaagac tccggctctg ctatcggaca cagatcctga tccctcttgc 2820
ttcttttccc tcctgcaccg cagcttgcca tccctgccct tctgtcctgg agaagagact 2880

ggtgcttctc cgcacacacg agggagggcg cccttgaggc atgccctctg agggagggag 2940

accagagatg cagggattgg ccagctgggt tggtttgctc tggaatggct aactcttgcc 3000

tgctttggtt ttagcttttc agcatgccaa agtcatgtaa gtttgtgtct tgtggaagaa 3060

atcctctttg tggaaaaaga aacagggttt tgaactctgt taacatttga aaaatatatt 3120

ttcaaattca ctttctaatt ggccaaaaga gatgagttcc agtctgaata caggtagata 3180

ttaaagggct aataaaaaat gagaaaccgg tcgtccaagg tggatgctgt caatgcccga 3240

gtgacacatg agagctgtat gaattgagag aaaaggcaac aagtagcatt cttcatcatt 3300

caagttctac ctggacacaa aggcgaggac cctggggttc caacaaagct cagctcccag 3360

attctctttc cagtttcatc ctaagttcct agcataaaca ctatttattt tctgcagcag 3420

tgtgttattt ttgcgcactt atacaaaatg gtagtactac tgtgttgtgg tttttaaaca 3480

ttaaacatgt aaagttatat acgaaatatc tgcttttgga ataagcagaa tgaggctaaa 3540

catgggttat acaaagggta tctggaaact gaagagcaac ttgttagaaa actgacaatg 3600

tcgcaagatg tactcagttt tgtttctgtg tgacatgcaa tggcaactca tgtggacact 3660

attgaaggga tgtgacatta cctcctgtag atatgctaac agtgttattc tttcatttcc 3720

aagggttctc tgtggctttg tgtatatgtt tcccagaggt catttgatta cctaatttac 3780

tgaactgatt tagcagggaa tggaatccat tccaactatt gcacgtggat ttcccagctg 3840

cccctaaata tatatacttg tgagtggcaa agtggcacta atgaagcttt tgccttttgt 3900

acatttgaga tttttgtata tagtgtttgc tgcaaggcct gtggaattaa ttcgttgcat 3960

atagaggtat caactgctgc atgttcaggc atattataaa actttagtct atgaaagaat 4020

aattataata atgtccaggt gcaatactct gtaagtctat tggttcaagt taccgagaga 4080

taggtgtgtt cctttatggg ggatgggggg gtgtgttggg gattctttgt attgtttatt 4140

tcattttggt ttattttaaa agatgtaaac atatattaag ctatattaaa tctcacatac 4200

agttcttctg tgctctatta taccctgata gagatggggg agagaaagga atgtttttga 4260

tggtggtttc aaagctcgga cagtaactat cttgagccca ttagagagtc tgtgtccata 4320

tttgcatctg gctggtcata gcctttgtta ctaatgatga cattcagttc tcttttgttt 4380

ttatttttta aaaactcagg tgtaattatt atctgttctt aagataattg caaatattaa 4440

atattatgat atatcaattc atgtgtttgg cataccagtg aatgatgaag aacatgagat 4500

taatttaatt tatcttcggt aacttgacat tctggagaga gactatcttc tggagttgag 4560

tacaagcaca gaaacatctt tacggtggca tcatctcatt ttttaggaag acatgataat 4620

actgcccatc atattcatgt gtaactactg ttctttcttc tgctttcttc accataataa 4680

actttggaca accaagcaag ctctaaccgc aatgccagat ggccttgtcc gagggcctag 4740

tgtttgcacg gcagtgggaa ctgggccttt cctacaggac aactggcaag tttgctggga 4800

agtcaaataa tacattccac ctggcagctg aaggcagcca gtcagtctgt cccagaaagg 4860

gcccttttca gcacccaaag ctgggctggc tgggatgcct ctggctggtg aagttctcac 4920

ataggctgat ttaaatccag caaaggtcta tagaaaaagg cttgcgtgtt cgttgagtaa 4980

tcattgtttc attttcattt ttacgagagt ttgaaaatag acacactgtt aacacttctg 5040

ccagtttttt ctgatctttc cagccccacc ccctttctct ttctctctct ctctcaaaga 5100

aaaaaaaaat gggagtgcaa aaaaaacaaa gccaaaaaat atatgaagga tagctgttct 5160

tctgtgttct ctcattatgg actttgtgaa gtagaaacat aatttttttt cctccaaagg 5220

tgaaaaaaca atgcattctt gctttaaaaa aaaaaaagaa ggctaaaaaa ttacctcttt 5280

ttaaattatg tgcaaaataa ttctggctaa ctgtaaaatg tattcaattt taggattttt 5340

tttttttgta ttgtgatgct ttatttgtac atttttttcc tttctggatg taattttaat 5400

ctcttgccat tcattagtgt tatttcattg taaacgttat tgtgccaaat gtactgtatt 5460

caaaaggatg tgaatgtgta ttgtttcaga acctaataaa tacaatgacg ttaagtctta 5520

aaaaaaaaaa aaaaaaa 5537

<210> 125
<211> 50
<212> DNA
<213> homo sapiens

<400> 125
cctccagttc cttggcagtt ctcccccaaa ccaggtctgt acaggtgttc 50

<210> 126
<211> 2530
<212> DNA
<213> homo sapiens

<400> 126
aaaacaggag ctgattcgag ctggcagagc tgggccatgg aaccggtaga gacctggacc 60

cccggaaagg tggcaacttg gctgagaggt cttgacgact ccctgcagga ctatcccttt 120

gaggactggc agctgcctgg caagaacctg ctccagctct gcccccaaag cctcgaggct 180

ctggctgtgc ggtctctggg acaccaggag ctcatcctgg gcggggtgga acagctccag 240

gccctgagct ccaggctaca gacagagaac ctgcaaagcc tgacagaggg acttctgggg 300

gcaacccatg acttccagag catagtccaa ggctgcctgg gggactgtgc caagacccct 360

attgatgtcc tctgtgcagc tgtggagctg ttgcatgaag ctgacgccct cctcttctgg 420

ctcagcaggt acctcttctc ccacttaaat gatttctcag catgccagga gatccgagac 480

ttgttggagg agctgagcca ggtcttgcat gaggatggtc cagcggctga gaaggagggc 540

acagtcctga ggatctgcag ccacgtggct gggatctgcc acaacatcct ggtctgctgc 600

cccaaggagc tgctggaaca gaaggccgtg ctcgagcagg tgcagctgga cagtccattg 660

ggcctagaaa ttcacaccac cagcaattgc cagcactttg tgtcccaagt ggacacccag 720

gttcccactg actcccgact gcagatccag cctggagacg aggttgtcca gatcaacgag 780

caggtggtgg tgggatggcc ccgtaagaac atggtgaggg aactgctgcg ggagccagcc 840

ggactcagct tagtgctgaa gaagatcccg ataccggaga cccccccaca gacgccccct 900

caggtcctgg actccccgca ccagaggagc ccatcactgt ctctggcccc actgtctccc 960

agggccccat ctgaagacgt ctttgccttt gacctgtctt caaaccccag tcccggaccc 1020

agccctgcct ggacagactc tgcctccctt ggccctgagc ccctgcccat ccccccggaa 1080

cccccagcca tactcccagc aggggtagca gggactccag ggctccctga atcccctgac 1140

aagagtcctg ttggtcggaa gaaatcaaaa ggcctggcga cccggctgag ccgccggcgg 1200

gtgtcatgcc gtgagctggg ccggccggac tgtgacggct ggctcctgtt gcgaaaggca 1260

ccgggcggct tcatgggccc gcgctggcgc cgccgctggt ttgtgctcaa gggacacacg 1320

ctctactggt accgccagcc ccaggatgag aaggctgagg gcctcatcaa tgtctccaac 1380

tatagtctgg aaagtggaca tgatcagaag aagaaatatg tgtttcagct cacccatgat 1440

gtgtacaaac ccttcatctt cgctgctgat accctgacag atctgagcat gtgggtgcgt 1500

catctcatta cctgcatctc caagtaccag tctccaggcc gggccccccc accccgagag 1560

gaagactgct acagtgagac cgaagcagag gacccggacg atgaggctgg gtcccactca 1620

gcctcgccca gccctgctca agctgggagt cccctccatg gagacacatc acctgcagcc 1680

acccccacac agcgcagccc acggacctcc tttggctctc tgacagacag cagtgaagag 1740

gcactggaag gaatggtacg ggggctgagg cagggtggcg tgtccctcct aggccagcca 1800

cagcccctga cccaggaaca gtggcggagc tctttcatgc ggcgcaaccg agaccctcag 1860

ctcaatgagc gagtgcaccg tgtgcgggcg ctacagagca cactcaaggc aaagctgcag 1920

gagctgcagg tcctagaaga agtgctgggt gaccctgagc tgacaggaga gaagttccgc 1980

cagtggaagg agcagaaccg ggagctgtac tcagagggcc tgggggcctg gggagtggca 2040

caggctgaag gcagctccca catcttgacc tctgactcca cagaacagtc cccaaactcc 2100

ctgccctctg accctgaaga gcactcccat ctctgccccc tgacctcaga gagcagcctc 2160

cgacctcctg acctctgacc ctggccagca ctctagctcc tgacctttga cccgagggcc 2220

acctcaaccc cagcttctga cgtgtccagg acagagcatc cctggattct gttcagggtg 2280

ggaagtagta ctgctagtca tggtctcacc ccgagctgac ccctctgcct gggctttgtg 2340

ccaccctctc ccttgccaaa gaagaaactc tccccccaaa tcctccaacc tctggggcca 2400

cagccctgcc cctccagttc cttggcagtt ctcccccaaa ccaggtctgt acaggtgttc 2460

tttattttac atgagggcta ctttccaacc aaataaagtc aatttttcta agaaaaaaaa 2520

aaaaaaaaaa 2530

<210> 127
<211> 50
<212> DNA
<213> homo sapiens

<400> 127
ccacagacac catatatcct tctgcatcct ttggccaata aaagttgctg 50

<210> 128
<211> 1135
<212> DNA
<213> homo sapiens

<400> 128
aaaatgaaaa attgagtttt tccgtgaact ttatactgtc cagctctgtt gattttaaag 60

cctcttcatc caggtcagtt caggaagtat atctggagta cctgctctgt ttttggctgt 120

gagactagca ctaaggattc tggtaccttt acccaaacct actgggctac taatacttct 180

ctcagcagtt gatcaaatac aatagaccat gtaagctggg gccgctcatc cacttccagt 240

ttgctggtct ccctgctaga aaacacattg tactgtgctt tttctggaat tcagtataat 300

ggcatcactg cctgtttttc acatcttttg tttcctgttc attttaagga aacctactaa 360

atccagttaa tattaaatgg acaccactca ttaagaaatt tctttatggc ttctgctgaa 420

tacttaaatg ccttactaca gttatccagt tgacatgttt ttaattcata taaggtatat 480

tgggtatatt gaagtatata ttgtattaca aagacttgtt cttgtatttt aaaatgtcag 540

tgcaaaaaat atatggtgga acctttcttt aaagttgaaa tgcagtatta tttaaatctg 600

aaaggttaaa aagctttctt caccttatat atgttcttcc actgtgactt tttagttgaa 660

gactagtaaa ttaactttta gttagaagat gcctactgct tttgttgttt attttaatca 720

gcagagcaca gagacacata aaaactctgg gaaatgacta ggataaaaat atcagtatgt 780

atctgtttta gatattttga gttttgcttt ttttatgcct tgaatatttt atttcaaaaa 840

gtatctgaag caaattctca gactgaacta cttcttagac ctcactgtaa gaatatttta 900

ttcaatgtct catttatgat agatttgcaa gctgctcatt tttgaacagc tttttgcatg 960

ggataggagc atgtctattc taacacatca gcttattcaa aagcaagaat tttaaaaata 1020

agataaatgt aaagttgttt tataaacgat cctgttaatt aaaccacaga caccatatat 1080

ccttctgcat cctttggcca ataaaagttg ctggagaacc aaaaaaaaaa aaaaa 1135

<210> 129
<211> 50
<212> DNA
<213> homo sapiens

<400>  129
ccctgcgaac ccacaatctc tgccagcaga actggttttg ttctgaatag          50

<210>  130
<211>  4535
<212>  DNA
<213>  homo sapiens

<400>  130
gtgcgcgtgc gccctgactg ttggggggcg ggcggcgtcc gcggcgaggg agcgggggcg     60

gggaggcgcc gtctggctca ggctggggcc ggcgggagcg cgaacgagcg acggcggaac    120

ccgtgggcct gtggactgca gcctcggcgg tcagcagagt gcttgtgatt tcacgtattt    180

ttgctgaact cgtaaaagag acacttggat ggtggattaa ccagaacact aacattctgt    240

gaaaagtttc aaattggaga atattgaatt ttcaccctag tccagcagct ccgctgctca    300

cttaaataca gatgaatgaa gaccccattc ggaaagacac ctggccagcg gtccagagct    360

gatgcaggcc atgctggagt atctgccaac atgatgaaga agaggacatc ccacaaaaaa    420

catcggagca gtgtgggtcc gagcaaacct gtttcccagc cccggcggaa catcgtaggc    480

tgcaggattc agcatgggtg gaaagagggg aatggccctg ttacccagtg gaaaggaacc    540

gttctggacc aggtgcctgt aaatccttct ttgtatctta taaaatacga tggatttgac    600

tgtgtttatg gactagaact taataaagat gaaagagttt ctgcgcttga agtcctccct    660

gatagagttg cgacatctcg aatcagcgat gcacacttgg cagacacaat gattggcaaa    720

gcagtggaac atatgtttga gacagaggat ggttctaaag atgagtggag gggaatggtc    780

ttagcacgtg cacctgtcat gaacacatgg ttttacatta cctatgagaa agaccctgtc    840

ttgtacatgt accaactctt agatgattac aaagaaggcg accttcgcat tatgcctgat    900

tccaatgatt cacctccagc agaaagggaa ccaggagaag ttgtggacag cctggtaggc    960

aaacaagtgg aatatgccaa agaagatggc tcgaaaagga ctggcatggt cattcatcaa   1020

gtagaagcca agccctccgt ctatttcatc aagtttgatg atgatttcca tatttatgtc   1080

tacgatttgg tgaaaacatc ctagatgtca tcacaaactc tgccaaattt gtggaactat   1140

gaaatgtatt atttgtagac ataaagactt gattgctttc cagtttaatg aaagcttaaa   1200

tgtccctgcg aacccacaat ctctgccagc agaactggtt ttgttctgaa tagtacagat   1260

tgatgtgaac acaaagcatt ttgtgtaagg agaacccctt tcttttaaaa gaagtctgtc   1320

tatttcgagg ggagttacag gcaagtttgg taaaagttaa gctagtatca tagtcattta   1380

aaattgtaat agatcttaac cattttcccc ctcaccctaa ctctcttatt ctgccgccac   1440

aatgcaagca tagtttgatg ttttcgttat tgcctttttt gagatgtatg tatctgtatc   1500

tacctatatc tatatgtgtg tatacatata taatatatac acacagatac aagtgtacac   1560

acacacacca cacaccacac acaccccact ggcagtcttt gtggattagg atgggggtga 1620

cataattttc ttgagtttaa caggagtgct ttacccaagt cagtcatgga tatgatatca 1680

ctgctcttta tttaaaagta aactttgggc acaggaacca tatgggaaca ttcagcttgt 1740

ttaaaaaaaa aaaatcagaa gttcagagca ccttttcaat ctggagccct taaaccataa 1800

gtccaaatat gaattatttg tgaacagtta atgacccata ttagaatttt acaatctggt 1860

gaacaatctg ggtaaaaggc tacctttaat tctaagcttc attttttttt gggggggggt 1920

tacattttaa tcataaattc ttttttgcaa agcccaggtt cttgttccac acagtgtaat 1980

gtaggtgtat tttggacagc acatatggtc cattgtgtac aagtcagtaa tctggatttc 2040

agtacatgga tttaaaaggc aacaatccat cactggtttg tgtgtttttg gttaagtttc 2100

tggaagaata caatgctttt atattggaag tataagtttt gagtggcatt gttgccttct 2160

aacaagctct ctgggacttt taaagtttta ttactattat tgctaatgta tgattatggt 2220

cagagacttt caacccaccc cttcctgcct acccctggct tttcctcaca aagaatgtct 2280

agacaaacag cagcttagtc tcctgacacc tcttgtgggg tgcatgttcc tgctccaggt 2340

atgctgttca tgtggattca ttccattcag tggccccttg gggtttgcat gccagtgaag 2400

cgttaccata gaagagccag ccgtttgtgt tcgcttggta ggtgtaagta atagcagccc 2460

ttagccacgg agtctttatg tggtttattc ctgcaaggtt gttttaaact gaaatagaac 2520

tgctagtgtg attggttgat cagcaaaaca gctgctctca ttttgttttg gaggaaccct 2580

ggtggtttga cagccaggag aaaggccata tgtgaaacca gtctcttgtg gagattggaa 2640

atcctccctg atatttgggc agagcctaaa cgtgcatgct tgtcactcaa acaccagaag 2700

tttgaatcag aggtctgagt gacctctttt gtggctgttc ttgatggttt ccaggcctcg 2760

ttatgcatgg tttgcttgat gcccattttt tgtgccttgt gctgttggat ggtaacgacc 2820

actcaccatg taaacacagt acctcagttt tctgtccaca gctgcagtcg ctctgtccct 2880

cgtgcttcac tgtgggcagc cagggacaag cttctgaaag cagtgtgata cccaagtcat 2940

aggtgcttct cgtatgtgaa caggtgtcat tctacagatc tgttatgtgt tttcctgttg 3000

actcttaaga gtccctgcat gtaaatctca aagctgagcc tggctcccag agtccagact 3060

gacattacag cgcaagagtt tgcaagtgtg tccagcatgc accaactgtg cagaacttgg 3120

ctcggtaact ttgcgtggaa aaataaagta cattttgaag tatcttgagg gttgggtcaa 3180

ttgagacatt tctagcatta cttaatgact tgcattgtgg tttttctgca agcaacttta 3240

atgacttttt ttataccaca tggtctccca gtttctagat gaatgcaaca tgatgatggt 3300

gatgatgacg atgagtttaa tcattgttca tttattgcct ttagggctga gggaaaggga 3360

aggtttgttt ttttttctcc ccattttccc ccattctgtc tttcttttgg tgacttacac 3420

cacatgtaat gacgctatga ctaattctgc tcccaagccc ttgtatcttg ggcttcattt 3480

taggctcatg tgtcagatct gcatgcattg cttgcatttt tctggtatct gaatgttggt 3540

tccttgttcc aggaattcaa cattaatttc caaaagtatc atgggacttg tgacaataca 3600

agacatgaat ctatgtataa aatttatcgg cctttctcat ttacctgctc tagtattatt 3660

gtattgtgtg tgcgtgcgtg tgtgatgtca ggctgccacg taaaacttca gagaaaaatc 3720

ttaaaagcag accatccttt tttgcatgct ctattctaag tagaatgttc aatgtaactg 3780

actaaaattg catgttaaag atatttaggt ttttttgttt tctttatttt tatttgtttt 3840

cagtttcctg tatatttgct tactgtgccg ttttagtggt tttaggataa aaatgcactg 3900

gtgaagcaaa tgtagtgcca acagaaggtg attttccagt tgtaaatgtc atgcagcatt 3960

tgaagggact gtgttttctt aaaaaaaaat cacagttact tctaaaccag atttcatttc 4020

ttttattgtt ttatgtgcca aaccacgaag tgcattgggc ttcaatctct gaacactgta 4080

gacccattag aagactgttc cgattgttac aaattgtagt gcctgaaaac actcttaagc 4140

tgattgtctt aacaaaatga aagttctcca aagacaaaac agaacaatta ttataacaaa 4200

ataattatgg ttgaaatgtc tgtggttcct tggaaatgct gcgctctttg tgtttttcca 4260

tcattagtgc agttggaatg aatgtgtata ggtcagaggt cttcgtgttc acattttaaa 4320

attaggtaaa tgacctcatc tttcaagctt gaattcattt ttaattttaa ttttatttta 4380

tacaatgtgt agacagttcc ctgttctctg catttagaag tatacacaat ataaatctgt 4440

taattctgta agtaattttt ataattatga tgtaactcta tcttatcctt aaaacattta 4500

aaataaaccc tttatgtgca actcctgact gtaaa 4535

<210> 131
<211> 50
<212> DNA
<213> homo sapiens

<400> 131
ggccaaaacc tgagaagcgg tgggctaaga taggtcctac tgcaaaccac 50

<210> 132
<211> 5889
<212> DNA
<213> homo sapiens

<400> 132
gctgccagct gagttttttt gctgctttga gtctcagttt tctttctttc ctagagtctc 60

tgaagccaca gatctcttaa gaactttctg tctccaaacc gtggctgctc gataaatcag 120

acagaacagt taatcctcaa tttaagcctg atctaacccc tagaaacaga tatagaacaa 180

tggaagtgac aacaagattg acatggaatg atgaaaatca tctgcgcaag ctgcttggaa 240

atgtttcttt gagtcttctc tataagtcta gtgttcatgg aggtagcatt gaagatatgg 300

ttgaaagatg cagccgtcag ggatgtacta taacaatggc ttacattgat tacaatatga 360
ttgtagcctt tatgcttgga aattatatta atttacatga aagttctaca gagccaaatg 420
attccctatg gttttcactt caaaagaaaa atgacaccac tgaaatagaa actttactct 480
taaatacagc accaaaaatt attgatgagc aactggtgtg tcgtttatcg aaaacggata 540
ttttcattat atgtcgagat aataaaattt atctagataa aatgataaca agaaacttga 600
aactaaggtt ttatggccac cgtcagtatt tggaatgtga agtttttcga gttgaaggaa 660
ttaaggataa cctagacgac ataaagagga taattaaagc cagagagcac agaaataggc 720
ttctagcaga catcagagac tataggccct atgcagactt ggtttcagaa attcgtattc 780
ttttggtggg tccagttggg tctggaaagt ccagtttttt caattcagtc aagtctattt 840
ttcatggcca tgtgactggc caagccgtag tggggtctga tatcaccagc ataaccgagc 900
ggtataggat atattctgtt aaagatggaa aaaatggaaa atctctgcca tttatgttgt 960
gtgacactat ggggctagat ggggcagaag gagcaggact gtgcatggat gacattcccc 1020
acatcttaaa aggttgtatg ccagacagat atcagtttaa ttcccgtaaa ccaattacac 1080
ctgagcattc tacttttatc acctctccat ctctgaagga caggattcac tgtgtggctt 1140
atgtcttaga catcaactct attgacaatc tctactctaa aatgttggca aaagtgaagc 1200
aagttcacaa agaagtatta aactgtggta tagcatatgt ggccttgctt actaaagtgg 1260
atgattgcag tgaggttctt caagacaact ttttaaacat gagtagatct atgacttctc 1320
aaagccgggt catgaatgtc cataaaatgc taggcattcc tatttccaat attttgatgg 1380
ttggaaacta tgcttcagat ttggaactgg accccatgaa ggatattctc atcctctctg 1440
cactgaggca gatgctgcgg gctgcagatg attttttaga agatttgcct cttgaggaaa 1500
ctggtgcaat tgagagagcg ttacagccct gcatttgaga taagttgcct tgattctgac 1560
atttggccca gcctgtactg gtgtgccgca atgagagtca atctctattg acagcctgct 1620
tcagattttg cttttgttcg ttttgccttc tgtccttgga acagtcatat ctcaagttca 1680
aaggccaaaa cctgagaagc ggtgggctaa gataggtcct actgcaaacc acccctccat 1740
atttccgtac catttacaat tcagtttctg tgacatcttt ttaaaccact ggaggaaaaa 1800
tgagatattc tctaatttat tcttctataa cactctatat agagctatgt gagtactaat 1860
cacattgaat aatagttata aaattattgt atagacatct gcttcttaaa cagattgtga 1920
gttctttgag aaacagcgtg gattttactt atctgtgtat tcacagagct tagcacagtg 1980
cctggtaatg agcaagcata cttgccatta cttttccttc ccactctctc caacatcaca 2040
ttcactttaa atttttctgt atatagaaag gaaaactagc ctgggcaaca tgatgaaacc 2100
ccatctccac tgcaaaaaaa aaaaaaaaaa ataagaaaga acaaaacaaa ccccacaaaa 2160
attagctggg tatgatggca cgtgcctgta gtcccagtta ctcaggatga ttgattgagc 2220

cttggaggtg gaggctacag tgagctgaga ttgtgccact gtactctagc cagggagaaa 2280

gagtgagatc ctggctcaaa aaaaccaaat aaaacaaaac aaacaaacga aaaacagaaa 2340

ggaagactga aagagaatga aaagctgggg agaggaaata aaaataaaga aggaagagtg 2400

tttcatttat atctgaatga aaatatgaat gactctaagt aattgaatta attaaaatga 2460

gccaactttt ttttaacaat ttacatttta tttctatggg aaaaaataaa tattcctctt 2520

ctaacaaacc catgcttgat tttcattaat tgaattccaa atcatcctag ccatgtgtcc 2580

ttccatttag gttactgggg caaatcagta agaaagttct tatatttatg ctccaaataa 2640

ttctgaagtc ctcttactag ctgtgaaagc tagtactatt aagaaagaaa acaaaattcc 2700

caaaagatag ctttcacttt tttttttcct taaagacttc ctaattctct tctccaaatt 2760

cttagtcttc ttcaaaataa tatgctttgg ttcaatagtt atccacattc tgacagtcta 2820

atttagtttt aatcagaatt atactcatct tttgggtagt catagatatt aagaaagcaa 2880

gagtttctta tgtccagtta tggaatattt cctaaagcaa ggctgcaggt gaagttgtgc 2940

tcaagtgaat gttcaggaga cacaattcag tggaagaaat taagtcttta aaaaagacct 3000

aggaatagga gaaccatgga aattgaggag gtaggcctac aagtagatat tgggaacaaa 3060

attagagagg caaccagaaa aagttatttt aggctcacca gagttgttct tattgcacag 3120

taacacacca atataccaaa acagcaggta ttgcagtaga gaaagagttt aataattgaa 3180

tggcagaaaa atgaggaagg ttgaggaaac ctcaaatcta cctccctgct gagtctaagt 3240

ttaggatttt taagagaaag gcaggtaagg tgctgaaggt ctggagctgc tgatttgttg 3300

gggtataggg aatgaaatga aacatacaga gatgaaaact ggaagttttt ttttgtttgt 3360

tttgtttttt ttttgttgtt gttttttttt ttttttgttt ttttgctgag tcaattcctt 3420

ggagggggtc ttcagactga ctggtgtcag cagacccatg ggattccaag atctggaaaa 3480

ctttttagat agaaacttga tgtttcttaa cgttacatat attatcttat agaaataact 3540

aagggaagtt agtgccttgt gaccacatct atgtgacttt taggcagtaa gaaactataa 3600

ggaaaggagc taacagtcat gctgtaagta gctacaggga attggcttaa agggcaagtt 3660

ggttagtact tagctgtgtt tttattcaaa gtctacattt tatgtagtgg ttaatgtttg 3720

ctgttcatta ggatggtttc acagttacca tacaaatgta gaagcaacag gtccaaaaag 3780

tagggcatga ttttctccat gtaatccagg gagaaaacaa gccatgacca ttgttggttg 3840

ggagactgaa ggtgattgaa ggttcaccat catcctcacc aacttttggg ccataattca 3900

cccaaccctt tggtggagcc tgaaaaaaat ctgggcagaa tgtaggactt ctttattttg 3960

tttaaagggg taacacagag tgcccttatg aaggagttgg agatcctgca aggaagagaa 4020

ggagtgaagg agagatcaag agagagaaac aatgaggaac atttcatttg acccaacatc 4080

ctttaggagc ataaatgttg acactaagtt atcccttttg tgctaaaatg gacagtattg 4140

gcaaaatgat accacaactt cttattctct ggctctatat tgctttggaa acacttaaac 4200

atcaaatgga gttaaataca tatttgaaat ttaggttagg aaatattggt gaggaggcct 4260

caaaaagggg gaaacatctt ttgtctggga ggatattttc cattttgtgg atttccctga 4320

tctttttcta ccaccctgag gggtggtggg aattatcatt ttgctacatt ttagaggtca 4380

tccaggattt ttgaaacttt acattcttta cggttaagca agatgtacag ctcagtcaaa 4440

gacactaaat tcttcttaga aaaatagtgc taaggagtat agcagatgac ctatatgtgt 4500

gttggctggg agaatatcat cttaaagtga gagtgatgtt gtggagacag ttgaaatgtc 4560

aatgctagag cctctgtggt gtgaatgggc acgttaggtt gttgcattag aaagtgactg 4620

tttctgacag aaatttgtag ctttgtgcaa actcacccac catctacctc aataaaatat 4680

agagaaaaga aaaatagagc agtttgagtt ctatgaggta tgcaggccca gagagacata 4740

agtatgttcc tttagtcttg cttcctgtgt gccacactgc ccctccacaa ccatagctgg 4800

gggcaattgt ttaaagtcat tttgttcccg actagctgcc ttgcacatta tcttcatttt 4860

cctggaattt gatacagaga gcaatttata gccaattgat agcttatgct gtttcaatgt 4920

aaattcgtgg taaataactt aggaactgcc tcttcttttt ctttgaaaac ctacttataa 4980

ctgttgctaa taagaatgtg tattgttcag gacaacttgt ctccatacag ttgggttgta 5040

accctcatgc ttggcccaaa taaactctct acttatatca gtttttccta cacttcttcc 5100

ttttaggtca acaataccaa gaggggttac tgtgctgggt aatgtgtaaa cttgtgtctt 5160

gtttagaaag ataaatttaa agactatcac attgcttttt cataaaacaa gacaggtcta 5220

caattaattt attttgacgc aaattgatag gggggccaag taagccccat atgcttaatg 5280

atcagctgat gaataatcat ctcctagcaa cataactcaa tctaatgcta aggtacccac 5340

aagatggcaa ggctgatcaa agtcgtcatg gaatcctgca accaaaagcc atgggaattt 5400

ggaagccctc aaatcccatt cctaatctga tgagtctatg gaccaatttg tggaggacag 5460

tagattaaat agatctgatt tttgccatca atgtaaggag gataaaaact tgcataccaa 5520

ttgtacaccc ttgcaaaatc tttctctgat gttggagaaa atgggccagt gagatcatgg 5580

atatagaagt acagtcaatg ttcagctgta ccctcccaca atcccacttc cttcctcaac 5640

acaattcaaa caaatagact cagactgttt caggctccag gacaggaagt gcagtgtagg 5700

caaaattgca aaaattgagg gcacaggggt ggaggtgggg gggttgaata acaagctgtg 5760

ctaaataatt acgtgtaaat atattttttc atttttaaaa attgatttct tttgcacatt 5820

ccatgacaat atatgtcaca tttttaaaat aaatgcaaag aagcatacat ccaaaaaaaa 5880

aaaaaaaaa 5889

<210>  133
<211>  50
<212>  DNA
<213>  homo sapiens

<400>  133
gtgagtggct aatgagaaga gggacagacc gaccaccagc acagtagggc          50


<210>  134
<211>  9285
<212>  DNA
<213>  homo sapiens

<400>  134
actgtctcca agatggcggc cgtgtcagtt tggggcatct ccgcggtccg gcccggggcc      60

ccgggatctc ggctgtcctt cctcccggat tctttctact aatccagata cttgttgaag     120

tgctgactag tttcttgggg aaagatgtca aggaagagtt gaaattccta gactggtgag     180

cagagattgc ggagtacaga atagccatca cccactctgg cataagatgc acatttttct     240

gctctggagc cgggaatgaa atattcttga gttcttacaa ctttatgacg agacccatgt     300

gtggtgctat tgagaaattc attgggaagt tggaagacat ttcaatcaac aggttgtttt     360

ggtttctata gtacaattgg ggtggcattc tgttttgtga aaggaggaag gacttaggcc     420

agaaaactca tatgctatgg ttaactggtt cccagcctcc gagaatcttg ttttccatgg     480

tgtaaaactt actcagcatc aggataaggg ataacgactc tatggatata cagaatcctt     540

caccatggta aaactcgcaa acccgcttta tactgagtgg attttggagg ccatcaaaaa     600

agtgaaaaag cagaaacagc gtccttcaga agaaaggata tgcaatgctg tgtcttcatc     660

ccatggcttg gatcgtaaaa ctgttttaga acaattggag ttgagtgtta aagatggaac     720

aattttaaaa gtctcaaata aaggactcaa ttcctataaa gatcctgata atcctgggcg     780

aatagcactt cctaagcctc ggaaccatgg aaaattggat aataaacaaa atgtggattg     840

gaataaactg ataaagcggg cagttgaggg cttggcagag tctggtggct caactttgaa     900

aagcattgaa cgttttttga aaggtcagaa ggatgtgtct gcattattcg gaggcagtgc     960

tgcctctggc tttcaccagc agttacgatt ggctatcaaa cgtgccattg gccacggcag    1020

actccttaaa gatggacctc tttatcggct caacactaaa gcaaccaacg tggatgggaa    1080

agagagttgt gagtctcttt cctgtttacc tccagtgtcc cttcttccac atgaaaagga    1140

taagccggtt gctgaaccaa tccccatctg tagtttctgt cttggtacaa aagaacaaaa    1200

ccgagaaaag aagccagagg aactcatctc ctgtgccgac tgtggcaaca gtggccatcc    1260

atcctgttta aagttttccc ctgaactaac ggttcgagtg aaggccttac ggtggcagtg    1320

catcgagtgt aaaacatgca gctcctgtcg agatcaaggc aaaaatgcgg ataacatgct    1380

cttttgtgat tcatgtgacc gaggttttca catggagtgt tgtgatccgc cactcacccg    1440

tatgccaaaa ggcatgtgga tatgtcaaat atgtcgacct aggaaaaaag gacgaaaact 1500

tctacaaaag aaggcagcac agataaaacg gcgctatact aatccaatag gacgtccaaa 1560

aaacaggtta aagaaacaaa acacggtatc aaaaggtccc ttcagcaaag ttcgaactgg 1620

ccctggaagg ggtaggaaac gaaaaatcac tctttccagc caatcagcat catcatcatc 1680

agaagaagga tatttagagc ggatagatgg cttggacttc tgcagagata gcaatgtctc 1740

cttgaagttc aacaagaaaa ccaaagggct cattgatggc cttaccaaat tttttacccc 1800

ttcccctgat gggcggaaag ctcgggggga agtggtggac tactctgagc aatatcgaat 1860

cagaaagagg ggcaacagga aatcaagcac ttcagattgg cccacagaca atcaggatgg 1920

ctgggatggc aaacaagaaa atgaggagcg actttttggg agccaggaaa tcatgactga 1980

gaaagatatg gaattatttc gtgatatcca agaacaagca ctgcagaaag ttggagtgac 2040

tggtccccct gatccacaag tccgctgtcc ctctgtcatt gagtttggga agtatgaaat 2100

tcacacctgg tactcctccc catatcctca agaatactca aggctgccca aattgtatct 2160

ttgtgaattt tgtctaaaat atatgaaaag tagaactatt ctgcagcagc acatgaagaa 2220

atgtggttgg ttccatcctc ctgccaatga gatttacaga aagaataata tttctgtctt 2280

tgaggttgat gggaatgtga gtaccattta ttgtcaaaac ctgtgtcttt tggcaaagtt 2340

gtttcttgac cacaaaaccc tctattacga tgtggagcca tttctttttt atgtactaac 2400

acagaatgat gtcaagggct gccaccttgt tggctacttt tctaaggaaa agcactgcca 2460

acagaagtac aatgtttcct gtataatgat tcttcctcaa taccagcgta agggctatgg 2520

caggtttctc atcgatttca gttatttgtt atcaaagcgt gaaggccaag cagggtctcc 2580

agagaaaccg ttatctgatc tgggtcgtct ttcctacatg gcatattgga aaagtgtaat 2640

attggagtgc ctttatcacc aaaatgacaa gcagatcagc attaagaagt taagcaagtt 2700

gactggaatc tgccctcaag acatcacttc cacactccac cacctacgaa tgctggactt 2760

ccgtagtgac caatttgtga ttatccgccg ggaaaaactt atccaggatc acatggcaaa 2820

gcttcagctg aatttgcgac ctgtagatgt agatccagaa tgtttgcgct ggactccagt 2880

catagtgtcc aactctgtgg tctcagagga ggaagaagag gaggctgagg aaggagaaaa 2940

cgaagagcca cagtgccagg aaagagaatt agagatcagt gtgggaaagt ctgtgtctca 3000

tgagaacaaa gaacaagatt cttattcagt agaaagtgaa aagaaaccag aagttatggc 3060

tccagtcagt tctacacgtt tgagcaaaca agtccttcct catgatagtc ttcctgcaaa 3120

tagccagcca tctcggaggg gccgctgggg gaggaagaac agaaaaaccc aggaacgttt 3180

tggtgataaa gattctaaac tgctcttgga agagacgtct tcagctcctc aggaacaata 3240

tggagaatgt ggggagaaat cagaagccac ccaggaacaa tacactgaaa gtgaagaaca 3300

gctggtggct tctgaggagc agccaagcca ggacgggaaa cctgaccttc ccaagagaag 3360

actcagtgag ggggttgagc cctggcgagg acagctcaag aaaagccctg aggctctgaa 3420
gtgcagatta acagaaggaa gtgagaggct gccccgtcgc tacagtgagg gtgacagggc 3480
tgtcctcagg ggcttcagtg agagcagcga ggaggaggag gagccggaaa gccctcggtc 3540
aagctcgcca ccaattctca caaagcccac gctgaagcga aagaaaccat ttctccaccg 3600
aaggaggaga gtccgaaagc gcaaacacca caatagcagt gtagtcacag aaactatttc 3660
tgagaccact gaagtgttag atgaaccttt tgaagattct gactccgaga ggccaatgcc 3720
aagattagaa cccacgtttg agatcgatga agaagaggag gaagaggatg aaaatgaact 3780
tttccctaga gaatacttcc gtcgtttgtc ttcgcaggat gtactcaggt gtcagtcctc 3840
ttctaagagg aagtctaaag atgaagaaga agatgaagag tcagatgatg ctgatgacac 3900
tcctatctta aagccagtat ctcttttgcg aaaacgtgat gtgaagaatt ctcctcttga 3960
gccagataca tccacacctt tgaaaaagaa aaaggatggg cccaaaggca agagccgcaa 4020
accaatccac tggaagaaaa gacctggtcg aaaaccagga tttaagttga gtcgggaaat 4080
catgccagtt tctactcaag catgcgtcat tgagcccatc gtttccattc ctaaagctgg 4140
acgtaaaccc aagatccagg agagtgaaga aactgttgag ccaaaagaag acatgcccct 4200
acccgaggag aggaaggagg aggaggagat gcaagcagag gcagaagagg ctgaagaggg 4260
tgaggaagag gatgcagcca gcagtgaagt cccagcagcc tctccagcag acagcagcaa 4320
tagtcctgag accgaaacca aggagcctga ggtggaggag gaagaagaga agccccgtgt 4380
ctcagaggag cagaggcagt cagaggagga gcagcaggaa ttagaggagc cagagccaga 4440
ggaggaggaa gatgcagctg cagagactgc ccagaatgac gaccacgacg ctgatgatga 4500
ggatgatggc cacctggagt ccacaaagaa aaaggagcta gaggaacagc ccacgaggga 4560
agatgtcaag gaggagcctg gtgttcaaga gtctttttta gatgctaata tgcagaagag 4620
tagggaaaag ataaaggata aagaggaaac cgagctggat tccgaagagg agcagccttc 4680
ccatgacacg tccgtggtgt cagagcagat ggctgggtct gaggacgacc acgaagaaga 4740
ctcccacact aaggaagagt taatcgaatt aaaagaggag gaagagattc ctcatagtga 4800
gctggatctg gaaactgtac aggcagtgca gtctttgact caagaagaaa gcagtgagca 4860
tgagggcgcc taccaggact gtgaggaaac tcttgcggcg tgtcagaccc tgcagagtta 4920
cacccaggct gacgaggacc ctcagatgtc catggttgaa gactgtcatg cgtcagaaca 4980
taatagccct atctcctccg ttcagtctca ccccagccag tcagtccgtt cggtcagcag 5040
tcccaacgtg cctgcccttg agagtggcta cacccagatc agcccagaac aaggatccct 5100
gtccgcaccc tctatgcaga acatggagac cagccccatg atggatgtgc cttccgtatc 5160
agaccactct cagcaggtgg tggacagcgg cttcagtgac ctgggcagca ttgagagcac 5220

cactgaaaac tatgagaacc caagcagtta cgactccacg atgggcggca gcatctgtgg 5280

gaacagctct tcccagagca gctgctccta cggtgggctg tcgtcctcca gcagcctcac 5340

ccagagcagc tgtgtggtca ctcagcagat ggccagcatg ggcagcagct gcagcatgat 5400

gcagcagagc agcgtccagc ctgctgccaa ctgcagcatc aagtcacctc agagctgcgt 5460

ggtggagagg cctcccagta accagcagca gcagccgcca ccaccgcctc cacagcagcc 5520

acagccgccg ccgccacaac cacaaccagc accacagcct ccaccacccc agcagcagcc 5580

gcaacagcag ccgcagcctc agccccagca gcctccaccc ccaccccctc cccagcagca 5640

gcccccgctg tcacagtgta gtatgaataa cagtttcacc ccagctccta tgatcatgga 5700

gataccagaa tctggaagca ctgggaacat aagtatctat gagaggattc caggggattt 5760

tggtgccggc agctactctc aaccatcagc caccttcagc ctagccaagc tgcagcagct 5820

gaccaacacc attatggacc ctcatgccat gccttatagc cattctcctg ctgtgacttc 5880

ctatgcaacc agtgtttctc tgtccaatac aggactggct cagctggctc catctcatcc 5940

cttagctggg actcctcaag cacaagccac catgacgcca cccccaaact tggcatccac 6000

taccatgaac ctcacatctc ctctgcttca gtgcaacatg tctgccacca acattggcat 6060

tcctcacacg cagagattgc aagggcaaat gccagtgaag ggccacattt ccatccgctc 6120

caagtctgcg ccactgccct ctgcggctgc tcaccagcag cagctgtatg gccgtagccc 6180

atcggcagtt gccatgcagg ctggccctcg cgcactggct gttcagcgtg gcatgaacat 6240

gggggttaat ctgatgccta ctcccgccta taatgtcaat tccatgaata tgaacacctt 6300

gaatgccatg aacagctatc gaatgacaca gcccatgatg aacagcagtt accatagtaa 6360

ccctgcctac atgaaccaga cagcacagta tcctatgcag atgcagatgg gaatgatggg 6420

gagccaggcc tatacccagc agcctatgca gcctaaccct catgggaaca tgatgtacac 6480

aggcccctcc catcacagct acatgaacgc tgctggcgtg cccaagcagt cactcaacgg 6540

accttacatg agaagatgag caagatgaac ttgcaatcaa aaacttaaat atatataaat 6600

aaaggaacct tttatactga caaaccagag aaaaatggac ctttttccag ttaaaatatt 6660

gctgtagatt tagaggaatt tttctttggt ttattttatt ttttagaaaa cctgatcttc 6720

tctttttttg ggttcatttt gttctgggtt ttggttttct tcacaatctt gaacatttta 6780

cagtagaact catctaaaaa tggatttggg gatggggaaa catgcacaaa atcttttcat 6840

aattaaaaag agccttactt tctttacata ccacatggac agaatttgtg taaaagtgaa 6900

ttatctttat tttaaaatgt atgtttcccc tcactgtttg cagctcccaa tgttgtcatt 6960

tttaaatgtt atatacatct caagggttaa ccagaccctt tcctccaaac ccaacctttc 7020

atttcctact tcattccagc aggaggcact taggggagac tcggatgggg acatggagaa 7080

caacccaagc tccttaaact tattattatt gttaatatta ttattattat tattaataaa 7140

gtgaggcagg aaaatgcttc tccttttaaa atcccctcca ctcctcacac acacacacct 7200

cttgaaaccc ttccccaaga atgtttcttt atagacggac ttcattgaaa tctttgttgt 7260

tcttgaatca agtgtaatat aatttttttc ttctttttta aaatattccc actcagcact 7320

cagagacaca aaaatactgt aagtctcaat taacagcaga atctcagaga aaagctgttt 7380

gcaatccaaa tccagccttt ggaggaatag agatggtcaa ttaacaatca aaaagaggag 7440

attaacctct tgttttttta ccacctggtg aatcagccat aacgcacaca cacgccaccc 7500

agcctcttgt ttctagtatg tactttgaaa tgctaactga gggtcttgat gcttgagcct 7560

ttgactgata aaactcaaat agcagtcccc agtgatttgc ctcttaggtt ctttcttaaa 7620

ttgttggtgg atgactgtac attttagtga tttgaaaaat aactgacaaa ccattgaaac 7680

agtttatttt atgttggaag agatggcgca gatgtgtgtc agaagggaga tcacggtgtg 7740

agtttcgtag ctatttaagt gatacatacc tctagttttt gtatgtcttt tgagatcctg 7800

agttcatccc ctgtgaatca gagtgcacaa gcacctctcc tgtgagtggc taatgagaag 7860

agggacagac cgaccaccag cacagtaggg cagatctgga cagcagaatg ttataacgca 7920

agttcatgtg ttgctcccaa ctccattctc ttttctctcg tgcaaccagt ttgcccattc 7980

tcttcctatt acttgctcca gggataggta aaaaaaaaaa aaaaaaaaaa aaaaatatat 8040

atatatatat atacacacac acacacacac acacacacac atatatatat atatatattc 8100

catcattaga agatggaaac tattatacca cttggtatgt gcagtcaaat atccaaaagg 8160

gggaagtact gcttagagaa atatctgtaa gcattaaatt ctctccttta tttgtacatt 8220

ttatatagat aaatttttaa agtactaatt aggcattaac ttttttcaaa tgcacaggtt 8280

tgttggtttt tttaatacac tttaattgac tttctcaagc gtgctaggta gaaacgcaga 8340

attccacatc ttcccgctct cagctctgag catgtgcaag gctgtgtagg acccagtttc 8400

tctgtatata ctattccagt tcccagtcat ctatgtagaa agtgcactgt gctgccatcg 8460

ccctacatct tcagctgtgt cattgcttcc tggttggggt ggggacgggg tgggtcggga 8520

gggaggtata ttgggaggag ggtgggtcaa ggcagaagga gaagctgttg aaatgagggc 8580

cgtgaaatca gtttctgttg gtttgcggtt gtgtttggtt ttgtttgttt ctttataaaa 8640

aattcaatcg ggcagctccc ttttccacaa gcctctttgt gactgtagaa ctattgtaga 8700

aaaaaaaagt tcttttctat attataaaag aaattatcca acacagtaat attggtacct 8760

gtcatttttt cacttctgtt taaaaaaaat gtatttttag caagataact tgggtaatct 8820

tctaaaaaag aaatttaaaa actcactgtt agtgactttg atgcctttta aaataagagc 8880

tttttcattt cattccatct ttaaaatttt ttatctttgt tgtagaatat taaaaactat 8940

tttaagaaag ataaattctc tttaaagaga tctctagcgt gtgtgaatag agctccagat 9000

gcctctaaaa gccgcatgta caaaggaagc cacgtctatc ctgtctgttt atatttgctt 9060

ttcctgtttt gtaacctctt tgtactttgt tcatggtgac ttgtaagcta aggggaaggg 9120

gtgcctagat gcctttgtat ctctccatgt cacgcgctcc tgggccagcc ggcctccctt 9180

cctctccttg tatgtaatac ctttttttcc cctttctagc aagtactttc aaaagaactc 9240

tgtacatttt aacataaaaa ataaattatg ttgagccatt ttgga 9285

<210> 135
<211> 50
<212> DNA
<213> homo sapiens

<400> 135
tgtgctggtg tgtgtgctgg cccgcaactt tggcaaggaa ttcaccccac 50

<210> 136
<211> 774
<212> DNA
<213> homo sapiens

<400> 136
agggcaagtt aagggaatag tggaatgaag gttcattttt cattctcaca aactaatgaa 60

accctgctta tcttaaacca acctgctcac tggagcaggg aggacaggac cagcataaaa 120

ggcagggcag agtcgactgt tgcttacact ttcttctgac ataacagtgt tcactagcaa 180

cctcaaacag acaccatggt gcatctgact cctgaggaga agactgctgt caatgccctg 240

tggggcaaag tgaacgtgga tgcagttggt ggtgaggccc tgggcagatt actggtggtc 300

tacccttgga cccagaggtt ctttgagtcc tttggggatc tgtcctctcc tgatgctgtt 360

atgggcaacc ctaaggtgaa ggctcatggc aagaaggtgc taggtgcctt tagtgatggc 420

ctggctcacc tggacaacct caagggcact ttttctcagc tgagtgagct gcactgtgac 480

aagctgcacg tggatcctga gaacttcagg ctcttgggca atgtgctggt gtgtgtgctg 540

gcccgcaact ttggcaagga attcacccca caaatgcagg ctgcctatca gaaggtggtg 600

gctggtgtgg ctaatgccct ggctcacaag taccattgag atcctggact gtttcctgat 660

aaccataaga agaccctatt tccctagatt ctattttctg aacttgggaa cacaatgcct 720

acttcaaggg tatggcttct gcctaataaa gaatgttcag ctcaacttcc tgat 774

<210> 137
<211> 50
<212> DNA
<213> homo sapiens

<400> 137
aggcccacaa tgcctgtgtc ctcaccatta gtcccgtgca gcctgaagac 50

<210> 138

<211>  774
<212>  DNA
<213>  homo sapiens

<400>  138
agggcaagtt aagggaatag tggaatgaag gttcattttt cattctcaca aactaatgaa      60

accctgctta tcttaaacca acctgctcac tggagcaggg aggacaggac cagcataaaa     120

ggcagggcag agtcgactgt tgcttacact ttcttctgac ataacagtgt tcactagcaa     180

cctcaaacag acaccatggt gcatctgact cctgaggaga agactgctgt caatgccctg     240

tggggcaaag tgaacgtgga tgcagttggt ggtgaggccc tgggcagatt actggtggtc     300

tacccttgga cccagaggtt ctttgagtcc tttggggatc tgtcctctcc tgatgctgtt     360

atgggcaacc ctaaggtgaa ggctcatggc aagaaggtgc taggtgcctt tagtgatggc     420

ctggctcacc tggacaacct caagggcact ttttctcagc tgagtgagct gcactgtgac     480

aagctgcacg tggatcctga gaacttcagg ctcttgggca atgtgctggt gtgtgtgctg     540

gcccgcaact ttggcaagga attcacccca caaatgcagg ctgcctatca gaaggtggtg     600

gctggtgtgg ctaatgccct ggctcacaag taccattgag atcctggact gtttcctgat     660

aaccataaga agaccctatt tccctagatt ctattttctg aacttgggaa cacaatgcct     720

acttcaaggg tatggcttct gcctaataaa gaatgttcag ctcaacttcc tgat           774


<210>  139
<211>  50
<212>  DNA
<213>  homo sapiens

<400>  139
gatgtggctg aagatcagag gctcagttag caacctgtgt tgtagcagtg                 50


<210>  140
<211>  6335
<212>  DNA
<213>  homo sapiens

<400>  140
agtgctgagg gagcaaagtt cattttctcg ggtaggagaa gatgattctc ttgcaacacg      60

tgcggattgt gacaaaatct ttcattaaca aggggagttt cggtgaagtg gaggtttggg     120

gaaaggcgag gaagtcggtc tggagcaagc aagcaaagtg cggaagctgt actgggattc     180

ttctagaaag tggggtggga aaggagctag ggagggcgtg tggagggacg agatctgtgt     240

cagaacgtgc gtgtgagcgg atacaaaacc cgagagaggc gtgagcagcg ctgtgtttgc     300

gagcgggagc gaggggcgcc ggctggggtg tgtgctcctg agctcttcag aaaccaggct     360

gctttcagga acattgctgt ggattcccag ggcctattcc actagaagca agatggctga     420

actcaatact catgtgaatg tcaaggaaaa gatctatgca gttagatcag ttgttcccaa     480

caaaagcaat aatgaaatag tcctggtgct ccaacagttt gattttaatg tggataaagc 540

cgtgcaagcc tttgtggatg gcagtgcaat tcaagttcta aaagaatgga atatgacagg 600

aaaaaagaag aacaataaaa gaaaaagaag caagtccaag cagcatcaag gcaacaaaga 660

tgctaaagac aaggtggaga ggcctgaggc agggcccctg cagccgcagc caccacagat 720

tcaaaacggc cccatgaatg gctgcgagaa ggacagctcg tccacagatt ctgctaacga 780

aaaaccagcc cttatccctc gtgagaaaaa gatctcgata cttgaggaac cttcaaaggc 840

acttcgtggg gtcacagaag gcaacagact actgcaacag aaactatcct tagatggaaa 900

ccccaaacct atacatggaa caacagagag gtcagatggc ctacagtggt cagctgagca 960

gccttgtaac ccaagcaagc ctaaggcaaa aacatctcct gttaagtcca atacccctgc 1020

agctcatctt gaaataaagc cagatgagtt ggcaaagaaa agaggcccaa atattgagaa 1080

atcagtgaag gatttgcaac gctgcaccgt ttctctaact agatatcgcg tcatgattaa 1140

ggaagaagtg gatagttccg tgaagaagat caaagctgcc tttgctgaat tacacaactg 1200

catcattgac aaagaagttt cattaatggc agaaatggat aaagttaaag aagaagccat 1260

ggaaatcctg actgctcgtc agaagaaagc agaagaacta aagagactca ctgaccttgc 1320

cagtcagatg gcagagatgc agctggccga actcagggca gaaattaagc actttgtcag 1380

cgagcgtaaa tatgacgagg agctcgggaa agctgcccgg ttttcctgtg acatcgaaca 1440

gctgaaggcc caaatcatgc tctgcggaga aattacacat ccaaagaaca actattcctc 1500

aagaactccc tgcagctccc tgctgcctct gctgaatgcg cacgcagcaa cctctgggaa 1560

acagagtaac ttttcccgaa aatcatccac tcacaataag ccctctgaag gcaaagcggc 1620

aaaccccaaa atggtgagca gtctccccag caccgccgac ccctctcacc agaccatgcc 1680

ggccaacaag cagaatggat cttctaacca aagacggaga tttaatccac agtatcataa 1740

caacaggcta aatgggcctg ccaagtcgca gggcagtggg aatgaagccg agccactggg 1800

aaagggcaac agccgccacg aacacagaag acagccgcac aacggcttcc ggcccaaaaa 1860

caaaggcggt gccaaaaatc aagaggcttc cttggggatg aagacccccg aggccccggc 1920

ccattctgaa aagccccggc gaaggcagca cgctgcagac acctcggagg ccaggccctt 1980

ccggggtagt gtcggtaggg tttcacagtg caatctctgc cccacgagaa tagaagtttc 2040

cacagatgca gcagttctct cagtcccggc tgtgacgttg gtggcctgag ctaggaggaa 2100

aaagagcagt tttcactcag ttttggttcc ctgcccgagg tgctgaccca attcgctgcc 2160

aaaagagtgt caatcagaat atacaaatcc cgtatggttg tgtcatcctc tcttaatcat 2220

ttttactaat tctaataatc agctctagct tgcttcataa ttttcatggc tttgcttgat 2280

ctgttgatgc tttctctcat caagactttg cagcatttta gccaggcagt atttactcat 2340

tattaggaaa atcaagatgt ggctgaagat cagaggctca gttagcaacc tgtgttgtag 2400

cagtgatgtc agtccattga ttgtctttag agagttaatg ttacaaaaaa gaattcttaa 2460

taatcagaca aacatgatct gctgaggaca catgcgcttt tgtagaattt aacatctggt 2520

gtttttctga aaaaatatat atacatatat tgctttattt gaaacaaatt aaaatatgct 2580

gcatttgaca cctggctagt ttcttttatt gatacccacc tagttattga atgtactgtt 2640

tagtgctttc aaaaaaaact ttagagacta gaggttgtgg tgcaaagctg tgtacaataa 2700

atactgtttc tgttgagaca agtactcttt caggaaaata tatatatgcc ctttcaatta 2760

gattacacaa atagatggat atgcaccctg atcatcttag acacaccatg tggcagttgg 2820

gcagttggaa acattggttg caagtactac aaacctcagc tgagcctagc ttcaacaata 2880

agaatttatt ggttcagtga gtgaaaagtc cagttggtcc aatttgatca ggatttcagc 2940

tcccatctct tttttttttt tcttttttct ttttcttttt ttgagacagt cttgctttgt 3000

cacccaggtg gagtgcaatg gcatgatctc ggctcactgc aacctccgcc tcctgggttc 3060

aagccattct cctgcccggc cttccgagta gctgggatta caggcatgtg ccaccacgcc 3120

ctgctaattt ttatattatt agtacagaca ggatttcacc atgttgtcca ggctggtctc 3180

aaacccctga cctcaggtga tccacctgct ttggcctccg aaactgccgg aattacaggc 3240

atgagccacc gtacccggcc agctcccatc tcttaattat cttagctctc ctttcctcct 3300

tgggttgaca ttgcattcag aattgtggca aagggccacc atgctcttaa gactcaagtc 3360

tattttccac actgtccaga ggagagaagt tatcctagta gcttctacac agagcaagtg 3420

tctttctcag aaatcccagc aaaggtcttg cataccattg ctggtaggcc tgtctcttaa 3480

accaatcatg aaagggggga gagaagtgaa tgggatgaac taatttgcat agactaatta 3540

gagcccaccc ctggagcctg ggtgtggcca tcttcccaga gttcctgagc tagatggaga 3600

aggggtactt ctcagaaagg gaaaatggat acccaatggc ccaaacccaa aatagcccca 3660

gttcccctaa ctttgactac agggcagtcc agtttgggtg ccgcttccgt tgcactcaca 3720

tgtcctacat atctgttgac tactcacaag tgcaaatgct tattctcaac tcaacattaa 3780

cattttttct ggcaacccag gttcactggt tctcttccac agagcggccc tgagcagctg 3840

agcctgcaag ccacgcaagc atctgtttct tcttttgcca agtacaggag gatgtttgct 3900

ctctctgtag agagctttct gaggtctctg ggtgtaccca gagatttaat agaaattctt 3960

aacgttaagt cacattccag gaaggaagga agagttgttc gttcaaataa gaaagataaa 4020

tgttcggcac tgtaggccct gtttacccca tctgaggccc tgaattcata tattacaaga 4080

cggaaggatt ttgcacagtt ttttatgtag caagattttg ctcaccactg aaaaatgtca 4140

gtgtaaatgt gaccgcttta aagatgagtc aagtaattct tggaacaggg aaaaaaatga 4200

atttgccagg tcaggagttc acctgccttt gtcagagttg aacccaacca ctcttgacct 4260

cgactcactc ccttagggtt aagaaagccc aaacacattc ctgagcacag agcaaacact 4320

cccatgtcac tgaaaagaaa caaaagaatg ctaaaaagtg ctcagacctg atcacatttt 4380

ttccaatatt ttttcctttt tttttttttt tttgagacag ggtcttgctc tatcacctag 4440

gctggagtgc atgtttttga gacagggtct tgctctatca cctaggctgg agtgcatgat 4500

catggctcac tatagccttg aactcctggg ctcaagccat cctcagcctc ccaagttgcg 4560

aggactacag gtgtgcacca caacgtccca gctacttttt aaatttttag tagaaacgag 4620

gtctcactat ggtgcccaga ctggtctcga actccggagc tcaagtgatc ttcctgcttt 4680

ggcctcccaa agtgttagaa ttacaggcat gagccacctc gcctggctgg tttttgcctt 4740

tcttatagac cctgggcatg taagcattta ttagtttgca tttttgaaac agtaatttca 4800

atattttagt gccaatgtca ggccgcttaa acactgtatt acatatcttc atctgtctgg 4860

tggaactatt ggtgtgatcc tagagaactg agtcctattc tgccattcat ttaaagtgtt 4920

ttaaactcta atctctctac ttaatgcaca gtagtcagat tattctctta aacatttgcc 4980

tagtagaggt taaaatagtt taatccttat gaagatggaa taacttcaaa ctcacattgt 5040

ggcacttaga tcttccacca agacttcatc cgtgaaatcc acacctccct gttgggttcc 5100

caattacatt ccaaatttac atttcttttg agaatctctg catactccag ctctgtcctg 5160

ttgatcctat tctagaagtg cttaatgcag caagacacag aaagttaaac gcaaattgct 5220

gcaaaattca ccctcagtgg aggactagaa acacaacatg tccaatttaa agctcagttc 5280

acaagcagtt caattctgct ggcatcagaa aaggagattc taattaaaca ttcttaggga 5340

aggacatcaa atgaggttaa tgggaaacgt taccagatta aaagcagttt tttgacaaag 5400

taacagattt ggaaattctg actctctgaa agccttgatt tgaacctcaa acttgatttc 5460

accatgagaa gtggggatca agggcctgcg cagttctttt cctaaatcga ttcggtgctc 5520

cccaccccga cgcaggcaca ggtccgcaac cagataggga gatgcctgaa tttcaggcta 5580

cctttgacaa agctttcttc ctccctccct ccctttgcac gctgcatccc acgctgcctg 5640

cttaaagcgc cctcatgtgt ctacagatgg taaaacgttt atttctcaac agacattcca 5700

gtgatagcat ccaatgacct atgtaacggc accctttttt gctgtacgcg ttttttgaga 5760

tggagtctcg ctctgtcgcc caggctggag tgcagtggcg cggtctcggc tcactgcaag 5820

ctccgcctcc cgggttcacg ccattctcct gcctcagcct cccgagtagc tgggactaca 5880

ggcacccgcc accacacctg gctaattttt tgtattttta gtagatacag ggtttcaccg 5940

tgttagccag gatggttttg atctcctgac ctcgtgatcc acccccctcg gcctcccaaa 6000

gtgctgggat tacaggggtg agccactgcg cccggcccca gtcacttgtt cttaagtttc 6060

ttaagcaaac tataaaatag caaaagacca aaaaaaaagg aaaaaaagca gttcgcctaa 6120

tacattgttc cagcatttcc ttgaaagtac tgagccatct caattgctct gattttgtga 6180

gaaaattatg aagagttgca aagtcccagt gattctcttg ttacttagct aagaatttga 6240

aatgtaattt aaatactatt ctttacaatc cattataagg attttaaaat ctttttgctt 6300

ctttaataaa ttctaacaaa gaaaaaaaaa aaaaa 6335

<210> 141
<211> 50
<212> DNA
<213> homo sapiens

<400> 141
agctgggtgt ggtggcatgc gcatgtagtc ccagctactc agaaggatga 50

<210> 142
<211> 1533
<212> DNA
<213> homo sapiens

<400> 142
tctgattggt gacgggtgag gcggcccgaa atcgtaggac ttccgaaagc agcggcggcg 60

tttgcttcac tgcttggaag tgtgagtgcg cgaagatgcg aaaggtggtt ttgatcaccg 120

gggctagcag tggcattggc ctggccctct gcaagcggct gctggcggaa gatgatgagc 180

ttcatctgtg tttggcgtgc aggaacatga gcaaggcaga agctgtctgt gctgctctgc 240

tggcctctca ccccactgct gaggtcacca ttgtccaggt ggatgtcagc aacctgcagt 300

cggtcttccg ggcctccaag gaacttaagc aaaggtttca gagattagac tgtatatatc 360

taaatgctgg gatcatgcct aatccacaac taaatatcaa agcacttttc tttggcctct 420

tttcaagaaa agtgattcat atgttctcca cagctgaagg cctgctgacc cagggtgata 480

agatcactgc tgatggactt caggaggtgt ttgagaccaa tgtctttggc cattttatcc 540

tgattcggga actggagcct ctcctctgtc acagtgacaa tccatctcag ctcatctgga 600

catcatctcg cagtgcaagg aaatctaatt tcagcctcga ggacttccag cacagcaaag 660

gcaaggaacc ctacagctct tccaaatatg ccactgacct tttgagtgtg gctttgaaca 720

ggaacttcaa ccagcagggt ctctattcca atgtggcctg tccaggtaca gcattgacca 780

atttgacata tggaattctg cctccgttta tatggacgct gttgatgccg gcaatattgc 840

tacttcgctt ttttgcaaat gcattcactt tgacaccata taatggaaca gaagctctgg 900

tatggctttt ccaccaaaag cctgaatctc tcaatcctct gatcaaatat ctgagtgcca 960

ccactggctt tggaagaaat tacattatga cccagaagat ggacctagat gaagacactg 1020

ctgaaaaatt ttatcaaaag ttactggaac tggaaaagca cattagggtc actattcaaa 1080

aaacagataa tcaggccagg ctcagtggct catgcctata attccagcac tttgggaggc 1140

caaggcagaa ggatcacttg agaccaggag ttcaagacca gcctgagaaa catagtgagc 1200

ccttgtctct acaaaaagaa ataaaaataa tagctgggtg tggtggcatg cgcatgtagt 1260

cccagctact cagaaggatg aggtgggagg atctcttgag gctgggaggc agaggttgca 1320

gtgagctgag attgtgccac tgcactccag cctgggtgac agcgagaccc tgtctcaaaa 1380

tatgtatata tttaatatat atataaaacc agagctgaca atgacactct ggaacattgc 1440

ataccttctg tacattctgg ggtacatgga tttctactga gttggataat atgcatttgt 1500

aataaactat gaactatgaa aaaaaaaaaa aaa 1533

<210> 143
<211> 50
<212> DNA
<213> homo sapiens

<400> 143
tccggctcct tctatcccac ggagcaaatc ctaacctgct gtggagtggc 50

<210> 144
<211> 3251
<212> DNA
<213> homo sapiens

<400> 144
gtaggcgcag ggctggcaag cagtcgggac gggagcgcgg gcgtccgcgg tggctgcagt 60

cccgtcggtc tccctgctgt ccggcgcgag ctcttcgagt cttgcttggg atgtttcagc 120

agcccctgag aaggaagagg aggaagctga gggcccgctg agggcgcagg acctgaggga 180

gtcctacatc cagctcgtcc agggtgtgca ggagtggcag gatggttgca tgtaccaggg 240

ggagtttggg ttgaacatga agcttggata tggcaaattc tcttggccca caggcgagtc 300

ataccatggg cagttttacc gggaccactg ccatggcctg ggtacctaca tgtggccaga 360

tggctccagt ttcacgggca cattttacct cagccaccga gaaggctacg gcaccatgta 420

catgaagaca cggcttttcc aggggctata caaagcggac cagcggtttg ggccaggtgt 480

cgagacctac cccgatggca gccaggacgt ggggctgtgg ttccgagagc agctcatcaa 540

gctgtgcacc cagatcccca gtggcttctc cctcctcaga taccctgagt tctccagctt 600

catcacccac agccctgcca ggatcagcct ctcagaagag gagaaaacgg agtggggact 660

gcaggaggga caggatccct ttttctatga ctataagcgg tttcttctga atgacaacct 720

aacgctgcct ccagaaatgt atgtctactc gaccaacagt gaccacctgc ccatgacaag 780

ctctttccgc aaagagctgg acgcccgcat cttcctcaat gaaattcctc cgttcgttga 840

ggatggagaa ccatggttca taatcaatga gacccctttg ttggtcaaaa tccagaagca 900

aacttacaag ttcaggaaca agccagctca caccagctgg aacatgggcg ccatcctgga 960

ggggaagcgc agtggctttg caccctgtgg gcccaaagag caactttcca tggagatgat 1020

cctaaaggct gaggaaggga accacgaatg gatttgtagg atcctgaagg acaactttgc 1080

tagtgctgac gtggcggacg caaagggcta cactgtgctt gctgcggctg ctactcactg 1140

ccacaacgac attgtcaacc ttctcctgga ctgtggggcc gacgtgaaca agtgctcaga 1200

tgagggtctc acggcactca gcatgtgttt cctcctccac taccccgccc agtccttcaa 1260

gcccaatgtt gctgaacgga ccatacctga gccccaggaa cctccaaaat tcccagttgt 1320

tccaatcctt tcatcatcat ttatggacac aaacctggag tctctgtact atgaggtgaa 1380

cgtgccttcc cagggtagct atgagctgag gccaccgcca gcaccactgc tcctgccacg 1440

cgtctcaggc agccacgagg gcggccactt ccaggacacc gggcagtgtg gggggtccat 1500

agaccacagg agcagctctc tgaaggggga ctccccgttg gtgaagggca gccttggcca 1560

tgtggaaagc gggcttgagg acgtgttggg aaacacagac cggggcagtc tgtgcagtgc 1620

tgagacgaaa tttgagtcca acgtgtgtgt gtgcgacttc tccatcgagc tctcgcaggc 1680

catgctggag agaagcgccc agtcccacag cttgctgaag atggcctcgc cctcaccgtg 1740

caccagcagc ttcgacaaag ggaccatgcg gaggatggcg ctgtccatga tcgagcggag 1800

gaagcgctgg cggaccatca agctgctgct gcgccggggc gcggacccca acctgtgctg 1860

cgtgcccatg caggtcctgt tccttgctgt gaaggccggg gacgtggatg gggtgaggct 1920

gctgctggag cacggggcga ggaccgacat ctgctttccg ccgcagctga gcaccctgac 1980

accactccac atcgctgccg cccttcctgg ggaggagggg gtacagattg tggagctgct 2040

gttgcatgcc atcaccgatg tggacgccaa ggcatccgac gaggacgaca cttacaagcc 2100

cggcaagctg gacctgctgc cctcaagtct gaagctcagc aatgagccag gccctcccca 2160

agcctactac agcacggaca cagccctccc ggaggagggg ggcaggacgg ctctgcacat 2220

ggcctgcgag cgggaggatg acaacaagtg tgccagggac atagtccggc tccttctatc 2280

ccacggagca aatcctaacc tgctgtggag tggccactcc ccgctctccc tgtccattgc 2340

cagtgggaat gagctggttg tgaaggagct cctgacccag ggagctgacc ccaacctgcc 2400

cctgaccaaa ggcttgggca gtgccctgtg tgttgcctgt gacctgacct acgagcacca 2460

gaggaacatg gacagcaagc tggccctgat tgaccgactc atcagtcacg gggccgacat 2520

cctgaagcct gtaatgctca ggcagggaga aaaggaggca gtgggcacag ccgtggacta 2580

tggctacttc agattcttcc aggaccggag gattgcccgc tgccccttcc acacgctgat 2640

gccagcagag cgcgagacgt tcctggcgcg gaagcggctc ctggagtaca tgggcttgca 2700

gctacggcag gctgtctttg ccaaggagag ccagtgggac cccacgtggc tgtacctgtg 2760

caagagagcg gagctgatcc ccagccacag gatgaagaag aagggcccca gcctgcccag 2820

gggcctggat gtgaaggagc aggggcaaat tcccttcttc aagttctgct accagtgtgg 2880

ccgctccatc ggggtccgcc tcttgccctg ccctcgctgc tacgggatcc tgacctgcag 2940

caagtactgc aagaccaagg cctggaccga gttccacaag aaggactgcg gggacctggt 3000

ggccatcgtg acacaactgg agcaagtttc caggaggaga gaagaattcc agtgaagcag 3060

cagctgcacg tccgaggctt ggggaggacc caggactgtg tgggtttctt acctgcctga 3120

gccacctcag ggaatcttcc agcctaatgc aggcatttct gcacctttgg ggtcatgctt 3180

tgtagcagtg tctcccttgc gacctcgcaa taaattggcc ccacggggtg attttgacag 3240

tcaaaaaaaa a 3251

<210> 145
<211> 50
<212> DNA
<213> homo sapiens

<400> 145
ttctgtctca tcaaaaactg aagttagctg ggtgtggtgg ctcatgcctg 50

<210> 146
<211> 2247
<212> DNA
<213> homo sapiens

<400> 146
taaatgtgcc acgtcttcta agaaggggga gtcctgaact tgtctgaagc ccttgtccgt 60

aagccttgaa ctacgttctt aaatctatga agtcgaggga cctttcgctg cttttgtagg 120

gacttctttc cttgcttcag caacatgagg cttttcttgt ggaacgcggt cttgactctg 180

ttcgtcactt ctttgattgg ggctttgatc cctgaaccag aagtgaaaat tgaagttctc 240

cagaagccat tcatctgcca tcgcaagacc aaaggagggg atttgatgtt ggtccactat 300

gaaggctact tagaaaagga cggctcctta tttcactcca ctcacaaaca taacaatggt 360

cagcccattt ggtttaccct gggcatcctg gaggctctca aaggttggga ccagggcttg 420

aaaggaatgt gtgtaggaga gaagagaaag ctcatcattc ctcctgctct gggctatgga 480

aaagaaggaa aaggtaaaat tcccccagaa agtacactga tatttaatat tgatctcctg 540

gagattcgaa atggaccaag atcccatgaa tcattccaag aaatggatct taatgatgac 600

tggaaactct ctaaagatga ggttaaagca tatttaaaga aggagtttga aaaacatggt 660

gcggtggtga atgaaagtca tcatgatgct ttggtggagg atatttttga taaagaagat 720

gaagacaaag atgggtttat atctgccaga gaatttacat ataaacacga tgagttatag 780

agatacatct acccttttaa tatagcactc atctttcaag agagggcagt catctttaaa 840

gaacatttta tttttataca atgttctttc ttgctttgtt ttttattttt atatattttt 900

tctgactcct atttaaagaa ccccttaggt ttctaagtac ccatttcttt ctgataagtt 960

attgggaaga aaaagctaat tggtctttga atagaagact tctggacaat ttttcacttt 1020

cacagatatg aagctttgtt ttactttctc acttataaat ttaaaatgtt gcaactggga 1080

atataccacg acatgagacc aggttatagc acaaattagc accctatatt tctgcttccc 1140

tctattttct ccaagttaga ggtcaacatt tgaaaagcct tttgcaatag cccaaggctt 1200

gctattttca tgttataatg aaatagttta tgtgtaactg gctctgagtc tctgcttgag 1260

gaccagagga aaatggttgt tggacctgac ttgttaatgg ctactgcttt actaaggaga 1320

tgtgcaatgc tgaagttaga aacaaggtta atagccaggc atggtggctc atgcctgtaa 1380

tcccagcact ttgggaggct gaggcgggcg gatcacctga ggttgggagt tcgagaccag 1440

cctgaccaac acggagaaac cctatctcta ctaaaaatac aaaagtagcc gggcgtggtg 1500

atgcgtgcct gtaatcccag ctacccagga aggctgaggc ggcagaatca cttgaacccg 1560

gaggcggagg ttgcggtaag ccgagatcac ctccagcctg gacactctgt ctcgaaaaaa 1620

agaaaagaaa cacggttaat aacatataaa tatgtatgca ttgagacatg ctacctagga 1680

cttaagctga tgaagcttgg ctcctagtga ttggtggcct attatgataa ataggacaaa 1740

tcatttatgt gtgagtttct ttgtaataaa atgtatcaat atgttataga tgaggtagaa 1800

agttatattt atattcaata tttacttctt aaggctagcg gaatatcctt cctggttctt 1860

taatgggtag tctatagtat attatactac aataacattg tatcataaga taaagtagta 1920

aaccagtcta cattttccca tttctgtctc atcaaaaact gaagttagct gggtgtggtg 1980

gctcatgcct gtaatcccag cactttgggg gccaaggagg gtggatcact tgagatcagg 2040

agttcaagac cagcctggcc aacatggtga aaccttgtct ctactaaaaa tacaaaaatt 2100

agccaggcgt ggtggtgcac acctgtagtc ccagctactc gggaggctga gacaggagat 2160

ttgcttgaac ccgggaggcg gaggttgcag tgagccaaga ttgtgccact gcactccagc 2220

ctgggtgaca gagcaagact ccatctc 2247

<210> 147
<211> 50
<212> DNA
<213> homo sapiens

<400> 147
gactatggaa cagactatgg aaccagacta ctggaccacg ggccacaggg 50

<210> 148
<211> 1489
<212> DNA
<213> homo sapiens

<400> 148
atggttgctg ctgccctgag gaggatgagg gggcccgcac aagcaaaact gctgcccaag 60

tcggccatcc aagcctttgc ggggctggtg cggccgctgg tcttggcgct cctccttgtg 120

tccgccactc tatccagtgt tgtatcatgg actgattcac tgattcaaac tgtactcaac 180

tcagatattt ctaccccaaa tgtgaatgct ttaacacatg aaaaacaaac caaatcttct 240

atttcccaaa tcagtaccac cctccctccc acaatgaata ctgagaaaag tggaggagca 300

tctgtggccc ctcatccctc acctacacct ctgtctcaag aggaagctga taacaatgaa 360

gatcctagca tagaggagga ggatcttctc acgctgaaca gttctccatc cacagccaaa 420

gacactctgg acagtggcaa ttatggagaa ccagactatg gaacagacta tggaaccaga 480

ctactggacc acgggccaca gggatgacga cgagtctgat gacaccttgg aagaaaacag 540

gggttacatg gaaattgaac agtcagtgaa atcttttaag atgccatcct caaatataga 600

agaggaagat agccatttct tttttcatct tattattttt gctttttgca ttgctattgt 660

ctacattaca tatcacaaaa aaaggaagat ttttcttcta gttcaaagca gaaaatggcg 720

tgatggcctt tgttccaaaa cagtagaata ccattgccta gatcagaatg ttaatgaggc 780

aatgtcttct ttgaagatta ccaataatta tattttttgc cctgtgattt gagtttgctt 840

attatgtaat tttatttgct tgacttttta tatgatattg tgcagatgtt tgccataggc 900

aattggtact taaatgagag gtgggtctct cttttgcctt ggtgctttgg aaattaaatg 960

tcataaacaa ggagtatata attttttgat ctgtactttt agagttgagt ttaatcaggt 1020

gtccaaaatg tgagttaagc attaccttat atttacactg ttagttttta ttgttttaga 1080

tttattatgc ttcttctgga agtattagtg atgctacttt taaaagatcc caaacctgta 1140

actaaattct aacatatctg tcactgctga ctcacattca ttctctgcca ttcaaatact 1200

attttttaac cacacatttt tttgtggtcc caaactgtaa tgtacaagga tatgtgtgat 1260

aatgctttac atttgaataa tatttttttt ccttccaaga aaactgcttt ggatattttt 1320

agataattta aacataattt aggataacga tattgctcaa tctgaccaca attttaggta 1380

aaacattaaa tgtgtcaaga aatcttggca acagagactc tgcagcttgc agtggacata 1440

cataaaacgt tacagagata ctattttttt tggtttgaat tactatgtt 1489